(19) Europäisches Patentamt
European Patent Office
Office européen des brevets

(11) Veröffentlichungsnummer: **0 143 746**
**A2**

# (12) EUROPÄISCHE PATENTANMELDUNG

(21) Anmeldenummer: **84810562.3**

(22) Anmeldetag: **19.11.84**

(51) Int. Cl.[4]: **C 07 K 5/06**, A 61 K 37/02, A 61 K 37/64

(30) Priorität: **23.11.83 CH 6285/83**

(43) Veröffentlichungstag der Anmeldung: **05.06.85 Patentblatt 85/23**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Fuhrer, Walter, Dr., Munzacherweg 11, CH-4402 Frenkendorf (CH)**
Erfinder: **Bühlmayer, Peter, Dr., Im Baumgarten 3, CH-4144 Arlesheim (CH)**
Erfinder: **Rasetti, Vittorio, Dr., Passwangstrasse 6, CH-4059 Basel (CH)**
Erfinder: **Riniker, Bernhard, Dr., Adlergasse 14, CH-4402 Frenkendorf (CH)**

## (54) Substituierte 5-Amino-4-hydroxyvalerylderivate.

(57) Verbindungen der Formel

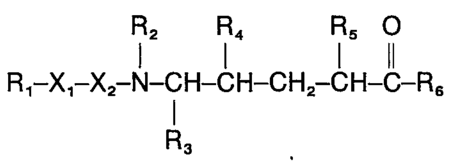

(I)

worin $R_1$ Wasserstoff oder Acyl, $X_1$ einen gegebenenfalls N-alkylierten Aminosäurerest, der N-terminal mit $R_1$ und C-terminal mit $X_2$ verbunden ist, $X_2$ einen gegebenenfalls N-alkylierten Aminosäurerest, der N-terminal mit $X_1$ und C-terminal mit der Gruppe $-NR_2-$ verbunden ist, $R_2$ Wasserstoff oder Niederalkyl, $R_3$ Wasserstoff, Alkyl, Cycloalkyl, Arylniederalkyl oder Aryl, $R_4$ Hydroxy oder veräthertes oder verestertes Hydroxy, $R_5$ Alkyl, Cycloalkyl, Arylniederalkyl oder Aryl, und $R_6$ substituiertes Amino oder substituiertes Hydroxy darstellen, und Salze von solchen Verbindungen mit salzbildenden Gruppen hemmen die blutdrucksteigernde Wirkung des Enzyms Renin und können als Antihypertensiva verwendet werden.

EP 0 143 746 A2

CIBA-GEIGY AG
Basel (Schweiz)

4-14661/+

## Substituierte 5-Amino-4-hydroxyvalerylderivate

Die Erfindung betrifft substituierte 5-Amino-4-hydroxyvalerylderivate der Formel

$$R_1-X_1-X_2-\overset{R_2}{\overset{|}{N}}-\underset{\underset{R_3}{|}}{CH}-\overset{R_4}{\overset{|}{CH}}-CH_2-\overset{R_5}{\overset{|}{CH}}-\overset{O}{\overset{\|}{C}}-R_6 \qquad (I),$$

worin $R_1$ Wasserstoff oder Acyl, $X_1$ einen gegebenenfalls N-alkylierten natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$ verbunden ist, $R_2$ Wasserstoff oder Niederalkyl, $R_3$ Wasserstoff, Alkyl, Cycloalkyl, Arylniederalkyl oder Aryl, $R_4$ Hydroxy oder veräthertes oder verestertes Hydroxy, $R_5$ Alkyl, Cycloalkyl, Arylniederalkyl oder Aryl und $R_6$ substituiertes oder freies Amino oder substituiertes Hydroxy darstellen, mit Ausnahme der Verbindungen, worin $R_1$ einen gegebenenfalls N-acylierten Rest der Aminosäure L-Prolin oder worin $X_1$ und $X_2$ zusammen -Val-Val-, -Gly-Gly- oder -Tyr-Gly- bedeuten, ferner Salze von solchen Verbindungen mit salzbildenden Gruppen, Verfahren zu ihrer Herstellung, pharmazeutische Präparate mit diesen Verbindungen und die Verwendung dieser Verbindungen als Arzneimittel oder zur Herstellung von pharmazeutischen Präparaten, sowie Zwischenprodukte.

In der Beschreibung der vorliegenden Erfindung bedeutet der bei der Definition von Gruppen oder Resten, z.B. Niederalkyl, Niederalkoxy, Niederalkanoyl etc., verwendete Ausdruck "Nieder", dass die so

definierten Gruppen oder Reste, falls nicht ausdrücklich anders definiert, bis einschliesslich 7 und bevorzugt bis einschliesslich 4 C-Atome enthalten.

Die durch $R_3$, $R_4$ und $R_5$ substituierten C-Atome können die R-, S- oder R,S-Konfiguration haben. Bevorzugt sind Verbindungen der Formel I, worin diese C-Atome die S-Konfiguration aufweisen.

Die in der Beschreibung der vorliegenden Erfindung verwendeten allgemeinen Ausdrücke und Bezeichnungen haben vorzugsweise die folgenden Bedeutungen:

Acyl $R_1$ hat z.B. bis zu 19 C-Atome und ist in erster Linie die Acylgruppe einer Carbonsäure, eines Halbesters der Kohlensäure, der Carbaminsäure, einer N-substituierten Carbaminsäure, der Thiocarbaminsäure, einer Sulfonsäure, der Amidosulfonsäure oder einer N-substituierten Amidosulfonsäure.

Acyl $R_1$ hat beispielsweise die Teilformeln: $R^b$-CO-, $R^a$-O-CO-($R^b$)($R^b$)N-CO-, ($R^b$)($R^b$)N-CS-, $R^b$-$SO_2$- oder ($R^b$)($R^b$)N-$SO_2$-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen oder einen unsubstituierten oder substituierten, fünf- oder sechsgliedrigen Heterocyclus darstellt und $R^b$ Wasserstoff bedeutet oder die Bedeutungen von $R^a$ hat.

Ein unsubstituierter oder substituierter, gesättigter oder ungesättigter, aliphatischer, cycloaliphatischer oder cycloaliphatischaliphatischer Kohlenwasserstoffrest $R^a$ oder $R^b$ ist beispielsweise

Alkyl, z.B. Niederalkyl, Niederalkenyl, Niederalkinyl, Mono-, Bi- oder Tricycloalkyl, Monocycloalkenyl, Bicycloalkenyl, Cycloalkylniederalkyl, Cycloalkylniederalkenyl oder Cycloalkenylniederalkyl.

Niederalkyl $R^a$ oder $R^b$ hat vorzugsweise 1-7 C-Atome und ist z.B. Methyl, Aethyl, n-Propyl, Isopropyl, n-Butyl oder tert-Butyl, welche durch eine oder mehrere funktionelle Gruppen, beispielsweise Hydroxy, veräthertes Hydroxy, z.B. Niederalkoxy, wie Methoxy oder Aethoxy, Phenyloxy oder durch einen gewünschtenfalls acetylierten oder als Acetal geschützten natürlichen Zucker veräthertes Hydroxy, z.B. Glucofuranosyl, verestertes Hydroxy, z.B. Niederalkanoyloxy, wie Acetoxy, oder Halogen, z.B. Chlor oder Brom, Hydroxysulfonyloxy, Carboxy, verestertes Carboxy, z.B. Niederalkoxycarbonyl, wie Methoxy- oder Aethoxycarbonyl, amidiertes Carboxy, z.B. Carbamoyl oder Mono- oder Diniederalkylcarbamoyl, wie Methyl- oder Dimethylcarbamoyl, Amino, Niederalkylamino, z.B. Methylamino, Hydroxyniederalkylamino, z.B. 2-Hydroxyäthylamino, Diniederalkylamino, z.B. Dimethylamino, Acylamino oder N-Acyl-N-niederalkylamino, worin Acyl eine weiter unten definierte Gruppe $R^c$-CO- oder $R^d$-O-CO- bedeutet, Guanidino, Mercapto, Niederalkylthio, z.B. Methylthio, oder durch Oxo substituiert sein können, wobei die Substituenten nur dann in 1-Stellung des Niederalkylrest stehen, wenn dieser in der Teilformel $R^b$-CO- an die Carbonylgruppe gebunden ist.

$R^d$ bedeutet Niederalkyl mit vorzugsweise 1-7 C-Atomen, z.B. Methyl, Aethyl, n-Propyl, Isopropyl, n-Butyl, tert-Butyl oder tert-Pentyl, 2-Halogenniederalkyl, z.B. 2-Chlor-, 2-Brom-, 2-Jod- oder 2,2,2-Trichloroäthyl, oder Arylniederalkyl, z.B. unsubstituiertes oder substituiertes Phenylniederalkyl, worin Phenyl durch Niederalkyl, Niederalkoxy, Hydroxy, Halogen und/oder Nitro mono-, di- oder trisubstituiert sein kann, beispielsweise Benzyl, p-Methoxybenzyl, Diphenylmethyl oder Trityl. $R^c$ hat die Bedeutungen von $R^d$ oder ist Niederalkyl substituiert durch eine oder mehrere funktionelle Gruppen, beispielsweise Hydroxy, veräthertes Hydroxy, z.B. Niederalkoxy, wie Methoxy oder Aethoxy, verestertes Hydroxy, z.B. Niederalkanoyloxy, wie Acetoxy oder Pivaloyloxy, Carboxy,

verestertes Carboxy, z.B. Niederalkoxycarbonyl, wie Methoxy- oder Aethoxycarbonyl, amidiertes Carboxy, z.B. Carbamoyl oder Mono- oder Diniedercarbamoyl, wie Methyl- oder Dimethylcarbamoyl, Amino, Niederalkylamino, z.B. Methylamino, Hydroxyniederalkylamino, z.B. 2-Hydroxyäthylamino, Diniederalkylamino, z.B. Dimethylamino, Acylamino oder N-Acyl-N-niederalkylamino, worin Acyl eine Gruppe $R^d$-CO- oder $R^d$-O-CO- bedeutet, Guanidino, Mercapto oder durch Niederalkylthio, z.B. Methylthio, ferner durch Phenyl, p-Hydroxyphenyl, Indolyl oder Imidazolyl.

Alkyl $R^a$ oder $R^b$ hat vorzugsweise 1-10 C-Atome und ist z.B. Niederalkyl mit den oben genannten Bedeutungen, z.B. Methyl, Aethyl, n-Pentyl, Neopentyl, n-Hexyl oder n-Heptyl, oder n-Octyl, n-Nonyl oder N-Decyl, und kann durch eine oder mehrere der obengenannten funktionellen Gruppen substituiert sein.

Substituiertes Niederalkyl oder Alkyl $R^a$ oder $R^b$ ist beispielsweise Hydroxyniederalkyl, z.B. 2-Hydroxyäthyl, Niederalkoxyniederalkyl, z.B. Niederalkoxymethyl oder Niederalkoxyäthyl, wie Methoxymethyl oder 2-Methoxyäthyl, Mono- oder Diisopropyliden-glucofuranosyl-O-methyl, Niederalkanoyloxyniederalkyl, z.B. Niederalkanoyloxymethyl oder Niederalkanoyloxyäthyl, z.B. Acetoxymethyl oder 2-Acetoxyäthyl, Halogenniederalkyl, z.B. Halogenmethyl oder Halogenäthyl, wie 2-Chlor- oder 2-Bromäthyl, Hydroxysulfonyloxyniederalkyl, z.B. Hydroxysulfonyloxymethyl oder 2-Hydroxysulfonyloxyäthyl, Carboxyniederalkyl, z.B. Carboxymethyl oder 2-Carboxyäthyl, Niederalkoxycarbonylniederalkyl, z.B. Niederalkoxycarbonylmethyl oder Niederalkoxycarbonyläthyl, wie Methoxycarbonylmethyl, 2-Methoxycarbonyläthyl, Aethoxycarbonylmethyl oder 2-Aethoxycarbonyläthyl, Carbamoylniederalkyl, z.B. Carbamoylmethyl oder 2-Carbamoyläthyl, Niederalkylcarbamoylniederalkyl, z.B. Methylcarbamoylmethyl, Diniederalkylcarbamoylniederalkyl, z.B. Dimethylcarbamoylmethyl, Aminoalkyl, z.B. Aminomethyl, 1-Aminoäthyl, 3-Aminopropyl oder 7-Aminoheptyl, Niederalkylaminoniederalkyl, z.B. Methylaminomethyl, Diniederalkylaminoniederalkyl, z.B. Dimethylaminomethyl, Acylaminoalkyl, z.B. Niederalkanoylaminoalkyl, wie Acetylaminomethyl, 1-Acetylaminoäthyl

oder Pivaloylaminomethyl, Niederalkoxycarbonylaminoalkyl, wie tert-Butoxycarbonylaminomethyl, 3-tert-Butoxycarbonylaminopropyl oder 7-tert-Butoxycarbonylaminoheptyl, Arylniederalkoxycarbonylaminoalkyl, wie Benzyloxycarbonylaminomethyl, 3-Benzyloxycarbonylaminopropyl oder 7-Benzyloxycarbonylaminoheptyl, substituiertes Niederalkanoylaminoniederalkyl, wie γ-Aminobutyryl-aminomethyl, γ-Benzyloxycarbonylaminobutyryl-aminomethyl, α-Amino-δ-guanidinovalerylaminomethyl oder α-Benzyloxycarbonylamino-δ-guanidinovalerylaminomethyl, ferner Mercaptoniederalkyl, z.B. 2-Mercaptoäthyl, Niederalkylthioniederalkyl, z.B. 2-Methylthioäthyl, oder Oxoniederalkyl, z.B. 2-Oxopropyl oder 2-Oxobutyl.

Substituiertes Niederalkyl $R^a$ oder $R^b$ mit zwei oder mehr Substituenten ist beispielsweise Hydroxy-carboxy-niederalkyl, z.B. Hydroxy-carboxy-methyl oder 1-Hydroxy-2-carboxy-äthyl, Hydroxy-niederalkoxycarbonyl-niederalkyl, z.B. Hydroxy-äthoxy- oder -methoxycarbonyl-äthyl, verestertes Hydroxy-niederalkoxycarbonyl-niederalkyl, z.B. Acetoxy-methoxycarbonyl-methyl, Dihydroxy-carboxy-niederalkyl, z.B. 1,2-Dihydroxy-2-carboxy-äthyl, Dihydroxy-niederalkoxycarbonyl-niederalkyl, z.B. 1,2-Dihydroxy-2-äthoxy- oder -methoxycarbonyl-äthyl, verestertes Dihydroxy-niederalkoxycarbonyl-niederalkyl, z.B. 1,2-Diacetoxy-2-äthoxy- oder -methoxycarbonyl-äthyl, Hydroxy-amino-niederalkyl, z.B. 2-Hydroxy-1-amino-äthyl, 2-Hydroxy-1-amino-propyl oder 2-Hydroxy-3-amino-5-methylhexyl, Hydroxy-acylamino-niederalkyl, z.B. 2-Hydroxy-1-benzyloxycarbonyl-amino-5-methylhexyl, Carboxy-amino-niederalkyl, z.B. Carboxy-amino-methyl, 2-Carboxy-1-amino-äthyl oder 3-Carboxy-1-amino-propyl, Niederalkoxycarbonyl-amino-niederalkyl, z.B. Methoxy-oder Aethoxycarbonyl-amino-methyl, Carboxy-acylamino-niederalkyl, z.B. 2-Carboxy-1-benzyloxycarbonylamino-äthyl oder 3-Carboxy-1-benzyloxycarbonylamino-propyl, Niederalkoxycarbonyl-acylamino-niederalkyl, z.B. Methoxy- oder Aethoxycarbonyl-benzyloxycarbonylamino-methyl oder Methoxy- oder Aethoxycarbonyl-tert-butoxycarbonylamino-methyl, Diamino-niederalkyl, z.B. 1,5-Diaminopentyl, oder Diacylamino-niederalkyl, z.B. 1,5-Bis(benzyloxycarbonylamino)pentyl.

Niederalkenyl $R^a$ oder $R^b$ enthält z.B. 2-7, insbesondere 2-4, C-Atome und ist z.B. Vinyl, Allyl oder 2- oder 3-Butenyl. Niederalkenyl $R^a$ oder $R^b$ kann durch die gleichen Substituenten substituiert sein wie Niederalkyl.

Niederalkinyl $R^a$ oder $R^b$ enthält z.B. 2-7, insbesondere 2-4, C-Atome und ist beispielsweise Aethinyl, 1-Propinyl oder 2-Propinyl.

Cycloalkyl $R^a$ oder $R^b$ enthält z.B. 3-8, insbesondere 3-6, C-Atome und ist z.B. Cyclopropyl, Cyclobutyl, Cyclopentyl oder Cyclohexyl.

Bicycloalkyl $R^a$ oder $R^b$ enthält z.B. 5-10, insbesondere 6-9, C-Atome und ist z.B. Bicyclohexyl, -heptyl, -octyl, -nonyl oder -decyl, z.B. Bicyclo[3.1.0]hex-1-, -2- oder -3-yl, Bicyclo[4.1.0]hept-1- oder -4-yl, Bicyclo[2.2.1]hept-2-yl, z.B. endo- oder exo-Norbornyl, Bicyclo[3.2.1]oct-2-yl, Bicyclo[3.3.0]oct-3-yl oder Bicyclo[3.3.1]-non-9-yl.

Tricycloalkyl $R^a$ oder $R^b$ enthält z.B. 8-10 C-Atome und ist z.B. Tricyclo[5.2.1.$0^{2,6}$]dec-8-yl oder Adamantyl.

Monocycloalkenyl $R^a$ oder $R^b$ enthält z.B. 3-8, insbesondere 3-6, C-Atome und ist beispielsweise Cyclohexenyl, z.B. 1-Cyclohexenyl, oder Cyclohexadienyl, z.B. 1,4-Cyclohexadienyl.

Bicycloalkenyl $R^a$ oder $R^b$ enthält z.B. 5-10, insbesondere 7-10, C-Atome und ist z.B. Bicyclo[2.2.1]hept-5-en-yl, z.B. 5-Norbornen-2-yl, Bicyclo[2.2.2]octen-2-yl oder Hexahydro-4,7-methanoind-1-en-6-yl.

Cycloalkylniederalkyl $R^a$ oder $R^b$ enthält z.B. 4-10, insbesondere 4-7, C-Atome und ist beispielsweise Cyclopropylmethyl, Cyclobutylmethyl, Cyclopentylmethyl oder Cyclohexylmethyl.

Cycloalkylniederalkenyl $R^a$ oder $R^b$ enthält z.B. 5-10, insbesondere 5-9, C-Atome und ist beispielsweise Cyclohexylvinyl oder Cyclohexylallyl.

Cycloalkenylniederalkyl $R^a$ oder $R^b$ enthält z.B. 4-10, insbesondere 4-7, C-Atome und ist beispielsweise 1-Cyclohexenylmethyl oder 1,4-Cyclohexadienylmethyl.

Die genannten cycloaliphatischen oder cycloaliphatisch-aliphatischen Reste können durch die gleichen Substituenten wie Niederalkyl $R^a$ substituiert sein.

Substituiertes Cycloalkyl $R^a$ oder $R^b$ ist beispielsweise Hydroxycycloalkyl, z.B. 4-Hydroxycyclohexyl oder 3,4,5-Trihydroxycyclohexyl, Carboxycycloalkyl, z.B. 4-Carboxycyclohexyl, Aminocycloalkyl, z.B. 1-Aminocyclopropyl oder 4-Aminocyclohexyl, oder Acylaminocycloalkyl, z.B. 1-tert-Butoxycarbonylaminocyclopropyl oder 1-Benzyloxycarbonylaminocyclopropyl.

Substituiertes Cycloalkenyl $R^a$ oder $R^b$ ist beispielsweise Hydroxycycloalkenyl, z.B. 3,4,5-Trihydroxycyclohex-1-enyl.

Ein aromatischer bzw. aromatisch-aliphatischer Kohlenwasserstoffrest $R^a$ oder $R^b$ ist beispielsweise unsubstituiertes oder substituiertes Phenyl bzw. Phenylniederalkyl, z.B. Halophenyl, wie 4-Chlorophenyl, Niederalkoxyphenyl, wie 4-Methoxyphenyl oder Nitrophenyl, Benzyl, Niederalkylbenzyl wie 4-Methylbenzyl, Niederalkoxybenzyl, wie 4-Methoxybenzyl, substituiertes Anilinobenzyl, wie 2-(o,o-Dichloroanilino)-benzyl oder 2-(o,o-Dichloro-N-benzylanilino)-benzyl, 2-Phenyläthyl, 2-(p-Hydroxyphenyl)-äthyl, Diphenylmethyl, Di-(4-methoxyphenyl)methyl oder Trityl, oder Phenylniederalkenyl, z.B. 3-Phenylallyl.

In einem heteroaromatischen bzw. heteroaromatisch-aliphatischen Kohlenwasserstoffrest $R^a$ oder $R^b$ ist der Heterocyclus fünf- oder sechsgliedrig und enthält ein bis zwei Stickstoffatome und/oder ein Sauerstoff- oder Schwefelatom und ist mit einem seiner Ring-Kohlenstoffatome mit der Gruppe -CO- bzw. -O-CO-, $>N-CO-$, $>N-CS-$, $-SO_2-$ oder $>N-SO_2-$ verknüpft. Dieser Heterocyclus kann an einem Stickstoffatom durch Niederalkyl, z.B. Methyl oder Aethyl, Phenyl, oder Phenylniederalkyl, z.B. Benzyl, substituiert und/oder benz-annelliert sein und ist beispielsweise 2- oder 3-Pyrrolyl, 2-Furanyl, 2-Thiophenyl, 4-Imidazolyl, 2-, 3- oder 4-Pyridyl, 3-Indolyl, 2-, 3- oder 4-Chinolinyl, 1-, 3- oder 4-Isochinolinyl, 2-Benzoxazolyl oder 2-Benzthiazolyl.

Die aromatisch-aliphatischen oder heteroaromatisch-aliphatischen Reste können im aliphatischen Teil durch die gleichen Substituenten wie Niederalkyl $R^a$ substituiert sein.

Substituiertes Phenylniederalkyl $R^a$ oder $R^b$ ist beispielsweise Phenyl-hydroxy-niederalkyl, z.B. α-Hydroxybenzyl, substituiertes oder unsubstituiertes Phenyl-amino-niederalkyl, z.B. 2-Phenyl-1-aminoäthyl oder 2-(p-Hydroxyphenyl)-1-amino-äthyl oder substituiertes oder unsubstituiertes Phenyl-acylamino-niederalkyl, z.B. 2-Phenyl-1-benzyloxycarbonylamino-äthyl oder 2-(p-Acetoxyphenyl)-1-benzyloxycarbonylamino-äthyl.

Ein fünf- oder sechsgliedriger Heterocyclus $R^a$ oder $R^b$ hat mindestens ein C-Atom, 1-3 Stickstoffatome und gegebenenfalls ein Sauerstoff- oder Schwefelatom als Ringglieder und ist mit einem seiner Ring-C-Atome mit der Gruppe -CO- bzw. -OCO-, $>N-CO-$, $>N-CS-$, $-SO_2-$ oder $>N-SO_2-$ verknüpft. Dieser Hetero-

cyclus kann an einem seiner C-Atome oder an einem Ringstickstoffatom durch Niederalkyl, z.B. Methyl oder Aethyl, Phenyl oder Phenylniederalkyl, z.B. Benzyl, an einem seiner C-Atome durch Hydroxy oder Oxo oder an einem Ringstickstoffatom durch Acyl $R^d$-CO- oder $R^d$-O-CO-substituiert sein.

Ein solcher Heterocyclus ist beispielsweise Pyrrolidin-3-yl, Hydroxypyrrolidin-2- oder -3-yl, z.B. 4-Hydroxypyrrolidin-2-yl, Hydroxy-1-acylpyrrolidin-2- oder -3-yl, z.B. 1-Benzyloxycarbonyl-4-hydroxypyrrolidin-2-yl, Oxopyrrolidin-2-yl, z.B. 5-Oxopyrrolidin-2-yl, Piperidin-2- oder -3-yl-, 1-Niederalkylpiperidin-2-, -3- oder -4-yl, z.B. 1-Methylpiperidin-2-, -3- oder -4-yl, 1-Acylpiperidin-2-, -3- oder -4-yl, z.B. 1-Benzyloxycarbonylpiperidin-2-, -3- oder -4-yl, Morpholin-2-oder -3-yl, Thiomorpholin-2- oder -3-yl oder 1- und/oder 4-Niederalkylpiperazin-2- oder -3-yl, z.B. 1,4-Dimethylpiperazin-2-oder -3-yl. Ausgeschlossen als Heterocyclus $R^b$ in einem Acylrest $R^b$-CO- ist gegebenenfalls N-acyliertes Pyrrolidin-2-yl, d.h. der Rest der Aminosäure Prolin.

Bevorzugte Acylgruppe $R_1$ sind beispielsweise Niederalkanoyl, z.B. Formyl, Acetyl, Propionyl oder Pivaloyl, Niederalkoxyniederalkanoyl, z.B. Methoxyacetyl, Mono- oder Diisopropyliden-glucofuranosyl-O-acetyl, z.B. 1,2-Mono- oder 1,2:5,6-Diisopropyliden-glucofuranosyl-3-O-acetyl, Halogenniederalkanoyl, z.B. α-Halogenacetyl, z.B. α-Chlor-, α-Brom-, α-Jod- oder α,α,α-Trichloracetyl, Carboxyniederalkanoyl, z.B. Carboxyacetyl, Niederalkoxycarbonylniederalkanoyl, z.B. Methoxycarbonylacetyl, Aminoalkanoyl, z.B. Aminoacetyl, α-Aminopropionyl, γ-Aminobutyryl oder 8-Aminooctanoyl, Acylaminoalkanoyl, z.B. Acetylaminoacetyl, Pivaloylaminoacetyl, tert-Butoxycarbonylaminoacetyl, γ-(tert-Butoxycarbonylamino)-butyryl, 8-(tert-Butoxycarbonylamino)-octanoyl, Benzyloxycarbonylaminoacetyl, γ-(Benzyloxycarbonylamino)-butyryl, 8-(Benzyloxycarbonylamino)-octanoyl, γ-Aminobutyryl-aminoacetyl, γ-Benzyloxycarbonylamino-butyryl-aminoacetyl, (α-Amino-δ-guanidinovaleryl)-aminoacetyl, oder (α-Benzyloxycarbonylamino-δ-guanidinovaleryl)-aminoacetyl, Oxoniederalkanoyl, z.B. Acetoacetyl, Hydroxy-carboxy-niederalkanoyl, z.B.

α-Hydroxy-ß-carboxypropionyl, Hydroxy-niederalkoxycarbonyl-niederalkanoyl, z.B. α-Hydroxy-ß-äthoxy- oder -methoxycarbonylpropionyl, Dihydroxy-carboxy-niederalkanoyl, z.B. α,ß-Dihydroxy-ß-carboxypropionyl, Dihydroxy-niederalkoxycarbonyl-niederalkanoyl, z.B. α,ß-Dihydroxy-ß-äthoxy- oder -methoxycarbonylpropionyl, verestertes Dihydroxy-niederalkoxycarbonyl-niederalkanoyl, z.B. α,ß-Diacetoxy-ß-methoxycarbonylpropionyl, Hydroxy-amino-niederalkanoyl, z.B. ß-Hydroxy-α-aminopropionyl oder 3-Hydroxy-4-amino-6-methylheptanoyl, Hydroxy-acylamino-niederalkanoyl, z.B. ß-Hydroxy-α-benzyloxycarbonylaminopropionyl oder 3-Hydroxy-4-benzyloxycarbonylamino-6-methylheptanoyl, Carboxy-amino-niederalkanoyl, z.B. α-Carboxy-α-aminoacetyl, Niederalkoxycarbonyl-amino-niederalkanoyl, z.B. α-Methoxy- oder Aethoxycarbonyl-α-aminoacetyl, Niederalkoxycarbonyl-acylamino-niederalkanoyl, z.B. α-Methoxy- oder Aethoxycarbonyl-α-benzyloxycarbonylaminoacetyl, Niederalkenoyl, z.B. Acryloyl oder Crotonoyl, Niederalkinoyl, z.B. Propiolyl, Cyclopentyl- oder Cyclohexylcarbonyl, Hydroxycyclohexylcarbonyl, z.B. 3,4,5-Trihydroxycyclohexylcarbonyl, Acylaminocycloalkylcarbonyl, z.B. 1-tert-Butoxycarbonylaminocyclopropylcarbonyl oder 1-Benzyloxycarbonylaminocyclopropylcarbonyl, 3,4,5-Trihydroxycyclohex-1-enylcarbonyl, Benzoyl oder durch Halogen, z.B. Chlor, Niederalkoxy, z.B. Methoxy, und/oder Nitro substituiertes Benzoyl, z.B. 4-Chlor-, 4-Methoxy- oder 4-Nitrobenzoyl, 2-(o,o-Dichloroanilino)-phenylacetyl, 2-(o,o-Dichloro-N-benzylanilino)-phenylacetyl, Hydroxypyrrolidinylcarbonyl, z.B. 3- oder 4-Hydroxypyrrolidinyl-2-carbonyl, Hydroxy-1-acylpyrrolidinylcarbonyl, z.B. 3- oder 4-Hydroxy-1-benzyloxycarbonylpyrrolidinyl-2-carbonyl, Niederalkoxycarbonyl, z.B. tert-Butoxycarbonyl, 2-Halogenniederalkoxycarbonyl, z.B. 2-Chlor-, 2-Brom-, 2-Jod-oder 2,2,2-Trichloräthoxycarbonyl, Arylmethoxycarbonyl mit einem oder zwei Arylresten, worin Aryl gegebenenfalls durch Niederalkyl, z.B. Methyl oder tert-Butyl, Niederalkoxy, z.B. Methoxy, Hydroxy, Halogen, z.B. Chlor, und/oder Nitro mono-, di-oder trisubstituiertes Phenyl ist, z.B. Benzyloxycarbonyl, 4-Nitrobenzyloxycarbonyl, Diphenylmethoxycarbonyl oder Di-(4-methoxyphenyl)-methoxycarbonyl.

Die Aminosäurereste $X_1$ und $X_2$ sind durch eine Peptidbindung miteinander verbunden und leiten sich von den natürlichen Aminosäuren ab, die normalerweise in Eiweissen vorkommen, und die beispielsweise in Houben-Weyl, Methoden der Organischen Chemie, Band XV/1 (1974), "Synthese von Peptiden", Seite 2, aufgezählt sind. Diese Aminosäuren sind α-Aminosäuren mit der L-Konfiguration und umfassen insbesondere die 20 α-Aminosäuren, welche besonders häufig in Proteinen vorkommen, nämlich Glycin (H-Gly-OH), Alanin (H-Ala-OH), Prolin (H-Pro-OH), Serin (H-Ser-OH), Cystein (H-Cys-OH), Tyrosin (H-Tyr-OH), Asparagin (H-Asn-OH), Glutamin (H-Gln-OH), Asparaginsäure (H-Asp-OH), Glutaminsäure (H-Glu-OH), Arginin (H-Arg-OH), Histidin (H-His-OH), einschliesslich jene 8 Aminosäuren, die für den Menschen essentiell sind, nämlich Valin (H-Val-OH), Leucin (H-Leu-OH), Isoleucin (H-Ile-OH), Lysin (H-LysOH), Phenylalanin (H-Phe-OH), Tryptophan (H-Trp-OH), Methionin (H-Met-OH) und Threonin (H-Thr-OH), ferner trans-3- und trans-4-Hydroxyprolin, α-Aminobuttersäure, α,ß-Diaminopropionsäure, α,γ-Diaminobuttersäure, Ornithin, und δ-Hydroxylysin.

Die Aminosäurereste $X_1$ und $X_2$ können N-terminal zur Erhöhung der Stabilität der Verbindung der Formel I gegen enzymatischen Abbau durch Niederalkyl, z.B. Methyl oder Aethyl, substituiert sein.

Eine Carboxy-Funktion in der Seitenkette eines Aminosäurerestes $X_1$ und $X_2$, wie sie in Glutaminsäure und Asparaginsäure vorliegt, ist gewünschtenfalls mit einem Niederalkanol, z.B. mit Methanol oder tert-Butanol verestert. Eine Amino-Funktion in der Seitenkette eines Aminosäurerestes $X_1$ und $X_2$, wie sie in Lysin, α,ß-Diaminopropionsäure, α,γ-Diaminobuttersäure, Ornithin und δ-Hydroxylysin vorliegt, ist gewünschtenfalls durch einen Rest $R^d$-O-CO-, z.B. tert-Butoxycarbonyl oder Benzyloxycarbonyl, acyliert.

$X_1$ ist vorzugsweise der bivalente Rest von Phenylalanin, Histidin, Arginin oder gegebenenfalls veresterter Glutaminsäure. Ganz besonders bevorzugt ist der bivalente Rest von Phenylalanin.

$X_2$ ist vorzugsweise der bivalente Rest von Histidin, Phenylalanin, Leucin, Alanin oder Arginin. Ganz besonders bevorzugt ist der bivalente Rest von Histidin oder von Phenylalanin.

Niederalkyl $R_2$, $R_3$ oder $R_5$ hat die weiter vorn für Niederalkyl $R^a$ genannten Bedeutungen. Niederalkyl $R_2$ ist vorzugsweise Methyl oder Aethyl. Alkyl $R_3$ oder $R_5$ ist vorzugsweise Niederalkyl, z.B. Isopropyl oder Isobutyl, oder n-Octyl.

Cycloalkyl $R_3$ oder $R_5$ hat die weiter vorn für Cycloalkyl $R^a$ oder $R^b$ genannten Bedeutungen und ist vorzugsweise Cyclohexyl.

Arylniederalkyl $R_3$ oder $R_5$ hat die weiter vorn für Arylniederalkyl $R^a$ oder $R^b$ genannten Bedeutungen und ist vorzugsweise Benzyl.

Aryl $R_3$ oder $R_5$ ist vorzugsweise Phenyl.

Eine verätherte Hydroxygruppe $R_4$ ist vorzugsweise durch solche organischen Reste veräthert, die unter physiologischen Bedingungen abspaltbar sind und nach der Abspaltung in der betreffenden Konzentration pharmakologisch unbedenkliche Spaltprodukte liefern.

Veräthertes Hydroxy $R_4$ ist z.B. Acyloxyniederalkoxy, worin Acyl die Acylgruppe einer gegebenenfalls verzweigten Niederalkancarbonsäure oder der durch gegebenenfalls verzweigtes Niederalkyl monoveresterten Kohlensäure darstellt, z.B. Niederalkanoyloxyniederalkoxy, wie Acetoxymethoxy, 1-Acetoxyäthoxy, Pivaloyloxymethoxy oder 1-Pivaloyloxyäthoxy, oder Niederalkoxycarbonyloxyniederalkoxy, wie Aethoxycarbonyloxymethoxy, 1-Aethoxycarbonyloxyäthoxy, tert-Butoxycarbonyloxymethoxy oder 1-tert-Butoxycarbonyloxyäthoxy.

Veräthertes Hydroxy $R_4$ ist ferner Niederalkoxy, z.B. Methoxy oder Aethoxy, Aryloxy, z.B. Phenoxy, oder Arylniederalkoxy, z.B. Benzyloxy.

Verestertes Hydroxy $R_4$ ist z.B. aliphatisches Acyloxy, z.B. Niederalkanoyloxy, wie Acetoxy oder Pivaloyloxy, cycloaliphatisches Acyloxy, z.B. Cycloalkanoyloxy, wie Cyclohexanoyloxy, oder aromatisches Acyloxy, z.B. Benzoyloxy.

$R_6$ mit der Bedeutung "substituiertes Amino" oder "substituiertes Hydroxy" ist beispielsweise eine Amino- oder Hydroxygruppe, welche durch einen oder gegebenenfalls zwei unsubstituierte oder substituierte, gesättigte oder ungesättigte, aliphatische Kohlenwasserstoffreste bis einschliesslich 18, bevorzugt bis einschliesslich 10, C-Atomen oder einen unsubstituierten oder substituierten, aromatischen, heteroaromatischen, aromatisch-aliphatischen oder heteroaromatisch-aliphatischen Kohlenwasserstoffrest mit bis zu 18, bevorzugt bis einschliesslich 10, C-Atomen substituiert ist.

$R_6$ mit der Bedeutung "substituiertes Amino" oder "substituiertes Hydroxy" kann ausserdem einen N-terminal mit der Gruppe -CO-verbundenen und C-terminal gegebenenfalls amidierten oder veresterten Rest einer natürlichen α-Aminosäure oder einen N-terminal mit der Gruppe -CO-verbundenen und C-terminal gegebenenfalls amidierten oder veresterten Di- oder Tripeptidrest, der aus natürlichen α-Aminosäuren und gewünschtenfalls aus Statin (abgekürzt H-Sta-OH, 4-Amino-3-hydroxy-6-methylheptansäure) besteht, darstellen.

Ein unsubstituierter oder substituierter, gesättigter oder ungesättigter, aliphatischer Kohlenwasserstoffrest, welcher die Aminogruppe oder Hydroxygruppe $R_6$ substituiert, ist beispielsweise Alkyl mit bis zu 10 C-Atomen, sowie Niederalkenyl oder Niederalkinyl bis einschliesslich 7 C-Atomen.

Diese Reste können wie Niederalkyl $R^a$ durch eine oder mehrere der weiter vorn genannten funktionellen Gruppen, sowie durch Sulfo, Cyano oder substituiertes Amino, worin die Aminogruppe Teil eines fünf- oder sechsgliedrigen Heterocyclus enthaltend ein bis zwei Stickstoffatome und gewünschtenfalls ein Sauerstoff- oder Schwefelatom ist, substituiert sein.

Als Substituenten sind Hydroxy, substituiertes oder unsubstituiertes Phenyloxy, z.B. Carbamoylphenyloxy oder Carbamoyl-hydroxy-phenyloxy, Carboxy, verestertes Carboxy, z.B. Niederalkoxycarbonyl, wie tert-Butoxycarbonyl, oder ein physiologisch spaltbares verestertes Carboxy, z.B. 1-(Niederalkanoyloxy)-niederalkoxycarbonyl, wie Acetoxymethoxycarbonyl, Pivaloyloxymethoxycarbonyl oder 1-Propionyloxyäthoxycarbonyl, 1-(Niederalkoxycarbonyloxy)-niederalkoxycarbonyl, wie 1-(Aethoxycarbonyloxy)-äthoxycarbonyl, oder α-Aminoniederalkanoyloxymethoxycarbonyl, wie α-Aminoacetoxymethoxycarbonyl oder (S)-α-Amino-ß-methylbutyryloxymethoxycarbonyl, Carbamoyl, substituiertes oder unsubstituiertes Niederalkylcarbamoyl, z.B. Hydroxyniederalkylcarbamoyl wie 2-Hydroxyäthylcarbamoyl, Triniederalkylsilyloxyniederalkylcarbamoyl, oder Methoxycarbonyl-hydroxy-niederalkylcarbamoyl, Amino, substituiertes oder unsubstituiertes Niederalkylamino, z.B. Hydroxyniederalkylamino, Diniederalkylamino, über ein Stickstoff-Atom gebundenes fünf- oder sechsgliedriges, gewünschtenfalls durch Oxo substituiertes Heterocyclyl, z.B. 1-Piperidyl, 4-Morpholinyl oder 2-Oxo-1-pyrrolidinyl, und Acylamino, z.B. Niederalkoxycarbonylamino, wie tert-Butoxycarbonylamino, bevorzugt.

Alkyl mit 1-10 C-Atomen ist z.B. Niederalkyl mit den unter $R^a$ genannten Bedeutungen, z.B. Methyl oder Aethyl, sowie n-Pentyl, Neopentyl, n-Hexyl, n-Heptyl, n-Octyl, n-Nonyl oder n-Decyl.

Substituiertes Alkyl ist z.B. Hydroxyniederalkyl, z.B. 2-Hydroxyäthyl, Niederalkoxyniederalkyl, z.B. Niederalkoxymethyl oder Niederalkoxyäthyl, z.B. Methoxymethyl oder 2-Methoxyäthyl, Niederalkanoyloxyniederalkyl, z.B. Niederalkanoyloxymethyl oder Niederalkanoyloxyäthyl, z.B. Acetoxymethyl oder 2-Acetoxyäthyl, Halogenniederalkyl, z.B. Halogenmethyl oder Halogenäthyl, z.B. Trifluormethyl, Chlor- oder Brommethyl, 2-Chlor-, 2-Brom- oder 2,2,2-Trichloräthyl, Hydroxysulfonyloxyniederalkyl, z.B. Hydroxysulfonyloxymethyl oder 2-Hydroxysulfonyloxyäthyl, Phenyloxyniederalkyl, z.B. 2-Phenyloxyäthyl, substituiertes Phenyloxyniederalkyl, z.B. 2-(3-Carbamyol-4-hydroxyphenoxy)-äthyl, substituiertes Phenyloxy-hydroxy-

niederalkyl, z.B. 3-(3-Carbamoylphenoxy)-2-hydroxypropyl, Carboxyalkyl, z.B. 4-Carboxy-n-butyl, 5-Carboxy-n-pentyl, 6-Carboxy-n-hexyl, 7-Carboxy-n-heptyl oder 8-Carboxy-n-octyl, ferner Carboxymethyl, 2-Carboxyäthyl, 3-Carboxy-n-propyl oder 1- oder 2-Carboxyprop-2-yl, verestertes Carboxyalkyl, z.B. Niederalkoxycarbonylalkyl, z.B. 4-tert-Butoxycarbonyl-n-butyl, 7-tert-Butoxycarbonyl-n-heptyl oder 8-tert-Butoxycarbonyl-n-octyl, ferner tert-Butoxycarbonylmethyl oder 2-tert-Butoxycarbonyläthyl, oder physiologisch spaltbares verestertes Carboxyalkyl, z.B. 7-Pivaloyloxymethoxycarbonyl-n-heptyl, 7-(1-Aethoxycarbonyloxyäthoxycarbonyl)-n-heptyl oder 4-Pivaloyloxymethoxycarbonyl-n-butyl, Carbamoylniederalkyl, z.B. Carbamoylmethyl, 2-Carbamoyläthyl oder Dicarbamoylmethyl, Niederalkylcarbamoylniederalkyl, z.B. Methylcarbamoylmethyl, Hydroxyniederalkylcarbamoylniederalkyl, z.B. 7-(2-Hydroxyäthyl)-carbamoyl-n-heptyl, 4-(2-Hydroxyäthyl)-carbamoyl-n-butyl, 7-(Tris-[hydroxymethyl]-methyl)-carbamoyl-n-heptyl oder 4-(Tris-[hydroxymethyl]-methyl)-carbamoyl-n-butyl, Triniederalkylsilyloxyniederalkylcarbamoylniederalkyl, z.B. 4-(Tris-[tert-butyldimethylsilyloxymethyl]-methyl)-carbamoyl-n-butyl, Niederalkoxycarbonyl-hydroxyniederalkylcarbamoylniederalkyl, z.B. Carbamoyl-(2-hydroxy-1-isobutyl-3-methoxycarbonylpropyl)-carbamoyl-methyl, Diniederalkylcarbamoylniederalkyl, z.B. Dimethylcarbamoylmethyl, Sulfoniederalkyl, z.B. Sulfomethyl oder 2-Sulfoäthyl, Aminoalkyl, z.B. 4-Amino-n-butyl, 5-Amino-n-pentyl, 6-Amino-n-hexyl, 7-Amino-n-heptyl oder 8-Amino-n-octyl, ferner 2-Aminoäthyl oder 3-Amino-n-propyl, Niederalkylaminoniederalkyl, z.B. 2-Methylaminoäthyl oder 3-Methylamino-n-propyl, Hydroxyniederalkylaminoniederalkyl, z.B. 2-(2-Hydroxyäthylamino)-äthyl, Diniederalkylaminoniederalkyl, z.B. 2-Dimethylaminoäthyl oder 3-Dimethylamino-n-propyl, über ein Stickstoff-Atom gebundenes fünf- oder sechsgliedriges Heterocyclylniederalkyl, z.B. 3-(1-Piperidinyl)-propyl, 2-(1-Piperidinyl)-äthyl, 3-(4-Morpholinyl)-propyl, 2-(4-Morpholinyl)-äthyl, 3-(2-Oxo-1-pyrrolidinyl)-propyl oder 2-(2-Oxo-1-pyrrolidinyl)-äthyl, oder Acylaminoalkyl, z.B. Niederalkanoylaminoalkyl, z.B. Pivaloylaminoalkyl oder Acetylaminoalkyl, wie 4-Pivaloylamino-n-butyl oder 4-Acetylamino-n-butyl, oder Niederalkoxycarbonylaminoalkyl, z.B.

tert-Butoxycarbonylaminoalkyl, wie 4-tert-Butoxycarbonylamino-n-butyl, 5-tert-Butoxycarbonylamino-n-pentyl, 6-tert-Butoxycarbonylamino-n-hexyl oder 7-tert-Butoxycarbonylamino-n-heptyl, ferner 2-tert-Butoxycarbonylaminoäthyl.

Niederalkenyl und Niederalkinyl sind wie die unter $R^a$ und $R^b$ genannten Reste definiert und können durch die gleichen Substituenten wie Niederalkyl $R^a$ substituiert sein.

Ein aromatischer oder aromatisch-aliphatischer Kohlenwasserstoffrest hat die gleichen wie unter $R^a$ oder $R^b$ genannten Bedeutungen und ist vorzugsweise Phenyl oder Phenylniederalkyl.

Diese Reste können im Aromaten z.B. durch Niederalkyl, wie Methyl oder Aethyl, Hydroxy, veräthertes Hydroxy, z.B. Niederalkoxy, wie Methoxy oder tert-Butoxy, verestertes Hydroxy, z.B. Niederalkanoyloxy, wie Acetoxy, oder Halogen, z.B. Fluor oder Chlor, Carboxy, verestertes Carboxy, z.B. Niederalkoxycarbonyl, wie tert-Butoxycarbonyl, Carbamoyl, Amino, Niederalkylamino, z.B. Methylamino, Diniederalkylamino, z.B. Dimethylamino, acyliertes Amino, z.B. Niederaloxycarbonylamino, wie tert-Butoxycarbonylamino, sowie durch Nitro substituiert sein.

Niederalkyl in einem Rest Phenylniederalkyl kann durch die gleichen Substituenten wie Alkyl in einem Rest $R_6$ substituiert sein.

Substituiertes Phenyl ist beispielsweise 2-, 3- oder 4-Niederalkylphenyl, z.B. 2-, 3- oder 4-Methylphenyl, 2-, 3- oder 4-Hydroxyphenyl, 2-, 3- oder 4-Niederalkoxyphenyl, z.B 2-, 3- oder 4-Methoxyphenyl, 2,3-, 2,4- oder 2,5-Dimethoxyphenyl oder 2-, 3- oder 4-tert-Butoxyphenyl, 2-, 3- oder 4-Halogenphenyl, z.B. 2-, 3- oder 4-Chlorphenyl, 2,3-, 3,4- oder 2,5-Dihalogenphenyl, z.B. 2,3-, 3,4- oder 2,5-Dichlorphenyl, 2-, 3- oder 4-Carboxyphenyl, 2-, 3- oder 4-Niederalkoxycarbonylphenyl, z.B. 2-, 3- oder 4-tert-Butoxycarbonylphenyl, 2-, 3-oder 4-Carbamoylphenyl, 2-, 3-oder 4-Aminophenyl, 2-, 3- oder 4-Niederalkoxycarbonylaminophenyl, z.B. 2-, 3- oder 4-tert-Butoxycarbonylaminophenyl, oder 2-, 3- oder 4-Nitrophenyl.

Substituiertes oder unsubstituiertes Phenylniederalkyl ist beispielsweise Benzyl, 2-Phenyläthyl, 1-Phenylprop-2-yl, 3-Phenylpropyl, oder 1-Hydroxymethyl-2-phenyl-äthyl, 2-Hydroxy-1- oder -2-phenyl-äthyl oder 1-(2-Cyano- oder 2-Carboxy-1-hydroxyäthyl)-2-phenyläthyl.

Ein heteroaromatischer oder heteroaromatisch-aliphatischer Kohlenwasserstoffrest hat die gleichen wie unter $R^a$ und $R^b$ genannten Bedeutungen und ist vorzugsweise Oxazolyl, Thiazolyl, Pyridylniederalkyl, Imidazolylniederalkyl oder Indolylniederalkyl.

Diese Reste können im Heteroaromaten z.B. durch Niederalkyl, z.B. Methyl oder Aethyl, Carboxy, verestertes Carboxy, z.B. Niederalkoxycarbonyl, wie tert-Butoxycarbonyl, Carbamoyl, Carboxyniederalkyl, z.B. Carboxymethyl, verestertes Carboxyniederalkyl, z.B. Niederalkoxycarbonyl, wie Methoxycarbonylmethyl, oder Carbamoylniederalkyl, z.B. Carbamoylmethyl substituiert sein.

Substituiertes oder unsubstituiertes Heteroaryl ist beispielsweise 2-Oxazolyl, 2-Thiazolyl oder 4- oder 5-Carbamoylmethyl-2-thiazolyl.

Substituiertes oder unsubstituietes Heteroarylniederalkyl ist beispielsweise 2-, 3- oder 4-Pyridylmethyl, 2-(2-, 3- oder 4-Pyridyl)-äthyl, 3-(2-, 3- oder 4-Pyridyl)-propyl, 4-Imidazolylmethyl, 2-(4-Imidazolyl)-äthyl, 3-Indolylmethyl oder 2-(3-Indolyl)-äthyl.

Die Amino- oder Hydroxygruppe $R_6$ kann ausserdem durch einen fünf- oder sechsgliedrigen Heterocyclus substituiert sein, der die unter $R^a$ oder $R^b$ genannten Bedeutungen hat. Ein solcher Heterocyclus ist

beispielsweise 1-Benzylpiperidin-2-, -3- oder -4-yl, oder 1-Niederalkoxycarbonylpiperidinyl, z.B. 1-Aethoxy- oder 1-n-Butoxycarbonylpiperidin-2-, -3- oder -4-yl.

Substituiertes Amino $R_6$ ist vorzugsweise Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino oder Tris(hydroxymethyl)-methylamino, Niederalkoxyniederalkylamino, z.B. 2-Methoxyäthylamino, substituiertes Phenoxyniederalkylamino, z.B. 2-(3-Carbamoyl-4-hydroxyphenoxy)-äthylamino, substituiertes Phenoxy-hydroxy-niederalkylamino, z.B. 3-(3-Carbamoylphenoxy)-2-hydroxypropylamino, Carboxyalkylamino, z.B. 4-Carboxy-n-butylamino, 5-Carboxy-n-pentylamino, 6-Carboxy-n-hexylamino, 7-Carboxy-n-heptylamino, 8-Carboxy-n-octylamino oder 1-Carboxy-prop-2-ylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 4-Pivaloyloxymethoxycarbonyl-n-butylamino, 7-(1-Aethoxycarbonyloxyäthoxycarbonyl)-n-heptylamino oder 7-Pivaloyloxymethoxycarbonyl-n-heptylamino, Carbamoylniederalkylamino, z.B. Carbamoylmethylamino oder Dicarbamoyl-methylamino, Hydroxyniederalkylcarbamoylniederalkylamino, z.B. 4-(Tris[hydroxymethyl]-methyl)-carbamoyl-n-butylamino, Niederalkoxycarbonyl-hydroxy-niederalkylcarbamoyl-niederalkylamino, z.B. α-Carbamoyl-α-(2-hydroxy-1-isobutyl-3-methoxycarbonylpropyl)-carbamoyl-methylamino, Aminoalkylamino, z.B. 4-Amino-n-butylamino, 5-Amino-n-pentylamino, 6-Amino-n-hexylamino, 7-Amino-n-heptylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf- oder sechsgliedriges Heterocyclylniederalkylamino, z.B. 3-(4-Morpholinyl)-propylamino oder 3-(2-Oxo-1-pyrrolidinyl)-propylamino, Acylaminoniederalkylamino, z.B. Niederalkanoylaminoniederalkylamino, wie 4-Pivaloylamino-n-butylamino, oder Niederalkoxycarbonylaminoniederalkylamino, wie 4-tert-Butoxycarbonylamino-n-butylamino, 5-tert-Butoxycarbonylamino-n-pentylamino, 6-tert-Butoxycarbonylamino-n-hexylamino oder 8-tert-Butoxycarbonylamino-n-octylamino, Benzylamino, Carboxybenzylamino, z.B. 3-Carboxybenzylamino, sowie Niederalkoxycarbonylbenzylamino, z.B. 3-tert-Butoxycarbonylbenzylamino.

Substituiertes Hydroxy $R_6$ ist vorzugsweise Niederalkoxy, z.B. Methoxy, Aethoxy, n-Propoxy, Isopropoxy, Butoxy, Isobutoxy, tert-Butoxy oder tert-Pentoxy, oder physiologisch spaltbares Alkoxy, z.B. Pivaloyloxymethoxy.

Ein N-terminal mit der -CO-Gruppe verbundener und C-terminal gegebenenfalls amidierter oder veresterter natürlicher Aminosäurerest $R_6$ besteht vorzugsweise aus einer der 20 weiter vorn genannten α-Aminosäuren, die besonders häufig in Proteinen vorkommen, z.B. -His-, -Phe- und besonders -Ala- oder -Ile- und ist gegebenenfalls C-terminal durch Amino, substituiertes Amino oder substituiertes Hydroxy substituiert. Solches substituiertes Amino oder substituiertes Hydroxy hat die weiter vorn für $R_6$ genannten Bedeutungen und ist beispielsweise Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Niederalkoxycarbonylalkylamino, z.B. 4-tert-Butoxycarbonyl-n-butylamino oder 7-tert-Butoxycarbonyl-n-heptylamino, physiologisch spaltbares, verestertes Carboxyalkylamino, z.B. 4-Pivaloyloxymethoxycarbonyl-n-butylamino, Carbamoylniederalkylamino, z.B. Carbamoylmethylamino oder Dicarbamoyl-methylamino, Hydroxyniederalkylcarbamoyl-niederalkylamino, z.B. 2-Hydroxyäthylcarbamoyl-methylamino oder Di-(2-hydroxyäthylcarbamoyl)-methylamino, Aminoalkylamino, z.B. 4-Amino-n-butylamino, 5-Amino-n-pentylamino oder 8-Amino-n-octylamino, Hydroxyniederalkylamino-niederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf- oder sechsgliedriges Heterocyclylniederalkylamino, z.B. 3-(4-Morpholinyl)-propylamino,

2-(4-Morpholinyl)-äthylamino oder 3-(2-Oxo-1-pyrrolidinyl)-propylamino, Acylaminoniederalkylamino, z.B. Niederalkoxycarbonylaminoniederalkylamino, wie 4-tert-Butoxycarbonylamino-n-butylamino, Phenylniederalkylamino, z.B. Benzylamino, Carboxybenzylamino, z.B. 3-Carboxybenzylamino, Niederalkoxycarbonylbenzylamino, z.B. 3-tert-Butoxycarbonylbenzylamino, Carbamoylbenzylamino, z.B. 3-Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1- oder 2-phenyläthylamino, 1-(2-Cyano- oder 2-Carboxy-1-hydroxyäthyl)-2-phenyl-äthylamino, Thiazolylamino, z.B. 2-Thiazolylamino oder 4- oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino, 3-(2-, 3- oder 4-Pyridyl)-propylamino, Imidazolylniederalkylamino, z.B. 4-Imidazolylmethylamino, 2-(4-Imidazolyl)-äthylamino, Indolylniederalkylamino, z.B. 3-Indolylmethylamino, 2-(3-Indolyl)-äthylamino, Piperidinylamino, z.B. 1-Benzylpiperidin-2-, -3- oder -4-ylamino, 1-Niederalkoxycarbonylpiperidinylamino, z.B. 1-Aethoxy- oder 1-n-Butoxycarbonylpiperidin-2-, -3- oder -4-ylamino, ausserdem Niederalkoxy, z.B. Methoxy, Aethoxy oder tert-Butoxy, oder physiologisch spaltbares substituiertes Alkoxy, z.B. Pivaloyloxymethoxy.

In einem N-terminal mit der -CO-Gruppe verbundenen und C-terminal gegebenenfalls amidierten oder veresterten Di- oder Tripeptidrest sind die Aminosäurebestandteile vorzugsweise von den 20 weiter vorn genannten α-Aminosäuren, die besonders häufig in Proteinen vorkommen, sowie vom Statin abgeleitet. Ein solcher Di- oder Tripeptidrest ist beispielsweise -Ile-His-, -Ile-Phe-, -Ala-His-, -Gly-Gly-, -Gly-His-, -Gly-Ala-, -Gly-Ser-, -Ala-Sta-, -Ile-Sta-, -Gly-Gly-Gly-, -Gly-His-Lys-, -Gly-Ala-Gly- oder Ile-His-Lys.

Die Aminosäurereste können N-terminal zur Erhöhung der Stabilität der Verbindung der Formel I gegen enzymatischen Abbau durch Niederalkyl, z.B. Methyl oder Aethyl, substituiert sein. Die Carboxy-Funktion in der Seitenkette von -Glu- und -Asp- ist gewünschtenfalls mit einem Niederalkanol, z.B. mit Methanol oder tert-Butanol verestert.

Die Amino-Funktion in der Seitenkette von -Lys- ist gewünschtenfalls durch einen Rest $R^d$-CO-, z.B. Pivaloyl, oder durch einen Rest $R^d$-O-CO-, z.B. tert-Butoxycarbonyl oder Benzyloxycarbonyl, acyliert.

Ein Di- oder Tripeptidrest ist C-terminal gegebenenfalls durch Amino, substituiertes Amino oder substituiertes Hydroxy substituiert. Solches substituiertes Amino oder substituiertes Hydroxy hat die weiter vorn für $R_6$ genannten Bedeutungen und ist beispielsweise Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy.

Salze sind in erster Linie die pharmazeutisch verwendbaren oder nicht-toxischen Salze von Verbindungen der Formel I.

Solche Salze werden beispielsweise von Verbindungen der Formel I mit einer sauren Gruppe, z.B. einer Carboxygruppe, gebildet und sind in erster Linie geeignete Alkalimetall-, wie Natrium- oder Kalium-, oder Erdalkalimetallsalze, z.B. Magnesium- oder Calciumsalze, ferner Zinksalze, oder Ammoniumsalze, inkl. solche Salze, welche mit organischen Aminen, wie gegebenenfalls durch Hydroxy substituierten Mono-, Di- oder Trialkylaminen gebildet werden, z.B. Diäthylamin, Di-(2-hydroxyäthyl)-amin, Triäthylamin, N,N-Dimethyl-N-(2-hydroxyäthyl)-amin, Tri-(2-hydroxy-äthyl)-amin oder N-Methyl-D-glucamin. Die Verbindungen der Formel I mit einer basischen Gruppe, z.B. einer Aminogruppe, können Säureadditionssalze bilden, z.B. mit anorganischen Säuren, z.B. Salzsäure, Schwefelsäure oder Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren,

wie z.B. den weiter vorn genannten α-Aminosäuren, sowie Methansulfonsäure, Aethansulfonsäure, 2-Hydroxyäthansulfonsäure, Aethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung weisen Enzym-hemmende Wirkungen auf; insbesondere hemmen sie die Wirkung des natürlichen Enzyms Renin. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Letzteres erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmern.

Die Wirkung von Renin-Hemmern wird unter anderem experimentell mittels in vitro-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende in vitro Test verwendet: Ein Extrakt von menschlichem Renin aus der Niere (0,5 mGU [Milli-Goldblatt-Einheiten]/ml) wird eine Stunde lang bei 37°C und pH 7,2 in 1-molarer wässriger 2-N-(Tris-hydroxymethyl-

methyl)-amino-äthansulfonsäure-Pufferlösung inkubiert mit 23 µg/ml synthetischem Renin-Substrat, dem Tetradecapeptid H-Asp-Arg-Val-Tyr-Ile-His-Pro-Phe-His-Leu-Leu-Val-Tyr-Ser-OH. Die Menge des gebildeten Angiotensins I wird in einem Radioimmunoassay ermittelt. Die erfindungsgemässen Hemmstoffe werden dem Inkubationsgemisch jeweils in verschiedenen Konzentrationen zugefügt. Als $IC_{50}$ wird diejenige Konzentration des jeweiligen Hemmstoffes bezeichnet, die die Bildung von Angiotensin I um 50% reduziert. Die Verbindungen der vorliegenden Erfindung zeigen in den in vitro-Systemen Hemmwirkungen bei minimalen Konzentrationen von etwa $10^{-6}$ bis etwa $10^{-9}$ Mol/l.

An salzverarmten Tieren bewirken Renin-Hemmer einen Blutdruckabfall. Das menschliche Renin unterscheidet sich von Renin anderer Spezies. Zur Prüfung von Hemmern des humanen Renins werden Primaten (Marmosets, Callithrix jacchus) verwendet, weil humanes Renin und Primaten-Renin im enzymatisch aktiven Bereich weitgehend homolog sind. Unter anderem wird der folgende in vivo Test benutzt: Die Testverbindungen werden an normotensiven Marmosets beider Geschlechter mit einem Körpergewicht von etwa 300 g, die bei Bewusstsein sind, geprüft. Blutdruck und Herzfrequenz werden mit einem Katheter in der Oberschenkelarterie gemessen. Die endogene Freisetzung von Renin wird durch die intravenöse Injektion von Furosemid (5 mg/kg) angeregt. 30 Minuten nach der Injektion von Furosemid werden die Testsubstanzen entweder über ein Katheter in der lateralen Schwanzvene durch einmalige Injektion oder durch kontinuierliche Infusion verabreicht und ihre Wirkung auf Blutdruck und Herzfrequenz ausgewertet. Die Verbindungen der vorliegenden Erfindung sind in dem beschriebenen in vivo Test bei Dosen von etwa 0,1 bis etwa 1,0 mg/kg i.v. wirksam.

Die Verbindungen der vorliegenden Erfindung können als Antihypertensiva, ferner zur Behandlung von Herzinsuffizienz verwendet werden.

Die Erfindung betrifft insbesondere Verbindungen der Formel I, worin $R_1$ Wasserstoff oder Acyl mit den Teilformeln $R^b$-CO- oder $R^a$-O-CO-, worin $R^a$ einen unsubstituierten oder substituierten, gesättigten

oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen oder einen unsubstituierten oder substituierten, fünf- oder sechsgliedrigen Heterocyclus darstellt und $R^b$ Wasserstoff bedeutet oder die Bedeutungen von $R^a$ hat, $X_1$ einen natürlichen Aminosäurerest, der N-terminal mit $R_1$ und C-terminal mit $X_2$ verbunden ist, $X_2$ einen gegebenenfalls N-alkylierten natürlichen Aminosäurerest, N-terminal mit $X_1$ und C-terminal mit der Gruppe $-NR_2-$ verbunden ist, $R_2$ Wasserstoff oder Niederalkyl, $R_3$ Wasserstoff, Niederalkyl, Cycloalkyl, Arylniederalkyl oder Aryl, $R_4$ Hydroxy, $R_5$ Alkyl, Cycloalkyl, Arylniederalkyl oder Aryl, und $R_6$ Amino oder eine Amino-oder Hydroxygruppe, welche durch einen oder gegebenenfalls zwei unsubstituierte oder substituierte, gesättigte oder ungesättigte, aliphatische Kohlenwasserstoffreste bis einschliesslich 18, bevorzugt bis einschliesslich 10, C-Atomen oder einen aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer natürlichen α-Aminosäure oder einen N-terminal mit der Gruppe -CO- verbundenen und C-terminal gegebenenfalls amidierten oder veresterten Di- oder Tripeptidrest, der aus natürlichen α-Aminosäuren und gewünschtenfalls aus Statin besteht, darstellen, mit Ausnahme der Verbindungen, worin $R_1$ einen gegebenenfalls N-acylierten Rest der Aminosäure L-Prolin oder worin $X_1$ und $X_2$ zusammen -Val-Val-, -Gly-Gly- oder -Tyr-Gly- bedeuten, ferner pharmazeutisch annehmbare Salze von Verbindungen der Formel I mit salzbildenden Gruppen.

Die Erfindung betrifft hauptsächlich Verbindungen der Formel I, worin $R_1$ Wasserstoff, Niederalkanoyl mit 1-7 C-Atomen, z.B. Formyl, Acetyl, Propionyl oder Pivaloyl, Hydroxyniederalkanoyl, z.B.

ß-Hydroxypropionyl, Niederalkoxyniederalkanoyl, z.B. Niederalkoxyacetyl oder Niederalkoxypropionyl, wie Methoxyacetyl oder ß-Methoxypropionyl, durch einem gewünschtenfalls acetylierten oder als Acetal geschützten natürlichen Zucker veräthertes Niederalkanoyl, z.B. Glucofuranosyl-O-niederalkanoyl, wie Mono- oder Diisopropylidenglucofuranosyl-O-acetyl, Niederalkanoyloxyniederalkanoyl, z.B. Niederalkanoyloxyacetyl oder Niederalkanoyloxypropionyl, wie Acetoxyacetyl oder ß-Acetoxypropionyl, Halogenniederalkanoyl, z.B. Halogenacetyl, wie α-Chlor-, α-Brom-, α-Iod oder α,α,α-Trichloracetyl, oder Halogenpropionyl, wie ß-Chlor- oder ß-Brompropionyl, Hydroxysulfonyloxyniederalkanoyl, z.B. Hydroxysulfonyloxyacetyl oder ß-Hydroxysulfonyloxypropionyl, Carboxyniederalkanoyl, z.B. Carboxyacetyl oder ß-Carboxypropionyl, Niederalkoxycarbonylniederalkanoyl, z.B. Niederalkoxycarbonylacetyl oder Niederalkoxycarbonylpropionyl, wie Methoxycarbonylacetyl, ß-Methoxycarbonylpropionyl, Aethoxycarbonylacetyl oder ß-Aethoxycarbonylpropionyl, Carbamoylniederalkanoyl, z.B. Carbamoylacetyl oder ß-Carbamoylpropionyl, Niederalkylcarbamoylniederalkanoyl, z.B. Methylcarbamoylacetyl, Diniederalkylcarbamoylniederalkanoyl, z.B. Dimethylcarbamoylacetyl, Aminoalkanoyl mit 1-10 C-Atomen, z.B. Aminoacetyl, α-Aminopropionyl, γ-Aminobutyryl oder 8-Aminooctanoyl, Niederalkylaminoniederalkanoyl, z.B. Methylaminoacetyl, Diniederalkylaminoniederalkanoyl, z.B. Dimethylaminoacetyl, Acylaminoalkanoyl, z.B. Niederalkanoylaminoalkanoyl, wie Acetylaminoacetyl, α-Acetylaminopropionyl oder Pivaloylaminoacetyl, Niederalkoxycarbonylaminoalkanoyl, wie tert-Butoxycarbonylaminoacetyl, γ-tert-Butoxycarbonylaminobutyryl oder 8-tert-Butoxycarbonylaminooctanoyl, Arylniederalkoxycarbonylaminoalkanoyl, wie Benzyloxycarbonylaminoacetyl, γ-Benzyloxycarbonylaminobutyryl oder 8-Benzyloxycarbonylaminooctanoyl, substituiertes Niederalkanoylaminoniederalkanoyl, wie γ-Aminobutyrylaminoacetyl, γ-Benzyloxycarbonylaminobutyrylaminoacetyl, (α-Amino-δ-guanidinovaleryl)-aminoacetyl oder (α-Benzyloxycarbonylamino-δ-guanidinovaleryl)-aminoacetyl, ferner Mercaptoniederalkanoyl, z.B. ß-Mercaptopropionyl, Niederalkylthioniederalkanoyl, z.B. ß-Methylthiopropionyl, Oxoniederalkanoyl, z.B. Acetoacetyl oder Propionylacetyl, Hydroxy-carboxy-niederalkanoyl, z.B. α-Hydroxy-α-carboxy-

acetyl oder α-Hydroxy-ß-carboxypropionyl, Hydroxy-niederalkoxycarbonyl-niederalkanoyl, z.B. α-Hydroxy-α-äthoxy- oder -methoxycarbonylacetyl oder α-Hydroxy-ß-äthoxy- oder -methoxycarbonylpropionyl, verestertes Hydroxy-niederalkoxycarbonyl-niederalkanoyl, z.B. α-Acetoxy-α-methoxycarbonyl-acetyl, Dihydroxy-carboxy-niederalkanoyl, z.B. α,ß-Dihydroxy-ß-carboxypropionyl, Dihydroxy-niederalkoxycarbonyl-niederalkanoyl, z.B. α,ß-Dihydroxy-ß-äthoxy- oder -methoxycarbonylpropionyl, verestertes Dihydroxy-niederalkoxycarbonyl-niederalkanoyl, z.B. α,ß-Diacetoxy-2-äthoxy- oder -methoxycarbonylpropionyl, Hydroxy-amino-niederalkanoyl, z.B. ß-Hydroxy-α-aminopropionyl, ß-Hydroxy-α-amino-butyryl oder 3-Hydroxy-4-amino-6-methylheptanoyl, Hydroxy-acylamino-niederalkanoyl, z.B. ß-Hydroxy-α-benzyloxycarbonylaminopropionyl oder 3-Hydroxy-4-benzyloxycarbonylamino-6-methylheptanoyl, Carboxy-amino-niederalkanoyl, z.B. α-Carboxy-α-aminoacetyl, ß-Carboxy-α-aminopropionyl oder γ-Carboxy-α-aminobutyryl, Niederalkoxycarbonyl-amino-niederalkanoyl, z.B. α-Methoxy- oder Aethoxycarbonyl-α-aminoacetyl, Carboxy-acylamino-niederalkanoyl, z.B. ß-Carboxy-α-benzyloxycarbonylaminopropionyl, oder γ-Carboxy-α-benzyloxycarbonylaminobutyryl, Niederalkoxycarbonyl-acylamino-niederalkanoyl, z.B. α-Methoxy- oder Aethoxycarbonyl-α-benzyloxycarbonylaminoacetyl oder α-Methoxy- oder Aethoxycarbonyl-α-tert-butoxycarbonylaminoacetyl, Diaminoniederalkanoyl, z.B. 2,6-Diaminohexanoyl, Diacylaminoniederalkanoyl, z.B. 2,6-Bis-(benzyloxycarbonylamino)-hexanoyl, Niederalkenoyl mit 3-7 C-Atomen, z.B. Acryloyl, Vinylacetyl, Crotonyl oder 3- oder 4-Pentenoyl, Niederalkinoyl mit 3-7 C-Atomen, z.B. Propiolyl oder 2- oder 3-Butinoyl, Cycloalkylcarbonyl mit 4-9 C-Atomen, z.B. Cyclopropylcarbonyl, Cyclobutylcarbonyl, Cyclopentylcarbonyl oder Cyclohexylcarbonyl, Bicycloalkylcarbonyl mit 6-11 C-Atomen, z.B. Bicyclo[3.1.0]hex-1-, -2- oder -3-ylcarbonyl, Bicyclo[4.1.0]hept-1- oder -4-ylcarbonyl, Bicyclo[3.3.0]oct-3-ylcarbonyl oder Bicyclo[3.3.1]-non-9-ylcarbonyl, Tricycloalkylcarbonyl mit 9-11 C-Atomen, z.B. Tricyclo[$5.2.1.0^{2,6}$]dec-8-ylcarbonyl oder Adamantylcarbonyl, Monocycloalkenylcarbonyl mit 4-9 C-Atomen, z.B. 1-Cyclohexenylcarbonyl oder 1,4-Cyclohexadienylcarbonyl, Bicycloalkenylcarbonyl mit 6-11 C-Atomen, z.B. 5-Norbornen-2-ylcarbonyl, Bicyclo[2.2.2]-octen-2-ylcarbonyl oder Hexahydro-4,7-methano-ind-1-en-6-ylcarbonyl, Cycloalkylniederalkanoyl mit 5-11 C-Atomen, z.B. Cyclopropylacetyl, Cyclobutylacetyl, Cyclopentylacetyl oder Cyclohexylacetyl, Cycloalkylniederalkenoyl mit 6-11 C-Atomen, z.B. Cyclohexylacryloyl, Cycloalkenylniederalkanoyl mit 5-11 C-Atomen, z.B. 1-Cyclohexenylacetyl oder 1,4-Cyclohexadienylacetyl, Hydroxycycloalkylcarbonyl, z.B. 4-Hydroxy-cyclohexylcarbonyl oder 3,4,5-Trihydroxycyclohexylcarbonyl, Carboxycycloalkylcarbonyl, z.B. 4-Carboxycyclohexylcarbonyl, Aminocycloalkylcarbonyl, z.B. 1-Aminocyclopropylcarbonyl oder 4-Aminocyclohexylcarbonyl, Acylaminocycloalkylcarbonyl, z.B. 1-tert-Butoxycarbonylaminocyclopropylcarbonyl oder 1-Benzyloxycarbonylaminocyclopropylcarbonyl, Hydroxycycloalkenylcarbonyl, z.B. 3,4,5-Trihydroxycyclohex-1-enylcarbonyl, Benzoyl, unsubstituiert, mono- oder mehrfachsubstituiert durch Niederalkyl, Hydroxy, Niederalkoxy, Halo und/oder Nitro, z.B. 4-Chlorobenzoyl, 4-Methoxybenzoyl oder 4-Nitrobenzoyl, Phenylniederalkanoyl, worin Phenyl unsubstituiert, mono- oder mehrfachsubstituiert durch Niederalkyl, Hydroxy, Niederalkoxy, Halo und/oder Nitro sein kann, z.B. Phenylacetyl, Niederalkylphenylacetyl, z.B. 4-Methylphenylacetyl, Niederalkoxyphenylacetyl, z.B. 4-Methoxyphenylacetyl, ß-Phenylpropionyl, ß-(p-Hydroxyphenyl)-propionyl, Diphenylacetyl, Di-(4-methoxyphenyl)-acetyl, Triphenylacetyl, ferner substituiertes Anilinophenylacetyl, z.B. 2-(o,o-Dichloroanilino)-phenylacetyl oder 2-(o,o-Dichloro-N-benzylanilino)-phenylacetyl, Phenylniederalkenoyl, z.B. ß-Phenylacryloyl oder ß-Phenylvinylacetyl, 2- oder 3-Pyrrolylcarbonyl, 2-, 3- oder 4-Pyridylcarbonyl, 2-, 3- oder 5-Indolylcarbonyl, 2-, 3- oder 4-Chinolinylcarbonyl, 1-, 3- oder 4-Isochinolinylcarbonyl, Phenyl-hydroxy-niederalkanoyl, z.B. α-Phenyl-α-hydroxy-acetyl, unsubstituiertes oder substituiertes Phenylamino-niederalkanoyl, z.B. ß-Phenyl-α-aminopropionyl oder ß-(4-Hydroxyphenyl)-α-aminopropionyl, unsubstituiertes oder substituiertes Phenyl-acylamino-niederalkanoyl, z.B. ß-Phenyl-α-benzyloxycarbonylaminopropionyl oder ß-(4-Acetoxyphenyl)-α-benzyloxycarbonylaminopropionyl, Pyrrolidinyl-3-carbonyl, Hydroxypyrrolidinylcarbonyl, z.B. 3- oder 4-Hydroxypyrrolidinyl-2-carbonyl, Hydroxy-1-acylpyrrolidinylcarbonyl, z.B. 3- oder 4-Hydroxy-1-benzyl-

oxycarbonyl-pyrrolidinyl-2-carbonyl, Oxopyrrolidinylcarbonyl, z.B. 5-Oxopyrrrolidinyl-2-carbonyl, Piperidinyl-2-, -3- oder -4-carbonyl, 1-Niederalkylpiperidinylcarbonyl, z.B. 1-Methylpiperidinyl-2-, -3- oder -4-carbonyl, 1-Acylpiperidinylcarbonyl, z.B. 1-Benzyloxycarbonylpiperidinyl-2-, -3- oder -4-carbonyl, Morpholinyl-2- oder -3-carbonyl, Thiomorpholinyl-2- oder -3-carbonyl, 1- und/oder 4-Niederalkylpiperazinylcarbonyl, z.B. 1,4-Dimethylpiperazinyl-2-oder -3-carbonyl, ferner Niederalkoxycarbonyl, z.B. Methoxycarbonyl, Aethoxycarbonyl oder tert-Niederalkoxycarbonyl, wie tert-Butoxycarbonyl, 2-Halogenniederalkoxycarbonyl, z.B. 2-Chlor-, 2-Brom-, 2-Iod- oder 2,2,2-Trichloräthoxycarbonyl, Arylniederalkoxycarbonyl, worin Aryl Phenyl, 1- oder 2-Naphthyl oder durch Niederalkyl, z.B. Methyl oder tert-Butyl, Niederalkoxy, z.B. Methoxy, Aethoxy oder tert-Butoxy, Hydroxy, Halogen, z.B. Chlor oder Brom, und/oder Nitro mono-oder mehrfach substituiertes Phenyl ist, z.B. Benzyloxycarbonyl, 4-Methoxybenzyloxycarbonyl, 4-Nitrobenzyloxycarbonyl, Diphenylmethoxycarbonyl, Di-(4-methoxyphenyl)-methoxycarbonyl oder Trityloxycarbonyl,

$X_1$ und $X_2$ unabhängig voneinander Ala, Arg, Asn, Asp, Cys, Gln, Glu, Gly, His, Ile, Leu, Lys, Met, Phe, Pro, Ser, Thr, Trp, Tyr oder Val, $-X_1-X_2-$ aber nicht -Val-Val-, -Gly-Gly- oder -Tyr-Gly-, und worin gegebenenfalls die Carboxylfunktion der Seitenkette in Asp und Glu mit einem Niederalkanol, z.B. Methanol oder tert-Butanol, verestert und/oder die Aminofunktion der Seitenkette in Lys durch Niederalkanoyl, z.B. Pivaloyl, durch Niederalkoxycarbonyl, z.B. tert-Butoxycarbonyl, oder durch Arylniederalkoxycarbonyl, z.B. Benzyloxycarbonyl, acyliert ist, $X_1$ N-terminal mit $R_1$ und C-terminal mit $X_2$ und $X_2$ N-terminal mit $X_1$ und C-terminal mit $-NR_2$-verbunden ist und $X_2$ am N-Atom durch Niederalkyl substituiert sein kann,

$R_2$ Wasserstoff oder Niederalkyl,

$R_3$ Wasserstoff, Niederalkyl, z.B. Isopropyl oder Isobutyl, Cycloalkyl, Phenylniederalkyl oder Phenyl,

$R_4$ Hydroxy,

$R_5$ Alkyl mit 1-10 C-Atomen, z.B. Niederalkyl, wie Isopropyl oder Isobutyl, oder n-Octyl, Cycloalkyl, z.B. Cyclohexyl, Phenylniederalkyl, z.B. Benzyl, oder Phenyl,

und $R_6$ Amino, Alkylamino mit 1 bis 10 C-Atomen, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino oder Diäthylamino, Hydroxyniederalkylamino, z.B. 2-Hydroxyäthylamino, 3-Hydroxypropylamino oder Tris-(hydroxymethyl)-methylamino, Di-(hydroxyniederalkyl)-amino, z.B. Di-(2-hydroxyäthyl)-amino, Niederalkoxyniederalkylamino, z.B. Methoxymethylamino oder 2-Methoxyäthylamino, Niederalkanoyloxyniederalkylamino, z.B. Acetoxymethylamino oder 2-Acetoxyäthylamino, Halogenniederalkylamino, z.B. Halogenmethyl- oder Halogenäthylamino, wie Trifluoromethyl-, Chlormethyl-, Brommethyl-, 2-Chloräthyl, 2-Bromäthyl- oder 2,2,2-Trichloräthylamino, Hydroxysulfonyloxyniederalkylamino, z.B. Hydroxysulfonyloxymethylamino oder 2-Hydroxysulfonyloxyäthylamino, Phenyloxyniederalkylamino oder Phenyloxy-hydroxy-niederalkylamino, worin Phenyl gegebenenfalls durch Niederalkyl, Niederalkoxy, Hydroxy, Carboxy, Niederalkoxycarbonyl oder Carbamoyl substituiert ist, z.B. 2-Phenyloxyäthyl-, 2-(3-Carbamoyl-4-hydroxyphenoxy)-äthyl- oder 3-(3-Carbamoylphenoxy)-2-hydroxypropylamino, Carboxyalkylamino, z.B. 4-Carboxy-n-butyl-, 5-Carboxy-n-pentyl-, 6-Carboxy-n-hexyl, 7-Carboxy-n-heptyl-, 8-Carboxy-n-octyl-, 2-Carboxyäthyl-, 2-Carboxy-n-propyl oder 1- oder 2-Carboxyprop-2-yl-amino, Niederalkoxycarbonylalkylamino, z.B. 4-tert-Butoxycarbonyl-n-butyl-, 7-tert-Butoxycarbonyl-n-heptyl-, 8-tert-Butoxycarbonyl-n-octyl-, 2-tert-Butoxycarbonyläthyl- oder 1-tert-Butoxycarbonylprop-2-yl-amino, physiologisch spaltbares verestertes Carboxyalkylamino, z.B. 4-Pivaloyloxymethoxycarbonyl-n-butylamino, 7-(1-Aethoxycarbonyloxyäthoxycarbonyl)-n-heptylamino oder 7-Pivaloyloxymethoxycarbonyl-n-heptylamino, Carbamoylniederalkylamino, z.B. Carbamoylmethylamino, 2-Carbamoyläthylamino oder Dicarbamoyl-methylamino, Niederalkylcarbamoylniederalkylamino, z.B. Methylcarbamoylmethylamino, Hydroxyniederalkylcarbamoylniederalkylamino, z.B. 7-(2-Hydroxyäthyl)-carbamoyl-n-heptyl-, 4-(2-Hydroxyäthyl)-carbamoyl-n-butyl-, 7-(Tris-[hydroxymethyl]-methyl)-carbamoyl-n-heptyl- oder 4-(Tris-[hydroxymethyl]-methyl)-carbamoyl-n-butylamino, Triniederalkylsilyloxyniederalkylcarbamoylniederalkylamino, z.B. 4-(Tris-[tert-butyldimethylsilyloxymethyl]-methyl)-carbamoyl-n-butylamino, Niederalkoxycarbonyl-hydroxy-niederalkylcarbamoylniederalkylamino, z.B. α-Carbamoyl-α-(2-hydroxy-1-isobutyl-3-methoxycarbonylpropyl)-carbamoyl-methylamino, Diniederalkylcarbamoylniederalkylamino, z.B. Dimethylcarbamoylmethylamino oder Bis-dimethylcarbamoyl-methylamino, Sulfoniederalkylamino, z.B. Sulfomethylamino oder 2-Sulfoäthylamino, Aminoalkylamino, z.B. 4-Amino-n-butyl-, 5-Amino-n-pentyl-, 6-Amino-n-hexyl-, 7-Amino-n-heptyl-, 8-Amino-n-octyl-, 2-Amino-äthyl- oder 1-Amino-prop-2-yl-amino, Niederalkylaminoniederalkylamino, z.B. 2-Methylaminoäthylamino oder 3-Methylamino-n-propylamino, Hydroxyniederalkylaminoniederalkylamino, z.B. 2-(2-Hydroxyäthylamino)-äthylamino oder 3-(2-Hydroxyäthylamino)-n-propylamino, Diniederalkylaminoniederalkylamino, z.B. 2-Dimethylaminoäthylamino oder 3-Dimethylamino-n-propylamino, 1-Piperidinylniederalkylamino, z.B. 2-(1-Piperidinyl)-äthlyamino oder 3-(1-Piperidinyl)-propylamino, 4-Morpholinylniederalkylamino, z.B. 2-(4-Morpholinyl)-äthylamino oder 3-(4-Morpholinyl)-propylamino, 1-Pyrrolidinylniederalkylamino, worin Pyrrolidin gegebenenfalls durch Hydroxy und/oder Oxo substituiert ist, z.B. 2-(2-Oxopyrrolidin-1-yl)-äthylamino oder 3-(2-Oxo-pyrrolidin-1-yl)-propylamino, Acylaminoalkylamino, z.B. Niederalkanoylaminoalkylamino, z.B. Pivaloylaminoalkylamino oder Acetylaminoalkylamino, wie 4-Pivaloylamino-n-butylamino, 2-Pivaloylamino-äthylamino oder 4-Acetylamino-n-butylamino, Niederalkoxycarbonylaminoalkylamino, wie 4-tert-Butoxycarbonylamino-n-butyl-, 5-tert-Butoxycarbonylamino-n-pentyl-, 6-tert-Butoxycarbonylamino-n-hexyl-, 7-tert-Butoxycarbonylamino-n-heptyl oder 2-tert-Butoxycarbonylaminoäthyl-amino, Niederalkenylamino, z.B. Allylamino oder 2- oder 3-Butenylamino, Niederalkinylamino, z.B. Propargylamino, Phenylamino oder Phenylniederalkylamino, worin Phenyl gegebenenfalls durch Niederalkyl, z.B. Methyl, Hydroxy, Niederalkoxy, z.B. Methoxy oder tert-Butoxy, Niederalkanoyloxy, z.B. Acetoxy, Halogen, z.B. Fluor

oder Chlor, Carboxy, Niederalkoxycarbonyl, z.B. tert-Butoxycarbonyl, Carbamoyl, Amino, Niederalkylamino, z.B. Methylamino, Diniederalkylamino, z.B. Dimethylamino, Acylamino, z.B. tert-Butoxycarbonylamino und/oder durch Nitro mono- oder mehrfach substituiert ist, z.B. Phenyl-, 2-, 3- oder 4-Methylphenyl-, 4-Hydroxyphenyl-, 4-Methoxyphenyl-, 2,3-, 2,4- oder 2,5-Dimethoxyphenyl-, 4-Chlorphenyl, 2-, 3- oder 4-Carboxyphenyl-, 2-, 3- oder 4-Methoxy- oder tert-Butoxycarbonylphenyl-, 2-, 3- oder 4-Carbamoylphenyl-, 4-Aminophenyl-, 4-tert-Butoxycarbonylaminophenyl- oder 4-Nitrophenyl-amino, ferner z.B. Benzylamino, 4-Methylbenzylamino, 4-Methoxybenzylamino, 2-, 3- oder 4-Carboxybenzylamino, 2-, 3- oder 4-tert-Butoxycarbonylbenzylamino, 2-, 3- oder 4-Carbamoylbenzylamino, 2-Phenyläthylamino, 2-Phenylprop-2-ylamino oder 3-Phenylpropylamino, Phenylniederalkylamino, worin Niederalkyl durch Hydroxy, Cyano, Carboxy, Niederalkoxy und/oder Carbamoyl substituiert ist, z.B. 1-Hydroxymethyl-2-phenyläthylamino, 2-Hydroxy-1- oder -2-phenyläthylamino, oder 1-(2-Cyano- oder -2-Carboxy-1-hydroxyäthyl)-2-phenyläthylamino, Oxazolylamino, z.B. 2-Oxazolylamino, Thiazolylamino, das gegebenenfalls durch Carboxyniederalkyl, z.B. Carboxymethyl, Niederalkoxycarbonylniederalkyl, z.B. Methoxycarbonylmethyl, oder Carbamoylniederalkyl, z.B. Carbamoylmethyl, substituiert ist, z.B. 4-oder 5-Carboxymethyl-2-thiazolylamino oder 4- oder 5-Carbamoylmethyl-2-thiazolylamino, Pyridylniederalkylamino, z.B. 2-, 3- oder 4-Pyridylmethyl-, 2-(2-, 3- oder 4-Pyridyl)-äthyl- oder 3-(2-, 3- oder 4-Pyridyl)-propylamino, Imidazolylniederalkylamino, z.B. 4-Imidazolylmethylamino oder 2-(4-Imidazolyl)-äthylamino, Indolylniederalkylamino, z.B. 3-Indolylmethylamino oder 2-(3-Indolyl)-äthylamino, 1-Benzylpiperidinylamino, z.B. 1-Benzylpiperidin-4-ylamino, 1-Niederalkoxycarbonyl piperidinylamino, z.B. 1-Aethoxy- oder 1-n-Butoxycarbonylpiperidin-2-, -3- oder -4-ylamino, ferner Niederalkoxy, z.B. Methoxy, Aethoxy, n-Propoxy, Isopropoxy, Butoxy, Isobutoxy, tert-Butoxy oder tert-Pentoxy, Phenyloxy, Phenylniederalkoxy, z.B. Benzyloxy oder Diphenylmethoxy, oder physiologisch spaltbares Alkoxy, z.B. Pivaloyloxymethoxy,

ferner -His-, -Phe-, -Ala-, -Ile-, C-terminal substituiert durch Amino, Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Niederalkoxycarbonylalkylamino, z.B. 4-tert-Butoxycarbonyl-n-butylamino oder 7-tert-Butoxycarbonyl-n-heptylamino, physiologisch spaltbares, verestertes Carboxyalkylamino, z.B. 4-Pivaloyloxymethoxycarbonyl-n-butylamino, Carbamoylniederalkylamino, z.B. Carbamoylmethylamino oder Dicarbamoyl-methylamino, Hydroxyniederalkylcarbamoyl-niederalkylamino, z.B. 2-Hydroxyäthylcarbamoyl-methylamino oder Di-(2-hydroxyäthylcarbamoyl)-methylamino, Aminoalkylamino, z.B. 4-Amino-n-butylamino, 5-Amino-n-pentylamino oder 8-Amino-n-octylamino, Hydroxyniederalkylamino-niederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf oder sechsgliedriges Heterocyclylniederalkylamino, z.B. 3-(4-Morpholinyl)-propylamino, 2-(4-Morpholinyl)-äthylamino oder 3-(2-Oxo-1-pyrrolidinyl)-propylamino, Acylaminoniederalkylamino, z.B. Niederalkoxycarbonylaminoniederalkylamino, wie 4-tert-Butoxycarbonylamino-n-butylamino, Phenylniederalkylamino, worin Phenyl durch Carboxy, Niederalkoxycarbonyl oder Carbamoyl und Niederalkyl durch Hydroxy, Cyano oder Carboxy substituiert sein kann, z.B. Benzylamino, Carboxybenzylamino, z.B. 3-Carboxybenzylamino, Niederalkoxycarbonylbenzylamino, z.B. 3-tert-Butoxycarbonylbenzylamino, Carbamoylbenzylamino, z.B. 3-Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1- oder 2-phenyläthylamino, 1-(2-Cyano- oder 2-Carboxy-1-hydroxyäthyl)-2-phenyl-äthylamino, unsubstituiertes oder substituiertes Thiazolylamino, z.B. 2-Thiazolylamino oder 4-oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino, 3-(2-, 3- oder 4-Pyridyl)-propylamino, Imidazolylniederalkylamino, z.B. 4-Imidazolylmethylamino oder 2-(4-Imidazolyl)-äthylamino, Indolylniederalkylamino, z.B. 3-Indolylmethylamino oder 2-(3-Indolyl)-äthylamino,

Piperidinylamino, z.B. 1-Benzylpiperidin-2-, -3- oder -4-ylamino, 1-Niederalkoxycarbonylpiperidinylamino, z.B. 1-Aethoxy- oder 1-n-Butoxycarbonylpiperidin-2-, -3- oder -4-ylamino, ausserdem durch Niederalkoxy, z.B. Methoxy, Aethoxy oder tert-Butoxy, oder physiologisch spaltbares substituiertes Alkoxy, z.B. Pivaloyloxymethoxy,

ferner -Ile-His-, -Ile-Phe-, -Ala-His-, -Gly-Gly-, -Gly-His-, -Gly-Ala-, -Gly-Ser-, -Ala-Sta-, -Ile-Sta-, -Gly-Gly-Gly-, -Gly-His-Lys-, -Gly-Ala-Gly- oder -Ile-His-Lys-, worin die Aminofunktion in der Seitenkette von -Lys- gegebenenfalls durch Niederalkanoyl, z.B. Pivaloyl, Niederalkoxycarbonyl, z.B. tert-Butoxycarbonyl, oder Phenylniederalkoxycarbonyl, z.B. Benzyloxycarbonyl, acyliert ist, C-terminal substituiert durch Amino, Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy, darstellt, sowie pharmazeutisch annehmbare Salze von diesen Verbindungen mit salzbildenden Gruppen.

Die Erfindung betrifft ganz besonders Verbindungen der Formel I, worin $R_1$ Wasserstoff, Niederalkanoyl, z.B. Formyl, Acetyl, Propionyl oder Pivaloyl, Niederalkoxyniederalkanoyl, z.B. Methoxyacetyl, Mono- oder Diisopropyliden-glucofuranosyl-O-acetyl, z.B. 1,2-Mono- oder 1,2:5,6-Diisopropyliden-glucofuranosyl-3-O-acetyl, Halogenniederalkanoyl, z.B. α-Halogenacetyl, z.B. α-Chlor-, α-Brom-, α-Jod- oder α,α,α-Trichloracetyl, Carboxyniederalkanoyl, z.B. Carboxyacetyl, Niederalkoxycarbonylniederalkanoyl, z.B. Methoxycarbonylacetyl, Aminoalkanoyl, z.B. Aminoacetyl, α-Aminopropionyl, γ-Aminobutyryl oder 8-Aminooctanoyl, Acylaminoalkanoyl, z.B. Niederalkanoylaminoalkanoyl, wie Acetylaminoacetyl oder Pivaloylaminoacetyl, Niederalkoxycarbonylaminoalkanoyl, wie tert-Butoxycarbonylaminoacetyl, γ-(tert-Butoxycarbonylamino)-butyryl oder 8-(tert-Butoxycarbonylamino)-octanoyl, Phenylniederalkoxycarbonylaminoalkanoyl, wie Benzyloxycarbonylaminoacetyl, γ-(Benzyloxycarbonylamino)-butyryl

oder 8-(Benzyloxycarbonylamino)-octanoyl, γ-Aminobutyryl-aminoacetyl, γ-Benzyloxycarbonylaminobutyryl-aminoacetyl, (α-Amino-δ-guanidinovaleryl)-aminoacetyl, oder (α-Benzyloxycarbonylamino-δ-guanidinovaleryl)-aminoacetyl, Oxoniederalkanoyl, z.B. Acetoacetyl, Hydroxycarboxy-niederalkanoyl, z.B. α-Hydroxy-ß-carboxypropionyl, Hydroxy-niederalkoxycarbonyl-niederalkanoyl, z.B. α-Hydroxy-ß-äthoxy- oder -methoxycarbonylpropionyl, Dihydroxy-carboxy-niederalkanoyl, z.B. α,ß-Dihydroxy-ß-carboxypropionyl, Dihydroxy-niederalkoxycarbonyl-niederalkanoyl, z.B. α,ß-Dihydroxy-ß-äthoxy- oder -methoxycarbonylpropionyl, verestertes Dihydroxy-niederalkoxycarbonyl-niederalkanoyl, z.B. α,ß-Diacetoxy-ß-methoxycarbonylpropionyl, Hydroxy-amino-niederalkanoyl, z.B. ß-Hydroxy-α-aminopropionyl oder 3-Hydroxy-4-amino-6-methylheptanoyl, Hydroxy-acylamino-niederalkanoyl, z.B. ß-Hydroxy-α-benzyloxycarbonylaminopropionyl oder 3-Hydroxy-4-benzyloxycarbonylamino-6-methylheptanoyl, Carboxy-amino-niederalkanoyl, z.B. α-Carboxy-α-aminoacetyl, Niederalkoxycarbonyl-amino-niederalkanoyl, z.B. α-Methoxy- oder Aethoxycarbonyl-α-aminoacetyl, Niederalkoxycarbonyl-acylamino-niederalkanoyl, z.B. α-Methoxy- oder Aethoxycarbonyl-α-benzyloxycarbonylaminoacetyl, Niederalkenoyl, z.B. Acryloyl oder Crotonoyl, Niederalkinoyl, z.B. Propiolyl, Cyclopentyl- oder Cyclohexylcarbonyl, Hydroxycyclohexylcarbonyl, z.B. 3,4,5-Trihydroxycyclohexylcarbonyl, Acylaminocycloalkylcarbonyl, z.B. 1-tert-Butoxycarbonylaminocyclopropylcarbonyl oder 1-Benzyloxycarbonylaminocyclopropylcarbonyl, 3,4,5-Trihydroxycyclohex-1-enylcarbonyl, Benzoyl oder durch Halogen, z.B. Chlor, Niederalkoxy, z.B. Methoxy, und/oder Nitro substituiertes Benzoyl, z.B. 4-Chlor-, 4-Methoxy- oder 4-Nitrobenzoyl, 2-(o,o-Dichloroanilino)-phenylacetyl, 2-(o,o-Dichloro-N-benzylanilino)-phenylacetyl, Hydroxypyrrolidinylcarbonyl, z.B. 3- oder 4-Hydroxypyrrolidinyl-2-carbonyl, Hydroxy-1-acylpyrrolidinylcarbonyl, z.B. 3- oder 4-Hydroxy-1-benzyloxycarbonylpyrrolidinyl-2-carbonyl, Oxopyrrolidinylcarbonyl, z.B. 5-Oxopyrrolidinyl-2-carbonyl, Niederalkoxycarbonyl, z.B. tert-Butoxycarbonyl, 2-Halogenniederalkoxycarbonyl, z.B. 2-Chlor-, 2-Brom-, 2-Jod-oder 2,2,2-Trichloräthoxycarbonyl, Arylmethoxycarbonyl mit einem oder zwei Arylresten, worin Aryl gegebenenfalls

durch Niederalkyl, z.B. Methyl oder tert-Butyl, Niederalkoxy, z.B. Methoxy, Hydroxy, Halogen, z.B. Chlor, und/oder Nitro mono-, di-oder trisubstituiertes Phenyl ist, z.B. Benzyloxycarbonyl, 4-Nitrobenzyloxycarbonyl, Diphenylmethoxycarbonyl oder Di-(4-methoxyphenyl)-methoxycarbonyl,

$X_1$ und $X_2$ zusammen -Phe-His-, -Phe-Phe-, -Phe-(N-methyl-Phe)-, -Phe-Leu-, -Phe-Ala-, -Phe-Arg-, -Phe-Gly-, -His-His-, -Arg-Arg-, -Arg-His-, -Arg-Phe-oder -Glu-His-, welche N-terminal mit $R_1$ und C-terminal mit der Gruppe $-NR_2-$ verbunden sind, $R_2$ Wasserstoff, $R_3$ Niederalkyl, z.B. Isopropyl oder Isobutyl, $R_4$ Hydroxy, $R_5$ Alkyl, z.B. Niederalkyl wie Isopropyl oder Isobutyl, oder n-Octyl, Cyclohexyl oder Phenyl, und

$R_6$ Amino, Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino oder Tris(hydroxymethyl)-methylamino, Niederalkoxyniederalkylamino, z.B. 2-Methoxyäthylamino, substituiertes Phenoxyniederalkylamino, z.B. 2-(3-Carbamoyl-4-hydroxyphenoxy)-äthylamino, substituiertes Phenoxy-hydroxy-niederalkylamino, z.B. 3-(3-Carbamoylphenoxy)-2-hydroxypropylamino, Carboxyalkylamino, z.B. 4-Carboxy-n-butylamino, 5-Carboxy-n-pentylamino, 6-Carboxy-n-hexylamino, 7-Carboxy-n-heptylamino, 8-Carboxy-n-octylamino oder 1-Carboxy-prop-2-ylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 4-Pivaloyloxymethoxycarbonyl-n-butylamino, 7-(1-Aethoxy-carbonyloxyäthoxycarbonyl)-n-heptylamino oder 7-Pivaloyloxymethoxycarbonyl-n-heptylamino, Carbamoylniederalkylamino, z.B. Carbamoylmethylamino oder Dicarbamoyl-methylamino, Hydroxyniederalkylcarbamoylniederalkylamino, z.B. 4-(Tris[hydroxymethyl]-methyl)-carbamoyl-n-butylamino, Niederalkoxycarbonyl-hydroxy-niederalkylcarbamoyl-niederalkylamino, z.B. α-Carbamoyl-α-(2-hydroxy-1-isobutyl-3-methoxycarbonylpropyl)-carbamoyl-methylamino, Aminoalkylamino, z.B. 4-Amino-n-butylamino,

5-Amino-n-pentylamino, 6-Amino-n-hexylamino, 7-Amino-n-heptylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, über ein Stickstoffatom gebundenes fünf- oder sechsgliedriges Heterocyclylniederalkylamino, z.B. 3-(4-Morpholinyl)-propylamino oder 3-(2-Oxo-1-pyrrolidinyl)-propylamino, Acylaminoniederalkylamino, z.B. Niederalkanoylaminoniederalkylamino, wie 4-Pivaloylamino-n-butylamino, oder Niederalkoxycarbonylaminoniederalkylamino, wie 4-tert-Butoxycarbonylamino-n-butylamino, 5-tert-Butoxycarbonylamino-n-pentylamino, 6-tert-Butoxycarbonylamino-n-hexylamino oder 8-tert-Butoxycarbonylamino-n-octylamino, Benzylamino, Carboxybenzylamino, z.B. 3-Carboxybenzylamino, Niederalkoxycarbonylbenzylamino, z.B. 3-tert-Butoxycarbonylbenzylamino, sowie Niederalkoxy, z.B. Methoxy, Aethoxy, n-Propoxy, Isopropoxy, Butoxy, Isobutoxy, tert-Butoxy oder tert-Pentoxy, oder physiologisch spaltbares Alkoxy, z.B. Pivaloyloxymethoxy,

ferner -Ala- oder -Ile-, C-terminal substituiert durch Amino, Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Carbamoylniederalkylamino, z.B. Carbamoylmethylamino oder Dicarbamoyl-methylamino, Hydroxyniederalkylcarbamoylniederalkylamino, z.B. 2-Hydroxyäthylcarbamoylmethylamino oder Di-(2-hydroxyäthylcarbamoyl)-methylamino, Aminoalkylamino, z.B. 4-Amino-n-butylamino, 5-Amino-n-pentylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, 4-Morpholinylniederalkylamino, z.B. 3-(4-Morpholinyl)-propylamino oder 2-(4-Morpholinyl)-äthylamino, Phenylniederalkylamino, z.B. Benzylamino, Carboxybenzylamino, z.B. 3-Carboxybenzylamino, Niederalkoxycarbonylbenzylamino, z.B. 3-tert-Butoxycarbonylbenzylamino, Carbamoylbenzylamino, z.B. 3-Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1- oder 2-phenyläthylamino oder 1-(2-Cyano- oder 2-Carboxy-1-hydroxyäthyl)-2-phenyläthylamino, Thiazolylamino, z.B. 4- oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino oder 3-(2-, 3- oder 4-Pyridyl)-propylamino, Imidazolylniederalkylamino, z.B. 4-Imidazolylmethylamino, oder 2-(4-Imidazolyl)-äthylamino, Indolylniederalkylamino, z.B. 3-Indolylmethylamino oder 2-(3-Indolyl)-äthylamino, 1-Niederalkoxycarbonylpiperidinylamino, z.B. 1-Aethoxycarbonylpiperidin-4-ylamino, Niederalkoxy, z.B. Methoxy, Aethoxy oder tert-Butoxy, oder physiologisch spaltbares substituiertes Alkoxy, z.B. Pivaloyloxymethoxy,

ferner -Ile-His-, -Ile-Phe, -Ile-Sta-, -Ala-His-, -Ala-Sta- oder -Ile-His-Lys-, worin die Aminofunktion in der Seitenkette von -Lys- gegebenenfalls durch Niederalkanoyl, z.B. Pivaloyl, Niederalkoxcarbonyl, z.B. tert-Butoxycarbonyl, oder Phenylniederalkoxycarbonyl, z.B. Benzyloxycarbonyl, acyliert ist, C-terminal substituiert durch Amino, Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy, darstellt, sowie pharmazeutisch annehmbare Salze von diesen Verbindungen mit salzbildenden Gruppen.

Die Erfindung betrifft bevorzugt Verbindungen der Formel I, worin $R_1$ Wasserstoff, Niederalkanoyl, z.B. Formyl, Acetyl, Propionyl oder Pivaloyl, Mono- oder Diisopropyliden-glucofuranosyl-O-acetyl, z.B. 1,2-Mono- oder 1,2:5,6-Diisopropyliden-glucofuranosyl-3-O-acetyl, Aminoalkanoyl, z.B. Aminoacetyl, α-Aminopropionyl, γ-Aminobutyryl oder 8-Aminooctanoyl, Acylaminoalkanoyl, z.B. Niederalkanoylaminoalkanoyl, wie Acetylaminoacetyl oder Pivaloylaminoacetyl, Niederalkoxycarbonylaminoalkanoyl, wie tert-Butoxycarbonylaminoacetyl, γ-(tert-Butoxycarbonylamino)-butyryl oder 8-(tert-Butoxycarbonylamino)-octanoyl, Phenylniederalkoxycarbonylaminoalkanoyl, wie

Benzyloxycarbonylaminoacetyl, γ-(Benzyloxycarbonylamino)-butyryl oder 8-(Benzyloxycarbonylamino)-octanoyl, γ-Aminobutyryl-amino-acetyl, γ-Benzyloxycarbonylaminobutyryl-aminoacetyl, (α-Amino-δ-guanidinovaleryl)-aminoacetyl, oder (α-Benzyloxycarbonylamino-δ-guanidinovaleryl)-aminoacetyl, Hydroxy-carboxy-niederalkanoyl, z.B. α-Hydroxy-ß-carboxypropionyl, Hydroxy-niederalkoxycarbonyl-niederalkanoyl, z.B. α-Hydroxy-ß-äthoxy- oder -methoxycarbonylpropionyl, Dihydroxy-carboxy-niederalkanoyl, z.B. α,ß-Dihydroxy-ß-carboxypro-pionyl, Dihydroxy-niederalkoxycarbonyl-niederalkanoyl, z.B. α,ß-Dihydroxy-ß-äthoxy- oder -methoxycarbonylpropionyl, verestertes Dihydroxy-niederalkoxycarbonyl-niederalkanoyl, z.B. α,ß-Diacetoxy-ß-methoxycarbonylpropionyl, Hydroxy-amino-niederalkanoyl, z.B. ß-Hydroxy-α-aminopropionyl oder 3-Hydroxy-4-amino-6-methylheptanoyl, Niederalkoxycarbonyl-acylamino-niederalkanoyl, z.B. α-Methoxy- oder Aethoxycarbonyl-α-benzyloxycarbonylaminoacetyl, Hydroxycyclohexyl-carbonyl, z.B. 3,4,5-Trihydroxycyclohexylcarbonyl, Hydroxycyclohexenylcarbonyl, z.B. 3,4,5-Trihydroxycyclohex-1-enylcarbonyl, 2-(o,o-Dichloroanilino)-phenylacetyl, 2-(o,o-Dichloro-N-benzyl-anilino)-phenylacetyl, Hydroxypyrrolidinylcarbonyl, z.B. 3-oder 4-Hydroxypyrrolidinyl-2-carbonyl, Hydroxy-1-acylpyrrolidinylcarbonyl, z.B. 3- oder 4-Hydroxy-1-benzyloxycarbonylpyrrolidinyl-2-carbonyl, Oxopyrrolidinylcarbonyl, z.B. 5-Oxopyrrolidinyl-2-carbonyl, Niederalkoxycarbonyl, z.B. tert-Butoxycarbonyl, Phenylmethoxycarbonyl mit ein oder zwei Phenylresten, die gegebenenfalls durch Niederalkyl, z.B. Methyl, Niederalkoxy, z.B. Methoxy und/oder Nitro substituiert sind, z.B. Benzyloxycarbonyl, 4-Nitrobenzyloxycarbonyl, Diphenylmethoxycarbonyl oder Di-(4-methoxyphenyl)-methoxy-carbonyl,

$X_1$ und $X_2$ zusammen -Phe-His-, -Phe-Phe-, -Phe-Leu-, -Arg-His- oder -Arg-Phe-, welche N-terminal mit $R_1$ und C-terminal mit der Gruppe $-NR_2-$ verbunden sind, $R_2$ Wasserstoff, $R_3$ Isopropyl oder Isobutyl, $R_4$ Hydroxy, $R_5$ Isopropyl, n-Octyl, Cyclohexyl oder Phenyl,

und $R_6$ Amino, Alkylamino, z.B. Methyl-, Aethyl-, n-Propyl-, n-Butyl-, Isobutyl-, tert-Butyl-, n-Pentyl-, n-Hexyl-, n-Octyl- oder n-Decylamino, Diniederalkylamino, z.B. Dimethylamino, Hydroxyniederalkylamino, z.B. 2-Hydroxyäthylamino oder Tris(hydroxymethyl)-methylamino, Niederalkoxyniederalkylamino, z.B. 2-Methoxyäthylamino, substituiertes Phenoxyniederalkylamino, z.B. 2-(3-Carbamoyl-4-hydroxyphenoxy)-äthylamino, substituiertes Phenoxy-hydroxy-niederalkylamino, z.B. 3-(3-Carbamoylphenoxy)-2-hydroxypropylamino, Carboxyalkylamino, z.B. 4-Carboxy-n-butylamino, 5-Carboxy-n-pentylamino, 6-Carboxy-n-hexylamino, 7-Carboxy-n-heptylamino, 8-Carboxy-n-octylamino oder 1-Carboxy-prop-2-ylamino, Niederalkoxycarbonylalkylamino, z.B. 4-tert-Butoxycarbonyl-n-butylamino, 7-tert-Butoxycarbonyl-n-heptylamino oder 8-tert-Butoxycarbonyl-n-octylamino, physiologisch spaltbares verestertes Carboxyalkylamino, z.B. 4-Pivaloyloxymethoxycarbonyl-n-butylamino, 7-(1-Aethoxy-carbonyloxyäthoxycarbonyl)-n-heptylamino oder 7-Pivaloyloxymethoxycarbonyl-n-heptylamino, Carbamoylniederalkylamino, z.B. Carbamoylmethylamino oder Dicarbamoyl-methylamino, Hydroxyniederalkylcarbamoylniederalkylamino, z.B. 4-(Tris[hydroxymethyl]-methyl)-carbamoyl-n-butylamino, Niederalkoxycarbonyl-hydroxy-niederalkoxycarbamoyl-niederalkylamino, z.B. α-Carbamoyl-α-(2-hydroxy-1-isobutyl-3-methoxycarbonylpropyl)-carbamoyl-methylamino, Aminoalkylamino, z.B. 4-Amino-n-butylamino, 5-Amino-n-pentylamino, 6-Amino-n-hexylamino, 7-Amino-n-heptylamino oder 8-Amino-n-octylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, über ein Stickstoff-Atom gebundenes fünf- oder sechsgliedriges Heterocyclylniederalkylamino, z.B. 3-(1-Morpholinyl)-propylamino oder 3-(2-Oxo-1-pyrrolidinyl)-propylamino, Acylaminoniederalkylamino, z.B. Niederalkanoylaminoniederalkylamino, wie 4-Pivaloylamino-n-butylamino, oder Niederalkoxycarbonylaminoniederalkylamino, wie 4-tert-Butoxycarbonylamino-n-butylamino, 5-tert-Butoxycarbonylamino-n-pentylamino, 6-tert-Butoxycarbonylamino-n-hexylamino oder 8-tert-Butoxycarbonylamino-n-octylamino, Benzylamino, Carboxybenzylamino, z.B. 3-Carboxybenzylamino, sowie Niederalkoxycarbonylbenzylamino, z.B. 3-tert-Butoxycarbonylbenzylamino, Niederalkoxy, z.B. Methoxy, Aethoxy,

n-Propoxy, Isopropoxy, Butoxy, Isobutoxy, tert-Butoxy oder tert-Pentoxy, oder physiologisch spaltbares Alkoxy, z.B. Pivaloyloxymethoxy, ferner -Ala- oder -Ile-, C-terminal substituiert durch Amino, Alkylamino, z.B. Methyl-, Aethyl-, n-Propyl-, n-Butyl- oder n-Octylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino oder 1-Carboxy-prop-2-ylamino, Carbamoylniederalkylamino, z.B. Carbamoylmethylamino oder Dicarbamoyl-methylamino, Hydroxyniederalkylcarbamoylniederalkylamino, z.B. 2-Hydroxyäthylcarbamoylmethylamino oder Di-(2-hydroxyäthylcarbamoyl)-methylamino, Aminoalkylamino, z.B. 4-Amino-n-butylamino, 5-Amino-n-pentylamino oder 8-Amino-n-octylamino, Hydroxyniederalkylaminoniederalkylamino, z.B. 2-(2-Hydroxyäthylamino)-äthylamino, 4-Morpholinylniederalkylamino, z.B. 2-(4-Morpholinyl)-äthylamino, Carboxybenzylamino, z.B. 3-Carboxybenzylamino, Carbamoylbenzylamino, z.B. 3-Carbamoylbenzylamino, Thiazolylamino, z.B. 4- oder 5-Carbamoylmethyl-2-thiazolylamino, Pyridylniederalkylamino, z.B. 2-, 3- oder 4-Pyridylmethylamino, 2-(2-, 3- oder 4-Pyridyl)-äthylamino oder 3-(2-, 3- oder 4-Pyridyl)-propylamino, Imidazolylniederalkylamino, z.B. 4-Imidazolylmethylamino, oder 2-(4-Imidazolyl)-äthylamino, Indolylniederalkylamino, z.B. 3-Indolylmethylamino oder 2-(3-Indolyl)-äthylamino, Niederalkoxy, z.B. Methoxy, Aethoxy oder tert-Butoxy, oder physiologisch spaltbares substituiertes Alkoxy, z.B. Pivaloyloxymethoxy, ferner -Ile-His-, -Ile-Sta- oder -Ala-Sta-, C-terminal substituiert durch Amino, Niederalkylamino, z.B. Methyl-, Aethyl-, n-Propyl- oder n-Butylamino, Diniederalkylamino, z.B. Dimethylamino, Hydroxyniederalkylamino, z.B. 2-Hydroxyäthylamino oder Tris-(hydroxymethyl)-methylamino, ferner Niederalkoxy, z.B. Methoxy, Aethoxy oder tert-Butoxy, oder physiologisch spaltbares substituiertes Alkoxy, z.B. Pivaloyloxymethoxy, darstellt, sowie pharmazeutisch annehmbare Salze von diesen Verbindungen mit salzbildenden Gruppen.

Die Erfindung betrifft in erster Linie Verbindungen der Formel I, worin $R_I$ Wasserstoff, Niederalkanoyl, z.B. Formyl, Acetyl, Propionyl oder Pivaloyl, Aminoalkanoyl, z.B. Aminoacetyl, γ-Aminobutyryl oder

8-Aminooctanoyl, Niederalkoxycarbonylaminoalkanoyl, z.B. tert-Butoxycarbonylaminoacetyl, γ-tert-Butoxycarbonylaminobutyryl oder 8-tert-Butoxycarbonylaminooctanoyl, Benzyloxycarbonylaminoniederalkanoyl, z.B. γ-Benzyloxycarbonylaminobutyryl oder 8-Benzyloxycarbonylaminooctanoyl, Amino- oder Acylamino-niederalkanoylaminoacetyl, z.B. γ-Aminobutyrylaminoacetyl, γ-tert-Butoxycarbonylaminobutyrylaminoacetyl oder γ-Benzyloxycarbonylaminobutyrylaminoacetyl, Dihydroxy-carboxy-niederalkanoyl, z.B. α,ß-Dihydroxy-ß-carboxypropionyl, Dihydroxyniederalkoxycarbonyl-niederalkanoyl, z.B. α,ß-Dihydroxy-ß-methoxycarbonylpropionyl, verestertes Dihydoxyniederalkoxycarbonyl-niederalkanoyl, z.B. α,ß-Diacetoxy-ß-methoxycarbonylpropionyl, Hydroxycyclohexenylcarbonyl, z.B. 3,4,5-Trihydroxycyclohex-1-enylcarbonyl, 2-(o,o-Dichloroanilino)-phenylacetyl, 2-(o,o-Dichloro-N-benzylanilino)-phenylacetyl, Hydroxypyrrolidinylcarbonyl, z.B. 3-oder 4-Hydroxypyrrolidinyl-2-carbonyl, Hydroxy-1-acylpyrrolidinylcarbonyl, z.B. 3- oder 4-Hydroxy-1-benzyloxycarbonylpyrrolidinyl-2-carbonyl, 5-Oxopyrrolidinyl-2-carbonyl, Niederalkoxycarbonyl, z.B. tert-Butoxycarbonyl, Phenylmethoxycarbonyl mit ein oder zwei Phenylresten, die gegebenenfalls durch Niederalkyl, z.B. Methyl, Niederalkoxy, z.B. Methoxy und/oder Nitro substituiert sind, z.B. Benzyloxycarbonyl, 4-Nitrobenzyloxycarbonyl, Diphenylmethoxycarbonyl oder Di-(4-methoxyphenyl)-methoxycarbonyl,

$X_1$ und $X_2$ zusammen -Phe-His- oder -Phe-Phe-, welche N-terminal mit $R_1$ und C-terminal mit der Gruppe $-NR_2-$ verbunden sind, $R_2$ Wasserstoff, $R_3$ Isobutyl, $R_4$ Hydroxy, $R_5$ Isopropyl, n-Octyl, Cyclohexyl oder Phenyl,

und $R_6$ Amino, Niederalkylamino, z.B. Methyl-, Aethyl- oder n-Butylamino, Diniederalkylamino, z.B. Dimethylamino, Hydroxyniederalkylamino, z.B. 2-Hydroxyäthylamino oder Tris-(hydroxymethyl)-methylamino, Carboxyalkylamino, z.B. 4-Carboxy-n-butylamino, 7-Carboxy-n-heptylamino oder 8-Carboxy-n-octylamino, Niederalkoxycarbonylalkylamino, z.B. 4-tert-Butoxycarbonyl-n-butylamino oder 7-tert-Butoxycarbonyl-n-heptylamino, physiologisch spaltbares verestertes Carboxyalkylamino, z.B. 4-Pivaloyloxymethoxycarbonyl-n-butylamino

oder 7-Pivaloyloxymethoxycarbonyl-n-heptylamino, Aminoalkylamino, z.B. 4-Amino-n-butylamino oder 8-Amino-n-butylamino, Hydroxyniederalkylaminoniederalkylamino, z.B. 2-(2-Hydroxyäthylamino)-äthylamino, Diniederalkylaminoniederalkylamino, z.B. 2-Dimethylaminoäthylamino, 4-Morpholinylniederalkylamino, z.B. 3-(4-Morpholinyl)-propylamino, 2-Oxo-1-pyrrolidinylniederalkylamino, z.B. 3-(2-Oxo-1-pyrrolidinyl)-propylamino, ferner -Ala- oder -Ile-, C-terminal substituiert durch Amino, Niederalkylamino, z.B. Methylamino, Aethylamino oder n-Butylamino, Diniederalkylamino, z.B. Dimethylamino, Hydroxyniederalkylamino, z.B. 2-Hydroxyäthylamino, Dicarbamoylmethylamino, Hydroxyniederalkylaminoniederalkylamino, z.B. 2-(2-Hydroxyäthylamino)-äthylamino, 4-Morpholinylniederalkylamino, z.B. 2-(4-Morpholinyl)-äthylamino, 3-Carbamoylbenzylamino, 4-Carbamoylmethyl-2-thiazolylamino, Pyridylniederalkylamino, z.B. 2-Pyridylmethylamino, 2-(2-Pyridyl)-äthylamino oder 3-(2-Pyridyl)-propylamino oder Imidazolylniederalkylamino, z.B. 2-(4-Imidazolyl)-äthylamino oder 2-(3-Indolyl)-äthylamino, ferner -Ile-His- oder -Ile-Sta-, C-terminal substituiert durch Amino, oder -Ala-Sta-, C-terminal substituiert durch Niederalkoxy, z.B. Methoxy, darstellt, sowie pharmazeutisch annehmbare Salze von diesen Verbindungen mit salzbildenden Gruppen.

Die Erfindung betrifft vor allem Verbindungen der Formel I, worin $R_1$ Wasserstoff, Acetyl, Pivaloyl, γ-Aminobutyryl, 8-Aminooctanoyl, γ-tert-Butoxycarbonylaminobutyryl, 8-tert-Butoxycarbonylaminooctanoyl, γ-Benzyloxycarbonylaminobutyryl, 8-Benzyloxycarbonylaminooctanoyl, γ-Aminobutyrylaminoacetyl, γ-tert-Butoxycarbonylaminobutyrylaminoacetyl, γ-Benzyloxycarbonylaminobutyrylaminoacetyl, α,ß-Dihydoxy-ß-carboxypropionyl, α,ß-Dihydroxy-ß-methoxycarbonylpropionyl, 4-Hydroxypyrrolidinyl-2-carbonyl, 1-Benzyloxycarbonyl-4-hydroxypyrrolidinyl-2-carbonyl, 5-Oxopyrrolidinyl-2-carbonyl, tert-Butoxycarbonyl oder Benzyloxycarbonyl,

$X_1$ und $X_2$ zusammen -Phe-His-, N-terminal mit $R_1$ und C-terminal mit der Gruppe $-NR_2-$ verbunden, $R_2$ Wasserstoff, $R_3$ Isobutyl, $R_4$ Hydroxy, $R_5$ Isopropyl,

und $R_6$ Amino, Niederalkylamino, z.B. Methylamino, Aethylamino oder n-Butylamino, Dimethylamino, 4-Carboxy-n-butylamino, 7-Carboxy-n-heptylamino, 4-tert-Butoxycarbonyl-n-butylamino, 7-tert-Butoxycarbonyl-n-heptylamino, 7-Pivaloyloxymethoxycarbonyl-n-heptylamino, 3-(2-Oxo-1-pyrrolidinyl)-propylamino, -Ala-, C-terminal substituiert durch 2-(4-Imidazolyl)-äthylamino oder 2-(2-Pyridyl)-äthylamino, -Ile-, C-terminal substituiert durch Dicarbamoylmethylamino, 3-Carbamoylbenzylamino, 4-Carbamoylmethyl-2-thiazolylamino, 2-(2-Pyridyl)-äthylamino, 2-Pyridylmethylamino oder 2-(3-Indolyl)-äthylamino, -Ile-His- oder -Ile-Sta-, C-terminal substituiert durch Amino, oder -Ala-Sta-, C-terminal substituiert durch Methoxy, darstellt, sowie pharmazeutisch annehmbare Salze von diesen Verbindungen mit salzbildenden Gruppen.

Die Erfindung betrifft zu allererst die in den Beispielen erwähnten Verbindungen und deren pharmazeutisch annehmbare Salze.

Die Erfindung betrifft vorallem folgende in den Beispielen beschriebenen Verbindungen:

Benzyloxycarbonyl-Phe-His-[5(S)-amino-4(S)-hydroxy-2(S)-isopropyl-7-methyloctanoyl]-Ile-His-$NH_2$,

Benzyloxycarbonyl-Phe-Phe-[5(S)-amino-4(S)-hydroxy-2(S)-isopropyl-7-methyloctanoyl]-Ile-His-$NH_2$,

H-Phe-His-[5(S)-amino-4(S)-hydroxy-2(S)-isopropyl-7-methyloctanoyl]-Ile-His-$NH_2$,

[(2S,4R)-4-Hydroxypyrrolidinyl-2-carbonyl]-Phe-His-[5(S)-amino-4(S)-hydroxy-2(S)-isopropyl-7-methyloctanoyl]-Ile-His-$NH_2$,

[(2S)-5-Oxopyrrolidinyl-2-carbonyl]-Phe-His-[5(S)-amino-4(S)-hydroxy-2(S)-isopropyl-7-methyloctanoyl]-Ile-His-$NH_2$,

tert-Butoxycarbonyl-Phe-His-[5(S)-amino-4(S)-hydroxy-2(S)-isopropyl-7-methyloctanoyl]-Ala-Sta-$OCH_3$,

Benzyloxycarbonyl-Phe-His-[5(S)-amino-4(S)-hydroxy-2(S)-isopropyl-7-methyloctanoyl]-Ala-Sta-$OCH_3$,

γ-Benzyloxycarbonylaminobutyrylaminoacetyl-Phe-His-[5(S)-amino-4(S)-hydroxy-2(S)-isopropyl-7-methyloctanoyl]-Ile-His-$NH_2$,

γ-Aminobutyrylaminoacetyl-Phe-His-[5(S)-amino-4(S)-hydroxy-2(S)-isopropyl-7-methyloctanoyl]-Ile-His-$NH_2$,

8-Aminooctanoyl-Phe-His-[5(S)-amino-4(S)-hydroxy-(2S)-isopropyl-7-methyloctanoyl]-Ile-His-$NH_2$ und

Benzyloxycarbonyl-Phe-His-[5(S)-amino-4(S)-hydroxy-(2S)-isopropyl-7-methyloctanoyl]-Ile-dicarbamoylmethylamid,

Benzyloxycarbonyl-Phe-His-[5(S)-amino-4(S)-hydroxy-2(S)-isopropyl-7-methyloctanoyl]-2-(3-carbamoyl-4-hydroxy-phenyloxy)-äthylamid,

α-Benzyloxycarbonylamino-α-methoxycarboyl-acetyl-Phe-His-[5(S)-amino-4(S)-hydroxy-2(S)-isopropyl-7-methyloctanoyl]-3-(2-pyrrolidinon-1-yl)-propylamid,

Benzyloxycarbonyl-Phe-His-[5(S)-amino-4(S)-hydroxy-2(S)-isopropyl-7-methyloctanoyl]-methylamid,

Benzyloxycarbonyl-Phe-His-[5(S)-amino-4(S)-hydroxy-2(S)-isopropyl-7-methyloctanoyl]-4-[tris-(hydroxymethyl)-methylaminocarbonyl]-butylamid,

Benzyloxycarbonyl-Glu(O-tert-butyl)-his-[5(S)-amino-4(S)-hydroxy-2(S)-isopropyl-7-methyloctanoyl]-Ile-His-$NH_2$,

Benzyloxycarbonyl-Phe-His-[5(S)-amino-4(S)-hydroxy-2(S)-isopropyl-7-methyloctanoyl]-carbamoyl-(3-methoxycarbonyl-2-hydroxy-1-isobutyl-propylaminocarbonyl)-methylamid.

Verfahren:

Die erfindungsgemässen Verbindungen der Formel I und Salze von solchen Verbindungen mit mindestens einer salzbildenden Gruppe werden nach an sich bekannten Verfahren erhalten, z.B. indem man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen

Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, die Ketogruppe in einer Verbindung der Formel

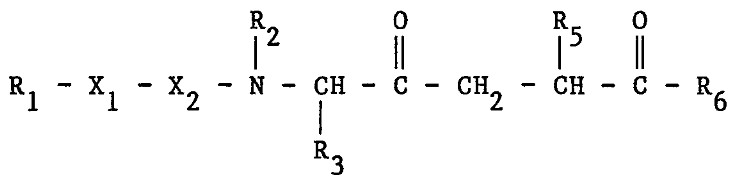 (II),

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) zur Herstellung von Verbindungen der Formel I, worin $R_4$ Hydroxy bedeutet, eine Aldehyd-Verbindung der Formel

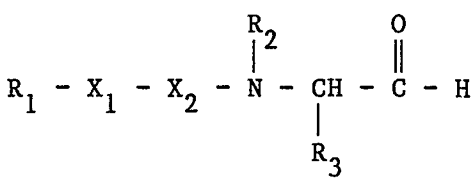 (III),

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

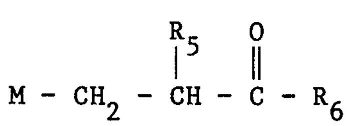 (IV),

worin die Substituenten die genannten Bedeutungen haben und M ein Metallradikal bedeutet, umsetzt und das gebildete Additionsprodukt hydrolysiert, oder

d) in einer Verbindung der Formel

$$R_1 - X_1 - X_2 - \overset{\overset{R_2}{|}}{N} - \underset{\underset{R_3}{|}}{CH} - \overset{\overset{X}{|}}{CH} - CH_2 - \overset{\overset{R_5}{|}}{CH} - \overset{\overset{O}{\|}}{C} - R_6 \quad (V),$$

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder

e) zur Herstellung einer Verbindung der Formel I, worin $R_6$ unsubstituiertes oder substituiertes Amino bedeutet, in einer Verbindung der Formel

$$R_1 - X_1 - X_2 - \overset{\overset{R_2}{|}}{N} - \underset{\underset{R_3}{|}}{CH} - \overset{\overset{R_4}{|}}{CH} - CH_2 - \overset{\overset{R_5}{|}}{CH} - CN \quad (VI),$$

worin die Substituenten die genannten Bedeutungen haben, bzw. in einer Verbindung der Formel

$$R_1 - X_1 - X_2 - \overset{\overset{R_2}{|}}{N} - \underset{\underset{R_3}{|}}{CH} - \overset{\overset{R_4}{|}}{CH} - CH_2 - \overset{\overset{R_5}{|}}{CH} - \overset{\overset{O}{\|}}{C} - R_6' - CN \quad (VIa),$$

worin die Gruppe $R_6'$-CN den Rest einer in der Natur vorkommenden α-Aminosäure, eines Di- oder eines Tripeptids, worin jeweils die terminale -COOH-Gruppe durch -CN ersetzt ist, bedeutet, die übrigen Substituenten die genannten Bedeutungen haben und vorhandene funktionelle Gruppen gegebenenfalls in geschützter Form vorliegen, die Cyanogruppe in eine unsubstituierte oder N-substituierte Carboxamidogruppe überführt, oder

f) zur Herstellung einer Verbindung der Formel I, worin $R_4$ freies Hydroxy bedeutet, ein Epoxid der Formel

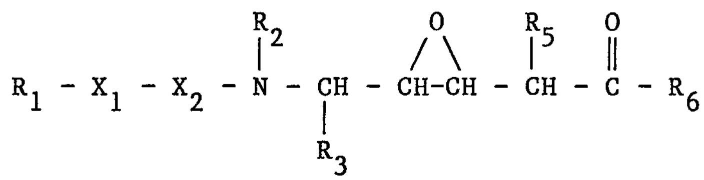

(VII),

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert,
und gewünschtenfalls in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

Die Erfindung betrifft auch die nach irgendeinem der oben genannten Verfahren erhältlichen anderen Verbindungen als Verbindungen der Formel I (Nebenprodukt), sowie Verbindungen der Formel I und ihre Salze, welche nach einem anderen Verfahren als einem der weiter vorn genannten hergestellt werden.

Verfahren a) (Herstellung einer Amidbindung):

Fragmente einer Verbindung der Formel I mit einer endständigen Carboxygruppe, welche mit einem zur Verbindung der Formel I komplementären Fragment unter Bildung einer Amidbindung kondensiert werden können, sind z.B. Verbindungen der Formeln: $R_1$-OH, $R_1$-$X_1$-OH, $R_1$-$X_1$-$X_2$-OH,

$$R_1 - X_1 - X_2 - \overset{R_2}{\overset{|}{N}} - \underset{\underset{R_3}{|}}{CH} - \overset{R_4}{\overset{|}{CH}} - CH_2 - \overset{R_5}{\overset{|}{CH}} - \overset{O}{\overset{\|}{C}} - OH ,$$

sowie gegebenenfalls

$$R_1 - X_1 - X_2 - \overset{R_2}{\overset{|}{N}} - \underset{\underset{R_3}{|}}{CH} - \overset{R_4}{\overset{|}{CH}} - CH_2 - \overset{R_5}{\overset{|}{CH}} - \overset{O}{\overset{\|}{C}} - R_6' - COOH,$$

worin die Gruppe $R_6'$-COOH den Rest einer α-Aminosäure, eines Dipeptids oder eines Tripeptids darstellt, wie er für $R_6$ oben definiert ist, oder die von diesen Verbindungen abgeleiteten aktivierten Ester oder reaktionsfähigen Anhydride, ferner reaktionsfähige cyclische Amide. Die reaktionsfähige Säurederivate können auch in situ gebildet werden.

Aktivierte Ester sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode), oder 1-Niederalkoxyvinylester (erhältlich z.B. durch Behandeln der entsprechenden Säure mit einem Niederalkoxyacetylen; Aethoxyacetylen-Methode), oder Ester vom Amidinotyp, wie

N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode), oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyamino- bzw. N-Hydroxyamido-Verbindung, z.B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benzotriazin-4-on, z.B. nach der Anhydrid-oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern, oder mit einem 1-Niederalkoxycarbonyl-2-niederalkoxy-1,2-dihydrochinolin, z.B. 1-Niederalkoxycarbonyl-2-äthoxy-1,2-dihydrochinolin; Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diäthylaminopropin; Methode der symmetrischen Anhyride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N'-Carbonyldiimidazol; Imidazol-Methode), oder Pyrazol, z.B. 3,5-Dimethylpyrazol (erhältlich z.B. über das Säurehydrazid durch Behandeln mit Acetylaceton; Pyrazolid-Methode).

Zur Verbindung der Formel I komplementäre Fragmente mit einer freien Aminogruppe sind z.B. je nach Bedeutung von $R_6$ Ammoniak, ein primäres oder sekundäres Amin, eine α-Aminosäure, ein Dipeptid oder ein Tripeptid mit einer freien α-Aminogruppe, ferner Verbindungen der Formeln:

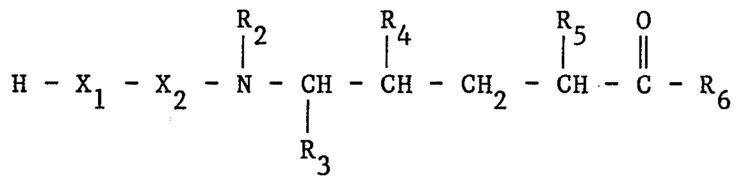 ,

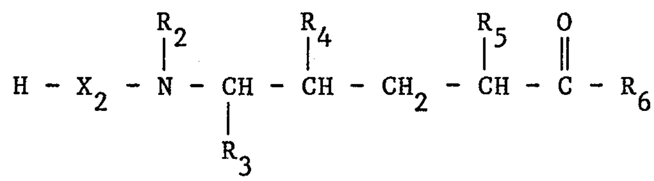

oder 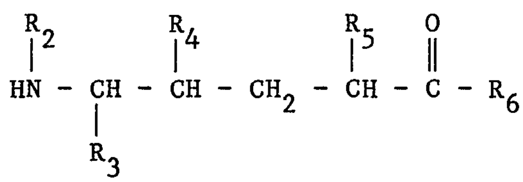

Die an der Reaktion teilnehmende Aminogruppe in einem zu einer Verbindung der Formel I komplementären Fragment liegt bevorzugt in freier Form vor, insbesondere wenn die damit reagierende Carboxygruppe in reaktionsfähiger Form vorliegt, sie kann aber auch selbst derivatisiert sein, z.B. durch Reaktion mit einem Phosphit, wie Diäthylchlorphosphit, 1,2-Phenylenchlorphosphit, Aethyldichlorphosphit, Aethylenchlorphosphit oder Tetraäthylpyrophosphit. Ein Derivat eines solchen komplementierenden Bruchstücks mit einer Aminogruppe ist z.B. auch ein Carbaminsäurehalogenid oder ein Isocyanat, wobei die an der Reaktion teilnehmende Aminogruppe durch Halogencarbonyl, z.B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Ist das komplementäre Fragment mit einer Aminogruppe Ammoniak selbst oder ein durch Niederalkyl oder Arylniederalkyl mono- oder disubstituiertes Amin, so stellt auch eine entsprechende Harnstoff-

Verbindung ein reaktionsfähiges Derivat dar. Beispielsweise erhält man beim Erhitzen äquimolarer Mengen dieser Harnstoffverbindung und der Komponente mit freier Carboxygruppe entsprechende Verbindungen der Formel I mit zum Teil guter Ausbeute.

Ist das komplementäre Fragment Dimethylamin, so ist auch Dimethylformamid ein reaktionsfähiges Derivat.

Funktionelle Gruppen in Ausgangsmaterialien, deren Umsetzung vermieden werden soll, insbesondere Carboxy-, Amino-, Hydroxy- und Mercaptogruppen, können durch geeignete Schutzgruppen (conventional protecting groups) geschützt sein, die üblicherweise bei der Synthese von Peptid-Verbindungen, aber auch von Cephalosporinen und Penicillinen verwendet werden. Diese Schutzgruppen können bereits in den Vorstufen vorhanden sein und sollen die betreffenden funktionellen Gruppen gegen unerwünschte Nebenreaktionen wie Acylierungen, Verätherungen, Veresterungen, Oxydationen, Solvolyse etc. schützen. Schutzgruppen können aber auch in den Endstoffen vorhanden sein. Verbindungen der Formel I mit geschützten funktionellen Gruppen können eine höhere metabolische Stabilität aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen, sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy. Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxyäthoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, sowie 2-Triniederalkylsilylniederalkoxycarbonyl, z.B. 2-Trimethylsilyläthoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und

die Aminogruppe oder die Carboxygruppe eines zweiten Moleküls der Formel I substituiert sein. Verbindungen mit solchen Schutzgruppen lassen sich z.B. mit Dimethylchlorsilan als Silylierungsmittel herstellen.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl und Diphenylmethoxycarbonyl.

Eine Aminogruppe kann z.B. in Form einer Acylamino-, Arylmethylamino-, verätherten Mercaptoamino- oder Silylaminogruppe oder als Azidogruppe geschützt sein.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure oder gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2,2,2-Trichloräthoxycarbonyl,

2-Bromäthoxycarbonyl oder 2-Jodäthoxycarbonyl, 2-Triniederalkylsilylniederalkoxycarbonyl, z.B. 2-Trimethlysilyläthoxycarbonyl, oder 2-Triarylsilylniederalkoxycarbonyl, z.B. 2-Triphenylsilyläthoxycarbonyl.

Eine Arylmethylaminogruppe ist z.B. Mono-, Di- oder insbesondere Triphenylmethylamino, z.B. Benzyl-, Diphenylmethyl- oder Tritylamino.

In einer verätherten Mercaptoaminogruppe ist die verätherte Mercaptogruppe in erster Linie substituiertes Arylthio, z.B. 4-Nitrophenylthio.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. mit Dimethylchlorsilan als Silylierungsmittel herstellen.

Bevorzugte Aminoschutzgruppen sind Acylreste von Kohlensäurehalbestern, insbesondere tert-Butoxycarbonyl, gegebenenfalls substituiertes Benzyloxycarbonyl, z.B. 4-Nitrobenzyloxycarbonyl, Diphenylmethoxycarbonyl, 2-Halogen-niederalkoxycarbonyl, z.B. 2,2,2-Trichloräthoxycarbonyl, ferner Trityl oder Formyl.

Eine Hydroxygruppe kann beispielsweise durch eine Acylgruppe, z.B. durch Halogen, z.B. Chlor, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichloräthoxycarbonyl, 4-Nitrobenzyloxycarbonyl, Diphenylmethoxycarbonyl oder Trityl. Eine Hydroxygruppe kann ferner durch Triniederalkylsilyl, z.B. Trimethylsilyl oder Dimethyl-tert-butylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl, oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Zwei benachbarte Hydroxylgruppen können beispielsweise durch eine vorzugsweise substituierte Methylengruppe geschützt sein, z.B. durch Niederalkyliden, z.B. Isopropyliden, Cycloalkyliden, z.B. Cyclohexyliden, oder Benzyliden.

Eine Mercaptogruppe, wie z.B. in Cystein, kann insbesondere durch S-Alkylierung mit gegebenenfalls substituierten Alkylresten, Thioacetalbildung, S-Acylierung oder durch die Bildung asymmetrischer Disulfid-Gruppierungen geschützt sein. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls am Phenylrest, z.B. durch Methoxy, substituiertes Diphenylmethyl, wie 4,4'-Dimethoxydiphenylmethyl, Triphenylmethyl, Trimethylsilyl, Benzylthiomethyl, Tetrahydropyranyl, Acylaminomethyl, Benzoyl, Benzyloxycarbonyl oder Niederalkylaminocarbonyl, wie Aethylaminocarbonyl.

Die Kondensation zur Herstellung der Amidbindung kann in an sich bekannter Weise durchgeführt werden, beispielsweise wie in Standardwerken, wie "Houben-Weyl, Methoden der organischen Chemie", 4. Auflage, Band 15/II, Georg Thieme Verlag, Stuttgart 1974, "The

Peptides" (Herausg. E. Gross und J. Meienhofer), Band 1 und 2, Academic Press, London und New York, 1979/1980, oder M. Bodanszky, "Priciples of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diäthyl-, Dipropyl-, N-Aethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Aethyl-5-phenyl-1,2-oxazolium-3'-sulfonat und 2-tert-Butyl-5-methylisoxazoliumperchlorat, oder eine geeignete Acylaminoverbindung, z.B. 2-Aethoxy-1-äthoxycarbonyl-1,2-dihydrochinolin, ferner aktivierte Phosphate, z.B. Diphenylphosphorylazid, Diäthylphosphorylcyanid oder Phenyl-N-phenylphosphoramidochloridat.

Gewünschtenfalls wird eine organische Base zugegeben, z.B. ein Triniederalkylamin mit voluminösen Resten, z.B. Aethyldiisopropylamin, oder eine heterocyclischen Base, z.B. Pyridin, 4-Dimethylaminopyridin oder bevorzugt N-Methylmorpholin.

Die Kondensation von Säureanhydriden mit Aminen kann z.B. in Gegenwart von Alkalimetallcarbonaten, z.B. Alkalimetallcarbonaten oder -hydrogencarbonaten, wie Natrium- oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril,

z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden. So kann man z.B. N,N'-disubstituierte Amidinoester in situ bilden, indem man das Gemisch des Fragments mit freier Carboxygruppe und des komplementierenden Fragments mit einer Aminogruppe in Gegenwart eines geeigneten disubstituierten Carbodiimids, z.B. Dicyclohexylcarbodiimid, umsetzt. Ferner kann man Amino- oder Amido-ester von solchen Säuren in Gegenwart der zu acylierenden Aminokomponente bilden, indem man das Gemisch der entsprechenden Säure- und Amino-Ausgangsstoffe in Gegenwart eines disubstituierten Carbodiimids, z.B. Dicyclohexylcarbodiimid, und eines N-Hydroxylamins oder N-Hydroxyamids, z.B. N-Hydroxybenztriazol, N-Hydroxysuccinimid oder N-Hydroxy-5-norbornen-2,3-dicarbonsäureimid, gegebenenfalls in Anwesenheit einer geeigneten Base, z.B. 4-Dimethylaminopyridin, N-Methylmorpholin oder Aethyldiisopropylamin, umsetzt.

Verfahren b) (Reduktion einer Ketogruppe):

In einem Ausgangsmaterial der Formel II sind funktionelle Gruppen mit Ausnahme der zu reduzierenden Ketogruppe gegebenenfalls durch eine der unter Verfahren a) genannten Schutzgruppen geschützt.

Zur Reduktion der Ketogruppe in einer Verbindung der Formel II eignen sich solche Reduktionsmittel, die unter den Reaktionsbedingungen des Verfahrens eine isolierte Ketogruppe selektiv oder schneller als die in Verbindungen der Formel I vorhandenen Amidgruppen reduzieren.

In erster Linie zu nennen sind geeignete Borhydride, wie Alkalimetallborhydride, insbesondere Natriumborhydrid, Lithiumborhydrid oder Natriumcyanborhydrid, oder geeignete Aluminiumhydride, wie Alkalimetallniederalkoxyaluminiumhydride mit voluminösen Resten, z.B. Lithium-tris-tert-butoxyaluminiumhydrid.

Die Reduktion kann auch mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, oder nach Meerwein-Ponndorf-Verley mit Hilfe von Aluminiumalkanolaten, bevorzugt Aluminium-2-propanolat oder -äthanolat, durchgeführt werden.

Die Reduktion kann vorzugsweise mit stöchiometrischen Mengen oder, wenn es bei gleichzeitigen Nebenreaktionen, z.B. mit dem Lösungsmittel, die allerdings unerwünscht sind, notwendig ist, einem sinnvoll bemessenen Ueberschuss des Reduktionsmittels in einem inerten Lösungsmittel bei Temperaturen zwischen -80°C und dem Siedepunkt des Lösungsmittels, vorzugsweise zwischen -20°C und +100°C, wenn nötig unter Schutzgas, z.B. Stickstoff oder Argon, durchgeführt werden.

Bei Verwendung von Natriumborhydrid eignen sich polare, protische Lösungsmittel, z.B. Methanol, Aethanol oder Isopropanol; bei Verwendung der anderen Reduktionsmittel die unter Verfahren a) genannten polaren, aprotischen Lösungsmittel, z.B. Tetrahydrofuran.

<u>Verfahren c):</u>
In einem Ausgangsmaterial der Formel III sind funktionelle Gruppen mit Ausnahme der Aldehydgruppe gegebenenfalls durch die unter Verfahren a) genannten Schutzgruppen geschützt. Ebenso sind vorhandene funktionelle Gruppen in einer Verbindung der Formel IV gegebenenfalls geschützt.

In einer Verbindung der Formel IV ist ein Metallradikal -M z.B. -Li oder -MgHal, z.B. -MgCl, -MgBr oder -MgJ.

Die Umsetzung einer Verbindung der Formel III mit einer Verbindung der Formel IV erfolgt in üblicher Weise in einem wasserfreien, inerten, aprotischen Lösungsmittel, z.B. in einem Aether, wie Diäthyläther oder Tetrahydrofuran, oder einem Kohlenwasserstoff, wie Benzol oder Toluol, oder Gemischen davon, gegebenenfalls unter Kühlen, insbesondere nach Beginn der Reaktion, z.B. bis etwa -30°C, oder unter Erwärmen, z.B. bis zur Siedetemperatur des Reaktionsgemisches, gegebenenfalls in einer Inertgas-, z.B. Stickstoffatmosphäre. Eine bevorzugte Ausführungsform des Verfahrens ist die Umsetzung des Aldehyds der Formel III mit einem Ueberschuss der Lithiumverbindung der Formel IV.

Die Hydrolyse des Additionsprodukts erfolgt mit $H^+$-Ionen liefernden Lösungsmitteln, z.B. Wasser (Eis-Wasser-Gemisch) oder verdünnten, wässrigen Säuren, z.B. verdünnten Mineralsäuren, wie verdünnter, wässriger Schwefelsäure, oder verdünnten organischen Säuren, z.B. verdünnter, wässriger Essigsäure.

Die Umsetzung einer Verbindung der Formel III kann auch mit einer in situ hergestellten Verbindung der Formel IV erfolgen, welche man z.B. aus dem entsprechenden Halogenid, z.B. Chlorid, durch Umsetzung mit einem Metallierungsmittel, z.B. Magnesium, Lithium oder tert-Butyllithium, erhält.

Verfahren d) (nukleophile Substitution):

In einem Ausgangsmaterial der Formel V sind funktionelle Gruppen gegebenenfalls durch die unter Verfahren a) genannten Schutzgruppen geschützt.

In einer Verbindung der Formel V ist die nukleofuge Abgangsgruppe X insbesondere mit einer starken anorganischen oder organischen Säure

verestertes Hydroxy, wie mit einer Mineralsäure, z.B. Halogenwasserstoffsäure, wie Chlor-, Brom- oder Jodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom und/oder Nitro substituierten Benzolsulfonsäure, z.B. einer Methansulfon-, Trifluormethansulfon- oder p-Toluolsulfonsäure, oder mit Stickstoffwasserstoffsäure verestertes Hydroxy.

Ein den Substituenten $R_4$ in nukleophiler Form einführendes Reagens ist abhängig von der Bedeutung von $R_4$ eine Hydroxid-haltige Base, z.B. Natrium- oder Kaliumhydroxid ($R_4$ = OH), ein Alkohol, z.B. Methanol oder Aethanol ($R_4$ = veräthertes Hydroxy) oder das Salz einer Carbonsäure, z.B. Silberacetat ($R_4$ = verestertes Hydroxy).

Vorzugsweise werden die Reaktionsbedingungen so gewählt, dass die Reaktion im wesentlichen als nukleophile Substitution zweiter Ordnung ($S_N2$) abläuft. Beispielsweise kann man eine Verbindung der Formel V, worin X für eine Abgangsgruppe mit hoher Polarisierbarkeit der Elektronenhülle, z.B. für Jod, steht, in einem polaren aprotischen Lösungsmittel, z.B. Aceton, Acetonitril, Nitromethan, Dimethylsulfoxid oder Dimethylformamid, mit dem Silbersalz einer Carbonsäure, z.B. Silberacetat, umsetzen. Die Reaktion mit einer Hydroxid-haltigen Base wird vorzugsweise in Wasser, dem gegebenenfalls als Lösungsvermittler ein organisches Lösungsmittel, z.B. Aethanol, Tetrahydrofuran oder Aceton, beigemischt ist, und die Reaktion mit einem Alkohol in einem Ueberschuss dieses Alkohols, gegebenenfalls in Gegenwart eines der obengenannten polaren aprotischen Lösungsmittel, durchgeführt. Die Substitutionsreaktion wird gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre, durchgeführt.

Verfahren e) (Ueberführung einer Cyanogruppe in eine Amidgruppe)

In einem Ausgangsmaterial der Formel VI bzw. VIa sind funktionelle Gruppen durch die unter a) genannten Schutzgruppen gegebenenfalls geschützt.

Die Ueberführung einer Verbindung der Formel VI bzw. VIa in eine Verbindung der Formel I kann durch partielle Hydrolyse, durch eine Ritter-Reaktion oder über Carbonsäureesterimid-Salze erfolgen.

Die Bedingungen der Hydrolyse einer Verbindung der Formel VI bzw. VIa können so gewählt werden, dass die Reaktion auf der Stufe des Amids stehen bleibt. Zu diesem Zweck wird das Nitril der Formel VI bzw. VIa mit Säure hydrolysiert, wobei man je nach thermischer Empfindlichkeit der in einer Verbindung der Formel VI bzw. VIa vorhandenen Substituenten insbesondere zwischen 80%iger Schwefelsäure (unter Erwärmen), Polyphosphorsäure (bei 110-150°), Bromwasserstoff/Eisessig oder Ameisensäure (Raumtemperatur), Chlorwasserstoffgas in ätherischer Lösung gefolgt von Wasser oder wässriger Salzsäure, oder Borhalogeniden, z.B. Bortrifluorid-Essigsäure-Komplex, gefolgt von Wasser, wählen kann. In einigen Fällen gelingt auch die alkalische Hydrolyse, insbesondere nach der Methode von Radziszewski, mit Wasserstoffperoxid in Gegenwart von Alkalimetallhydroxiden bei mässiger Temperatur, z.B. Raumtemperatur.

Mit Hilfe der Ritter-Reaktion gelingt die Herstellung N-substituierter Amide aus Nitrilen der Formel VI bzw. VIa. Hierzu setzt man die Nitrile in Gegenwart einer starken Säure, beispielsweise 85-90%iger Schwefelsäure, oder auch Polyphosphorsäure, Fluorwasserstoff, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Benzylalkohol, meist ohne Lösungsmittel oder beispielsweise in Eisessig.

In einer Variante der Ritter-Reaktion wird ein Nitril der Formel VI oder VIa mit einem Olefin und Quecksilber(II)-nitrat umgesetzt und die Organoquecksilberverbindung anschliessend mit Natriumborhydrid zu einer N-substituierten Verbindung der Formel I reduziert.

Die Carbonsäureesterimide erhält man z.B. durch säurekatalysierte Anlagerung von Alkoholen an die Nitrile, bevorzugt in Gegenwart von Chlorwasserstoff. Aus den so gebildeten Esterimid-Salzen erhält man die Amide durch thermische Umlagerung bei Temperaturen oberhalb von etwa 80°C.

Verfahren f) (Reduktion des Epoxids):

In einem Ausgangsmaterial der Formel VII sind funktionelle Gruppen durch die unter Verfahren a) genannten Schutzgruppen gegebenenfalls geschützt.

Es können solche Reduktionsmittel verwendet werden, die unter den Reaktionsbedingungen des Verfahrens die Epoxygruppe selektiv oder schneller als die vorhandenen Amidgruppen reduzieren und das Epoxid so öffnen, dass ein genügend und möglichst grosser Anteil der Reaktionsprodukte die neugebildete Hydroxygruppe in der der Formel I entsprechenden Position trägt. Beispiele für solche selektive Reduktionsmittel sind Lithiumborhydrid oder Natriumcyanborhydrid/Bortrifluoridätherat. Mit dem letztgenannten Reagens lässt sich die Reaktion z.B. so durchführen, dass man zu 1 Mol der Verbindung der Formel VII und einem Ueberschuss, z.B. 1,4-3 Mol, Natriumcyanborhydrid in Tetrahydrofuran bei erhöhter Temperatur, z.B. unter Rückfluss, eine Lösung von Bortrifluoridätherat, $BF_3 \cdot O(C_2H_5)_2$, in Tetrahydrofuran so zugibt, dass der pH-Wert der Reaktionslösung in der Nähe des Umschlagpunktes des ebenfalls zugegebenen Indikators Bromkresolgrün gehalten wird. Die Reduktion mit Lithiumborhydrid wird vorzugsweise in einem Aether, z.B. Tetrahydrofuran, 1,2-Dimethoxyäthan oder Diäthylenglykoldimethyläther, bei Temperaturen zwischen Raumtemperatur und Rückflusstemperatur durchgeführt.

Nachoperationen:

In einer erhältlichen Verbindung der Formel I, worin $R_1$, $X_1$, $X_2$, $R_2$, $R_3$, $R_4$ und $R_5$ die genannten Bedeutungen haben und $R_6$ den Rest einer Aminosäure, eines Dipeptids oder eines Tripeptids mit einer endständigen Carboxamid-, Carboxy- oder Carbonsäureestergruppe darstellt, kann man die endständige Carboxamidgruppe substituieren, die in freier oder in reaktionsfähiger Form vorliegende Carboxygruppe verestern bzw. eine veresterte Carboxygruppe in eine Carboxamidgruppe überführen.

Die Substitution der terminalen Carboxamidgruppe oder einer anderen Aminogruppe erfolgt z.B. durch Alkylierung.

Geeignete Mittel zur Alkylierung der terminalen Carboxamidgruppe in einer Verbindung der Formel I sind z.B. Diazoverbindungen, z.B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z.B. in Gegenwart eines Edelmetalls in fein verteilter Form, z.B. Kupfer, oder eines Edelmetallsalzes, z.B. Kupfer(I)-chlorid oder Kupfer(II)-sulfat.

Weitere Alkylierungsmittel sind die in der Deutschen Offenlegungsschrift 2 331 133 genannte Alkylierungsmittel, z.B. Alkylhalogenide, Sulfosäureester, Meerweinsalze oder 1-substituierte 3-Aryltriazene. welche man unter den dort genannten Reaktionsbedingungen mit einer Verbindung der Formel I mit endständiger Carboxamidgruppe umsetzen kann.

Zur Veresterung einer endständigen Carboxygruppe in einer Verbindung der Formel I kann man die freie Säure verwenden oder die freie Säure in eines der unter Verfahren a) genannten reaktionsfähigen Derivate überführen und mit einem Alkohol umsetzen, oder man kann die freie

Säure oder ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit dem Halogenid eines Alkohols umsetzen.

Die Veresterung einer endständigen Carboxygruppe kann mit den für die Substitution der Carboxamidgruppe oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

In einer erhältlichen Verbindung der Formel I kann man eine endständige veresterte Carboxygruppe durch Aminolyse mit Ammoniak oder einem primären oder sekundären Amin in eine gegebenenfalls substituierte Carboxamidgruppe überführen.

Die Aminolyse kann nach den im Organikum, letzte Auflage, VEB Deutscher Verlag der Wissenschaften, Berlin (Ost) für solche Umsetzungen genannten Reaktionsbedingungen erfolgen.

In einer erhältlichen Verbindung der Formel I, worin die Substituenten die genannten Bedeutungen haben und mindestens eine freie Hydroxygruppe vorhanden ist und die übrigen funktionellen Gruppen gegebenenfalls in geschützter Form vorliegen, kann man die freie Hydroxygruppe, z.B. die Hydroxygruppe $R_4$, veräthern oder verestern.

Die Verätherung dieser Hydroxygruppe kann mit den oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Die Veresterung der freien Hydroxygruppe kann mit den üblichen Acylierungsmitteln und den üblichen im Organikum angegebenen Reaktionsbedingungen erfolgen, z.B. mit Essigsäureanhydrid.

Die genannten Alkylierungsreaktionen, Verätherungen, Veresterungen etc. können statt im Endstoff auch in einem Ausgangsmaterial entsprechend durchgeführt werden.

In einer erhältlichen Verbindung der Formel I, worin eine oder mehrere funktionelle Gruppen geschützt sind, können diese Gruppen, z.B. Carboxy-, Amino-, Hydroxy- und/oder Mercaptogruppen, in an sich bekannter Weise, mittels Solvolyse, insbesondere Hydrolyse, gegebenenfalls enzymatische Hydrolyse, Alkoholyse oder Acidolyse, oder mittels Reduktion, insbesondere Hydrogenolyse, oder chemische Reduktion, gegebenenfalls stufenweise oder gleichzeitig, freigesetzt werden. Die Abspaltung der Schutzgruppen ist in den weiter vorn im Abschnitt "Schutzgruppen" genannten Standardwerken beschrieben.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch durch Reduktion, z.B. durch Behandeln mit einem Alkalimetall-, z.B. Natrium-dithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumjodid, gespalten werden. 2-Triniederalkylsilylniederalkoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie oben beschrieben, freigesetzt werden. Verestertes Carboxy kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure,

gespalten werden. Aroylmethoxycarbonylamino kann auch durch Behandeln mit einem nukleophilen, vorzugsweise salzbildenden Reagenz, wie Natriumthiophenolat, und 4-Nitrobenzyloxycarbonylamino auch durch Behandeln mit einem Alkalimetall-, z.B. Natrium-dithionit, gespalten werden. Gegebenenfalls substituiertes Diphenylmethoxycarbonylamino, tert-Niederalkoxycarbonylamino oder 2-Triniederalkylsilylniederalkoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die

katalytische Hydrierung wird vorzugsweise in einem inerten Lösungsmittel, wie einem halogenierten Kolenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert-Niederalkyl oder durch einen 2-oxa- oder 2-thia-aliphatischen oder -cycloaliphatischen Kohlenwasserstoffrest, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Aethylhexansäure, oder mit organischen Alkali- oder Erdalkalimetallsalzen, z.B. Natriumhydrogencarbonat, oder mit Ammoniak oder einem geeigneten organische Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscherreagenz. Innere Salze von Verbindungen der Formel I, welche z.B. eine freie Carboxygruppe und

eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen übergeführt werden: Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

An einzelnen Chiralitätszentren in einer Verbindung der Formel I, z.B. dem CH-$R_4$-C-Atom, kann die Konfiguration gezielt umgekehrt werden. Beispielsweise kann man die Konfiguration am CH-$R_4$-Atom durch nukleophile Substitution zweiter Ordnung gemäss Verfahren d) nach Ueberführung der Hydroxygruppe in eine Abgangsgruppe X und Reaktion mit einem den Substituenten $R_4$ einführendes Reagens umkehren.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Pharmazeutische Präparate:

Die pharmakologisch verwendbaren Verbindungen der vorliegenden Erfindung können z.B. zur Herstellung von pharmazeutischen Präparaten verwendet werden, welche eine wirksame Menge des Wirkstoffs zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, Alter und dem individuellen Zustand, der zu behandelnden Krankheit sowie von der Applikationsweise ab.

Die an Warmblüter, z.B. Menschen, von etwa 70 kg Körpergewicht, zu verabreichenden Dosismengen liegen zwischen etwa 3 mg und etwa 3 g, vorzugsweise zwischen etwa 10 mg und etwa 1 g, z.B. bei ungefähr 300 mg pro Person und Tag, verteilt auf vorzugsweise 1 bis 3 Einzeldosen, die z.B. gleich gross sein können. Ueblicherweise erhalten Kinder die halbe Dosis von Erwachsenen.

Die neuen pharmazeutischen Präparate enthalten von etwa 1% bis etwa 95%, vorzugsweise von etwa 20% bis etwa 90% des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffs, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8-22, besonders 12-22, Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren wie z.B. Oelsäure, Elaidinsäure, Erucasäure, Brasidinsäure oder Linolsäure, enthalten. Die Alkoholkomponente dieser Fettsäureester hat maximal 6 Kohlenstoffatome und ist ein ein- oder mehrwertiger, z.B. ein-, zwei- oder dreiwertiger Alkohol, z.B. Methanol, Aethanol, Propanol, Butanol oder Pentanol oder deren Isomere, vor allem aber Glycol oder Glyzerin. Als Fettsäureester sind daher beispielsweise zu nennen: Aethyloleat,

Isopropylmyristat, Isopropylpalmitat, "Labrafil M 2735" (Polyoxyäthylenglyzerintrioleat der Firma Gattefossé, Paris), "Myglyol 812" (Triglyzerid gesättigter Fettsäuren der Kettenlänge $C_8$ bis $C_{12}$ der Firma Chemische Werke Witten/Ruhr, Deutschland), besonders aber vegetabile Oele wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig, nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglykol

und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Aethylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Ausgangsmaterialien:

Neue Ausgangsmaterialien und/oder Zwischenprodukte, sowie Verfahren zu ihrer Herstellung, sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Die Ausgangsmaterialien zur Durchführung des Verfahrens a) sind bekannt oder können, falls sie neu sind, nach an sich bekannten Verfahren hergestellt werden, z.B. aus den betreffenden Aminosäuren, Di- oder Tripeptidresten durch Kondensation analog dem vorstehend beschriebenen Verfahren a).

Beispielsweise kann man eine Verbindung der Formel

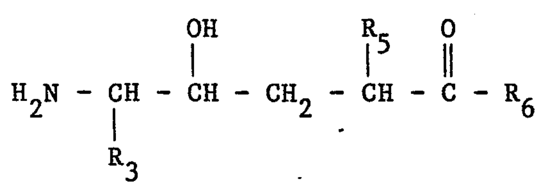

(VIII),

worin $R_3$, $R_5$ und $R_6$ die unter Formel I genannten Bedeutungen haben, herstellen, indem man eine Verbindung der Formel

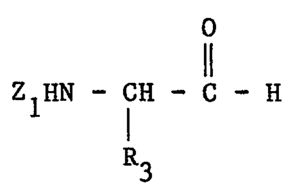

(IX),

worin $R_3$ die genannte Bedeutung hat und $Z_1$ eine Aminoschutzgruppe ist, mit einer Schwefel-Ylid-Verbindung in ein Epoxid der Formel

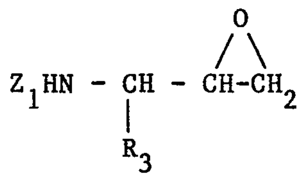
(X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

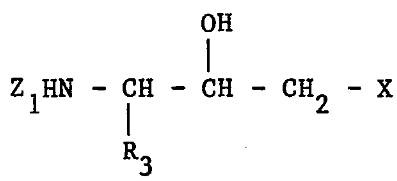
(XI),

worin $R_3$ und $Z_1$ die genannten Bedeutungen haben und X eine nukleofuge Abgangsgruppe ist, mit einer Carbonylverbindung der Formel

$$R_a - (C = O) - R_b \qquad (XII),$$

worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

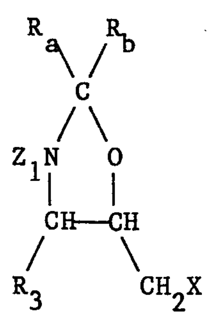
(XIII),

worin die Substituenten die genannten Bedeutungen haben, mit einem Carbonsäureestersalz der Formel

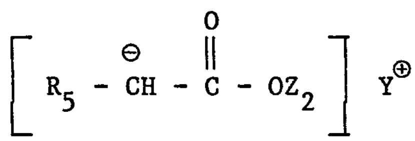 (XIV),

worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium- oder bevorzugt das Lithiumion, ist, umsetzt, und in einer erhältlichen Verbindung der Formel

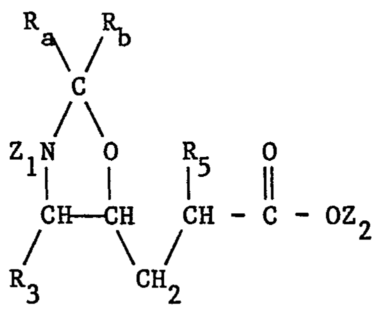 (XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2$=H) je nach Bedeutung des einzuführenden Rests $R_6$ amidiert, verestert oder mit einem C-terminal gegebenenfalls amidierten oder veresterten Rest einer Aminosäure, eines Di- oder Tripeptidrest substituiert und in einer erhältlichen Verbindung der Formel

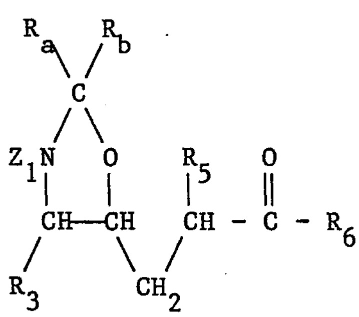 (XVI),

worin die Substituenten die genannten Bedeutungen haben, mit einem geeigneten Solvolysereagens den Ring öffnet und gegebenenfalls die Schutzgruppe $Z_1$ abspaltet.

Die Aminoschutzgruppe $Z_1$ ist eine übliche Schutzgruppe, wie sie weiter vorn genannt sind, z.B. tert-Butoxycarbonyl oder Benzyloxycarbonyl.

Eine geeignete Schwefel-Ylid-Verbindung ist beispielsweise ein Dialkylsulfoniummethylid, z.B. Dimethylsulfoniummethylid, ein Alkyl- oder Phenyl-dialkylaminosulfoxoniummethylid, z.B. Methyl- oder Phenyl-dimethylaminosulfoxoniummethylid, oder ein Dialkylsulfoxoniummethylid, z.B. Dimethyl- oder Diäthylsulfoxononiummethylid.

Die betreffende Schwefel-Ylid-Verbindung wird zweckmässigerweise in situ aus dem entsprechenden Sulfonium- bzw. Sulfoxoniumsalz und einer Base, z.B. Natriumhydrid, in einem dipolar aprotischen Lösungsmittel, z.B. Dimethylsulfoxid, oder einem Aether, z.B. Tetrahydrofuran oder 1,2-Dimethoxyäthan, hergestellt und anschliessend mit der Verbindung der Formel IX umgesetzt. Die Umsetzung erfolgt normalerweise bei Raumtemperatur, unter Kühlen z.B. bis auf -20°, oder unter leichtem Erwärmen, z.B. bis auf 40°. Das gleichzeitig gebildete Sulfid, Sulfinamid bzw. Sulfoxid wird bei der anschliessenden wässrigen Aufarbeitung entfernt.

Eine nukleofuge Abgangsgruppe X ist insbesondere mit einer starken anorganischen oder organischen Säure verestertes Hydroxy, wie mit einer Mineralsäure, z.B. Halogenwasserstoffsäure, wie Chlorwasserstoff-, Bromwasserstoff- oder Jodwasserstoffsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon-oder p-Toluolsulfonsäure verestertes Hydroxy oder mit Stickstoffwasserstoffsäure verestertes Hydroxy.

Ein die nukleofuge Abgangsgruppe X einführendes Reagens ist je nach der Bedeutung von X die freie Säure HX, z.B. Jod- oder Bromwasserstoffsäure oder ein Salz dieser Säure, z.B. Natrium- oder Kaliumjodid.

Vorzugsweise werden die Reaktionsbedingungen so gewählt, dass die Reaktion im wesentlichen als nukleophile Substitution zweiter Ordnung abläuft. Z.B. kann man zur Herstellung einer Verbindung der Formel XI, worin X für eine Abgangsgruppe mit hoher Polarisierbarkeit, z.B. für Jod, steht, eine Verbindung der Formel X in einem dipolaren aprotischen Lösungsmittel, z.B. Aceton, Acetonitril, Nitromethan oder Dimethylformamid, mit einem Alkalimetallsalz von HX, z.B. Natriumjodid, umsetzen.

Die Reaktion kann auch zweistufig durchgeführt werden, wobei eine Verbindung der Formel X in Wasser, das gegebenenfalls ein organisches Lösungsmittel, z.B. einen Alkohol, wie Aethanol, einen Aether, z.B. Tetrahydrofuran oder Dioxan, oder Aceton, als Lösungsvermittler enthält, mit einem Alkalimetallhydroxid, z.B. Natrium- oder Kaliumhydroxid, oder mit einer verdünnten Mineralsäure, z.B. Salzsäure oder Schwefelsäure, oder einer starken organischen Sulfonsäure, z.B. eine der oben im Zusammenhang mit der Abgangsgruppe X genannten Sulfonsäure, umsetzt und das dabei gebildete Diol der Formel XI, worin X OH bedeutet, mit einem den Rest X, wie er oben definiert ist, einführenden Reagens umsetzt, beispielsweise mit einem Halogenid einer organischen Sulfonsäure, z.B. Methansulfonsäurechlorid, Benzolsulfonsäurechlorid oder p-Toluolsulfonsäurechlorid, mit einem Thionylhalogenid, z.B. Thionylchlorid, oder einem Phosphortrihalogenid, z.B. Phosphortrichlorid oder Phosphortribromid. Dieses den Rest X einführende Reagens wird bevorzugt in äquimolarer Menge oder in einem geringen Ueberschuss in einem inerten Lösungsmittel, z.B. einem Halogenkohlenwasserstoff, wie Methylenchlorid, oder einem Aether, z.B. Diäthyläther, Tetrahydrofuran oder Dioxan, oder auch in Acetonitril oder Dimethylformamid bei Temperaturen zwischen -20° und

+50°, gegebenenfalls in Gegenwart einer organischen oder anorganischen Base, z.B. Triäthylamin, Aethyldiisopropylamin, Pyridin oder 4-Dimethylaminopyridin, oder Natriumbicarbonat, Calciumcarbonat oder Magnesiumcarbonat, umgesetzt.

Carbonylverbindungen der Formel XII sind Aldehyde oder vorzugsweise Ketone, worin jeder der Reste $R_a$ und $R_b$ Niederalkyl, vorzugsweise mit bis zu 4 Kohlenstoffatomen, z.B. Methyl oder Aethyl, Aryl oder Arylniederalkyl mit bis zu 10 Kohlenstoffatomen, z.B. Phenyl oder Benzyl darstellt, insbesondere Niederalkanone, z.B. Aceton, oder, wenn $R_a$ und $R_b$ zusammen gegebenenfalls überbrücktes Alkyliden bedeuten, zyklische Ketone, insbesondere Cycloalkanone, z.B. Cyclohexanon, oder Bicycloalkanone, z.B. Campher, ebenfalls optisch aktive Ketone oder Aldehyde.

Derivate der Carbonylverbindung der Formel XII sind z.B. Acetale oder Enoläther, vorallem Acetondimethylketal (2,2-Dimethoxypropan) oder 2-Methoxypropen.

Saure Reagenzien sind starke und schwache Brönstedt-Säuren, beispielsweise Mineralsäuren, z.B. Schwefelsäure oder Salzsäure, oder organische Säuren, z.B. p-Toluolsulfonsäure, oder Lewis-Säuren, z.B. Bortrifluoridätherat, Kupfersulfat oder Eisen(III)chlorid.

Als Lösungsmittel eignen sich unpolare, aprotische Lösungsmittel, z.B. Diäthyläther oder Kohlenwasserstoffe, z.B. Benzol oder Toluol.

Die Reaktion wird vorzugsweise bei erhöhten Temperaturen von etwa 40° bis etwa 150°, vor allem bei der Siedetemperatur des jeweiligen Reaktionsgemisches, durchgeführt.

Eine Carboxyschutzgruppe $Z_2$ ist eine der weiter vorn unter Verfahren a) genannten Carboxyschutzgruppen, z.B. tert-Butyl, Benzyl oder 4-Nitrobenzyl, sowie unverzweigtes Niederalkyl, z.B. Methyl oder Aethyl.

Das Salz eines Carbonsäureesters der Formel XIV wird durch Umsetzung des Carbonsäureesters der Formel

$$R_5 - CH_2 - \overset{\displaystyle O}{\overset{\|}{C}} - OZ_2 \qquad (XIVa)$$

mit einem geeigneten Metallisierungsmittel hergestellt.

Geeignete Metallisierungsreagenzien sind substituierte und unsubstituierte Alkalimetallamide, Alkalimetallhydride oder Alkalimetallniederalkylverbindungen, worin das Alkalimetall Natrium oder insbesondere Lithium ist, z.B. Natrium- oder Lithiumamid, Lithium-bis-trimethylsilylamid, Natriumhydrid, Lithiumhydrid oder bevorzugt Lithiumdiisopropylamid oder Butyllithium.

Für die Metallierungsreaktion geeignete Lösungsmittel dürfen keinen aktiven Wasserstoff enthalten und sind z.B. Kohlenwasserstoffe, z.B. Hexan, Benzol, Toluol oder Xylol, schwach polare Aether, z.B. Diäthyläther, Tetrahydrofuran oder Dioxan, oder Säureamide, z.B. Hexamethylphosphorsäuretriamid. Das metallierte Zwischenprodukt der Formel XIV braucht nicht isoliert, sondern kann im Anschluss an die Metallierungsreaktion mit der Verbindung der Formel XIII umgesetzt werden. Die Metallierungsreaktion erfolgt bei Temperaturen von etwa -100° bis ca. 0°, vorzugsweise unterhalb -30°. Die weitere Umsetzung unter Bildung von XV kann bei der gleichen Temperatur und gegebenenfalls langsamem Erwärmen bis zum Siedepunkt des Reaktionsgemisches erfolgen.

Die Abspaltung der Carboxyschutzgruppe $Z_2$ erfolgt unter den weiter vorn für die Abspaltung von Carboxyschutzgruppen genannten Reaktionsbedingungen. Wenn $Z_2$ beispielsweise unverzweigtes Niederalkyl ist, kann der Niederalkylester der Formel XV nach den üblichen Verseifungsmethoden, z.B. basisch, z.B. in Gegenwart von Kalium-tert-butylat, in Aether oder in einem anderen organischen Lösungsmittel, gegebenenfalls in Gegenwart von Wasser, gespalten werden. Bei basischer Verseifung des Niederalkylesters der Formel XV erhält man das Salz der freien Carbonsäure, welches man in die freie Carbonsäure umwandeln kann, z.B. in wässrig-alkoholischer Lösung. Die freie Carbonsäure der Formel XV oder das Salz dieser Carbonsäure kann nach den weiter vorn beschriebenen Verfahren amidiert, verestert oder mit einer am terminalen C-Atom gegebenenfalls amidierten oder veresterten Aminosäure bzw. Di- oder Tripeptidrest kondensiert werden.

Die Umsetzung einer erhältlichen Verbindung der Formel XVI mit einem geeigneten Solvolysereagens erfolgt unter Ringöffnung und Abspaltung von $R_a(C=O)R_b$ und unter Abspaltung der Schutzgruppe $Z_1$, wenn diese eine solvolytisch abspaltbare Schutzgruppe ist. Ist $Z_1'$ beispielsweise eine hydrogenolytisch abspaltbare Schutzgruppe, z.B. Benzyloxycarbonyl, kann die Ringöffnung und die Abspaltung dieser Schutzgruppe ebenfalls gleichzeitig erfolgen, indem die Hydrogenolyse in Gegenwart eines Katalysators, z.B. Palladium/Kohle, und eines Solvolysemittels, z.B. Methanol, durchgeführt wird.

Geeignete Solvolysereagentien sind beispielsweise organische Säuren, z.B. Niederalkancarbonsäuren, z.B. Eisessig oder Ameisensäure, Anhydride von Niederalkancarbonsäuren, z.B. Essigsäureanhydrid, oder Sulfonsäuren, z.B. p-Toluolsulfonsäure, Mineralsäuren, z.B. Schwefel- oder Salzsäure, Niederalkanole, z.B. Methanol oder Aethanol, oder Niederalkandiole, z.B. Aethylenglykol.

Die genannten Solvolysereagenzien werden unverdünnt oder mit Wasser verdünnt zugegeben. Die Solvolyse kann auch mit reinem Wasser durchgeführt werden. Die Solvolyse mit einem sauren Reagens findet bevorzugt in wässriger Lösung dieses Reagens und bei Temperaturen von etwa -20° bis etwa zum Siedepunkt des Reaktionsgemisches, bevorzugt bei Zimmertemperatur bis zum Siedepunkt des Reaktionsgemisches, statt.

Die gesamte Reaktionsfolge zur Herstellung der Zwischenprodukte der Formel VIII kann in situ oder nach Isolierung von einzelnen oder sämtlichen verfahrensgemäss erhältlichen Vorprodukten erfolgen.

Verbindungen der Formel II sind neu und ebenfalls Gegenstand der vorliegenden Erfindung. Diese werden beispielsweise hergestellt, indem man eine Carbonsäure der Formel

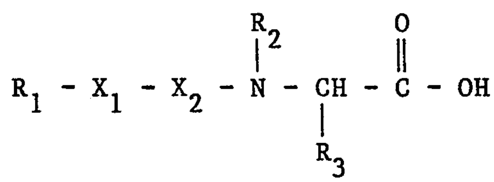

(XVII),

worin die Substituenten die genannten Bedeutungen haben und freie funktionelle Gruppen mit Ausnahme der Carboxygruppe gegebenenfalls in geschützter Form vorliegen, mit einer Organometallverbindung der Formel IV, worin $R_5$ und $R_6$ die genannten Bedeutungen haben und M ein Metallradikal, z.B. -Li oder -MgHal, z.B. -MgCl, -MgBr oder -MgJ bedeutet, umsetzt und das gebildete Additionsprodukt solvolysiert.

Die Herstellung von Verbindungen der Formel II kann nach den in Verfahren c) angegebenen Reaktionsbedingungen erfolgen.

Verbindungen der Formel III sind bekannt oder können, falls sie neu sind, nach an sich bekannten Verfahren hergestellt werden, beispielsweise indem man in einer Verbindung der Formel XVII, worin die Substituenten die genannten Bedeutungen haben und freie funktionelle Gruppen mit Ausnahme der Carboxygruppe gegebenenfalls in geschützter Form vorliegen, die Carboxygruppe nach an sich bekannten Methoden zur Aldehyd-Funktion reduziert.

Verbindungen der Formel IV sind bekannt oder können, falls sie neu sind, beispielsweise durch Umsetzung eines bekannten oder mit an sich bekannten Methoden herstellbaren Halogenids der Formel

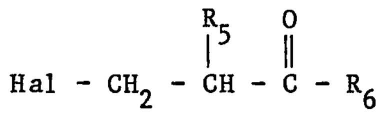 (XVIII),

z.B. des Chlorids, mit einem Metallierungsmittel, z.B. Magnesium, Lithium oder tert-Butyllithium, hergestellt werden.

Verbindungen der Formel V sind neu und ebenfalls Gegenstand der vorliegenden Erfindung. Diese werden beispielsweise hergestellt, indem man einen Aldehyden der Formel III mit einer Organometallverbindung der Formel IV gemäss Verfahren c) umsetzt und die gebildete Hydroxy-Verbindung der Formel I, gegebenenfalls nach Trennung der Isomeren, mit einer der Definition von X entsprechenden starken organische oder anorganischen Säure verestert, beispielsweise wie oben für die Herstellung der Verbindung der Formel XI aus dem entsprechenden Diol beschrieben.

Nitrile der Formeln VI und VIa sind neu und ebenfalls Gegenstand der vorliegenden Erfindung. Diese werden beispielsweise hergestellt, indem man eine Verbindung der Formel

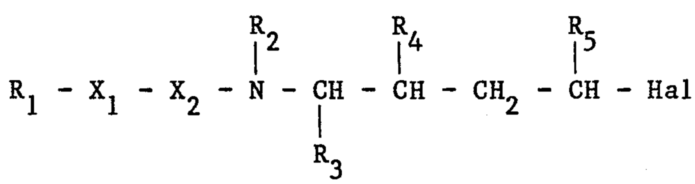 (XIX)

bzw.

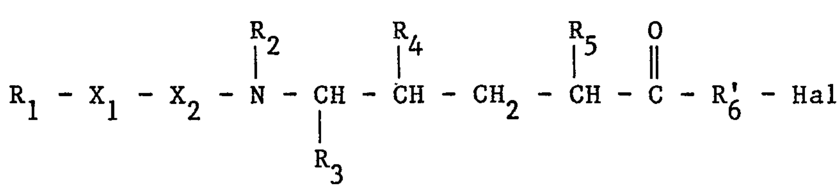 (XIXa),

worin die Substituenten die genannten Bedeutungen haben, mit einem Salz der Cyanwasserstoffsäure umsetzt.

Geeignete Salze der Cyanwasserstoffsäure sollten im gewählten inerten Lösungsmitel genügend löslich sein, damit eine Umsetzung

erfolgen kann. Solche Salze sind z.B. Ammoniumcyanid, Alkalimetall- oder Erdalkalimetallcyanide, z.B. Natrium- oder Kaliumcyanid, oder Uebergangsmetallcyanide, z.B. Kupfercyanid. Letztere eignen sich aufgrund ihrer im Vergleich zu den Alkalimetallcyaniden geringeren Basizität.

Je nach der Art des eingesetzten Cyanids und des Lösungsmittels stellt sich ein Gleichgewicht zwischen der isomeren Nitril- und der Isonitrilform ein. Die Nitrilform wird bevorzugt gebildet, wenn z.B. die Umsetzung mit solchen Metallcyaniden erfolgt, deren Metallkationen ein niedrigeres Atomgewicht als das von Kupfer besitzen.

Geeignete inerte Lösungsmittel sind vor allem polare, aprotische Lösungsmittel, beispielsweise Carbonsäureamide, z.B. Dimethylformamid oder Dimethylacetamid, Nitrile, z.B. Acetonitril oder Propionitril, oder Diniederalkylsulfoxide, z.B. Dimethylsulfoxid.

Die Umsetzung erfolgt bei Raumtemperatur, bei erniedrigter oder bei erhöhter Temperatur, z.B. in einem Temperaturbereich von etwa -40°C bis etwa +100°C, bevorzugt von etwa -10°C bis etwa +50°C und gewünschtenfalls in einer Inertgas-, z.B. Stickstoffatmosphäre.

Epoxide der Formel VII sind neu und ebenfalls Gegenstand der vorliegenden Erfindung. Diese werden z.B. hergestellt, indem man eine Verbindung der Formel IX mit einer Phosphoranylidenverbindung der Formel

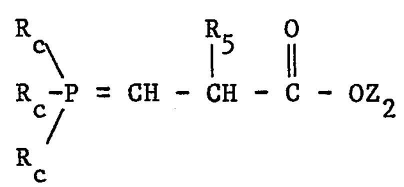

(XX),

worin $R_c$ einen gegebenenfalls substituierten Kohlenwasserstoffrest darstellt, $R_5$ die genannte Bedeutung hat und $Z_2$ eine Carboxyschutzgruppe ist, umsetzt und eine erhältliche Verbindung der Formel

$$Z_1HN - \underset{\substack{| \\ R_3}}{CH} - CH = CH - \overset{\substack{R_5 \\ |}}{CH} - \overset{\substack{O \\ \|}}{C} - OZ_2 \qquad (XXI),$$

mit einem die Peroxogruppe enthaltenden Oxydationsmittel in ein Epoxid überführt und in einer erhältlichen Verbindung in beliebiger Reihenfolge der Reaktionsschritte die Schutzgruppen $Z_1$ und $Z_2$ abspaltet und durch die Gruppen $R_1$-$X_1$-$X_2$- und $R_6$ ersetzt.

$R_c$ ist bevorzugt Phenyl. Die Umsetzung einer Verbindung der Formel IX mit einer Phosphoranylidenverbindung der Formel XX erfolgt unter den für Wittig-Reaktionen bekannten und z.B. im Organikum beschriebenen Reaktionsbedingungen. Das dabei erhältliche Olefin der Formel XXI wird gegebenenfalls in situ mit dem Oxydationsmittel, z.B. Peressigsäure oder m-Chlorperbenzoesäure, umgesetzt. Die Abspaltung der Schutzgruppen $Z_1$ und $Z_2$ und die Einführung der Gruppen $R_1$-$X_1$-$X_2$- bzw. $R_6$ ist weiter vorn unter Verfahren a) beschrieben.

Die folgenden Beispiele dienen zur Illustration der Erfindung, Temperaturen werden in Celsiusgraden angegeben.

Die $R_f$-Werte werden, wenn nicht anders angegeben, auf Kieselgeldünnschichtplatten in folgenden Lösungsmittelsystemen ermittelt:

| | | |
|---|---|---|
| N1 | Chloroform-Methanol | 9:1 |
| N2 | Chloroform-Methanol | 95:5 |
| N3 | Essigester-Hexan | 1:1 |
| N4 | Essigester-Hexan | 1:4 |
| N5 | Essigester-Hexan | 1:9 |
| N6 | Methylenchlorid-Aether | 4:1 |
| N7 | Methylenchlorid-Aether | 1:1 |
| N8 | Methylenchlorid-Methanol | 9:1 |
| N9 | Methylenchlorid-Methanol | 6:1 |

| | | |
|---|---|---|
| N10 | Methylenchlorid-Methanol | 20:1 |
| N11 | Methylenchlorid-Methanol | 10:1 |
| N12 | Methylenchlorid-Methanol | 5:1 |
| B1 | Chloroform-Methanol-Ammoniak konz. | 40:10:1 |
| B2 | Chloroform-Methanol-Ammoniak konz. | 350:50:1 |
| B3 | Methylenchlorid-Methanol-Ammoniak konz. | 1000:50:1 |
| B4 | Methylenchlorid-Methanol-Ammoniak konz. | 350:50:1 |
| B5 | Methylenchlorid-Methanol-Ammoniak konz. | 140:10:1 |
| B6 | Methylenchlorid-Methanol-Ammoniak konz. | 105:50:1 |
| B7 | Methylenchlorid-Methanol-Ammoniak konz. | 90:10:1 |
| B8 | Methylenchlorid-Methanol-Ammoniak konz. | 80:10:1 |
| B9 | Methylenchlorid-Methanol-Ammoniak konz. | 65:10:1 |
| B10 | Methylenchlorid-Methanol-Ammoniak konz. | 50:10:1 |
| B11 | Methylenchlorid-Methanol-Ammoniak konz. | 40:10:1 |
| B12 | Methylenchlorid-Methanol-Ammoniak konz. | 40:25:1 |
| B13 | Methylenchlorid-Methanol-Ammoniak konz. | 30:10:1 |
| B14 | Methylenchlorid-Methanol-Ammoniak konz. | 25:10:1 |
| B15 | Methylenchlorid-Methanol-Ammoniak konz. | 20:5:1 |
| B16 | Methylenchlorid-Methanol-Ammoniak konz. | 10:10:1 |
| B17 | Essigester-Pyridin-Eisessig-Wasser | 62:21:6:11 |
| B18 | n-Butanol-Pyridin-Eisessig-Wasser | 38:24:8:30 |
| B19 | Pyridin-n-Butanol-n-Amylalkohol-Methyläthylketon-Eisessig-Ameisensäure-Wasser | 25:20:15:10:3:3:25 |
| B20 | n-Butanol-Pyridin-Ameisensäure-Wasser | 42:24:4:20 |
| B21 | n-Butanol-Pyridin-Eisessig-Wasser | 42:24:4:30 |
| B22 | n-Butanol-Pyridin-Ameisensäure-Wasser | 44:24:2:20 |
| S1 | Essigester-Pyridin-Eisessig-Wasser | 62:21:6:11 |
| S2 | Essigester-Methanol-Eisessig-Wasser | 67:10:12:23 |
| S3 | Chloroform-Methanol-Wasser-Eisessig | 300:108:20:2 |
| S4 | Chloroform-Methanol-Wasser-Eisessig | 75:27:5:0,5 |
| S5 | Chloroform-Methanol-Wasser-Eisessig | 70:40:10:0,5 |
| S6 | Chloroform-Methanol-Wasser-Eisessig | 90:10:1:0,5 |
| S7 | Chloroform-Methanol-Wasser-Eisessig | 55:47:13:0,5 |
| S8 | Chloroform-Methanol-Wasser-Eisessig | 80:20:3:3 |

| | | |
|---|---|---|
| S9 | Chloroform-Methanol-Wasser-Eisessig | 70:30:3:3 |
| S10 | Methylenchlorid-Methanol-Wasser-Eisessig | 750:270:50:5 |
| S11 | n-Butanol-Eisessig-Wasser | 67:10:23 |

Beispielsweise bedeutet die Abkürzung "$R_f$(N1)", dass der $R_f$-Wert im System N1 ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

Abkürzungen für Aminosäuren und Aminosäurederivate:

| | |
|---|---|
| H-Ala-OH | L-Alanin |
| H-Arg-OH | L-Arginin |
| H-Glu-OH | L-Glutaminsäure |
| H-Glu(OR)-OH | L-Glutaminsäure, worin die Carbonsäure-Funktion der Seitenkette mit dem Rest R verestert ist |
| H-Gly-OH | Glycin |
| H-His-OH | L-Histidin |
| H-Ile-OH | L-Isoleucin |
| H-Leu-OH | L-Leucin |
| H-Lys-OH | L-Lysin |
| H-Lys(R)-OH | L-Lysin, worin die Amino-Funktion der Seitenkette mit dem Rest R substituiert ist |
| H-Phe-OH | L-Phenylalanin |
| H-(N-methyl-Phe)-OH | N-Methyl-L-phenylalanin |
| H-Sta-OH | Statin, (3S,4S)4-Amino-3-hydroxy-6-methylheptansäure |
| H-Val-OH | L-Valin |

$-NH_2$ statt -OH: C-terminales (Carbonsäure)Amid

-OMe statt -OH: C-terminaler (Carbonsäure)Methylester

Das Fragment mit der Bezeichnung -Leu$\overset{c}{-}$Val- bedeutet das zweiwertige Radikal von (2S,4S,5S)-2-Isopropyl-4-hydroxy-5-amino-7-methyloctansäure und hat die Formel

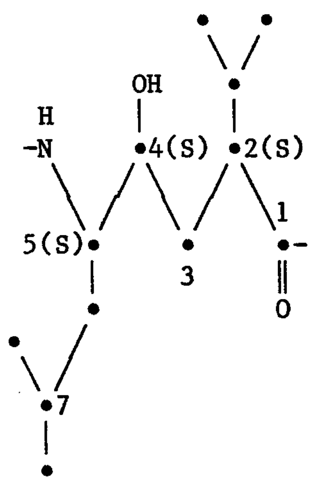

Das Fragment mit der Bezeichnung -Leu$\overset{cx}{-}$Val- leitet sich vom Fragment -Leu$\overset{c}{-}$Val- durch Ueberbrückung von NH und OH durch eine Isopropyliden-Gruppe ab und hat die Formel

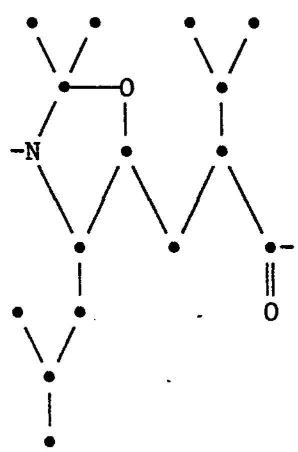

Das Fragment mit der Bezeichnung -Leu$\overset{c}{-}$Gly(R)- bedeutet das zweiwertige Radikal von (4S,5S)-2-R-4-hydroxy-5-amino-7-methyloctansäure mit (R)- oder (S)-Konfiguration am C-Atom 2 und hat die Formel

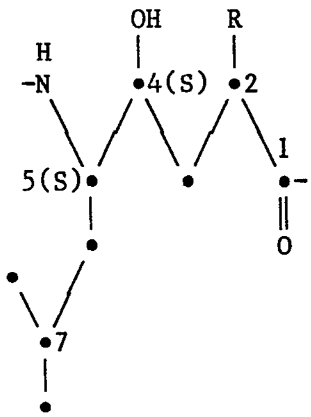

Das Fragment mit der Bezeichnung -Leu$\overset{cx}{-}$Gly(R)- leitet sich vom Fragment -Leu$\overset{c}{-}$Gly(R)- durch Ueberbrückung von NH und OH durch eine Isopropyliden-Gruppe ab.

<u>Weitere Abkürzungen:</u>

| | |
|---|---|
| Ac | = Acetyl |
| BOC | = tert-Butoxycarbonyl |
| DC | = Dünnschichtchromatographie, wenn nicht anders angegeben auf Kieselgel |
| DCCI | = Dicyclohexylcarbodiimid |
| DCH | = Dicyclohexylharnstoff |
| DMF | = Dimethylformamid |
| DMSO | = Dimethylsulfoxid |
| HOBt | = 1-Hydroxybenzotriazol |
| Min. | = Minute(n) |
| Sdp. | = Siedepunkt |
| Smp. | = Schmelzpunkt |
| Std. | = Stunde(n) |
| THF | = Tetrahydrofuran |
| Vol. | = Volumen(anteile) |
| Z | = Benzyloxycarbonyl |

Beispiel 1: Z-Phe-His-Leu$^{c}$Val-Ile-His-$NH_2$

1,7 g Z-Phe-His-OH (hergestellt nach der Vorschrift in Chem. Ber. 94, 2768 (1961)), 1,71 g H-Leu$^{c}$Val-Ile-His-$NH_2$ und 544 mg HOBt (Fluka purum, enthält 11-13% Wasser) werden in 60 ml DMF abs. auf 0° abgekühlt. Nach Zugabe von 1,03 g DCCI wird während 15 Std. bei Eisbadkühlung und anschliessend während 2 Tagen bei Raumtemperatur gerührt. Die Suspension wird im Hochvakuum eingeengt und der Rückstand während 60 Min. in einem Gemisch von Methanol-Wasser-Eisessig (94:3:3) in einem Oelbad von 60° gerührt. Nach Entfernen des Lösungsmittels im Hochvakuum wird der Rückstand mittels Flash-Chromatographie aufgetrennt (1000 g Kieselgel 60, 40-63 µm, Fliessmittel-System B8). Die produkthaltigen Fraktionen werden eingedampft. Nach dem Trocknen im Hochvakuum erhält man Z-Phe-His-Leu$^{c}$Val-Ile-His-$NH_2$ als farbloses Pulver. $R_f$(B4) = 0,07; $R_f$(S3) = 0,29. Das Produkt kann durch Aufrühren in Methanol und Versetzen mit 2 Aequivalenten wässriger 1N HCl in das Dihydrochlorid überführt werden.

Das Ausgangsmaterial H-Leu$^{c}$Val-Ile-His-$NH_2$ kann nach folgendem Reaktionsschema hergestellt werden:

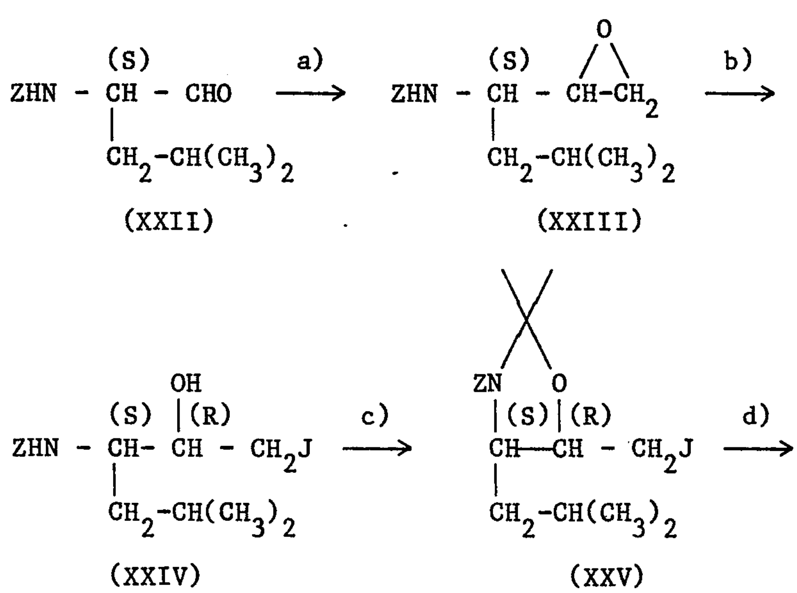


(XXII) (XXIII) (XXIV) (XXV)

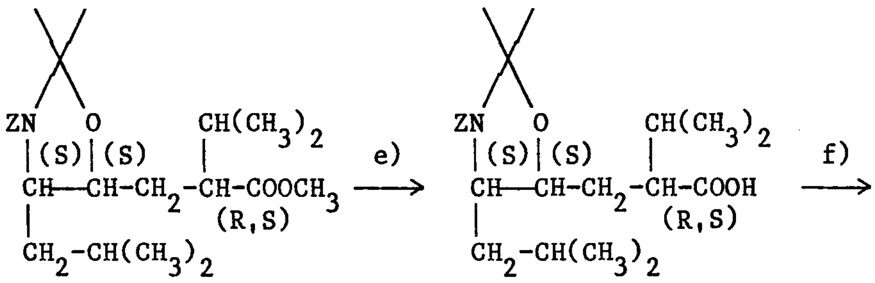


(XXVI)

(XXVII)
(S,S,S-Isomeres: Z-Leu$^{cx}$Val-OH)

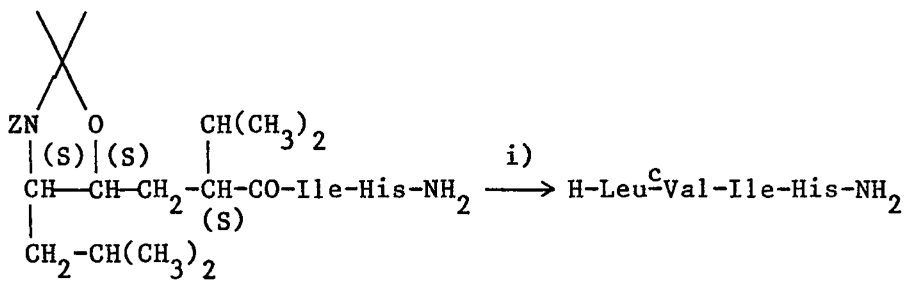


(Z-Leu$^{cx}$Val-Ile-His-$NH_2$)

a) 8,75 g Natriumhydriddispersion (55% in Oel) werden in einem trockenen Sulfierkolben unter Argon durch dreimaliges Aufrühren in 85 ml Petroläther (Sdp. 40-60°) und anschliessendem Abdekantieren des Lösungsmittels vom Oel befreit. Nach Trocknen im Hochvakuum wird ein graues Pulver erhalten, das tropfenweise mit einer Lösung von 44,1 g Trimethylsulfoxoniumiodid in 180 ml Dimethylsulfoxid versetzt wird, wobei die Temperatur auf ca. 40° steigt. Die graue Suspension wird bei Raumtemperatur 1 Std. gerührt und anschliessend innerhalb von 50 Min. bei 10° mit einer Lösung von 43,4 g (S)-2-Benzyloxycarbonylamino-4-methyl-pentanal (XXII) (Herstellung: Helvetica Chimica Acta 66, 450 (1983)) in 180 ml DMSO versetzt. Die gelbe Suspension wird 15 Min. bei 10° und anschliessend 1,5 Std. bei Raumtemperatur gerührt. Die gelblich-trübe Lösung wird auf 500 g Eis gegossen. Die wässrige Lösung wird mit Aether extrahiert, die organische Phase mit Wasser gewaschen und nach Trocknen über Natriumsulfat eingedampft. Der ölige Rückstand wird mittels Flash-Chromatographie an 2 kg Kieselgel (40-63 µm) mit einem Gemisch von Methylenchlorid/n-Hexan/ Essigsäureäthylester (5:10:3) aufgetrennt.

Die produkthaltigen Fraktionen werden vereinigt, eingedampft und im Hochvakuum getrocknet. Man erhält das Epoxid (XXIII) (Diastereomerengemisch, ca. 3:1) als leicht gelbliches Oel. $R_f$(B1) = 0,57; $R_f$(N4) = 0,16.

b) 33,8 g der Verbindung (XXIII) werden in 255 ml Acetonitril aufgenommen und die erhaltene Lösung auf 0° abgekühlt. Nach Zugabe von 19,24 g Natriumjodid werden während 30 Min. tropfenweise bei 0° 16,27 ml Trimethylchlorsilan zugegeben. Das Gemisch wird 40 Min. bei 0-3° gerührt und anschliessend auf 250 ml eiskaltes Wasser gegossen. Das wässrige Gemisch wird mit Aether extrahiert und die organische Phase mit 10%-iger wässriger Natriumthiosulfatlösung und gesättigter, wässriger Natriumchloridlösung gewaschen. Nach Trocknen über Natriumsulfat und Eindampfen erhält man ein öliges Gemisch des gewünschten Alkohols (XXIV) und des entsprechenden Trimethylsilyläthers (beides Diastereomerengemische). Der freie Alkohol wird mittels Flash-Chromatographie abgetrennt (2 kg Kieselgel 60, 40-63 µm, Laufmittel Essigsäureäthylester/Hexan 1:4). Nach Eindampfen der produkthaltigen, vereinigten Fraktionen erhält man den Alkohol (XXIV) als Oel. Nach Vereinigen und Eindampfen der Fraktionen, welche den Trimethylsilyläther enthalten, nimmt man den Rückstand in THF auf, versetzt mit einer Lösung von 2,67 g Tetrabutylammoniumfluorid in Wasser und rührt das so erhaltene Gemisch 3 Tage lang bei Raumtemperatur. Die Lösung wird anschliessend zwischen Wasser und Aether ausgeschüttelt. Die organische Phase wird mit Wasser gewaschen, über Natriumsulfat getrocknet und eingedampft. Nach Trocknen im Hochvakuum erhält man eine zusätzliche Menge des Diastereomerengemisches der Verbindung (XXIV) als Oel. Die gesamte Menge des Diastereomerengemisches (XXIV) wird zwecks weiterer Reinigung in zwei Teilen durch Mitteldruckchromatographie aufgetrennt (1,4 kg Lichroprep® Si 60, 25-40 µm, Laufmittel: Essigsäureäthylester/Hexan (1:8), Fraktionen von ca. 220 ml). Durch Kristallisation aus Petroläther (Sdp. 40-60°) lässt sich die stärker polare Komponente aus dem Rohmaterial und den Mischfraktionen teilweise

anreichern (Smp. 104-106°). Die beiden Diastereomeren fallen in einem Verhältnnis von ca. 3:1 an. Bei der weniger polaren, öligen Komponente handelt es sich um die gewünschte Verbindung (XXIV). $R_f$(N4) = 0,16; $R_f$(B1) = 0,54.

c) 66,6 g der Verbindung (XXIV) und 1,62 g p-Toluolsulfonsäuremonohydrat werden in 1500 ml 2,2-Dimethoxypropan 3 Std. lang unter Rückfluss erhitzt. Nach Abkühlen auf Raumtemperatur wird das Gemisch zwischen 1000 ml Aether und 500 ml gesättigter, wässriger Natriumhydrogencarbonatlösung ausgeschüttelt. Die organische Phase wird mit Wasser gewaschen, über Natriumsulfat getrocknet und eingedampft. Das halbkristalline Rohprodukt wird mittels Flash-Chromatographie gereinigt (2 kg Kieselgel 60, 40-63 µm, Laufmittel: Essigsäureäthylester/Hexan (1:6), Fraktionen zu ca. 400 ml). Durch Eindampfen der vereinigten, produkthaltigen Fraktionen erhält man die Verbindung (XXV) als leicht gelbliche Kristalle. $R_f$(N4) = 0,48; $R_f$(N5) = 0,26.

d) 10,82 ml Diisopropylamin werden in 100 ml absolutem Tetrahydrofuran unter Argon gelöst und auf 0° abgekühlt. Anschliessend wird bei 0-5° das Gemisch 20 Min. lang tropfenweise mit einer 1,6 M Lösung von n-Butyllithium in Hexan versetzt und 15 Min. gerührt. Dann werden bei -70° bis -75° 10,1 ml Isovaleriansäuremethylester zugetropft und die Mischung 1,5 Std. bei -75° gerührt. Bei -60° bis -75° werden unter Rühren 190 ml Hexamethylphosphorsäuretriamid zugetropft. Die entstandene Suspension wird 10 Min. lang gerührt und schliesslich bei -70° bis -75° in 5 Min. tropfenweise mit einer Lösung von 30,0 g der Verbindung (XXV) in 50 ml Tetrahydrofuran versetzt. Das Reaktionsgemisch wird bei Raumtemperatur während 2,5 Std. gerührt und schliesslich auf ein Gemisch von 450 ml gesättigter, wässriger Ammoniumchloridlösung und 500 g Eis gegossen. Die wässrige Phase wird mit Aether extrahiert und die organische Phase mit Wasser gewaschen und über Natriumsulfat getrocknet. Nach Eindampfen erhält man das Diastereomerengemisch der Verbindung (XXVI) als gelbes Oel. $R_f$(N5) = 0,20; $R_f$(N4) = 0,40 (Werte für die weniger polare Komponente).

e) 10,7 g Kalium-tert-butylat werden in 80 ml Aether bei ca. 5° mit 1,07 ml Wasser versetzt. Die weisse Suspension wird noch 10 Min. im Eisbad gerührt und darauf mit 10,0 g der Verbindung (XXVI) (Diastereomerengemisch) in 80 ml Aether versetzt, wobei die Temperatur unterhalb 10° gehalten wird. Das Reaktionsgemisch wird nun während 18 Stunden bei Raumtemperatur gerührt und schliesslich auf 1600 ml gesättigte, wässrige Ammoniumchloridlösung gegossen. Die wässrige Phase wird mit Aether extrahiert und die organische Phase mit gesättigter, wässriger Natriumchloridlösung gewaschen, über Natriumsulfat getrocknet und eingedampft. Das ölige Rohprodukt wird durch Mitteldruckchromatographie aufgetrennt (740 g Lichroprep® Si 60, 25-40 µm, Laufmittel Essigsäureäthylester/Hexan 1:5). Z-Leu$\overset{cx}{—}$Val-OH, die weniger polare Komponente der Verbindung (XXVII) mit der gewünschten Konfiguration des an die Isopropylgruppe gebundenen C-Atoms (S-Konfiguration) wird als gelbes Oel erhalten. $R_f$ (Methylenchlorid/Methanol/Wasser 500:10:1) = 0,13; $R_f$ (N6) = 0,58.

f) Z-Leu$\overset{cx}{—}$Val-Ile-His-$NH_2$: 1,96 g Z-Leu$\overset{cx}{—}$Val-OH, 1,42 g H-Ile-His-$NH_2$ (Herstellung siehe g) und 0,74 g HOBt werden in 15 ml DMF auf 0° abgekühlt. Nach Zugabe von 1,3 g DCCI wird 2 Std. lang bei 0° und dann 18 Std. lang bei Raumtemperatur gerührt. Der entstandene DCH wird abfiltriert und das Lösungsmittel des Filtrats im Hochvakuum eingedampft. Der Rückstand wird in einem Gemisch von 25 ml Methanol/Wasser/Essigsäure (94:3:3) aufgenommen und 1 Std. lang bei 60° gerührt. Dann wird bis zur Trockne eingedampft und der Rückstand in 40 ml Methanol im Eisbad während 30 Min. verrührt. Nach Filtrieren und Abdampfen des Lösungsmittels wird der Rückstand in n-Butanol aufgenommen und mit gesättigter Natriumhydrogencarbonatlösung und gesättigter Natriumchloridlösung gewaschen. Die organische Phase wird über Natriumsulfat getrocknet und eingedampft. Das Rohprodukt wird durch Mitteldruckchromatographie aufgetrennt (240 g Lichroprep® Si 60, 25-40 µm, Fraktionen zu ca. 40 ml, Druck

ca. 8 bar, Laufmittel: Methylenchlorid/Methanol/Wasser 80:10:1, Fliessgeschwindigkeit 40 ml/Min.). Nach ca. 20 Min. wird das Produkt eluiert. Durch Eindampfen der produkthaltigen Fraktionen erhält man die Titelverbindung. $R_f$(S3) = 0,19; $R_f$(B4) = 0,48.

g) H-Ile-His-$NH_2$: 2,5 g Z-Ile-His-$NH_2$ werden in 25 ml Methanol-Wasser (9:1) gelöst und in Gegenwart von 400 mg Palladium-Kohle (10%) unter $CO_2$-Absorption bis zur Sättigung hydriert. Der Katalysator wird abfiltriert, das Filtrat zur Trockne eingeengt und der Rückstand durch Flash-Chromatographie an Kieselgel im System S9 gereinigt. Die produkthaltigen Fraktionen ergeben nach Einengen im Rotationsverdampfer und Trocknen im Hochvakuum H-Ile-His-$NH_2$.

h) Z-Ile-His-$NH_2$: 3,0 g Z-Ile-His-$OCH_3$ werden in 120 ml einer 5 N methanolischen Ammoniak-Lösung gelöst und 16 Std. lang bei Raumtemperatur gerührt. Der gebildete, farblose Niederschlag wird abfiltriert und im Hochvakuum getrocknet. $R_f$(S5) = 0,58, Smp. 204-205°.

i) H-Leu$\overset{c}{-}$Val-Ile-His-$NH_2$: 1,352 g Z-Leu$\overset{cx}{-}$Val-Ile-His-$NH_2$ werden 1 Std. lang in 25 ml Methanol/Wasser (95:5) bei Raumtemperatur in Gegenwart von 150 mg Palladium-Kohle (5%) unter Normaldruck hydriert. Nach Abfiltrieren des Katalysators wird das Lösungsmittel abgedampft und der Rückstand in 30 ml Methanol/Wasser (1:1) während 30 Min. bei Raumtemperatur gerührt. Nach Abdampfen des Lösungsmittels und Trocknen im Hochvakuum erhält man H-Leu$\overset{c}{-}$Val-Ile-His-$NH_2$ als farbloses Pulver. $R_f$(B4) = 0,1; $R_f$(B11) = 0,42.

Beispiel 2: BOC-Phe-His-Leu$\overset{c}{-}$Val-Ile-His-$NH_2$

Analog Beispiel 1 wird ausgehend von 92 mg BOC-Phe-His-OH (hergestellt nach der Vorschrift in der US-Patentschrift 4 025 499) und 100 mg H-Leu$\overset{c}{-}$Val-Ile-His-$NH_2$ nach Zugabe von 35 mg HOBt und 56 mg DCCI und chromatographischer Reinigung an einer LOBAR®-Fertigsäule (Fliessmittel: Methylenchlorid-Methanol-Ammoniak konz. 390:60:2) die Titelverbindung erhalten. $R_f$(B11) = 0,32; $R_f$(S3) = 0,19.

Beispiel 3: Z-Phe-Phe-Leu$^{c}$-Val-Ile-His-$NH_2$

Analog Beispiel 1 wird ausgehend von 54 mg Z-Phe-Phe-OH (Bachem AG, Bubendorf, Schweiz) und 75 mg H-Leu$^{c}$-Val-Ile-His-$NH_2$ nach Zugabe von 26 mg HOBt und 42 mg DCCI und chromatographischer Reinigung an einer LOBAR®-Fertigsäule (Fliessmittel: Methylenchlorid-Methanol-Wasser 50:10:1) die Titelverbindung erhalten. $R_f$(B10) = 0,35.

Beispiel 4: H-Phe-His-Leu$^{c}$-Val-Ile-His-$NH_2$

3,14 g des Dihydrochlorid von Z-Phe-His-Leu$^{c}$-Val-Ile-His-$NH_2$ aus Beispiel 1 werden in 100 ml Wasser/Methanol (5:95) in Gegenwart von 350 mg Palladium-Kohle (5%) während 4 Std. bei Normaldruck hydriert. Nach Abfiltrieren des Katalysators, Abdampfen des Lösungsmittels und Trocknen im Hochvakuum erhält man H-Phe-His-Leu$^{c}$-Val-Ile-His-$NH_2$•2HCl als farbloses Pulver. $R_f$(B11) = 0,26.

Beispiel 5: BOC-Phe-His-Leu$^{c}$-Val-7-tert-butoxycarbonyl-heptylamid

Analog Beispiel 1 wird ausgehend von 81 mg BOC-Phe-His-OH, 79 mg H-Leu$^{c}$-Val-7-tert-butoxycarbonyl-heptylamid, 28 mg HOBt und 57 mg DCCI nach Flash-Chromatographie (30 g Kieselgel 60, 40-63 µm, System Methylenchlorid/Methanol/Ammoniak konz.800:50:1) die Titelverbindung als farbloses Pulver erhalten. $R_f$ (Methylenchlorid/Methanol/Ammoniak konz. 500:50:1) = 0,33.

Die Ausgangsmaterialien werden folgendermassen hergestellt:

a) H-Leu$^{c}$-Val-7-tert-butoxycarbonyl-heptylamid wird durch Hydrierung von 213 mg Z-Leu$^{cx}$-Val-7-tert-butoxycarbonyl-heptylamid in Gegenwart von 40 mg Palladium-Kohle (10%) analog Beispiel 1i erhalten. $R_f$(B2) = 0,13.

b) Z-Leu$\overset{cx}{-}$Val-7-tert-butoxycarbonyl-heptylamid wird analog Beispiel 1 ausgehend von 162 mg Z-Leu$\overset{cx}{-}$Val-OH und 103 mg 8-Amino-octansäure-tert-butylester nach Zugabe von 61 mg HOBt, 53 µl 4-Methylmorpholin und 107 mg DCCI erhalten. $R_f$(N4) = 0,20.

c) 8-Amino-octansäure-tert-butylester wird durch Hydrierung von 1,5 g 8-Benzyloxycarbonylamino-octansäure-tert-butylester in 15 ml Methanol nach Zugabe von 0,10 g Palladium-Kohle (10%) analog Beispiel 4 als farbloses Oel erhalten. $R_f$(B3) = 0,08.

d) 8-Benzyloxycarbonylamino-octansäure-tert-butylester: In einem Autoklaven werden 2 g 8-Benzyloxycarbonylamino-octansäure in 12 ml Dioxan in Gegenwart von 1,2 ml Schwefelsäure mit 7,5 g Isobutylen umgesetzt und 48 Stunden bei Raumtemperatur stehengelassen. Die Reaktionsmischung wird mit Eis und 40 ml 1,8 N wässriger Ammoniaklösung versetzt. Das Reaktionsgemisch wird mit Aether extrahiert. Die organische Phase wird mit Wasser und gesättigter, wässriger Kochsalzlösung gewaschen, über Natriumsulfat getrocknet und im Vakuum eingeengt. Man erhält die Titelverbindung als gelbes, klares Oel. $R_f$(N8) = 0,44.

e) 8-Benzyloxycarbonylamino-octansäure: Zu 7 g 8-Amino-octansäure werden 25 ml 2N NaOH-Lösung gegeben. Anschliessend gibt man gleichzeitig bei ca. 0°-5° 30 Min. lang 17,1 g Chlorameisensäurebenzylester (50% in Toluol) und 12,5 ml 4N NaOH-Lösung zu. Man beobachtet einen voluminösen weissen Niederschlag. Das Reaktionsgemisch wird mit 150 ml Aether und 60 ml Wasser versetzt. Die wässrige Phase wird mit Aether extrahiert, mit Eis versetzt und unter Rühren langsam mit verdünnter wässriger HCl-Lösung auf pH 2 gebracht. Die entstandene Suspension wird mit Essigester zweimal extrahiert. Man vereinigt die organischen Phasen, wäscht diese mit Wasser und gesättigter, wässriger Kochsalzlösung, trocknet über Natriumsulfat und dampft im Vakuum ein. Man erhält die Titelverbindung mit einem Schmelzpunkt von 63°-64°, $R_f$(N1) = 0,53.

Beispiel 6: [o-(2,6-Dichlor-N-benzyl-anilino)-phenyl]-acetyl-Phe-His-Leu$^{c}$-Val-Ile-His-$NH_2$

Analog Beispiel 1 wird ausgehend von 75 mg N-Benzyl-Diclofenac (hergestellt nach DOS 2'840'589), 150 mg H-Phe-His-Leu$^{c}$-Val-Ile-His-$NH_2$•2HCl, 27 mg HOBt, 0,5 ml Aethyldiisopropylamin und 52 mg DCCI nach Flash-Chromatographie (70 g Kieselgel 60, 40-63 µm, System B8) die Titelverbindung als farbloses Pulver erhalten. $R_f$ (B11) = 0,47.

Beispiel 7: [o-(2,6-Dichloranilino)-phenyl]-acetyl-Phe-His-Leu$^{c}$-Val-Ile-His-$NH_2$

134 mg [o-(2,6-Dichloro-N-benzyl-anilino)-phenyl]-acetyl-Phe-His-Leu$^{c}$-Val-Ile-His-$NH_2$ (Beispiel 6) werden in Gegenwart von 90 mg Palladium-Kohle (5%) und 0,4 ml o-Dichlorbenzol in 10 ml MeOH während 10 Std. unter Normaldruck hydriert. Nach Abfiltrieren des Katalysators und Abdampfen des Lösungsmittels wird der Rückstand durch Mitteldruckchromatographie (LOBAR®-Fertigsäule, Grösse B (Merck), 40-60 µm, System B10, ca. 30 ml/Min.) aufgetrennt. Nach Abdampfen des Lösungsmittels der produkthaltigen Fraktionen und Trocknen wird die Titelverbindung als farbloses Pulver erhalten. $R_f$(B11) = 0,44.

Beispiel 8: 3(R),4(S),5(R)-Trihydroxy-1-cyclohexen-1-carbonyl-Phe-His-Leu$^{c}$-Val-Ile-His-$NH_2$

Analog Beispiel 1 wird ausgehend von 17 mg Shikimisäure, 75 mg H-Phe-His-Leu$^{c}$-Val-Ile-His-$NH_2$•2HCl, 14 mg HOBt, 0,36 ml Diisopropyläthylamin und 26 mg DCCI nach Flash-Chromatographie (30 g Kieselgel 60, 40-63 µm, System Methylenchlorid/Methanol/Ammoniak konz. 40:20:1) die Titelverbindung als farbloses Pulver erhalten. $R_f$(B12) = 0,23.

Beispiel 9: Z-Arg-Gly-Phe-His-Leu$\overset{c}{-}$Val-Ile-His-$NH_2$

Analog Beispiel 1 wird ausgehend von 36 mg Z-Arg-Gly-OH (hergestellt nach J. Chem. Soc. 1965, 782), H-Phe-His-Leu$\overset{c}{-}$Val-Ile-His-$NH_2$•2HCl, 14 mg HOBt, 0,36 ml Aethyldiisopropylamin und 26 mg DCCI nach Flash-Chromatographie (110 g Kieselgel 60, 40-63 µm, System Methylenchlorid/ Methanol/Wasser/Eisessig 280:140:30:2) die Titelverbindung als farbloses Pulver erhalten. $R_f$ (Methylenchlorid/Methanol/-Wasser/ Eisessig 140:30:20:1) = 0,25.

Beispiel 10: (2S,4R)-N-Benzyloxycarbonyl-4-hydroxy-pyrrolidin-2-carbonyl-Phe-His-Leu$\overset{c}{-}$Val-Ile-His-$NH_2$

Analog Beispiel 1 wird ausgehend von 42 mg N-Benzyloxycarbonyl-4-hydroxy-L-prolin, 120 mg H-Phe-His-Leu$\overset{c}{-}$Val-Ile-His-$NH_2$•2HCl, 22 mg HOBt, 0,57 ml Aethyldiisopropylamin und 41 mg DCCI nach Flash-Chromatographie (70 g Kieselgel 60, 40-63 µm, System B11) die Titelverbindung als farbloses Pulver erhalten. $R_f$(B13) = 0,41.

Beispiel 11: (2S,4R)-4-Hydroxy-pyrrolidin-2-carbonyl-Phe-His-Leu$\overset{c}{-}$Val-Ile-His-$NH_2$

Analog Beispiel 4 wird durch Hydrierung von 39,6 mg (2S,4R)-N-Benzyloxycarbonyl-4-hydroxy-pyrrolidin-2-carbonyl-Phe-His-Leu$\overset{c}{-}$Val-Ile-His-$NH_2$ (Beispiel 10) nach Flash-Chromatographie (10 g Kieselgel 60, 40-63 µm, System B13) die Titelverbindung als farbloses Pulver erhalten. $R_f$(B13) = 0,38.

Beispiel 12: (5S)-2-Pyrrolidon-5-carbonyl-Phe-His-Leu$\overset{c}{-}$Val-Ile-His-$NH_2$

Analog Beispiel 1 wird ausgehend von 12,7 mg L-Pyroglutaminsäure, 75 mg H-Phe-His-Leu$\overset{c}{-}$Val-Ile-His-$NH_2$•2HCl, 14 mg HOBt, 0,36 ml Aethyldiisopropylamin und 26 mg DCCI nach Flash-Chromatographie (200 g Kieselgel 60, 40-63 µm, System B11) die Titelverbindung als farbloses Pulver erhalten. $R_f$(B11) = 0,21.

Beispiel 13: BOC-Phe-His-Leu$\overset{c}{-}$Val-Ala-Sta-$OCH_3$

Analog Beispiel 1 wird ausgehend von 52 mg BOC-Phe-His-OH, 60 mg H-Leu$\overset{c}{-}$Val-Ala-Sta-$OCH_3$•HCl, 18 mg HOBt, 0,24 ml Aethyldiisopropylamin und 34 mg DCCI nach Flash-Chromatographie (30 g Kieselgel 60, 40-63 µm, System N8) die Titelverbindung als farbloses Pulver erhalten. $R_f$(N9) = 0,38.

Die Ausgangsmaterialien werden folgendermassen hergestellt:

a) H-Leu$\overset{c}{-}$Val-Ala-Sta-$OCH_3$•HCl wird als farbloses Pulver erhalten durch Hydrierung von 602 mg Z-Leu$\overset{cx}{-}$Val-Ala-Sta-$OCH_3$ mit 65 mg Palladium-Kohle 5% bei pH 5,0 analog Beispiel 4. $R_f$(B11) = 0,5.

b) Z-Leu$\overset{cx}{-}$Val-Ala-Sta-$OCH_3$ wird als farbloses Pulver erhalten analog Beispiel 1 ausgehend von 677 mg Z-Leu$\overset{cx}{-}$Val-OH (Beispiel 1e), 546 mg H-Ala-Sta-$OCH_3$•HCl, 0,31 ml Aethyldiisopropylamin, 256 mg HOBt und 483 mg DCCI nach Mitteldruckchromatographie (245 g Lichroprep® Si60, 25-40 µm, System Methylenchlorid/Aether 2:1). $R_f$ (Essigester/Hexan 3:1) = 0,32.

c) H-Ala-Sta-$OCH_3$•HCl wird als farbloses Pulver erhalten durch Hydrierung von 750 mg Z-Ala-Sta-$OCH_3$ mit 80 mg Palladium-Kohle (10%) bei pH 5,0 analog Beispiel 4. $R_f$(B4) = 0,25.

d) Z-Ala-Sta-$OCH_3$ wird als farbloses Pulver erhalten analog Beispiel 1 ausgehend von 1,09 g Z-Ala-OH, 1,00 g Statin-methylester-hydrochlorid (hergestellt wie beschrieben in der Europäischen Patentanmeldung 111'266), 678 mg HOBt, 0,49 ml Aethyldiisopropylamin und 1,28 g DCCI nach Flash-Chromatographie (330 g Kieselgel 60, 40-63 µm, System Essigester/Hexan 3:2). $R_f$ (Essigester/Hexan 2:1) = 0,24.

Beispiel 14: Z-Phe-His-Leu$\overset{c}{-}$Val-Ala-Sta-$OCH_3$

Analog Beispiel 1 wird ausgehend von 56 mg Z-Phe-His-OH, 60 mg H-Leu$\overset{c}{-}$Val-Ala-Sta-$OCH_3$•HCl, 18 mg HOBt, 0,24 ml Aethyldiisopropylamin und 34 mg DCCI nach Flash-Chromatographie (30 g Kieselgel 60, 40-63 µm, System N8) die Titelverbindung als farbloses Pulver erhalten. $R_f$(N9) = 0,33.

Beispiel 15: 4-Benzyloxycarbonylamino-butyryl-Gly-Phe-His-Leu$\overset{c}{-}$Val-Ile-His-$NH_2$

Analog Beispiel 1 wird ausgehend von 29 mg 4-(Benzyloxycarbonylamino)-butyryl-Gly-OH, 75 mg H-Phe-His-Leu$\overset{c}{-}$Val-Ile-His-$NH_2$•2HCl, 14 mg HOBt, 18 mg N-Methylmorpholin und 26 mg DCCI nach Flash-Chromatographie (30 g Kieselgel 60, 40-63 µm, System B9) die Titelverbindung als farbloses Pulver erhalten. $R_f$(B11) = 0,21. Das Präparat kann in Methanol mit 2 Aequivalenten wässriger 1N HCl in das Dihydrochlorid überführt werden.

Die Ausgangsmaterialien werden folgendermassen hergestellt:

a) 4-(Benzyloxycarbonylamino)-butyryl-Gly-OH wird erhalten durch Behandeln von 1,6 g 4-(Benzyloxycarbonylamino)-butyryl-Gly-$OCH_3$ in 10 ml Methanol mit 7,8 ml 1N NaOH bei Raumtemperatur während 1 Std. Nach Zugabe von 7,8 ml 1N HCl wird das Lösungsmittel abgedampft und

der Rückstand in 50 ml $CH_2Cl_2$/MeOH (5:1) aufgerührt. Nach Abfiltrieren des ungelösten NaCl, Abdampfen des Lösungsmittels und Trocknen im Vakuum erhält man die Titelverbindung als farbloses Pulver. $R_f$(B11) = 0,05.

b) 4-(Benzyloxycarbonylamino)-butyryl-Gly-$OCH_3$ wird erhalten analog Beispiel 1 ausgehend von 4,0 g 4-Benzyloxycarbonylaminobuttersäure (hergestellt aus 4-Aminobuttersäure analog Beispiel 5e), 1,93 g Glycin-methylester-hydrochlorid, 1,58 ml N-Methylmorpholin, 2,58 g HOBt und 4,11 g DCCI nach Mitteldruckchromatographie (240 g Lichroprep® Si60, Merck, 25-40 µm, System Methylenchlorid/Methanol 95:5) als farbloses Oel. $R_f$(S3) = 0,79.

Beispiel 16: 4-Aminobutyryl-Gly-Phe-His-Leu$^{c}$-Val-Ile-His $NH_2$

Durch Hydrierung von 300 mg 4-(Benzyloxycarbonylamino)-butyryl-Gly-Phe-His-Leu$^{c}$-Val-Ile-His-$NH_2$ (Beispiel 15) analog Beispiel 4 erhält man nach Flash-Chromatographie (30 g Kieselgel 60, 40-63 µm, System B14) die Titelverbindung als farbloses Pulver. $R_f$(B10) = 0,18.

Beispiel 17 (2R,3R)-2,3-Bis-acetoxy-3-methoxycarbonyl-propionyl-Phe-His-Leu$^{c}$-Val-Ile-His-$NH_2$

Analog Beispiel 1 wird ausgehend von 24 mg L(+)-Weinsäure-monomethylester-bis-acetat (hergestellt nach der Vorschrift in J. Amer. Chem. Soc. 63, 1653 (1941)), 75 mg H-Phe-His-Leu$^{c}$-Val-Ile-His-$NH_2$•2HCl, 14 mg HOBt, 0,36 ml Aethyldiisopropylamin und 26 mg DCCI nach Flash-Chromatographie (30 g Kieselgel 60, 40-63 µm, System Methylenchlorid/Methanol/Ammoniak konz. 80:20:1) die Titelverbindung als leicht gelbliches Pulver erhalten. $R_f$(B11) = 0,31.

Beispiel 18: (2R,3R)-2,3-Dihydroxy-3-methoxycarbonyl-propionyl-Phe-His-Leu$\underline{c}$Val-Ile-His-$NH_2$

44 mg (2R,3R)-2,3-Bis-acetoxy-3-methoxycarbonyl-propionyl-Phe-His-Leu$\underline{c}$Val-Ile-His-$NH_2$ (Beispiel 16) werden mit 1N wässriger Ammoniaklösung/Methanol 1:1 bei 0° während 2,5 Std. gerührt. Nach Abdampfen des Lösungsmittels und Flash-Chromatographie (10 g Kieselgel 60, 40-63 µm, System B11) wird die Titelverbindung als farbloses Pulver erhalten. $R_f$(B13) = 0,31.

Beispiel 19: Z-Phe-His-Leu$\underline{c}$Val-4-[tris-(tert-butyldimethylsilyloxymethyl)-methylaminocarbonyl]-butylamid.

Analog Beispiel 1 wird ausgehend von 60 mg Z-Phe-His-OH, 98 mg H-Leu$\underline{c}$Val-4-[tris-(tert-butyldimethylsilyloxymethyl)-methylaminocarbonyl]-butylamid, 21 mg HOBt, 14 µl Aethyldiisopropylamin und 34 mg DCCI nach Mitteldruckchromatographie (60 g Lichroprep® Si60, 40-60 µm, System Methylenchlorid/Methanol/Ammoniak konz. 120:10:1) die Titelverbindung als farbloses Pulver erhalten. $R_f$ (Methylenchlorid/Methanol/Ammoniak konz. 120:10:1) = 0,16.

Die Ausgangsmaterialien werden folgendermassen hergestellt:

a) H-Leu$\underline{c}$Val-4-[tris-(tert-butyldimethylsilyloxymethyl)-methylaminocarbonyl]-butylamid wird erhalten durch Hydrierung von 900 mg Z-Leu$\underline{cx}$Val-4-[tris-(tert-butyldimethylsilyloxymethyl)-methylaminocarbonyl]-butylamid mit 90 mg Palladium-Kohle 5% analog Beispiel 1i und anschliessender Mitteldruckchromatographie (60 g Lichroprep® Si60, 40-60 µm, System B7). Farbloses Oel, $R_f$(B7) = 0,24.

b) Z-Leu$\underline{cx}$Val-4-[tris-(tert-butyldimethylsilyloxymethyl)-methylaminocarbonyl]-butylamid wird erhalten analog Beispiel 1 ausgehend von 500 mg Z-Leu$\underline{cx}$Val-OH, 694 mg 5-Aminovaleriansäure-tris-(tert-

butyldimethylsilyloxymethyl)-methylamid, 0,21 ml Diisopropyläthylamin, 189 mg HOBt und 331 mg DCCI nach Mitteldruckchromatographie (60 g Lichroprep® Si60, 40-60 µm, System N4) als farbloses Oel. $R_f$(N3) = 0,57.

c) 5-Aminovaleriansäure-tris-(tert-butyldimethylsilyloxymethyl)-methylamid wird erhalten durch Hydrierung von 4,03 g der entsprechenden 5-Benzyloxycarbonylamino-Verbindung mit 0,4 g Palladium-Kohle 5% analog Beispiel 4 und Mitteldruckchromatographie (60 g Lichroprep® Si60, 40-60 µm, System N12). Farbloses Oel, $R_f$(B7) = 0,28.

d) 5-Benzyloxycarbonylaminovaleriansäure-tris-(tert-butyldimethylsilyloxymethyl)-methylamid wird erhalten analog Beispiel 1 ausgehend von 291 mg 5-Benzyloxycarbonylaminovaleriansäure, 500 mg Tris-(tert-butyldimethylsilyloxymethyl)-methylamin, 182 mg HOBt, 0,2 ml Aethyldiisopropylamin und 189 mg DCCI nach Mitteldruckchromatographie (60 g Lichroprep® Si60, 40-60 µm, System N4) als farbloses Oel. $R_f$(N4) = 0,56.

e) Tris-(tert-butyldimethylsilyloxymethyl)-methylamin wird erhalten durch Hydrierung von 399 mg des entsprechenden Benzyloxycarbonyl-Derivates mit 50 mg Palladium-Kohle 5% als farbloses Oel. $R_f$(N4) = 0,41.

f) Benzyloxycarbonyl-tris-(tert-butyldimethylsilyloxymethyl)-methylamid wird hergestellt aus 250 mg Benzyloxycarbonyl-tris-(hydroxymethyl)-methylamid, 234 mg Imidazol und 472 mg tert-Butyldimethylsilylchlorid in 10 ml DMF während 15 Stunden bei Raumtemperatur.

g) Benzyloxycarbonyl-tris-(hydroxymethyl)-methylamid wird hergestellt aus 20 g Tris-(hydroxymethyl)-methylamin, 31,3 g Chlorameisensäurebenzylester und 42,4 ml Triäthylamin in 200 ml DMF

bei 0°. Nach Abdampfen des Lösungsmittels wird in Essigester aufgenommen und mit 2N HCl und gesättigter Natriumchlorid-Lösung gewaschen, die organische Phase getrocknet und das Lösungsmittel abgedampft. $R_f$(N8) = 0,27.

Beispiel 20: Z-Phe-His-Leu$^c$Val-4-[tris-(hydroxymethyl)-methyl-aminocarbonyl]-butylamid

83 mg Z-Phe-His-Leu$^c$Val-4-[tris-(tert-butyldimethylsilyloxymethyl)-methylaminocarbonyl]-butylamid (Beispiel 19) werden mit 6 ml Eisessig/Wasser 2:1 2 Std. bei Raumtemperatur gerührt. Nach Abdampfen des Lösungsmittels und Mitteldruckchromatographie (60 g Lichroprep® Si 60, 40-60 µm, System B8) wird die Titelverbindung als farbloses Pulver erhalten. $R_f$ (Methylenchlorid/Methanol/Ammoniak konz. 70:10:1) = 0,11.

Beispiel 21: 8-(Benzyloxycarbonylamino)-octanoyl-Phe-His-Leu$^c$Val-Ile-His-$NH_2$

Analog Beispiel 1 wird ausgehend von 75 mg H-Phe-His-Leu$^c$Val-Ile-His-$NH_2$•2HCl, 29 mg 8-Benzyloxycarbonylaminooctansäure, 14 mg HOBt, 0,36 ml Aethyldiisopropylamin und 26 mg DCCI in 2 ml DMF nach Flash-Chromatographie (30 g Kieselgel 60, 40-63 µm, System B8) die Titelverbindung als farbloses Pulver erhalten. $R_f$(B11) = 0,33.

Beispiel 22: 8-Aminooctanoyl-Phe-His-Leu$^c$Val-Ile-His-$NH_2$

Durch Hydrierung von 60 mg 8-(Benzyloxycarbonylamino)-octanoyl-Phe-His-Leu$^c$Val-Ile-His-$NH_2$ (Beispiel 21) analog Beispiel 4 erhält man nach Flash-Chromatographie (10 g Kieselgel 60, 40-63 µm, System B16) die Titelverbindung als farbloses Pulver.
$R_f$(B16) = 0,38.

<u>Beispiel 23</u>: <u>Z-Phe-His-Leu$\overset{c}{-}$Val-2-methoxycarbonyl-1-methyl-äthylamid</u>
Analog Beispiel 1 wird ausgehend von 161 mg Z-Phe-His-OH, 122 mg H-Leu$\overset{c}{-}$Val-2-methoxycarbonyl-1-methyl-äthylamid, 57 mg HOBt und 99 mg DCCI in 6 ml DMF nach Flash-Chromatographie (160 g Kieselgel 60, 40-63 µm, System N8) die Titelverbindung als weisses, amorphes Pulver erhalten. $R_f$(N8) = 0,28; $R_f$(B2) = 0,44.

Die Ausgangsmaterialien werden folgendermassen hergestellt:

a) H-Leu$\overset{c}{-}$Val-2-methoxycarbonyl-1-methyl-äthylamid wird durch Hydrierung von 187 mg Z-Leu$\overset{cx}{-}$Val-2-methoxycarbonyl-1-methyl-äthylamid in Gegenwart von 30 mg Palladium-Kohle 10% analog Beispiel 1i erhalten und als Rohprodukt weiterverarbeitet. $R_f$(B2) = 0,22.

b) Z-Leu$\overset{cx}{-}$Val-2-methoxycarbonyl-1-methyl-äthylamid wird analog Beispiel 1 ausgehend von 162 mg Z-Leu$\overset{cx}{-}$Val-OH (Beispiel 1e), 59 mg DL-3-Aminobuttersäuremethylester, 61 mg HOBt, 53 µl 4-Methylmorpholin und 107 mg DCCI nach Flash-Chromatographie mit System N10 erhalten. $R_f$(N10) = 0,64.

<u>Beispiel 24</u>: <u>Z-Phe-His-Leu$\overset{c}{-}$Val-2-carboxy-1-methyl-äthylamid</u>
Eine Mischung von 75 mg Z-Phe-His-Leu$\overset{c}{-}$Val-2-methoxycarbonyl-1-methyl-äthylamid (Beispiel 23), 4 ml Methanol, 2 ml Wasser und 0,2 ml 1N NaOH wird 24 Std. bei Raumtemperatur gerührt. Nach der Zugabe von 0,2 ml 1N HCl wird zur Trockene eingeengt. Die beiden entstandenen Produkte ($R_f$(B1) = 0,15 und $R_f$(B1) = 0,08) werden mittels Flash-Chromatographie (30 g Kieselgel 60, 40-63 µm, ca. 0,4 bar, Fliessmittel B1) isoliert. Die produkthaltigen Fraktionen werden vereinigt und eingedampft. Das unpolarere Diastereomere der Titelverbindung zeigt $R_f$(B1) = 0,08 und $R_f$(S10) = 0,46.

Beispiel 25: Pivaloyl-Phe-His-Leu$^{c}$Val-Ile-His-$NH_2$

Analog Beispiel 1 wird ausgehend von 9 mg Pivaloyl-Phe-His-OH und 48 mg H-Leu$^{c}$Val-Ile-His-$NH_2$ nach Zugabe von 17 mg HOBt und 27 mg DCCI und Reinigung durch Flash-Chromatographie (40 g Kieselgel 60, 40-63 µm, System B9) die Titelverbindung erhalten. $R_f$(B11) = 0,3 und $R_f$(S10) = 0,17.

Die Ausgangsmaterialien werden folgendermassen hergestellt:

a) Pivaloyl-Phe-His-OH wird erhalten durch Hydrolyse von 1,61 g Pivaloyl-Phe-His-OMe in 50 ml Methanol/Wasser (1:2) mit 6 ml 1N NaOH während 45 Min. bei Raumtemperatur. Nach der Zugabe von 6 ml 1N HCl, Einengen und Trocknen erhält man die Titelverbindung als weisses Pulver. $R_f$(B1) = 0,06; $R_f$(S4) = 0,14.

b) Pivaloyl-Phe-His-OMe wird analog Beispiel 1 ausgehend von 1,25 g Pivaloyl-L-phenylalanin und 1,21 g L-Histidin-methylester-dihydrochlorid nach Zugabe von 0,77 g HOBt, 1,01 g 4-Methylmorpholin und 1,34 g DCCI und Reinigung durch Flash-Chromatographie mit System B4 erhalten. $R_f$(B2) = 0,38; $R_f$(S4) = 0,61.

c) Pivaloyl-L-phenylalanin: 3,3 g L-Phenylalanin werden bei 0° in 20 ml 1N NaOH vorgelegt. Nach gleichzeitiger Zugabe von 30 ml NaOH und 3,38 g Pivalinsäurechlorid in 30 ml Aether wird noch 1 Std. nachgerührt. Die wässrige Phase wird mit 50 ml 1N Salzsäure versetzt und mit Essigester extrahiert. Durch Kristallisation aus Aether/Hexan wird die Titelverbindung vom Smp. 90-91° erhalten.

Beispiel 26: (1,2:5,6-Di-O-isopropyliden-α-D-glucofuranosyl-3-)-acetyl-Phe-His-Leu$^{c}$Val-Ile-His-$NH_2$

Analog Beispiel 1 wird ausgehend von 14 mg (1,2:5,6-Di-O-isopropyliden-α-D-glucofuranosyl-3)-essigsäure-Dicyclohexylamin-Salz

(hergestellt nach der Vorschrift in Arzneim.-Forsch./Drug Res. 29, 986 (1979)) und H-Phe-His-Leu$^{c}$-Val-Ile-HIS-$NH_2$•2HCl (Beispiel 4) nach Zugabe von 4 mg HOBt, 5,5 µl 4-Methylmorpholin und 7 mg DCCI und Reinigung durch Flash-Chromatographie (35 g Kieselgel 60, 40-63 µm, System B11) die Titelverbindung erhalten. $R_f$(B11) = 0,46; $R_f$(S10) = 0,16.

Beispiel 27: (1,2-O-Isopropyliden-α-D-glucofuranosyl-3)-acetyl-Phe-His-Leu$^{c}$-Val-Ile-His-$NH_2$-bis-acetat

25 mg (1,2:5,6-Di-O-isopropyliden-α-D-glucofuranosyl-3)-acetyl-Phe-His-Leu$^{c}$-Val-Ile-His-$NH_2$ (Beispiel 26) und 2,5 ml Essigsäure/Wasser 1:1 werden 24 Std. bei 50° gerührt. Nach dem Einengen am Hochvakuum wird der Rückstand in 4 ml tert-Butanol aufgenommen und lyophilisiert, wobei die Titelverbindung als weisses Pulver anfällt. $R_f$(B17) = 0,13; $R_f$(B1) = 0,26.

Beispiel 28: Z-Phe-His-Leu$^{c}$-Val-7-tert-butoxycarbonyl-heptylamid

Analog Beispiel 1 wird ausgehend von 153 mg Z-Phe-His-OH, 150 mg H-Leu$^{c}$-Val-7-tert-butoxycarbonyl-heptylamid (Beispiel 5a), 54 mg HOBt und 94 mg DCCI nach Flash-Chromatographie (100 g Kieselgel 60, 40-63 µm, System B3) die Titelverbindung erhalten. $R_f$(N8) = 0,40; $R_f$(B4) = 0,55.

Beispiel 29: Z-Phe-His-Leu$^{c}$-Val-7-carboxy-heptylamid

63 mg Z-Phe-His-Leu$^{c}$-Val-7-tert-butoxycarbonyl-heptylamid (Beispiel 28) werden in 1,5 ml Trifluoressigsäure 10 Min. bei Raumtemperatur gerührt. Die entstandene Lösung wird eingeengt. Der Rückstand wird mittels Flash-Chromatographie (30 g Kieselgel 60, 40-63 µm, ca. 0,4 bar, Fliessmittel B1) gereinigt. Die produkthaltigen Fraktionen werden vereinigt und eingedampft. Der Rückstand wird in 2 ml Wasser verrührt, der Festkörper abgenutscht und im Hochvakuum getrocknet, wobei die Titelverbindung als weisses Pulver erhalten wird. $R_f$(B11) = 0,14.

Beispiel 30: Z-Phe-His-Leu$\overset{c}{-}$Val-7-(pivaloyloxy-methyloxycarbonyl)-heptylamid

40 mg Z-Phe-His-Leu$\overset{c}{-}$Val-7-carboxy-heptylamid (Beispiel 29) werden in 1 ml Aethanol aufgeschlämmt und mit 100 µl 1N NaOH versetzt. Die Lösung wird eingedampft und durch zweimaliges Aufschlämmen in Toluol-Aethanol und Eindampfen getrocknet. Der Rückstand wird in 0,5 ml DMF gelöst, bei 0° unter Rühren im Abstand von 1 Std. zweimal mit je 12 mg Pivalinsäure-jodmethylester in 0,2 ml DMF versetzt, 16 Std. bei 0° und 1 Std. bei Raumtemperatur weitergerührt und dann am Hochvakuum zur Trockne eingedampft. Die Titelverbindung wird durch Flash-Chromatographie (5,5 g Kieselgel, Fliessmittel Methylenchlorid/Methanol/Ammoniak konz. 700:50:1) des Rückstands erhalten. $R_f$(B11) = 0,81; $R_f$ (Methylenchlorid/Methanol/Ammoniak konz. 700:50:1) = 0,23.

Beispiel 31: Z-Phe-His-Leu$\overset{c}{-}$Val-4-tert-butoxycarbonyl-butylamid

Analog Beispiel 1 wird ausgehend von 144 mg Z-Phe-His-OH, 138 mg H-Leu$\overset{c}{-}$Val-4-tert-butoxycarbonyl-butylamid, 51 mg HOBt und 89 mg DCCI nach Flash-Chromatographie (180 g Kieselgel 60, 40-63 µm, System N10) die Titelverbindung erhalten. $R_f$(B2) = 0,40; $R_f$(N8) = 0,41.

Die Ausgangsmaterialien werden folgendermassen hergestellt:

a) H-Leu$\overset{c}{-}$Val-4-tert-butoxycarbonyl-butylamid wird durch Hydrierung von 185 mg Z-Leu$\overset{cx}{-}$Val-4-tert-butoxycarbonyl-butylamid in Gegenwart von 30 mg Palladium-Kohle (10%) analog Beispiel 4 erhalten und als Rohprodukt weiterverarbeitet. $R_f$(B2) = 0,17.

b) Z-Leu$\overset{cx}{-}$Val-4-tert-butoxycarbonyl-butylamid wird analog Beispiel 1 ausgehend von 101 mg Z-Leu$\overset{cx}{-}$Val-OH, 52 mg 5-Amino-valeriansäure-tert-butylester, 38 mg HOBt, 33 µl N-Methylmorpholin und 68 mg DCCI nach Flash-Chromatographie mit System N4 erhalten. $R_f$(N4) = 0,20.

c) 5-Amino-valeriansäure-tert-butylester wird aus 5-Aminovaleriansäure über 5-Benzyloxycarbonylamino-valeriansäure und 5-Benzyloxycarbonylamino-valeriansäure-tert-butylester analog der in den Beispielen 5c, 5d und 5e beschriebenen Art und Weise hergestellt.

Beispiel 32: Z-Phe-His-Leu$\overset{c}{-}$Val-4-carboxy-butylamid

Analog Beispiel 29 wird durch Hydrolyse von 68 g Z-Phe-His-Leu$\overset{c}{-}$Val-4-tert-butoxycarbonyl-butylamid mit 1,5 ml Trifluoressigsäure nach Flash-Chromatographie (35 g Kieselgel 60, 40-63 µm, System B22) die Titelverbindung erhalten. $R_f$(B11) = 0,13.

Beispiel 33: Z-Phe-His-Leu$\overset{c}{-}$Val-$NH_2$

Analog Beispiel 1 wird ausgehend von 87 mg Z-Phe-His-OH, 46 mg H-Leu$\overset{c}{-}$Val-$NH_2$, 31 mg HOBt und 55 mg DCCI nach Flash-Chromatographie (35 g Kieselgel 60, 40-63 µm, Systsem B4) die Titelverbindung erhalten. $R_f$(S10) = 0,57; $R_f$(B2) = 0,37.

Die Ausgangsmaterialien werden folgendermassen hergestellt:

a) H-Leu$\overset{c}{-}$Val-$NH_2$ wird durch Hydrierung von 73 mg Z-Leu$\overset{cx}{-}$Val-$NH_2$ in Gegenwart von 15 mg Palladium-Kohle (10%) analog Beispiel 4 erhalten und als Rohprodukt weiterverarbeitet. $R_f$(B2) = 0,04; $R_f$(S10) = 0,18.

b) Z-Leu$\overset{cx}{-}$Val-$NH_2$: Eine Mischung aus 93 mg Z-Leu$\overset{cx}{-}$Val-OH (Beispiel 1e), 2 ml Methylenchlorid, 39 µl Aethyldiisopropylamin und 62 mg Phosphorsäure-phenylester-anilid-chlorid wird 30 Min. gerührt und bei Raumtemperatur mit 0,2 ml Ammoniaklösung 8N (in Methanol) versetzt. Nach weiteren 30 Min. filtriert man den Niederschlag ab. Das Filtrat wird eingeengt und der Rückstand mittels Flash-Chromatographie (30 g Kieselgel 60, 40-63 µm, ca. 0,4 bar, Fliessmittel N3) gereinigt. Die produkthaltigen Fraktionen werden vereinigt und eingeengt und die Titelverbindung als farbloses Oel isoliert. $R_f$(N3) = 0,20.

Beispiel 34: Z-Phe-His-Leu$\overset{c}{-}$Val-methylamid

Analog Beispiel 1 wird ausgehend von 50 mg Z-Phe-His-OH, 28 mg H-Leu$\overset{c}{-}$Val-methylamid, 18 mg HOBt und 31 mg DCCI nach Flash-Chromatographie (35 g Kieselgel 60, 40-63 µm, Fliessmittelsystem Methylenchlorid/Methanol/Ammoniak konz. 700:50:1) die Titelverbindung erhalten. $R_f$(B2) = 0,43; $R_f$(S10) = 0,68.

Die Ausgangsmaterialien werden folgendermassen hergestellt:

a) H-Leu$\overset{c}{-}$Val-methylamid wird durch Hydrierung von 48 mg Z-Leu$\overset{cx}{-}$Val-methylamid in Gegenwart von 10 mg Palladium-Kohle (10%) analog Beispiel 4 erhalten und als Rohprodukt weiterverarbeitet. $R_f$(B2) = 0,08; $R_f$(S10) = 0,27.

b) Z-Leu$\overset{cx}{-}$Val-methylamid: Eine Mischung aus 100 mg Z-Leu$\overset{cx}{-}$Val-OH (Beispiel 1e), 2 ml DMF, 49 mg HOBt und 66 mg DCCI wird 24 Std. bei 0° stehen gelassen. Die Mischung wird mit einem Ueberschuss an Methylamin vesetzt und 2 Std. bei 0° und 2 Std. bei Raumtemperatur gerührt. Die Titelverbindung wird nach Flash-Chromatographie mit System N3 als farbloses Oel erhalten. $R_f$(N7) = 0,55.

Beispiel 35: Z-Phe-His-Leu$\overset{c}{-}$Val-benzylamid

Analog Beispiel 1 wird ausgehend von 88 mg Z-Phe-His-OH, 65 mg H-Leu$\overset{c}{-}$Val-benzylamid, 31 mg HOBt und 54 mg DCCI nach Flash-Chromatographie (100 g Kieselgel 60, 40-62 µm, System B3) die Titelverbindung erhalten. $R_f$(B2) = 0,60; $R_f$(S10) = 0,68.

Die Ausgangsmaterialien werden folgendermassen hergestellt:

a) H-Leu$\overset{c}{-}$Val-benzylamid wird durch Hydrierung von 100 mg Z-Leu$\overset{cx}{-}$Val-benzylamid in Gegenwart von 20 mg Palladium-Kohle (10%) analog Beispiel 4 erhalten und als Rohprodukt weiterverarbeitet. $R_f$(B2) = 0,20; $R_f$(S10) = 0,37.

b) Z-Leu$\overset{cx}{—}$Val-benzylamid: Eine Mischung aus 150 mg Z-Leu$\overset{cx}{—}$Val-OH (Beispiel 1e), 3 ml DMF, 74 mg HOBt und 99 mg DCCI wird 24 Std. bei 0° stehengelassen. Die Mischung wird mit 55 µl Benzylamin versetzt und 6 Std. bei 0° und 24 Std. bei Raumtemperatur gerührt. Die Titelverbindung wird nach Flash-Chromatographie mit Essigester/Hexan (1:2) als farbloses Oel erhalten. $R_f$(N7) = 0,56.

Beispiel 36: Z-Phe-His-Leu$\overset{c}{-}$Val-dimethylamid

Analog Beispiel 1 wird ausgehend von 162 mg Z-Phe-His-OH, 95 mg H-Leu$\overset{c}{-}$Val-dimethylamid, 57 mg HOBt und 99 mg DCCI nach Flash-Chromatographie (100 g Kieselgel 60, 40-63 µm, System B3)die Titelverbindung erhalten. $R_f$(B2) = 0,57; $R_f$(S10) = 0,74.

Die Ausgangsmaterialien werden folgendermassen hergestellt:

a) H-Leu$\overset{c}{-}$Val-dimethylamid wird durch Hydrierung von 160 mg Z-Leu$\overset{cx}{—}$Val-dimethylamid in Gegenwart von 30 mg Palladium-Kohle (10%) analog Beispiel 4 erhalten und als Rohprodukt weiterverarbeitet. $R_f$(B2) = 0,15; $R_f$(S10) = 0,37.

b) Z-Leu$\overset{cx}{—}$Val-dimethylamid wird analog Beispiel 35b aus 100 mg Z-Leu$\overset{cx}{—}$Val-OH, 2 ml DMF, 49 mg HOBt und 66 mg DCCI gefolgt von einem Ueberschuss an Dimethylamin nach Flash-Chromatographie mit Essigester/Hexan (1:2) als farbloses Oel erhalten. $R_f$(N7) = 0,35.

Beispiel 37: Z-Phe-His-Leu$\overset{c}{-}$Val-3-(m-carbamoylphenyloxy)-2-hydroxy-propylamid

Analog Beispiel 1 wird ausgehend von 77 mg Z-Phe-His-OH, 37 mg H-Leu$\overset{c}{-}$Val-3-(-m-carbamoylphenyloxy)-2-hydroxy-propylamid, 27 mg HOBt und 36 mg DCCI nach Flash-Chromatographie (65 g Kieselgel 60, 40-63 µm, Fliessmittelsystem B7) die Titelverbindung erhalten. $R_f$(B7) = 0,15; $R_f$(N12) = 0,30.

Das Ausgangsmaterial wird folgendermassen hergestellt:

a) H-Leu$\overset{c}{-}$Val-3-(m-carbamoylphenyloxy)-2-hydroxy-propylamid: 80 mg Z-Leu$\overset{cx}{-}$Val-3-(m-carbamoylphenyloxy)-2-hydroxy-propylamid werden in 8 ml Methanol-Wasser 9:1 gelöst und nach Zugabe von 20 mg Palladium-Kohle (10%) unter $CO_2$- Absorption bis zur Sättigung hydriert. Nach 24 Std. bei Raumtemperatur wird der Katalysator abfiltriert, das Filtrat eingeengt und am Hochvakuum getrocknet. Die Titelverbindung wird durch Flash-Chromatographie (65 g Kieselgel 60, 40-63 µm, System B11) gereinigt. $R_f$(B11) = 0,28.

b) Z-Leu$\overset{cx}{-}$Val-3-(m-carbamoylphenyloxy)-2-hydroxy-propylamid wird analog Beispiel 1 ausgehend von 156 mg Z-Leu$\overset{cx}{-}$Val-OH, 89 mg m-(3-Amino-2-hydroxy-propyloxy)-benzoesäureamid, 71 mg HOBt und 111 mg DCCI nach Flash-Chromatographie im System N11 erhalten. $R_f$(N11) = 0,34; $R_f$(N12) = 0,59.

c) m-(3-Amino-2-hydroxy-propyloxy)-benzoesäureamid: 15 g m-(2,3-Epoxy-propyloxy)-benzoesäureamid werden gelöst in 150 ml Methanol langsam zu 155 ml Ammoniak conc. zugegeben. Anschliessend wird noch während 2 Std. auf 40° erwärmt, am Rotationsverdampfer zur Trockene eingeengt und das Produkt mittels Flash-Chromatographie im System B11 gereinigt. $R_f$(B11) = 0,14.

d) m-(2,3-Epoxy-propyloxy)-benzoesäureamid: 16,2 g m-Hydroxy-benzoesäureamid und 32,4 g Pottasche werden in 120 ml Epichlorhydrin während 3 Std. am Rückfluss gekocht. Das Reaktionsgemisch wird anschliessend auf Eis gegossen, das Produkt mit Essigester extrahiert und mittels Flash-Chromatographie im System N10 gereinigt. $R_f$(N10) = 0,28.

Beispiel 38: Z-Phe-His-Leu$^{c}$Val-3-(2-pyrrolidinon-1-yl)-propylamid
Analog Beispiel 1 wird ausgehend von 221 mg Z-Phe-His-OH, 150 mg H-Leu$^{c}$Val-3-(2-pyrrolidinon-1-yl)-propylamid, 77 mg HOBt und 122 mg DCCI nach Flash-Chromatographie (65 g Kieselgel 60, 40-63 µm, Fliessmittelsystem B7) die Titelverbindung erhalten. $R_f$(B7) = 0,26; $R_f$(N12) = 0,34.

Das Ausgangsmaterial wird folgendermassen hergestellt:

a) H-Leu$^{c}$Val-3-(2-pyrrolidinon-1-yl)-propylamid wird durch Hydrierung von 210 mg Z-Leu$^{cx}$Val-3-(2-pyrrolidinon-1-yl)-propylamid in Gegenwart von 30 mg Palladium-Kohle (10%) analog Beispiel 37a erhalten und durch Flash-Chromatographie mit System B7 gereinigt. $R_f$(B7) = 0,20; $R_f$(B11) = 0,47.

b) Z-Leu$^{cx}$Val-3-(2-pyrrolidinon-1-yl)-propylamid wird analog Beispiel 1 ausgehend von 150 mg Z-Leu$^{cx}$Val-OH, 64 mg N-(3-Aminopropyl)-2-pyrrolidinon, 68 mg HOBt und 107 mg DCCI erhalten und durch Flash-Chromatographie mit System N10 gereinigt.
$R_f$(N10) = 0,20; $R_f$(N11) = 0,55.

Beispiel 39: Z-Phe-His-Leu$^{c}$Val-3-(N-morpholino)-propylamid
Analog Beispiel 1 wird ausgehend von 159 mg Z-Phe-His-OH, 100 mg H-Leu$^{c}$Val-3-(N-morpholino)-propylamid, 60 mg HOBt und 92 mg DCCI nach Flash-Chromatographie (65 g Kieselgel 60, 40-63 µm, Fliessmittelsystem B7) die Titelverbindung erhalten. $R_f$(B7) = 0,22; $R_f$(B11) = 0,65.

Das Ausgangsmaterial wird folgendermassen hergestellt:

a) H-Leu$^{c}$Val-3-(N-morpholino)-propylamid wird durch Hydrierung von 210 mg Z-Leu$^{cx}$Val-3-(N-morpholino)-propylamid in Gegenwart von 30 mg Palladium-Kohle (10%) analog Beispiel 37a erhalten und durch Flash-Chromatographie mit System B11 gereinigt. $R_f$(B11) = 0,35; $R_f$(B7) = 0,11.

b) Z-Leu$\overset{cx}{-}$Val-3-(N-morpholino)-propylamid wird analog Beispiel 1 ausgehend von 150 mg Z-Leu$\overset{cx}{-}$Val-OH, 80 mg 4-(3-Aminopropyl)-morpholin, 68 mg HOBt und 115 mg DCCI erhalten und durch Flash-Chromatographie mit System N11 gereinigt. $R_f$(N11) = 0,35; $R_f$(B7) = 0,53.

<u>Beispiel 40</u>: <u>Z-Phe-His-Leu$\overset{c}{-}$Val-2-(2-hydroxyäthylamino)-äthylamid</u>

Analog Beispiel 1 wird ausgehend von 118 mg Z-Phe-His-OH, 78 mg H-Leu$\overset{c}{-}$Val-2-(2-hydroxyäthylamino)-äthylamid, 42 mg HOBt und 66 mg DCCI nach Flash-Chromatographie (65 g Kieselgel 60, 40-63 µm, Fliessmittelsystem B11) die Titelverbindung erhalten.
$R_f$(B11) = 0,32.

Das Ausgangsmaterial wird folgendermassen hergestellt:

a) H-Leu$\overset{c}{-}$Val-2-(2-hydroxyäthylamino)-äthylamid wird durch Hydrierung von 110 mg Z-Leu$\overset{cx}{-}$Val-2-(2-hydroxyäthylamino)-äthylamid in Gegenwart von 20 mg Palladium-Kohle (10%) analog Beispiel 37a erhalten und durch Flash-Chromatographie mit System S10 gereinigt. $R_f$(S10) = 0,25; $R_f$(B11) = 0,12.

b) Z-Leu$\overset{cx}{-}$Val-2-(2-hydroxyäthylamino)-äthylamid wird analog Beispiel 1 ausgehend von 150 mg Z-Leu$\overset{cx}{-}$Val-OH, 130 mg N-(2-Hydroxyäthyl)-äthylendiamin, 68 mg HOBt und 114 mg DCCI erhalten und durch Flash-Chromatographie mit System B7 gereinigt. $R_f$(B7) = 0,24; $R_f$(B11) = 0,67.

<u>Beispiel 41</u>: <u>Z-Phe-His-Leu$\overset{c}{-}$Val-2-(3-carbamoyl-4-hydroxy-phenyloxy)-äthylamid</u>

Analog Beispiel 1 wird ausgehend von 132 mg Z-Phe-His-OH, 95 mg H-Leu$\overset{c}{-}$Val-2-(3-carbamoyl-4-hydroxy-phenyloxy)-äthylamid, 46 mg HOBt und 72 mg DCCI nach Flash-Chromatographie (65 g Kieselgel 60, 40-63 µm, Fliessmittelsystem B7) die Titelverbindung erhalten.
$R_f$(B7) = 0,21; $R_f$(B11) = 0,56.

Das Ausgangsmaterial wird folgendermassen hergestellt:

a) H-Leu$\overset{c}{-}$Val-2-(3-carbamoyl-4-hydroxy-phenyloxy)-äthylamid wird durch Hydrierung von 240 mg Z-Leu$\overset{cx}{-}$Val-2-(3-carbamoyl-4-hydroxy-phenyloxy)-äthylamid in Gegenwart von 40 mg Palladium-Kohle (10%) analog Beispiel 37a erhalten und durch Flash-Chromatographie mit System B9 gereinigt. $R_f$(B9) = 0,30; $R_f$(B11) = 0,40.

b) Z-Leu$\overset{cx}{-}$Val-2-(3-carbamoyl-4-hydroxy-phenyloxy)-äthylamid wird analog Beispiel 1 ausgehend von 147 mg Z-Leu$\overset{cx}{-}$Val-OH, 107 mg 5-(1-Aminoäthyloxy)-2-hydroxy-benzoesäureamid (hergestellt nach J. Med. Chem. <u>27</u>, 831 (1984)), 61 mg HOBt und 97 mg DCCI erhalten und durch Flash-Chromatographie mit System B7 gereinigt. $R_f$(B7) = 0,41; $R_f$(N7) = 0,20.

<u>Beispiel 42</u>: <u>Z-Phe-His-Leu$\overset{c}{-}$Val-Ala-2-(4-imidazolyl)-äthylamid</u>

Analog Beispiel 1 wird ausgehend von 158 mg Z-Phe-His-OH, 110 mg H-Leu$\overset{c}{-}$Val-Ala-2-(4-imidazolyl)-äthylamid, 56 mg HOBt und 86 mg DCCI nach Flash-Chromatographie (65 g Kieselgel 60, 40-63 µm, Fliessmittelsystem B11) die Titelverbindung erhalten. $R_f$(B11) = 0,42; $R_f$(B9) = 0,22.

Das Ausgangsmaterial wird folgendermassen hergestellt:

a) H-Leu$\overset{c}{-}$Val-Ala-2-(4-imidazolyl)-äthylamid wird durch Hydrierung von 240 mg Z-Leu$\overset{cx}{-}$Val-Ala-2-(4-imidazolyl)-äthylamid in Gegenwart von 40 mg Palladium-Kohle (10%) analog Beispiel 37a erhalten und durch Flash-Chromatographie mit System B11 gereinigt.
$R_f$(B11) = 0,20; $R_f$(B9) = 0,09.

b) Z-Leu$\overset{cx}{-}$Val-Ala-2-(4-imidazolyl)-äthylamid wird analog Beispiel 1 ausgehend von 160 mg Z-Leu$\overset{cx}{-}$Val-OH, 100 mg L-Alanin-2-(4-imidazolyl)-äthylamid, 67 mg HOBt und 106 mg DCCI erhalten und durch Flash-Chromatographie mit System B7 gereinigt. $R_f$(B7) = 0,22; $R_f$(N11) = 0,17.

c) L-Alanin-2-(4-imidazolyl)-äthylamid wird durch Hydrierung von 300 mg Z-Ala-2-(4-imidazolyl)-äthylamid in Gegenwart von 50 mg Palladium-Kohle (10%) analog Beispiel 37a erhalten und durch Flash-Chromatographie mit System B11 gereinigt. $R_f$(B11) = 0,17.

d) Z-Ala-2-(4-imidazolyl)-äthylamid wird analog Beispiel 1 ausgehend von 940 mg Z-Ala-OH, 853 mg Histamin-dihydrochlorid, 1,6 ml Aethyldiisopropylamin, 709 mg HOBt und 1,13 g DCCI erhalten und durch Flash-Chromatographie (200 g Kieselgel 60, 40-63 µm, Fliessmitel-System B7) gereinigt. $R_f$(B7) = 0,26; $R_f$(N11) = 0,15.

Beispiel 43: Z-Phe-His-Leu$\overset{c}{-}$Val-Ala-2-(2-pyridyl)-äthylamid

Analog Beispiel 1 wird ausgehend von 140 mg Z-Phe-His-OH, 100 mg H-Leu$\overset{c}{-}$Val-Ala-2-(2-pyridyl)-äthylamid, 50 mg HOBt und 66 mg DCCI nach Flash-Chromatographie (65 g Kieselgel 60, 40-63 µm, Fliessmittelsystem B7) die Titelverbindung erhalten. $R_f$(B7) = 0,26; $R_f$(B11) = 0,45.

Das Ausgangsmaterial wird folgendermassen hergestellt:

a) H-Leu$\overset{c}{-}$Val-Ala-2-(2-pyridyl)-äthylamid wird durch Hydrierung von 320 mg Z-Leu$\overset{cx}{-}$Val-Ala-2-(pyridyl)-äthylamid in Gegenwart von 50 mg Palladium-Kohle (10%) analog Beispiel 37a erhalten und durch Flash-Chromatographie mit System B9 gereinigt. $R_f$(B9) = 0,26.

b) Z-Leu$\overset{cx}{-}$Val-Ala-2-(2-pyridyl)-äthylamid wird analog Beispiel 1 ausgehend von 200 mg Z-Leu$\overset{cx}{-}$Val-OH, 143 mg L-Alanin-2-(2-pyridyl)-äthylamid, 91 mg HOBt und 132 mg DCCI erhalten und durch Flash-Chromatographie mit System N10 gereinigt. $R_f$(N10) = 0,32; $R_f$(N11) = 0,46.

c) L-Alanin-2-(2-pyridyl)-äthylamid wird durch Hydrierung von 900 mg Z-Ala-2-(2-pyridyl)-äthylamid in Gegenwart von 100 mg Palladium-Kohle (10%) analog Beispiel 37a erhalten und durch Flash-Chromatographie mit System B7 gereinigt. $R_f$(B7) = 0,20.

d) Z-Ala-2-(2-pyridyl)-äthylamid wird analog Beispiel 1 ausgehend von 635 mg Z-Ala-OH, 400 mg 2-(2-Aminoäthyl)-pyridin, 436 mg HOBt und 762 mg DCCI erhalten und durch Flash-Chromatographie (130 g Kieselgel, System N11) gereinigt. $R_f$(N11) = 0,33.

Beispiel 44: Z-Phe-His-Leu$^{c}$Val-Ala-2-(2-hydroxyäthylamino)-äthylamid
Analog Beispiel 1 wird ausgehend von 88 mg Z-Phe-His-OH, 60 mg H-Leu$^{c}$Val-Ala-2-(2-hydroxyäthylamino)-äthylamid, 31 mg HOBt und 48 mg DCCI nach Flash-Chromatographie (65 g Kieselgel 60, 40-63 µm, Fliessmittelsystem B11) die Titelverbindung erhalten.
$R_f$(B11) = 0,24.

Das Ausgangsmaterial wird folgendermassen hergestellt:

a) H-Leu$^{c}$Val-Ala-2-(2-hydroxyäthylamino)-äthylamid wird durch Hydrierung von 89 mg Z-Leu$^{cx}$Val-Ala-2-(2-hydroxyäthylamino)-äthylamid in Gegenwart von 20 mg Palladium-Kohle (10%) analog Beispiel 37a erhalten und ohne Reinigung weiterverarbeitet.
$R_f$(B11) = 0,08; $R_f$(S10) = 0,10.

b) Z-Leu$^{cx}$Val-Ala-2-(2-hydroxyäthylamino)-äthylamid wird analog Beispiel 1 ausgehend von 100 mg Z-Leu$^{cx}$Val-OH, 150 mg L-Alanin-2-(2-hydroxyäthylamino)-äthylamid, 45 mg HOBt und 76 mg DCCI erhalten und durch Flash-Chromatographie mit System B9 gereinigt. $R_f$(B9) = 0,33; $R_f$(B11) = 0,53.

c) L-Alanin-2-(2-hydroxyäthylamino)-äthylamid wird durch Hydrierung von 540 mg Z-Ala-2-(2-hydroxyäthylamino)-äthylamid in Gegenwart von 70 mg Palladium-Kohle (10%) analog Beispiel 37a erhalten und ohne Reinigung direkt weiterverwendet. $R_f$(B14) = 0,17.

d) Z-Ala-2-(2-hydroxyäthylamino)-äthylamid wird analog Beispiel 1 ausgehend von 571 mg Z-Ala-OH, 400 mg N-(2-Hydroxyäthyl)-äthylendiamin, 470 mg HOBt und 788 mg DCCI erhalten und durch Flash-Chromatographie (160 g Kieselgel, System B11) gereinigt. $R_f$(B11) = 0,23.

<u>Beispiel 45</u>: <u>Z-Phe-His-Leu$\overset{c}{-}$Val-Ala-1(S)-benzyl-2-hydroxy-äthylamid</u>
Analog Beispiel 1 wird ausgehend von 110 mg Z-Phe-His-OH, 84 mg H-Leu$\overset{c}{-}$Val-Ala-1(S)-benzyl-2-hydroxy-äthylamid, 38 mg HOBt und 60 mg DCCI nach Flash-Chromatographie (65 g Kieselgel 60, 40-63 µm, Fliessmittelsystem B7) die Titelverbindung erhalten. $R_f$(B7) = 0,27.

Das Ausgangsmaterial wird folgendermassen hergestellt:

a) H-Leu$\overset{c}{-}$Val-Ala-1(S)-benzyl-2-hydroxy-äthylamid wird durch Hydrierung von 210 mg Z-Leu$\overset{cx}{-}$Val-Ala-1(S)-benzyl-2-hydroxy-äthylamid in Gegenwart von 20 mg Palladium-Kohle (10%) analog Beispiel 37a erhalten und durch Flash-Chromatographie mit System B7 gereinigt. $R_f$(B7) = 0,16.

b) Z-Leu$\overset{cx}{-}$Val-Ala-1(S)-benzyl-2-hydroxy-äthylamid wird analog Beispiel 1 ausgehend von 200 mg Z-Leu$\overset{cx}{-}$Val-OH, 160 mg L-Alanin-1(S)-benzyl-2-hydroxy-äthylamid, 110 mg HOBt und 160 mg DCCI erhalten und durch Flash-Chromatographie mit System N10 gereinigt. $R_f$(N10) = 0,27.

c) L-Alanin-1(S)-benzyl-2-hydroxy-äthylamid wird durch Hydrierung von 1,3 g Z-Ala-1(S)-benzyl-2-hydroxy-äthylamid in Gegenwart von 130 mg Palladium-Kohle (10%) analog Beispiel 37a erhalten und durch Flash-Chromatographie mit System B7 gereinigt. $R_f$(B7) = 0,28.

d) Z-Ala-1(S)-benzyl-2-hydroxy-äthylamid wird analog Beispiel 1 ausgehend von 1,0 g Z-Ala-OH, 680 mg 2(S)-Amino-3-phenylpropanol, 750 mg HOBt und 1,2 g DCCI erhalten und durch Flash-Chromatographie (220 g Kieselgel, System N11) gereinigt. $R_f$(N11) = 0,44.

Beispiel 46: Z-Phe-His-Leu$\overset{c}{-}$Val-Ala-1(S)-benzyl-3-cyano-2(R,S)-hydroxy-propylamid

Analog Beispiel 1 wird ausgehend von 142 mg Z-Phe-His-OH, 140 mg H-Leu$\overset{c}{-}$Val-Ala-1(S)-benzyl-3-cyano-2(R,S)-hydroxy-propylamid, 54 mg HOBt und 91 mg DCCI nach Flash-Chromatographie (65 g Kieselgel 60, 40-63 µm, Fliessmittelsystem B7) die Titelverbindung erhalten. $R_f$(B7) = 0,32.

Das Ausgangsmaterial wird folgendermassen hergestellt:

a) H-Leu$\overset{c}{-}$Val-Ala-1(S)-benzyl-3-cyano-2(R,S)-hydroxy-propylamid wird durch Hydrierung von 350 mg Z-Leu$\overset{cx}{-}$Val-Ala-1(S)-benzyl-3-cyano-2(R,S)-hydroxy-propylamid in Gegenwart von 50 mg Palladium-Kohle (10%) analog Beispiel 37a erhalten und durch Flash-Chromatographie mit System B11 gereinigt. $R_f$(B11) = 0,46; $R_f$(B7) = 0,16.

b) Z-Leu$\overset{cx}{-}$Val-Ala-1(S)-benzyl-3-cyano-2(R,S)-hydroxy-propylamid wird analog Beispiel 1 ausgehend von 240 mg Z-Leu$\overset{cx}{-}$Val-OH, 232 mg L-Alanin-1(S)-benzyl-3-cyano-2(R,S)-hydroxy-propylamid, 100 mg HOBt und 159 mg DCCI erhalten und durch Flash-Chromatographie mit System N7 gereinigt. $R_f$(N7) = 0,28.

c) L-Alanin-1(S)-benzyl-3-cyano-2(R,S)-hydroxy-propylamid wird durch Hydrierung von 350 mg Z-Ala-1(S)-benzyl-3-cyano-2(R,S)-hydroxy-propylamid in Gegenwart von 50 mg Palladium-Kohle (10%) analog Beispiel 37a erhalten und durch Flash-Chromatographie mit System B7 gereinigt. $R_f$(B7) = 0,27.

d) Z-Ala-1(S)-benzyl-3-cyano-2(R,S)-hydroxy-propylamid wird analog Beispiel 1 ausgehend von 394 mg Z-Ala-OH, 305 mg 4(S)-Amino-3(R,S)-hydroxy-5-phenyl-valeriansäurenitril, 270 mg HOBt und 430 mg DCCI erhalten und durch Flash-Chromatographie (130 g Kieselgel, System N11) gereinigt. $R_f$(N11) = 0,42; $R_f$(N7) = 0,20.

e) 4(S)-Amino-3(R,S)-hydroxy-5-phenyl-valeriansäurenitril: 840 mg N-(1(S)-Benzyl-3-cyano-2(R,S)-hydroxy-propyl)-phthalimid werden in 20 ml Dichlormethan/Methanol 3:1 gelöst, mit 1 ml Hydrazin-Hydrat versetzt und 20 Std. bei Raumtemperatur gerührt. Es wird vom ausgefallenen Hydrazid befreit und die Titelverbindung durch Flash-Chromatographie mit System B7 gereinigt. $R_f$(B7) = 0,35.

f) N-(1(S)-Benzyl-3-cyano-2(R,S)-hydroxy-propyl)-phthalimid: 1,5 g N-(1(S)-Benzyl-3-cyano-2-keto-propyl)-phthalimid werden in 20 ml Dichlormethan und 2 ml Methanol gelöst, auf -14° gekühlt und innerhalb von 10 Min. mit 90 mg Natriumborhydrid versetzt. Nach 20 Min. wird das Reaktionsgemisch auf eiskalte 0,1N $H_2SO_4$ gegossen, die Titelverbindung mit Dichlormethan extrahiert und mittels Flash-Chromatographie (System N6) gereinigt. Die Titelverbindung besteht aus einem Diastereomerengemisch im Verhältnis 1:1. $R_f$(N6) = 0,41.

g) N-(1(S)-Benzyl-3-cyano-2-keto-propyl)-phthalimid: 2,2 g Phthaloyl-L-phenylalanin (hergestellt nach Houben-Weyl, Band 15/1, S. 252), 0,64 ml Cyanessigsäure-tert-butylester, 1,1 ml Diäthylphosphorcyanidat (J. Org. Chem. 43, 3631 (1978)) und 3,3 ml Aethyldiisopropylamin werden in 40 ml DMF während 20 Std. bei 0°C intensiv gerührt. Die Reaktionslösung wird eingeengt, das resultierende gelbe Oel in 100 ml Methylenchlorid gelöst und zweimal mit 0,1 N $H_2SO_4$ gewaschen. Nach erneutem Einengen wird das Oel in 40 ml Trifluoressigsäure bei Raumtemperatur gelöst und nach 2 Std. am Rotationsverdampfer eingeengt. Das resultierende Rohprodukt wird in Methylenchlorid gelöst, einmal mit Eiswasser gewaschen und mittels Flash-Chromatographie im System Methylenchlorid-Aether 9:1 gereinigt. $R_f$ (Methylenchlorid-Aether 9:1) = 0,43.

Beispiel 47: Z-Phe-His-Leu$\overset{c}{-}$Val-Ile-2-(2-pyridyl)-äthylamid

Analog Beispiel 1 wird ausgehend von 162 mg Z-Phe-His-OH, 128 mg H-Leu$\overset{c}{-}$Val-Ile-2-(2-pyridyl)-äthylamid, 57 mg HOBt und 88 mg DCCI nach Flash-Chromatographie (65 g Kieselgel 60, 40-63 µm, Fliessmittelsystem B7) die Titelverbindung erhalten. $R_f$(B7) = 0,30; $R_f$(B11) = 0,62.

Das Ausgangsmaterial wird folgendermassen hergestellt:

a) H-Leu$\overset{c}{-}$Val-Ile-2-(2-pyridyl)-äthylamid wird durch Hydrierung von 260 mg Z-Leu$\overset{cx}{-}$Val-Ile-2-(2-pyridyl)-äthylamid in Gegenwart von 40 mg Palladium-Kohle (10%) analog Beispiel 37a erhalten und durch Flash-Chromatographie mit System B7 gereinigt. $R_f$(B7) = 0,23.

b) Z-Leu$\overset{cx}{-}$Val-Ile-2-(2-pyridyl)-äthylamid wird analog Beispiel 1 ausgehend von 189 mg Z-Leu$\overset{cx}{-}$Val-OH, 180 mg L-Isoleucin-2-(2-pyridyl)-äthylamid, 93 mg HOBt und 144 mg DCCI erhalten und durch Flash-Chromatographie mit System N11 gereinigt. $R_f$(N11) = 0,45.

c) L-Isoleucin-2-(2-pyridyl)-äthylamid wird durch Hydrierung von 440 mg Z-Ile-2-(2-pyridyl)-äthylamid in Gegenwart von 50 mg Palladium-Kohle (10%) analog Beispiel 37a erhalten und durch Flash-Chromatographie mit System B11 gereinigt. $R_f$(B11) = 0,65; $R_f$(N9) = 0,22.

d) Z-Ile-2-(2-pyridyl)-äthylamid wird analog Beispiel 1 ausgehend von 4,9 g Z-Ile-OH-Dicyclohexylaminsalz, 1,75 g 2-(2-Aminoäthyl)-pyridin, 1,69 g HOBt und 2,95 g DCCI erhalten und durch Flash-Chromatographie (250 g Kieselgel, System N11) gereinigt. $R_f$(N11) = 0,46.

Beispiel 48: Z-Phe-His-Leu$\overset{c}{-}$Val-Ile-2-pyridyl-methylamid

Analog Beispiel 1 wird ausgehend von 130 mg Z-Phe-His-OH, 100 mg H-Leu$\overset{c}{-}$Val-Ile-2-pyridyl-methylamid, 46 mg HOBt und 62 mg DCCI nach Flash-Chromatographie (65 g Kieselgel 60, 40-63 µm, Fliessmittelsystem B7) die Titelverbindung erhalten. $R_f$(B7) = 0,32.

Das Ausgangsmaterial wird folgendermassen hergestellt:

a) H-Leu$\overset{c}{-}$Val-Ile-2-pyridyl-methylamid wird durch Hydrierung von 340 mg Z-Leu$\overset{cx}{-}$Val-Ile-2-pyridyl-methylamid in Gegenwart von 50 mg Palladium-Kohle (10%) analog Beispiel 37a erhalten und durch Flash-Chromatographie mit System B9 gereinigt. $R_f$(B9) = 0,40.

b) Z-Leu$\overset{cx}{-}$Val-Ile-2-pyridyl-methylamid wird analog Beispiel 1 ausgehend von 220 mg Z-Leu$\overset{cx}{-}$Val-OH, 200 mg L-Isoleucin-2-pyridyl-methylamid, 91 mg HOBt und 146 mg DCCI erhalten und durch Flash-Chromatographie mit System N10 gereinigt. $R_f$(N10) = 0,23.

c) L-Isoleucin-2-pyridyl-methylamid wird durch Hydrierung von 500 mg Z-Ile-2-pyridyl-methylamid in Gegenwart von 50 mg Palladium-Kohle (10%) analog Beispiel 37a erhalten und durch Flash-Chromatographie mit System B7 gereinigt. $R_f$(B7) = 0,26; $R_f$(B11) = 0,65.

d) Z-Ile-2-pyridyl-methylamid wird analog Beispiel 1 ausgehend von 894 mg Z-Ile-OH-Dicyclohexylaminsalz, 265 mg 2-Aminomethylpyridin, 337 mg HOBt und 536 mg DCCI erhalten und durch Flash-Chromatographie (100 g Kieselgel, System N7) gereinigt. $R_f$(N7) = 0,22; $R_f$(N11) = 0,48.

Beispiel 49: Z-Phe-His-Leu$\overset{c}{-}$Val-Ile-2-(3-indolyl)-äthylamid

Analog Beispiel 1 wird ausgehend von 92 mg Z-Phe-His-OH, 79 mg H-Leu$\overset{c}{-}$Val-Ile-2-(3-indolyl)-äthylamid, 32 mg HOBt und 44 mg DCCI nach Flash-Chromatographie (65 g Kieselgel 60, 40-63 µm, Fliessmittelsystem B7) die Titelverbindung erhalten. $R_f$(B7) = 0,34.

Das Ausgangsmaterial wird folgendermassen hergestellt:

a) H-Leu$\overset{c}{-}$Val-Ile-2-(3-indolyl)-äthylamid wird durch Hydrierung von 210 mg Z-Leu$\overset{cx}{-}$Val-Ile-2-(3-indolyl)-äthylamid in Gegenwart von 30 mg Palladium-Kohle (10%) analog Beispiel 37a erhalten und durch Flash-Chromatographie mit System B7 gereinigt. $R_f$(B7) = 0,23.

b) Z-Leu$\overset{cx}{-}$Val-Ile-2-(3-indolyl)-äthylamid wird analog Beispiel 1 ausgehend von 121 mg Z-Leu$\overset{cx}{-}$Val-OH, 120 mg L-Isoleucin-2-(3-indolyl)-äthylamid, 68 mg HOBt und 92 mg DCCI erhalten und durch Flash-Chromatographie mit System N10 gereinigt. $R_f$(N10) = 0,22; $R_f$(B5) = 0,53.

c) L-Isoleucin-2-(3-indolyl)-äthylamid wird durch Hydrierung von 800 mg Z-Ile-2-(3-indolyl)-äthylamid in Gegenwart von 100 mg Palladium-Kohle (10%) analog Beispiel 37a erhalten und durch Flash-Chromatographie mit System B7 gereinigt. $R_f$(B7) = 0,43.

d) Z-Ile-2-(3-indolyl)-äthylamid wird analog Beispiel 1 ausgehend von 894 mg Z-Ile-OH-Dicyclohexylaminsalz, 385 mg Tryptamin, 337 mg HOBt und 536 mg DCCI erhalten und durch Flash-Chromatographie (160 g Kieselgel, System N10) gereinigt. $R_f$(N10) = 0,30; $R_f$(N11) = 0,70.

Beispiel 50: Z-Phe-His-Leu$\overset{c}{-}$Val-Ile-2-(N-morpholino)-äthylamid
Analog Beispiel 1 wird ausgehend von 67 mg Z-Phe-His-OH, 54 mg H-Leu$\overset{c}{-}$Val-Ile-2-(N-morpholino)-äthylamid, 24 mg HOBt und 37 mg DCCI nach Flash-Chromatographie (65 g Kieselgel 60, 40-63 µm, Fliessmittelsystem B7) die Titelverbindung erhalten. $R_f$(B7) = 0,28.

Das Ausgangsmaterial wird folgendermassen hergestellt:

a) H-Leu$\overset{c}{-}$Val-Ile-2-(N-morpholino)-äthylamid wird durch Hydrierung von 160 mg Z-Leu$\overset{cx}{-}$Val-Ile-2-(N-morpholino)-äthylamid in Gegenwart von 30 mg Palladium-Kohle (10%) analog Beispiel 37a erhalten und durch Flash-Chromatographie mit System B7 gereinigt. $R_f$(B7) = 0,14.

b) Z-Leu$\overset{cx}{-}$Val-Ile-2-(N-morpholino)-äthylamid wird analog Beispiel 1 ausgehend von 133 mg Z-Leu$\overset{cx}{-}$Val-OH, 120 mg L-Isoleucin-2-(N-morpholino)-äthylamid, 76 mg HOBt und 102 mg DCCI erhalten und durch Flash-Chromatographie mit System B5 gereinigt. $R_f$(B5) = 0,36.

c) L-Isoleucin-2-(N-morpholino)-äthylamid wird durch Hydrierung von 1,0 g Z-Ile-2-(N-morpholino)-äthylamid in Gegenwart von 150 mg Palladium-Kohle (10%) analog Beispiel 37a erhalten und durch Flash-Chromatographie mit System B7 gereinigt. $R_f$(B7) = 0,22.

d) Z-Ile-2-(N-morpholino)-äthylamid wird analog Beispiel 1 ausgehend von 1,34 g Z-Ile-OH-Dicyclohexylaminsalz, 430 mg 4-(2-Aminoäthyl)-morpholin, 500 mg HOBt und 801 mg DCCI erhalten und durch Flash-Chromatographie (200 g Kieselgel, System B7) gereinigt. $R_f$(B7) = 0,50.

<u>Beispiel 51:</u> Z-Phe-His-Leu$\overset{c}{-}$Val-Ile-4-(carbamoylmethyl)-2-thiazolylamid

Analog Beispiel 1 wird ausgehend von 81 mg Z-Phe-His-OH, 69 mg H-Leu$\overset{c}{-}$Val-Ile-4-(carbamoylmethyl)-2-thiazolylamid, 28 mg HOBt und 39 mg DCCI nach Flash-Chromatographie (65 g Kieselgel 60, 40-63 µm, Fliessmittelsystem B7) die Titelverbindung erhalten. $R_f$(B7) = 0,21.

Das Ausgangsmaterial wird folgendermassen hergestellt:

a) H-Leu$\overset{c}{-}$Val-Ile-4-(carbamoylmethyl)-2-thiazolylamid wird durch Hydrierung von 260 mg Z-Leu$\overset{cx}{-}$Val-Ile-4-(carbamoylmethyl)-2-thiazolylamid in Gegenwart von 100 mg Palladium-Kohle (10%) analog Beispiel 37a erhalten und durch Flash-Chromatographie mit System B9 gereinigt. $R_f$(B9) = 0,21.

b) Z-Leu$\overset{cx}{-}$Val-Ile-4-(carbamoylmethyl)-2-thiazolylamid wird analog Beispiel 1 ausgehend von 181 mg Z-Leu$\overset{cx}{-}$Val-OH, 181 mg L-Isoleucin-4-(carbamoylmethyl)-2-thiazolylamid, 103 mg HOBt und 138 mg DCCI erhalten und durch Flash-Chromatographie mit System B5 gereinigt. $R_f$(B5) = 0,20.

c) L-Isoleucin-4-(carbamoylmethyl)-2-thiazolylamid wird durch Hydrierung von 1,2 g Z-Ile-4-(carbamoylmethyl)-2-thiazolylamid in Gegenwart von 500 mg Palladium-Kohle (10%) analog Beispiel 37a erhalten und durch Flash-Chromatographie mit System N9 gereinigt. $R_f$(N9) = 0,29.

d) Z-Ile-4-(carbamoylmethyl)-2-thiazolylamid: 300 mg Z-Ile-4-(äthoxycarbonylmethyl)-2-thiazolylamid werden in 20 ml einer 5N Lösung von Ammoniak in Methanol gelöst und 24 Std. bei Raumtemperatur stehen gelassen. Das Reaktionsgemisch wird am Rotationsverdampfer eingeengt und die Titelverbindung durch Flash-Chromatographie mit System N11 erhalten. $R_f$(N11) = 0,33.

e) Z-Ile-4-(äthoxycarbonylmethyl)-2-thiazolylamid wird analog Beispiel 1 ausgehend von 2,23 g Z-Ile-OH-Dicyclohexylaminsalz, 930 mg (2-Amino-4-thiazolyl)-essigsäureäthylester, 842 mg HOBt und 1,34 g DCCI erhalten und durch Flash-Chromatographie (250 g Kieselgel, System N10) gereinigt. $R_f$(N10) = 0,53.

Beispiel 52: Z-Phe-His-Leu$\overset{c}{-}$Val-Ile-(m-carbamoylphenyl)-methylamid

Analog Beispiel 1 wird ausgehend von 127 mg Z-Phe-His-OH, 107 mg H-Leu$\overset{c}{-}$Val-Ile-(m-carbamoylphenyl)-methylamid, 45 mg HOBt und 70 mg DCCI nach Flash-Chromatographie (65 g Kieselgel 60, 40-63 µm, Fliessmittelsystem B7) die Titelverbindung erhalten. $R_f$(B7) = 0,20.

Das Ausgangsmaterial wird folgendermassen hergestellt:

a) H-Leu$\overset{c}{-}$Val-Ile-(m-carbamoylphenyl)-methylamid wird durch Hydrierung von 230 mg Z-Leu$\overset{cx}{-}$Val-Ile-(m-carbamoylphenyl)-methylamid in Gegenwart von 40 mg Palladium-Kohle (10%) analog Beispiel 37a erhalten und durch Flash-Chromatographie mit System B7 gereinigt. $R_f$(B7) = 0,11; $R_f$(B9) = 0,37.

b) Z-Leu$\overset{cx}{-}$Val-Ile(m-carbamoylphenyl)-methylamid wird analog Beispiel 1 ausgehend von 164 mg Z-Leu$\overset{cx}{-}$Val-OH, 160 mg L-Isoleucin-(m-carbamoylphenyl)-methylamid 93 mg HOBt und 126 mg DCCI erhalten und durch Flash-Chromatographie mit System N11 gereinigt. $R_f$(N11) = 0,39.

c) L-Isoleucin-(m-carbabmoylphenyl)-methylamid wird durch Hydrierung von 250 mg Z-Ile-(m-carbamoylphenyl)-methylamid in Gegenwart von 40 mg Palladium-Kohle (10%) analog Beispiel 37a erhalten und durch Flash-Chromatographie mit System B7 gereinigt. $R_f$(B7) = 0,27; $R_f$(B11) = 0,56.

d) Z-Ile-(m-carbamoylphenyl)-methylamid wird analog Beispiel 1 ausgehend von 1,78 g Z-Ile-OH-Dicyclohexylaminsalz, 600 mg 3-Aminomethyl-benzoesäureamid, 673 mg HOBt und 1071 mg DCCI erhalten und durch Flash-Chromatographie (220 g Kieselgel, System N11) gereinigt. $R_f$(N11) = 0,37.

<u>Beispiel 53: Z-Phe-His-Leu$\overset{c}{-}$Val-Ile-dicarbamoyl-methylamid</u>

Analog Beispiel 1 wird ausgehend von 82 mg Z-Phe-His-OH, 64 mg H-Leu$\overset{c}{-}$Val-Ile-dicarbamoyl-methylamid, 29 mg HOBt und 45 mg DCCI nach Flash-Chromatographie (65 g Kieselgel 60, 40-63 µm, Fliessmittelsystem B9) die Titelverbindung erhalten. $R_f$(B9) = 0,37; $R_f$(B11) = 0,46.

Das Ausgangsmaterial wird folgendermassen hergestellt:

a) H-Leu$\stackrel{c}{-}$Val-Ile-dicarbamoyl-methylamid wird durch Hydrierung von 150 mg Z-Leu$\stackrel{cx}{-}$Val-Ile-dicarbamoyl-methylamid in Gegenwart von 30 mg Palladium-Kohle (10%) analog Beispiel 37a erhalten und durch Flash-Chromatographie mit System B11 gereinigt. $R_f$(B11) = 0,28.

b) Z-Leu$\stackrel{cx}{-}$Val-Ile-dicarbamoyl-methylamid wird analog Beispiel 1 ausgehend von 150 mg Z-Leu$\stackrel{cx}{-}$Val-OH, 130 mg L-Isoleucin-dicarbamoyl-methylamid, 85 mg HOBt und 115 mg DCCI erhalten und durch Flash-Chromatographie mit System N11 gereinigt. $R_f$(N11) = 0,25.

c) L-Isoleucin-dicarbamoyl-methylamid wird durch Hydrierung von 560 mg Z-Ile-dicarbamoyl-methylamid in Gegenwart von 100 mg Palladium-Kohle (10%) analog Beispiel 37a erhalten und durch Flash-Chromatographie mit System B11 gereinigt. $R_f$(B11) = 0,22.

d) Z-Ile-dicarbamoyl-methylamid wird durch Reaktion von 1,00 g Z-Ile-bis(methoxycarbonyl)-methylamid mit 50 ml einer 5N Lösung von Ammoniak in Methanol bei 40° in 2 Std. erhalten und durch Abfiltrieren des Niederschlags und Trocknung am Hochvakuum isoliert. $R_f$(N9) = 0,48.

e) Z-Ile-bis(methoxycarbonyl)-methylamid wird analog Beispiel 1 ausgehend von 5,00 g Z-Ile-OH-Dicyclohexylaminsalz, 2,1 g Aminomalonsäuredimethylester-hydrochlorid, 1,72 g HOBt und 3,00 g DCCI erhalten und durch Flash-Chromatographie (250 g Kieselgel, System N6) gereinigt. $R_f$(N6) = 0,48; $R_f$(N10) = 0,66.

Beispiel 54: Z-Phe-His-Leu$\stackrel{c}{-}$Val-Ile-Sta-$NH_2$

Analog Beispiel 1 wird ausgehend von 125 mg Z-Phe-His-OH, 110 mg H-Leu$\stackrel{c}{-}$Val-Ile-Sta-$NH_2$, 44 mg HOBt und 68 mg DCCI nach Flash-Chromatographie (65 g Kieselgel 60, 40-63 µm, Fliessmittelsystem B7) die Titelverbindung erhalten. $R_f$(B7) = 0,20; $R_f$(B11) = 0,64.

Das Ausgangsmaterial wird folgendermassen hergestellt:

a) H-Leu$\overset{c}{-}$Val-Ile-Sta-$NH_2$ wird durch Hydrierung von 250 mg Z-Leu$\overset{cx}{-}$Val-Ile-Sta-$NH_2$ in Gegenwart von 50 mg Palladium-Kohle (10%) analog Beispiel 37a erhalten und durch Flash-Chromatographie mit System B7 gereinigt. $R_f$(B7) = 0,29.

b) Z-Leu$\overset{cx}{-}$Val-Ile-Sta-$NH_2$ wird analog Beispiel 1 ausgehend von 160 mg Z-Leu$\overset{cx}{-}$Val-OH, 136 mg H-Ile-Sta-$NH_2$,67 mg HOBt und 106 mg DCCI erhalten und durch Flash-Chromatographie mit System N11 gereinigt. $R_f$(N11) = 0,33.

c) H-Ile-Sta-$NH_2$ wird durch Hydrierung von 700 mg Z-Ile-Sta-$NH_2$ in Gegenwart von 100 mg Palladium-Kohle (10%) analog Beispiel 37a erhalten und durch Flash-Chromatographie mit System B7 gereinigt. $R_f$(B7) = 0,20.

d) Z-Ile-Sta-$NH_2$ wird analog Beispiel 1 ausgehend von 983 mg Z-Ile-OH-Dicyclohexylaminsalz, 348 mg Statinamid, 367 mg HOBt und 536 mg DCCI erhalten und durch Flash-Chromatographie (160 g Kiesgel, System N11) gereinigt. $R_f$(N11) = 0,38.

e) Statinamid wird ausgehend von 900 mg Z-Sta-$NH_2$ durch Hydrierung in Gegenwart von 120 mg Palladium-Kohle (10%) analog Beispiel 4 erhalten und durch Flash-Chromatographie mit System S9 gereinigt. $R_f$(S9) = 0,23.

f) Z-Sta-$NH_2$ wird durch Ammonolyse von 1,00 g Z-Sta-$OCH_3$ (hergestellt wie beschrieben in der Europäischen Patentanmeldung 111'266) in 20 ml 5N Ammoniak-Lösung in Methanol bei Raumtemperatur in 24 Std. erhalten. Die Reaktionslösung wird eingeengt und die Titelverbindung durch Flash-Chromatographie mit System S6 gereinigt. $R_f$(S6) = 0,15.

Beispiel 55: Z-Phe-His-Leu$\overset{c}{-}$Val-Ile-His-Lys(BOC)-OMe

375 mg Z-Phe-His-OH, 375 mg H-Leu$\overset{c}{-}$Val-Ile-His-Lys(BOC)-OMe und 180 mg N-Hydroxy-5-norbornen-2,3-dicarbonsäureimid werden in 5 ml DMF gelöst, auf 0° gekühlt und mit 200 mg DCCI versetzt. Man lässt während ca. 15 Std. bei 0° und 24 Std. bei Raumtemperatur stehen, filtriert den auskristallisierten DCH ab und engt das Filtrat im Hochvakuum bei 40° zur Trockne ein. Der Rückstand wird in 10 ml Methanol-Eisessig-$H_2O$ (94:3:3) während 1 Std. auf 60° erwärmt, wieder zur Trockne eingeengt und zur Vorreinigung einer Craig-Verteilung über 800 Stufen im System Methanol-Acetatpuffer-Aethylenchlorid-Chloroform 10:3:8:4 (Acetatpuffer: 14,3 ml Eisessig und 9,6 g Ammoniumacetat in 1000 ml Wasser) unterworfen. Die das Kondensationsprodukt enthaltenden Fraktionen (K-Wert = 0,70) werden vereinigt und zur Trockene eingeengt. Ein in der Craig-Verteilung nicht abtrennbares Nebenprodukt wird anschliessend noch durch Chromatographie an einer Säule mit 30 g Kieselgel (Merck Nr. 60, 40-63 μm) durch Elution mit Chloroform-Methanol-$H_2O$-Eisessig (150:54:10:1) entfernt. Die chromatographisch reinen Fraktionen werden vereinigt und zur Trockene eingedampft, wobei die Titelverbindung als amorphes Pulver anfällt; $R_f$(B17) = 0,41; $R_f$(S4) = 0,43.

Die Ausgangsmaterialien werden folgendermassen hergestellt:

a) H-His-Leu$\overset{c}{-}$Val-Ile-His-Lys(BOC)-OMe: 465 mg Z-Leu$\overset{cx}{-}$Val-Ile-His-Lys(BOC)-OMe werden in 10 ml Methanol/Wasser (95:5) gelöst und nach Zugabe von 50 mg Palladium-Kohle (10% Pd) unter $CO_2$-Absorption bis zur Sättigung hydriert. Der Katalysator wird abfiltriert, das Filtrat zur Tockene eingeengt, pulverisiert und im Hochvakuum bei 40° nachgetrocknet. Man erhält die Titelverbindung in amorpher, chromatographisch einheitlicher Form. $R_f$(B17) = 0,25; $R_f$(B21) = 0,6.

b) Z-Leu$\overset{cx}{-}$Val-Ile-His-Lys(BOC)-OMe: 300 mg Z-Leu$\overset{cx}{-}$Val-OH, 454 mg H-Ile-His-Lys(BOC)-OMe (hergestellt wie beschrieben in der Europäischen Patentanmeldung 111'266) und 136 mg HOBt werden in 5 ml DMF gelöst, auf 0° gekühlt und mit 198 mg DCCI versetzt. Nach kurzem Rühren lässt man über Nacht bei 0° und weitere 24 Std. bei Raumtemperatur stehen und filtriert dann den auskristallisierten DCH ab. Das Filtrat wird im Hochvakuum bei 40° eingeengt und der ölige Rückstand in 12 ml Methanol-Eisessig-$H_2O$ (94:3:3) gelöst und während 1 Std. auf 60° erwärmt. Die Lösung wird wiederum zur Trockne eingeengt und der Rückstand durch Craig-Verteilung im System Methanol-Acetatpuffer-Aethylenchlorid-Chloroform 10:3:8:4 (Acetatpuffer: 14,3 ml Eisessig und 9,6 g Ammoniumacetat in 1000 ml Wasser) über 800 Stufen gereinigt. Die dünnschichtchromatographisch reinen Fraktionen (K = 0,26) werden vereinigt, zur Trockne eingeengt, pulverisiert und im Hochvakuum bei 40° nachgetrocknet.
$R_f$(B17) = 0,70; $R_f$(S4) = 0,75.

Beispiel 56: Z-Phe-His-Leu$\overset{c}{-}$Val-Ile-His-Lys-OMe

80 mg Z-Phe-His-Leu$\overset{c}{-}$Val-Ile-His-Lys(BOC)-OMe (Beispiel 55) werden in 800 µl 95% ige Trifluoressigsäure gelöst (Dauer ca. 1 Min.) und während 2 Min. stehengelassen. Man fällt sodann das Produkt durch Zugabe von 8 ml Diisopropyläther aus, lässt 15 Min. bei 0° stehen, filtriert ab, wäscht den Niederschlag mit Diisopropyläther und trocknet über KOH im Hochvakuum. Man löst sodann das Pulver in 1 ml 90%igem Trifluoräthanol, fällt durch Zugabe von 10 ml 3%iger $NaHCO_3$-Lösung aus, filtriert ab, wäscht mit $H_2O$ und trocknet über Kaliumhydroxid und Phosphorpentoxid am Hochvakuum. Zur Umwandlung der so erhaltenen säurefreien Titelverbindung in das Acetat wird das Produkt noch in 2 ml 90%-iger Essigsäure gelöst und lyophilisiert. Die Titelverbindung ist ein chromatographisch einheitliches, amorphes Pulver; $R_f$(B17) = 0,08; $R_f$(B21) = 0,65.

Beispiel 57: Z-Arg-His-Leu$\overset{c}{-}$Val-Ile-His-$NH_2$

141 mg Z-Arg-His-OH (enthaltend ca. 20% NaCl), 72 mg H-Leu$\overset{c}{-}$Val-Ile-His-$NH_2$ (Beispiel 1i) und 30 mg HOBt werden in 1,5 ml DMF gelöst, auf 0° gekühlt, mit 46 mg DCCI versetzt und während 15 Std. bei 0°

und 1 Tag bei 24° stehengelassen. Das Unlösliche (DCH und NaCl) wird abfiltriert, das Filtrat zur Trockne eingedampft, in 3 ml Methanol-Eisessig-Wasser (94:3:3) 1 Std. auf 60° erwärmt, wieder eingeengt, in 3 ml Methanol/0,1 N Essigsäure (1:1) gelöst und langsam durch eine Säule (∅ = 1 cm, Länge = 12 cm) von schwach basischem Ionenaustauscher in der Acetatform filtriert. Der Trockenrückstand des Eluates wird einer Craig-Verteilung im System n-Butanol-Eisessig-Wasser (4:1:5) über 600 Stufen unterworfen. Die chromatographisch reinen Fraktionen (K-Wert ca. 0,3) werden vereinigt, zur Trockne eingeengt, in Wasser gelöst und lyophilisiert. $R_f$(S11) = 0,21; $R_f$(S5) = 0,20; $R_f$(B21) = 0,47.

Die Ausgangsmaterialien werden folgendermassen hergestellt:

a) Z-Arg-His-OH wird durch Hydrolyse von 1,84 g Z-Arg-His-OMe-Hydrochlorid in 18 ml Wasser und 7,2 ml 1N NaOH in 2 Min. bei 26° und nachfolgender Neutralisierung mit 7,2 ml 1N HCl und Lyophilisierung erhalten. $R_f$(S5) = 0,25; $R_f$(B21) = 0,47.

b) Z-Arg-His-OMe: 770 mg Z-Arg-OH, 500 mg L-Histidin-methylester-dihydrochlorid, 230 µl N-Methlymorpholin und 350 mg HOBt werden in 5 ml DMF unter Erwärmen gelöst, auf 20° gekühlt und mit 600 mg DCCI in fester Form versetzt. Die Lösung wird 6 Std. bei 20° stehengelassen, 1 Std. bei 0° gerührt, vom kristallinen DCH abfiltriert und das Filtrat zur Trockne eingeengt. Der halbfeste Rückstand wird in 5 ml Methanol-Eisessig-Wasser (93:3:3) während 1 Std. auf 60° erwärmt und dann wieder zur Trockne eingedampft. Zur Reinigung wird an einer Säule mit 25 g Kieselgel (Merck Nr. 60, 40-63 µm) chromatographiert. Die Titelverbindung wird als Hydrochlorid durch Elution mit Chloroform-Methanol (75:25) gewonnen. $R_f$(B17) = 0,13; $R_f$(B21) = 0,55.

Beispiel 58: Z-Arg-Phe-Leu$^{c}$Val-Ile-His-$NH_2$

130 mg Z-Arg-Phe-OH, 91 mg H-Leu$^{c}$Val-Ile-His-$NH_2$ (Beispiel 1i) und 38 mg HOBt werden in 1,4 ml DMF gelöst. Nach Zugabe von 57 µl einer 5N Lösung von HCl in Dioxan wird auf 0° gekühlt und dann 58 mg DCCI zugegeben. Man lässt während 20 Std. bei 0° und 2 Tage bei 25° stehen, filtriert den DCH bei 0° ab, engt das Filtrat zur Trockne ein, erwärmt den in 4 ml Methanol-Eisessig-Wasser (94:3:3) gelösten Rückstand während 1 Std. auf 60° und dampft wieder zur Trockne ein. Das Rohprodukt wird an einer Säule mit 20 g Kieselgel (Merck Nr. 60, 40-63 µm) im Lösungsmittelgemisch Chloroform-Methanol-Wasser-Eisessig (150:54:10:1) chromatographiert. Die reinen Fraktionen werden zur Trockne eingedampft, in $H_2O$ gelöst und lyophilisiert. $R_f$(S11) = 0,35; $R_f$(B17) = 0,23; $R_f$(S5) = 0,52.

Die Ausgangsmaterialien werden folgendermassen hergestellt:

a) Z-Arg-Phe-OH: 535 mg Z-Arg-Phe-OMe-Hydrochlorid werden in 20 ml Methanol-$H_2O$ (1:1) gelöst, mit 16,1 ml 0,1N NaOH versetzt, während 30 Min. gerührt und dann mit 16,1 ml 0,1N HCl neutralisiert. Die Lösung wird auf 8-10 ml eingeengt und lyophilisiert, der Rückstand in 5 ml Trifluoräthanol gelöst, vom unlöslichen NaCl abfiltriert und das Filtrat zur Trockne eingeengt. Die Titelverbindung vom Smp. 193-195° wird durch Kristallisation aus Methanol-Wasser erhalten. $R_f$(S11) = 0,48; $R_f$(S5) = 0,43.

b) Z-Arg-Phe-OMe: 1,71 g Z-Arg-OH, 1,0 g L-Phenylalanin-methylester-hydrochlorid und 0,85 g HOBt werden in 10 ml DMF unter leichtem Erwärmen gelöst und dann bei Raumtemperatur mit 1,34 g DCCI versetzt. Man rührt kurz und lässt während 6 Std. stehen. Der auskristallisierte DCH wird abfiltriert, das Filtrat zur Trockne eingedampft und der Rückstand in 20 ml Chloroform-Methanol (9:1) gelöst und an einer Säule von 75 g Kieselgel (Merck Nr. 60, 40-63 µm, Fliessmittel Chloroform-Methanol-Wasser-Eisessig 180:20:2:1),

gereinigt. Die chromatographisch reinen Fraktionen werden zur Trockne eingedampft, in $H_2O$ gelöst und lyophilisiert, nochmals in 0,2N HCl gelöst und erneut lyophilisiert, was die Titelverbindung als Hydrochlorid ergibt. $R_f$(S11) = 0,55; $R_f$(B17) = 0,39.

Beispiel 59: Z-His-His-Leu$^c$-Val-Ile-His-$NH_2$

Analog Beispiel 1 wird ausgehend von 23 mg Z-His-OH, 50 mg H-His-Leu$^c$-Val-Ile-His-$NH_2$•2HCl, 11 mg HOBt, 24,6 µl Aethyldiisopropylamin und 21 mg DCCI nach Flash-Chromatographie (32 g Kieselgel 60, 40-63 µm, Fliessmittelsystem Methylenchlorid/Methanol/Wasser/Eisessig 280:160:40:2) die Titelverbindung als graues Harz erhalten. $R_f$(B13) = 0,40.

Das Ausgangsmaterial wird folgendermassen hergestellt:

a) H-His-Leu$^c$-Val-Ile-His-$NH_2$•2HCl wird erhalten analog Beispiel 4 durch Hydrierung von 923 mg Z-His-Leu$^c$-Val-Ile-His-$NH_2$•2HCl in Gegenwart von 100 mg Palladium-Kohle (10%). Das Rohprodukt wird aus Methanol/Essigester (3:1) umkristallisiert, wobei die Titelverbindung in weissen Kristallen anfällt. $R_f$(B13) = 0,16.

b) Z-His-Leu$^c$-Val-Ile-His-$NH_2$ wird erhalten analog Beispiel 1 ausgehend von 662 mg Z-His-OH, 1000 mg H-Leu$^c$-Val-Ile-His-$NH_2$, 318 mg HOBt und 601 mg DCCI. Das Rohprodukt wird in 30 ml Methylenchlorid/ Methanol 1:1 aufgerührt und abfiltriert, dann in 20 ml Methanol und 3,67 ml 1N HCl bei Eisbadkühlung verrührt. Nach Abifltrieren von ungelöstem DCH wird das Filtrat auf 6 ml eingeengt, mit 35 ml warmem Essigester versetzt und im Eisbad gerührt, wobei die Titelverbindung in weissen Kristallen anfällt. Weitere Mengen der Titelverbindung werden aus dem eingedampften Filtrat der Methylenchlorid-Methanol-Aufschlämmung durch Flash-Chromatographie (70 g Kieselgel 60, 40-63 µm, Fliessmittelsystem Methylenchlorid/Methanol/Ammoniak konz. 1000:10:1) erhalten. $R_f$(B11) = 0,22.

Das Dihydrochlorid wird erhalten, indem man die Titelverbindung in Methanol löst und mit 2,0 Aequivalenten 1N HCl versetzt.

Beispiel 60: Z-Phe-Arg-Leu$\overset{c}{-}$Val-Ile-His-$NH_2$

Analog Beispiel 1 wird ausgehend von 69 mg Z-Phe-Arg-OH, 50 mg H-Leu$\overset{c}{-}$Val-Ile-His-$NH_2$, 16 mg HOBt und 40 mg DCCI wird die Titelverbindung nach Flash-Chromatographie (45 g Kieselgel 60, 40-63 µm, Fliessmittel-System Methylenchlorid/Methanol/Wasser/Eisessig 330:100:19:2), als farbloses Pulver erhalten. $R_f$(S3) = 0,16.

Das Ausgangsmaterial wird folgendermassen hergestellt:

a) Z-Phe-Arg-OH: 2,09 g Z-Phe-Arg-$OCH_3$ (Bachem AG, Bubendorf, Schweiz) werden in 80 ml Methanol/Wasser 1:1 mit 6,67 ml wässriger 1N NaOH während 3 Std. bei Raumtemperatur gerührt. Nach Zugabe von 6,67 ml wässriger 1N HCl wird zur Trockne eingedampft. Der Rückstand wird in wenig Methanol aufgerührt, NaCl abfiltriert und das Filtrat eingedampft, wobei die Titelverbindung als farbloses Pulver erhalten wird. $R_f$(B1) = 0,26.

Beispiel 61: Z-Glu-(O-tert-butyl)-His-Leu$\overset{c}{-}$Val-Ile-His-$NH_2$

Analog Beispiel 1 wird ausgehend von 64 mg Z-Glu-(O-tert-butyl)-OH, 120 mg H-His-Leu$\overset{c}{-}$Val-Ile-His-$NH_2$•2HCl, 0,104 ml Aethyldiisopropylamin, 29 mg HOBt und 47 mg DCCI die Titelverbindung nach Mitteldruckchromatographie (LOBAR®-Fertigsäule, Merck, Grösse B, 40-60 µm, System B8) als farbloses Pulver erhalten. $R_f$(B11) = 0,43.

Beispiel 62: Z-Phe-Phe-Leu$\overset{c}{-}$Val-tris-(tert-butyldimethylsilyloxymethyl)-methylamid

Analog Beispiel 1 wird ausgehend von 22 mg Z-Phe-Phe-OH, 31 mg H-Leu$\overset{c}{-}$Val-tris-(tert-butyldimethylsilyloxymethyl)-methylamid, 9 µl Aethyldiisopropylamin, 8 mg HOBt und 12 mg DCCI nach Mitteldruckchromatographie (LOBAR®-Fertigsäule, Merck, Grösse B, 40-60 µm, System B8) die Titelverbindung als gelbliches Oel erhalten.

Das Ausgangsmaterial wird folgendermassen hergestellt:

a) H-Leu$\overset{c}{-}$Val-tris-(tert-butyldimethylsilyloxymethyl)-methylamid wird erhalten analog Beispiel 4 durch Hydrierung von 139 mg Z-Leu$\overset{cx}{-}$Val-tris-(tert-butyldimethylsilyloxymethyl)-methylamid in Gegenwart von 50 mg Palladium-Kohle (5%) und Mitteldruckchromatographie (LOBAR®-Fertigsäule, Grösse B, 40-60 µm, System Methylenchlorid/Methanol/Ammoniak konz. 175:10:1) als farbloses Oel. $R_f$(B8) = 0,41.

b) Z-Leu$\overset{cx}{-}$Val-tris-(tert-butyldimethylsilyloxymethyl)-methylamid wird hergestellt durch Kondensation von 250 mg Z-Leu$\overset{cx}{-}$Val-OH mit 286 mg Tris-(tert-butyldimethylsilyloxymethyl)-methylamin (Beispiel 19c) und 106 µl Aethyldiisopropylamin, 94 mg HOBt und 165 mg DCCI analog Beispiel 1. Nach Mitteldruckchromatographie (LOBAR®-Fertigsäule, Grösse B, 40-60 µm, System N5) wird die Titelverbindung als farbloses Oel erhalten.

Beispiel 63: Z-Phe-Phe-Leu$\overset{c}{-}$Val-tris-(hydroxymethyl)-methylamid

39 mg Z-Phe-Phe-Leu$\overset{c}{-}$Val-tris-(tert-butyldimethylsilyloxymethyl)-methylamid (Beispiel 62) werden während 3 Std. in 3 ml Essigsäure/$H_2O$ 2:1 gerührt. Nach Abdampfen des Lösungsmittels und Mitteldruckchromatographie (LOBAR®-Fertigsäule, Grösse B, 40-60 µm, System B8) wird die Titelverbindung als farbloses Pulver erhalten. $R_f$(B8) = 0,36.

Beispiel 64: Z-Phe-His-Leu$\frac{c}{}$Gly(phenyl)-Ile-His-$NH_2$ (Diastereomer I)

49 mg Z-Phe-His-OH, 53 mg H-Leu$\frac{c}{}$Gly(phenyl)-Ile-His-$NH_2$ (Diastereomer I) und 17 mg HOBt werden bei Eisbadtemperatur in 2 ml DMF gelöst. Nach Zugabe von 28 mg DCCI wird während 8 Std. bei Eisbadtemperatur und anschliessend während 40 Std. bei Raumtemperatur gerührt. Nach Abdampfen des Lösungsmittels im Hochvakuum und Mitteldruckchromatographie (LOBAR®-Fertigsäule, Grösse B, 40-60 µm, System B9) wird die Titelverbindung als farbloses Pulver erhalten. $R_f$(B11) = 0,42.

Die Ausgangsmaterialien werden folgendermassen hergestellt:

a) H-Leu$\frac{c}{}$Gly(phenyl)-Ile-His-$NH_2$ (Diastereomer I) wird erhalten analog Beispiel 4 durch Hydrierung von 160 mg Z-Leu$\frac{cx}{}$Gly(phenyl)-Ile-His-$NH_2$ (Diastereomer I) in Gegenwart von 50 mg Palladium-Kohle (10%) nach Mitteldruckchromatographie (LOBAR®-Fertigsäule, Grösse B, 40-60 µm, System B10) als farbloses Pulver. $R_f$(B11) = 0,35.

b) Z-Leu$\frac{cx}{}$Gly(phenyl)-Ile-His-$NH_2$ (Diastereomer I) wird analog Beispiel 1f ausgehend von 205 mg Z-Leu$\frac{cx}{}$Gly(phenyl)-OH (Diastereomer I), 124 mg H-Ile-His-$NH_2$, 71 mg HOBt und 125 mg DCCI hergestellt und nach Mitteldruckchromatographie (LOBAR®-Fertigsäule, Grösse B, 40-60 µm, System B8) als farbloses Pulver erhalten. $R_f$(B9) = 0,36.

c) Z-Leu$\frac{cx}{}$Gly(phenyl)-OH (Diastereomer I und II) wird durch Hydrolyse von 1,56 g Z-Leu$\frac{cx}{}$Gly(phenyl)-$OCH_3$ (Diastereomerengemisch) mit 124 mg Wasser und 966 mg Kalium-tert-butylat in Aether analog Beispiel 1e erhalten und durch Mitteldruckchromatographie (240 g Lichroprep® Si 60, 25-40 µm, System N3) die Diastereomeren getrennt. Diastereomer I: $R_f$(N3) = 0,24. Diastereomer II: $R_f$(N3) = 0,15.

d) Z-Leu$\frac{cx}{}$Gly(phenyl)-$OCH_3$ (Diastereomerengemisch) wird ausgehend von 0,723 ml Diisopropylamin, 3,3 ml Butyllithium, 767 mg Phenylessigsäuremethylester und 2,00 g der Verbindung XXV analog Beispiel 1d hergestellt und nach Mitteldruckchromatographie (LOBAR®-Fertigsäule, Grösse B, 40-60 µm, Fliessmittelsystem Essigester/Hexan 1:5) als farbloses Oel erhalten. $R_f$(N4) = 0,35 und 0,33.

Beispiel 65: Z-Phe-His-Leu$\overset{c}{-}$Gly(phenyl)Ile-His-$NH_2$ (Diastereomer II)

Analog Beispiel 64 wird ausgehend von 47 mg Z-Phe-His-OH, 50 mg H-Leu$\overset{c}{-}$Gly(phenyl)-Ile-His-$NH_2$ (Diastereomer II), 16 mg HOBt und 26 mg DCCI die Titelverbindung als farbloses Pulver erhalten. $R_f$(B11) = 0,44.

Die Ausgangsmaterialien werden folgendermassen hergestellt:

a) H-Leu$\overset{c}{-}$Gly(phenyl)-Ile-His-$NH_2$ (Diastereomer II) wird erhalten analog Beispiel 64a durch Hydrierung von 167 mg Z-Leu$\overset{cx}{-}$Gly(phenyl)-Ile-His-$NH_2$ (Diastereomer II) in Gegenwart von 50 mg Palladium-Kohle (10%). $R_f$(B11) = 0,24.

b) Z-Leu$\overset{cx}{-}$Gly(phenyl)-Ile-His-$NH_2$ (Diastereomer II) wird erhalten analog Beispiel 64b ausgehend von 289 mg Z-Leu$\overset{cx}{-}$Gly(phenyl)-OH (Diastereomer II, Beispiel 64c), 175 mg H-Ile-His-$NH_2$, 100 mg HOBt und 175 mg DCCI. $R_f$(B9) = 0,41.

Beispiel 66: Z-Phe-His-Leu$\overset{c}{-}$Gly(cyclohexyl)-Ile-His-$NH_2$ (Diastereomer I)

Ausgehend von 98 mg Z-Phe-His-OH, 106 mg H-Leu$\overset{c}{-}$Gly(cyclohexyl)-Ile-His-$NH_2$ (Diastereomer I), 34 mg HOBt und 55 mg DCCI wird analog Beispiel 1 nach Mitteldruckchromatographie (LOBAR®-Fertigsäule, Grösse B, 40-60 µm, System B9) die Titelverbindung als farbloses Pulver erhalten. $R_f$(B11) = 0,52.

Die Ausgangsmaterialien werden folgendermassen hergestellt:

a) H-Leu$\overset{c}{-}$Gly(cyclohexyl)-Ile-His-$NH_2$ (Diastereomer I) wird erhalten analog Beispiel 4 durch Hydrierung von 200 mg Z-Leu$\overset{cx}{-}$Gly(cyclohexyl)-Ile-His-$NH_2$ in Gegenwart von 50 mg Palladium-Kohle (10%) nach Mitteldruckchromatographie (31 g Lichroprep® Si 60, 25-40 µm, System B8) als farbloses Pulver. $R_f$(B11) = 0,39.

b) Z-Leu$\overset{cx}{-}$Gly(cyclohexyl)-Ile-His-$NH_2$ (Diastereomer I) wird analog Beispiel 1f ausgehend von 250 mg Z-Leu$\overset{cx}{-}$Gly(cyclohexyl)-OH (Diastereomer I), 150 mg H-Ile-His-$NH_2$, 86 mg HOBt und 150 mg DCCI hergestellt und nach Mitteldruckchromatographie (LOBAR®-Fertigsäule, Grösse B, 40-60 µm, System B7) als farbloses Pulver erhalten. $R_f$(B9) = 0,35.

c) Z-Leu$\overset{cx}{-}$Gly(cyclohexyl)-OH (Diastereomer I und II) wird durch Hydrolyse von 1,67 g Z-Leu$\overset{cx}{-}$Gly(cyclohexyl)-$OCH_3$ (Diastereomerengemisch) mit 131 mg Wasser und 898 mg Kalium-tert-butylat in Aether analog Beispiel 1e erhalten und durch Mitteldruckchromatographie (244 g Lichroprep® Si 60, 25-40 µm, System Essigester/Hexan 1:4) die Diastereomeren getrennt. Diastereomer I: $R_f$(N4) = 0,14. Diastereomer II: $R_f$(N4) = 0,11.

d) Z-Leu$\overset{cx}{-}$Gly(cyclohexyl)-$OCH_3$ (Diastereomerengemisch) wird ausgehend von 0,723 ml Diisopropylamin, 3,3 ml Butyllithium, 796 mg Cyclohexylessigsäuremethylester und 2,00 g der Verbindung XXV analog Beispiel 1d hergestellt und nach Mitteldruckchromatographie (244 g Lichroprep® Si 60, 25-40 µm, System N5) als farbloses Oel erhalten. $R_f$(N4) = 0,35 und 0,33.

Beispiel 67: Z-Phe-His-Leu$\overset{c}{-}$Gly(cyclohexyl)-Ile-His-$NH_2$ (Diastereomer II)

Analog Beispiel 66 wird ausgehend von 91 mg Z-Phe-His-OH, 99 mg H-Leu$\overset{c}{-}$Gly(cyclohexyl)-Ile-His-$NH_2$ (Diastereomer II), 32 mg HOBt und 51 mg DCCI die Titelverbindung als farbloses Pulver erhalten. $R_f$(B11) = 0,61.

Die Ausgangsmaterialien werden folgendermassen hergestellt:

a) H-Leu$\overset{c}{-}$Gly(cyclohexyl)-Ile-His-$NH_2$ (Diastereomer II) wird erhalten analog Beispiel 66a durch Hydrierung von 180 mg Z-Leu$\overset{cx}{-}$Gly(cyclohexyl)-Ile-His-$NH_2$ (Diastereomer II) in Gegenwart von 50 mg Palladium-Kohle (10%). $R_f$(B11) = 0,38.

b) Z-Leu$\overset{cx}{-}$Gly(cyclohexyl)-Ile-His-$NH_2$ (Diastereomer II) wird erhalten analog Beispiel 66b ausgehend von 250 mg Z-Leu$\overset{cx}{-}$Gly(cyclohexyl)-OH (Diastereomer II, Beispiel 66c), 150 mg H-Ile-His-$NH_2$, 86 mg HOBt und 150 mg DCCI. $R_f$(B9) = 0,47.

Beispiel 68: Z-Phe-His-Leu$\overset{c}{-}$Gly(n-octyl)-Ile-His-$NH_2$

Ausgehend von 112 mg Z-Phe-His-OH, 128 mg H-Leu$\overset{c}{-}$Gly(n-octyl)-Ile-His-$NH_2$, 39 mg HOBt und 62 mg DCCI wird analog Beispiel 1 die Titelverbindung nach Mitteldruckchromatographie (LOBAR®-Fertigsäule, Grösse B, 40-60 µm, System B8) als farbloses Pulver erhalten. $R_f$(B9) = 0,29.

Die Ausgangsmaterialien werden folgendermassen erhalten:

a) H-Leu$\overset{c}{-}$Gly(n-octyl)-Ile-His-$NH_2$ wird erhalten durch Hydrierung von 269 mg Z-Leu$\overset{cx}{-}$Gly(n-octyl)-Ile-His-$NH_2$ in Gegenwart von 40 mg Palladium-Kohle analog Beispiel 4 nach Mitteldruckchromatographie (LOBAR®-Fertigsäule, Grösse B, 40-60 µm, System B9) als farbloses Harz. $R_f$(B11) = 0,35.

b) Z-Leu$\overset{cx}{-}$Gly(n-octyl)-Ile-His-$NH_2$ wird analog Beispiel 1f ausgehend von 413 mg Z-Leu$\overset{cx}{-}$Gly(n-octyl)-OH, 232 mg H-Ile-His-$NH_2$, 133 mg HOBt und 233 mg DCCI hergestellt und nach Mitteldruckchromatographie (LOBAR®-Fertigsäule, Grösse B, 40-60 µm, System B7) als farbloses Pulver erhalten. $R_f$(B11) = 0,64 und 0,58 (Diastereomerengemisch).

c) Z-Leu$\overset{cx}{-}$Gly(n-octyl)-OH wird durch Hydrolyse von 827 mg Z-Leu$\overset{cx}{-}$Gly(n-octyl)-$OCH_3$ mit 85 mg Wasser und 569 mg Kalium-tert-butylat in Aether analog Beispiel 1c nach Mitteldruckchromatographie (LOBAR®-Fertigsäule, Grösse B, 40-60 µm, System N5) als farbloses Oel erhalten. $R_f$ (N4, zweimal entwickelt) = 0,20 und 0,15 (Diastereomerengemisch).

d) Z-Leu$\overset{cx}{-}$Gly(n-octyl)-$OCH_3$ wird ausgehend von 1,21 ml Diisopropylamin, 5,5 ml Butyllithium, 1595 mg Decansäuremethylester und 738 mg der Verbindung XXV analog Beispiel 1d hergestellt und nach Mitteldruckchromatographie (LOBAR®-Fertigsäule, Grösse B, 40-60 µm, System: Hexan 100%) als farbloses Oel erhalten.

<u>Beispiel 69:</u> <u>BOC-Phe-(N-methyl-Phe)-Leu$\overset{c}{-}$Val-methylamid</u>
Analog Beispiel 1 wird ausgehend von 72 mg BOC-Phe-(N-methyl-Phe)-OH, 41 mg H-Leu$\overset{c}{-}$Val-methylamid (Beispiel 34a), 26 mg HOBt und 45 mg DCCI in 2,5 ml DMF nach Flash-Chromatographie (35 g Kieselgel 60, 40-63 µm, Essigester/Hexan 4:1) die Titelverbindung als weisses amorphes Pulver erhalten, $R_f$(N10) = 0,37.

Das Ausgangsmaterial wird folgendergemassen hergestellt:

a) BOC-Phe-(N-methyl-Phe)-OH wird hergestellt durch Kondensation von BOC-Phe-OH mit N-Methyl-L-phenylalanin-methylester (Bachem AG, Bubendorf, Schweiz) analog der Vorschrift in Chem. Pharm. Bull. <u>26</u>, 3588 (1978) und anschliessender Hydrolyse des Methylesters mit NaOH analog Beispiel 15a.

Beispiel 70: BOC-Phe-(N-methyl-Phe)-Leu$\overset{c}{-}$Val-Ile-His-$NH_2$

Analog Beispiel 1 wird ausgehend von 47 mg BOC-Phe-(N-methyl-Phe)-OH, 48 mg H-Leu$\overset{c}{-}$Val-Ile-His-$NH_2$, 17 mg HOBt und 27 mg DCCI in 2 ml DMF nach Flash-Chromatographie (35 g Kieselgel 60, 40-63 µm, System B4) die Titelverbindung als weisses amorphes Pulver erhalten. $R_f$(B11) = 0,77, $R_f$(S4) = 0,46.

Beispiel 71: Analog der vorstehenden Beispiele können hergestellt werden:

Z-Phe-His-Leu$\overset{c}{-}$Val-Ile-tert-butylester, $R_f$(B4) = 0,40.

Ac-Phe-His-Leu$\overset{c}{-}$Val-Ile-His-$NH_2$, $R_f$(B11) = 0,24 und $R_f$(S4) = 0,10.

Z-Phe-His-Leu$\overset{c}{-}$Val-Ile-(N-äthoxycarbonyl-4-piperidyl)-amid, $R_f$(B7) = 0,31.

α-(Benzyloxycarbonylamino)-α-methoxycarbonyl-acetyl-Phe-His-Leu$\overset{c}{-}$Val-3-(2-pyrrolidinon-1-yl)-propylamid, $R_f$(B7) = 0,30.

Z-Phe-His-Leu$\overset{c}{-}$Val-Ala-bis-(2-hydroxyäthylaminocarbamoyl)-methylamid, $R_f$(B9) = 0,25 und $R_f$(11) = 0,57.

Z-Phe-His-Leu$\overset{c}{-}$Val-Ala-1-hydroxymethyl-2-(4-imidazolyl)-äthylamid, $R_f$(B9) = 0,2 und $R_f$(B11) = 0,47.

H-Sta-Phe-His-Leu$\overset{c}{-}$Val-Ile-His-$NH_2$, $R_f$(B13) = 0,25.

(2R,3R)-2,3-Dihydroxy-3-carboxy-propionyl-Phe-His-Leu$\overset{c}{-}$Val-Ile-His-$NH_2$, $R_f$(Methylenchlorid/Methanol/Ammoniak konz. 5:3:1) = 0,38.

Z-Phe-His-Leu$\overset{c}{-}$Val-carbamoyl-(3-methoxycarbonyl-2-hydroxy-1-isobutyl-propylaminocarbonyl)-methylamid, $R_f$(B7) = 0,28.

Z-Phe-His-Leu$\overset{c}{-}$Val-Gly-Gly-tris(hydroxymethyl)-methylamid, $R_f$(B11) = 0,30.

Z-Arg-Arg-Leu$\overset{c}{-}$Val-Ile-His-$NH_2$, $R_f$(S11) = 0,18.

Beispiel 72: Gelatine-Lösung

Eine sterilfiltrierte wässrige Lösung von Z-Phe-His-Leu$\overset{c}{-}$Val-Ile-His-$NH_2$ wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| Z-Phe-His-Leu$\overset{c}{-}$Val-Ile-His-$NH_2$ | 3 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel 73: Sterile Trockensubstanz zur Injektion

Man löst 5 mg Z-Phe-His-Leu$\overset{c}{-}$Val-Ile-His-$NH_2$ in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 74: Nasenspray

In einer Mischung von 3,5 ml "Myglyol 812" und 0,08 g Benzylalkohol werden 500 mg fein gemahlenes ($<5,0$ µm) Z-Phe-His-Leu$\overset{c}{-}$Val-Ile-His-$NH_2$ suspendiert. Diese Suspension wird in einen Behälter mit Dosierventil eingefüllt. Es werden 5,0 g "Freon 12" unter Druck durch das Ventil in den Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

Patentansprüche für die Vertragsstaaten BE, CH, LI, DE, FE, GB, IT, LU, NL, SE

1. Substituierte 5-Amino-4-hydroxyvalerylderivate der Formel

$$R_1-X_1-X_2-\underset{\displaystyle R_3}{\underset{|}{\overset{\displaystyle R_2}{\overset{|}{N}}}}-CH-\overset{\displaystyle R_4}{\overset{|}{CH}}-CH_2-\overset{\displaystyle R_5}{\overset{|}{CH}}-\overset{O}{\overset{\|}{C}}-R_6 \qquad (I),$$

worin $R_1$ Wasserstoff oder Acyl, $X_1$ einen gegebenenfalls N-alkylierten natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$ verbunden ist, $R_2$ Wasserstoff oder Niederalkyl, $R_3$ Wasserstoff, Alkyl, Cycloalkyl, Arylniederalkyl oder Aryl, $R_4$ Hydroxy oder veräthertes oder verestertes Hydroxy, $R_5$ Alkyl, Cycloalkyl, Arylniederalkyl oder Aryl und $R_6$ substituiertes oder freies Amino oder substituiertes Hydroxy darstellen, mit Ausnahme der Verbindungen, worin $R_1$ einen gegebenenfalls N-acylierten Rest der Aminosäure L-Prolin oder worin $X_1$ und $X_2$ zusammen -Val-Val-, -Gly-Gly- oder -Tyr-Gly- bedeuten, ferner Salze von solchen Verbindungen mit salzbildenden Gruppen.

2. Verbindungen gemäss Anspruch 1 der Formel I, worin $R_1$ Wasserstoff oder Acyl mit den Teilformeln $R^b$-CO- oder $R^a$-O-CO-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen oder einen unsubstituierten oder substituierten, fünf- oder sechsgliedrigen Heterocyclus darstellt und $R^b$ Wasserstoff bedeutet oder

die Bedeutungen von $R^a$ hat, $X_1$ einen natürlichen Aminosäurerest, der N-terminal mit $R_1$ und C-terminal mit $X_2$ verbunden ist, $X_2$ einen gegebenenfalls N-alkylierten natürlichen Aminosäurerest, N-terminal mit $X_1$ und C-terminal mit der Gruppe $-NR_2$-verbunden ist, $R_2$ Wasserstoff oder Niederalkyl, $R_3$ Wasserstoff, Niederalkyl, Cycloalkyl, Arylniederalkyl oder Aryl, $R_4$ Hydroxy, $R_5$ Alkyl, Cycloalkyl, Arylniederalkyl oder Aryl, und $R_6$ Amino oder eine Amino-oder Hydroxygruppe, welche durch einen oder gegebenenfalls zwei unsubstituierte oder substituierte, gesättigte oder ungesättigte, aliphatische Kohlenwasserstoffreste bis einschliesslich 18, bevorzugt bis einschliesslich 10, C-Atomen oder einen aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer natürlichen α-Aminosäure oder einen N-terminal mit der Gruppe -CO- verbundenen und C-terminal gegebenenfalls amidierten oder veresterten Di- oder Tripeptidrest, der aus natürlichen α-Aminosäuren und gewünschtenfalls aus Statin besteht, darstellen, mit Ausnahme der Verbindungen, worin $R_1$ einen gegebenenfalls N-acylierten Rest der Aminosäure L-Prolin oder worin $X_1$ und $X_2$ zusammen -Val-Val-, -Gly-Gly- oder -Tyr-Gly- bedeuten, ferner pharmazeutisch annehmbare Salze von Verbindungen der Formel I mit salzbildenden Gruppen.

3. Verbindungen gemäss Anspruch 1 der Formel I, worin $R_1$ Wasserstoff, Niederalkanoyl mit 1-7 C-Atomen, Hydroxyniederalkanoyl, Niederalkoxyniederalkanoyl, durch einem gewünschtenfalls acetylierten oder als Acetal geschützten natürlichen Zucker veräthertes Niederalkanoyl, Niederalkanoyloxyniederalkanoyl, Halogenniederalkanoyl, Hydroxysulfonyloxyniederalkanoyl, Carboxyniederalkanoyl, Niederalkoxycarbonylniederalkanoyl, Carbamoylniederalkanoyl, Niederalkylcarbamoylniederalkanoyl, Diniederalkylcarbamoylniederalkanoyl, Aminoalkanoyl mit 1-10 C-Atomen, Niederalkylaminoniederalkanoyl, Diniederalkylaminoniederalkanoyl, Niederalkanoylaminoalkanoyl,

Niederalkoxycarbonylaminoalkanoyl, Arylniederalkoxycarbonylaminoalkanoyl, substituiertes Niederalkanoylaminoniederalkanoyl, Mercaptoniederalkanoyl, Niederalkylthioniederalkanoyl, Oxoniederalkanoyl, Hydroxy-carboxy-niederalkanoyl, Hydroxy-niederalkoxycarbonyl-niederalkanoyl, verestertes Hydroxy-niederalkoxycarbonylniederalkanoyl, Dihydroxy-carboxy-niederalkanoyl, Dihydroxy-niederalkoxycarbonyl-niederalkanoyl, verestertes Dihydroxy-niederalkoxycarbonyl-niederalkanoyl, Hydroxy-amino-niederalkanoyl, Hydroxyacylamino-niederalkanoyl, Carboxy-amino-niederalkanoyl, Niederalkoxycarbonyl-amino-niederalkanoyl, Carboxy-acylamino-niederalkanoyl, Niederalkoxycarbonyl-acylamino-niederalkanoyl, Diaminoniederalkanoyl, Diacylaminoniederalkanoyl, Niederalkenoyl mit 3-7 C-Atomen, Niederalkinoyl mit 3-7 C-Atomen, Cycloalkylcarbonyl mit 4-9 C-Atomen, Bicycloalkylcarbonyl mit 6-11 C-Atomen, Tricycloalkylcarbonyl mit 9-11 C-Atomen, Monocycloalkenylcarbonyl mit 4-9 C-Atomen, Bicycloalkenylcarbonyl mit 6-11 C-Atomen, Cycloalkylniederalkanoyl mit 5-11 C-Atomen, Cycloalkylniederalkenoyl mit 6-11 C-Atomen, Cycloalkenylniederalkanoyl mit 5-11 C-Atomen, Hydroxycycloalkylcarbonyl, Carboxycycloalkylcarbonyl, Aminocycloalkylcarbonyl, Acylaminocycloalkylcarbonyl, Hydroxycycloalkenylcarbonyl, Benzoyl, unsubstituiert, mono- oder mehrfachsubstituiert durch Niederalkyl, Hydroxy, Niederalkoxy, Halo und/oder Nitro, Phenylniederalkanoyl, worin Phenyl unsubstituiert, mono- oder mehrfachsubstituiert durch Niederalkyl, Hydroxy, Niederalkoxy, Halo und/oder Nitro sein kann, substituiertes Anilinophenylacetyl, Phenylniederalkenoyl, 2- oder 3-Pyrrolylcarbonyl, 2-, 3- oder 4-Pyridylcarbonyl, 2-, 3- oder 5-Indolylcarbonyl, 2-, 3- oder 4-Chinolinylcarbonyl, 1-, 3- oder 4-Isochinolinylcarbonyl, Phenyl-hydroxy-niederalkanoyl, unsubstituiertes oder substituiertes Phenyl-amino-niederalkanoyl, unsubstituiertes oder substituiertes Phenyl-acylamino-niederalkanoyl, Pyrrolidinyl-3-carbonyl, Hydroxypyrrolidinylcarbonyl, Hydroxy-1-acylpyrrolidinylcarbonyl, Oxopyrrolidinylcarbonyl, Piperidinyl-2-, -3- oder -4-carbonyl, 1-Niederalkylpiperidinylcarbonyl, 1-Acylpiperidinylcarbonyl, Morpholinyl-2- oder -3-carbonyl, Thiomorpholinyl-2- oder -3-carbonyl, 1-und/oder

4-Niederalkylpiperazinylcarbonyl, ferner Niederalkoxycarbonyl, 2-Halogenniederalkoxycarbonyl, Arylniederalkoxycarbonyl, worin Aryl Phenyl, 1- oder 2-Naphthyl oder durch Niederalkyl, Niederalkoxy, Hydroxy, Halogen und/oder Nitro mono- oder mehrfach substituiertes Phenyl ist,

$X_1$ und $X_2$ unabhängig voneinander Ala, Arg, Asn, Asp, Cys, Gln, Glu, Gly, His, Ile, Leu, Lys, Met, Phe, Pro, Ser, Thr, Trp, Tyr oder Val, $-X_1-X_2-$ aber nicht -Val-Val-, -Gly-Gly- oder -Tyr-Gly-, und worin gegebenenfalls die Carboxylfunktion der Seitenkette in Asp und Glu mit einem Niederalkanol verestert und/oder die Aminofunktion der Seitenkette in Lys durch Niederalkanoyl, durch Niederalkoxycarbonyl oder durch Arylniederalkoxycarbonyl, acyliert ist, $X_1$ N-terminal mit $R_1$ und C-terminal mit $X_2$ und $X_2$ N-terminal mit $X_1$ und C-terminal mit $-NR_2$-verbunden ist und $X_2$ am N-Atom durch Niederalkyl substituiert sein kann,

$R_2$ Wasserstoff oder Niederalkyl,

$R_3$ Wasserstoff, Niederalkyl, Cycloalkyl, Phenylniederalkyl oder Phenyl,

$R_4$ Hydroxy,

$R_5$ Alkyl mit 1-10 C-Atomen, Cycloalkyl, Phenylniederalkyl oder Phenyl,

und $R_6$ Amino, Alkylamino mit 1 bis 10 C-Atomen, Diniederalkylamino, Hydroxyniederalkylamino, Di-(hydroxyniederalkyl)-amino, Niederalkoxyniederalkylamino, Niederalkanoyloxyniederalkylamino, Halogenniederalkylamino, Hydroxysulfonyloxyniederalkylamino, Phenyloxyniederalkylamino oder Phenyloxy-hydroxy-niederalkylamino, worin Phenyl gegebenenfalls durch Niederalkyl, Niederalkoxy, Hydroxy, Carboxy, Niederalkoxycarbonyl oder Carbamoyl substituiert ist, Carboxyalkylamino, Niederalkoxycarbonylalkylamino, physiologisch

spaltbares verestertes Carboxyalkylamino, Carbamoylniederalkylamino, Niederalkylcarbamoylniederalkylamino, Hydroxyniederalkylcarbamoylniederalkylamino, Triniederalkylsilyloxyniederalkylcarbamoylniederalkylamino, Niederalkoxycarbonyl-hydroxy-niederalkylcarbamoylniederalkylamino, Diniederalkylcarbamoylniederalkylamino, Sulfoniederalkylamino, Aminoalkylamino, Niederalkylaminoniederalkylamino, Hydroxyniederalkylaminoniederalkylamino, Diniederalkylaminoniederalkylamino, 1-Piperidinylniederalkylamino, 4-Morpholinylniederalkylamino, 1-Pyrrolidinylniederalkylamino, worin Pyrrolidin gegebenenfalls durch Hydroxy und/oder Oxo substituiert ist, Niederalkanoylaminoalkylamino, Niederalkoxycarbonylaminoalkylamino, Niederalkenylamino, Niederalkinylamino, Phenylamino oder Phenylniederalkylamino, worin Phenyl gegebenenfalls durch Niederalkyl, Hydroxy, Niederalkoxy, Niederalkanoyloxy, Halogen, Carboxy, Niederalkoxycarbonyl, Carbamoyl, Amino, Niederalkylamino, Diniederalkylamino, Acylamino, und/oder durch Nitro mono- oder mehrfach substituiert ist, Phenylniederalkylamino, worin Niederalkyl durch Hydroxy, Cyano, Carboxy, Niederalkoxy und/oder Carbamoyl substituiert ist, Oxazolylamino, Thiazolylamino, das gegebenenfalls durch Carboxyniederalkyl, Niederalkoxycarbonylniederalkyl oder Carbamoylniederalkyl substituiert ist, Pyridylniederalkylamino, Imidazolylniederalkylamino, Indolylniederalkylamino, 1-Benzylpiperidinylamino, 1-Niederalkoxycarbonylpiperidinylamino, ferner Niederalkoxy, Phenyloxy, Phenylniederalkoxy, oder physiologisch spaltbares Alkoxy,

ferner -His-, -Phe-, -Ala-, -Ile-, C-terminal substituiert durch Amino, Alkylamino, Diniederalkylamino, Hydroxyniederalkylamino, Carboxyalkylamino, Niederalkoxycarbonylalkylamino, physiologisch spaltbares, verestertes Carboxyalkylamino, Carbamoylniederalkylamino, Hydroxyniederalkylcarbamoyl-niederalkylamino, Aminoalkylamino, Hydroxyniederalkylamino-niederalkylamino, Diniederalkylaminoniederalkylamino, über ein Stickstoff-Atom gebundenes fünf oder sechsgliedriges Heterocyclylniederalkylamino, Acylaminoniederalkylamino, Phenylniederalkylamino, worin Phenyl durch Carboxy, Niederalkoxycarbonyl oder Carbamoyl und Niederalkyl durch Hydroxy, Cyano oder Carboxy substituiert sein kann, unsubstituiertes oder substituiertes Thiazolylamino, Pyridylniederalkylamino, Imidazolylniederalkylamino, Indolylniederalkylamino, Piperidinylamino, 1-Niederalkoxycarbonylpiperidinylamino, ausserdem durch Niederalkoxy oder physiologisch spaltbares substituiertes Alkoxy,

ferner -Ile-His-, -Ile-Phe-, -Ala-His-, -Gly-Gly-, -Gly-His-, -Gly-Ala-, -Gly-Ser-, -Ala-Sta-, -Ile-Sta-, -Gly-Gly-Gly-, -Gly-His-Lys-, -Gly-Ala-Gly- oder -Ile-His-Lys-, worin die Aminofunktion in der Seitenkette von -Lys- gegebenenfalls durch Niederalkanoyl, Niederalkoxycarbonyl oder Phenylniederalkoxycarbonyl acyliert ist, C-terminal substituiert durch Amino, Niederalkylamino, Diniederalkylamino, Hydroxyniederalkylamino, ferner Niederalkoxy oder physiologisch spaltbares substituiertes Alkoxy darstellt, sowie pharmazeutisch annehmbare Salze von diesen Verbindungen mit salzbildenden Gruppen.

4. Verbindungen gemäss Anspruch 1 der Formel I, worin $R_1$ Wasserstoff, Niederalkanoyl, Niederalkoxyniederalkanoyl, Mono- oder Diisopropyliden-glucofuranosyl-O-acetyl, Halogenniederalkanoyl, Carboxyniederalkanoyl, Niederalkoxycarbonylniederalkanoyl, Aminoalkanoyl, Niederalkanoylaminoalkanoyl, Niederalkoxycarbonylaminoalkanoyl, Phenylniederalkoxycarbonylaminoalkanoyl, γ-Aminobutyrylaminoacetyl, γ-Benzyloxycarbonylaminobutyryl-aminoacetyl, (α-Amino-δ-guanidinovaleryl)-aminoacetyl, (α-Benzyloxycarbonylamino-δ-guanidinovaleryl)-aminoacetyl, Oxoniederalkanoyl, Hydroxycarboxyniederalkanoyl, Hydroxy-niederalkoxycarbonyl-niederalkanoyl, Dihydroxy-carboxy-niederalkanoyl, Dihydroxy-niederalkoxycarbonylniederalkanoyl, verestertes Dihydroxy-niederalkoxycarbonyl-niederalkanoyl, Hydroxy-amino-niederalkanoyl, Hydroxy-acylamino-niederalkanoyl, Carboxy-amino-niederalkanoyl, Niederalkoxycarbonyl-aminoniederalkanoyl, Niederalkoxycarbonyl-acylamino-niederalkanoyl, Niederalkenoyl, Niederalkinoyl, Cyclopentyl- oder Cyclohexylcarbonyl, Hydroxycyclohexylcarbonyl, Acylaminocycloalkylcarbonyl,

3,4,5-Trihydroxycyclohex-1-enylcarbonyl, Benzoyl oder durch Halogen, Niederalkoxy und/oder Nitro substituiertes Benzoyl, 2-(o,o-Dichloroanilino)-phenylacetyl, 2-(o,o-Dichloro-N-benzylanilino)-phenylacetyl, Hydroxypyrrolidinylcarbonyl, Hydroxy-1-acylpyrrolidinylcarbonyl, Oxopyrrolidinylcarbonyl, Niederalkoxycarbonyl, 2-Halogenniederalkoxycarbonyl, Arylmethoxycarbonyl mit einem oder zwei Arylresten, worin Aryl gegebenenfalls durch Niederalkyl, Niederalkoxy, Hydroxy, Halogen und/oder Nitro mono-, di- oder trisubstituiertes Phenyl ist,

$X_1$ und $X_2$ zusammen -Phe-His-, -Phe-Phe-, -Phe-(N-methyl-Phe)- -Phe-Leu-, -Phe-Ala-, -Phe-Arg-, -Phe-Gly-, -His-His-, -Arg-Arg-, -Arg-His-, -Arg-Phe-oder -Glu-His-, welche N-terminal mit $R_1$ und C-terminal mit der Gruppe $-NR_2-$ verbunden sind, $R_2$ Wasserstoff, $R_3$ Niederalkyl, $R_4$ Hydroxy, $R_5$ Alkyl, Cyclohexyl oder Phenyl, und

$R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkylamino, Niederalkoxycarbonylalkylamino, physiologisch spaltbares verestertes Carboxyalkylamino, Carbamoylniederalkylamino, Hydroxyniederalkylcarbamoylniederalkylamino, Niederalkoxycarbonyl-hydroxyniederalkoxycarbamoyl-niederalkylamino, Aminoalkylamino, Hydroxyniederalkylaminoniederalkylamino, Diniederalkylaminoniederalkylamino, über ein Stickstoffatom gebundenes fünf- oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, sowie Niederalkoxy, oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, Diniederalkylaminoniederalkylamino, 4-Morpholinylniederalkylamino, Benzylamino, Carboxybenzylamino, Niederalkoxycarbonylbenzylamino, Carbamoylbenzylamino, 2-Phenyläthylamino, 1-Phenylprop-2-ylamino, 3-Phenylpropylamino,

1-Hydroxymethyl-2-phenyläthylamino, 2-Hydroxy-1- oder 2-phenyläthylamino oder 1-(2-Cyano- oder 2-Carboxy-1-hydroxyäthyl)-2-phenyläthylamino, 4- oder 5-Carbamoylmethyl-2-thiazolylamino, Pyridylniederalkylamino, Imidazolylniederalkylamino, Indolylniederalkylamino, 1-Niederalkoxycarbonylpiperidinylamino, Niederalkoxy oder physiologisch spaltbares substituiertes Alkoxy, ferner -Ile-His-, -Ile-Phe, -Ile-Sta-, -Ala-His-, -Ala-Sta- oder -Ile-His-Lys-, worin die Aminofunktion in der Seitenkette von -Lys- gegebenenfalls durch Niederalkanoyl, Niederalkoxcarbonyl oder Phenylniederalkoxycarbonyl acyliert ist, C-terminal substituiert durch Amino, Niederalkylamino, Diniederalkylamino, Hydroxyniederalkylamino, Niederalkoxy oder physiologisch spaltbares substituiertes Alkoxy, darstellt, sowie pharmazeutisch annehmbare Salze von diesen Verbindungen mit salzbildenden Gruppen.

5. Verbindungen gemäss Anspruch 1 der Formel I, worin $R_1$ Wasserstoff, Niederalkanoyl, Mono- oder Diisopropyliden-glucofuranosyl-O-acetyl, Aminoalkanoyl, Niederalkanoylaminoalkanoyl, Niederalkoxycarbonylaminoalkanoyl, Phenylniederalkoxycarbonylaminoalkanoyl, γ-Aminobutyryl-aminoacetyl, γ-Benzyloxycarbonylaminobutyryl-aminoacetyl, (α-Amino-δ-guanidinovaleryl)-aminoacetyl, (α-Benzyloxycarbonylamino-δ-guanidinovaleryl)-aminoacetyl, Hydroxy-carboxyniederalkanoyl, Hydroxy-niederalkoxycarbonyl-niederalkanoyl, Dihydroxy-carboxy-niederalkanoyl, Dihydroxy-niederalkoxycarbonylniederalkanoyl, verestertes Dihydroxy-niederalkoxycarbonyl-niederalkanoyl, Hydroxy-amino-niederalkanoyl, Niederalkoxycarbonyl-acylamino-niederalkanoyl, 3,4,5-Trihydroxycyclohexylcarbonyl, 3,4,5-Trihydroxycyclohex-1-enylcarbonyl, 2-(o,o-Dichloroanilino)-phenylacetyl, 2-(o,o-Dichloro-N-benzylanilino)-phenylacetyl, 3-oder 4-Hydroxypyrrolidinyl-2-carbonyl, 3- oder 4-Hydroxy-1-benzyloxycarbonylpyrrolidinyl-2-carbonyl, 5-Oxopyrroldinyl-2-carbonyl, Niederalkoxycarbonyl, Phenylmethoxycarbonyl mit ein oder zwei Phenylresten, die gegebenenfalls durch Niederalkyl, Niederalkoxy und/oder Nitro substituiert sind, $X_1$ und $X_2$ zusammen -Phe-His-, -Phe-Phe-, -Phe-Leu-, -Arg-His- oder -Arg-Phe-, welche N-terminal

mit $R_1$ und C-terminal mit der Gruppe $-NR_2-$ verbunden sind, $R_2$ Wasserstoff, $R_3$ Isopropyl oder Isobutyl, $R_4$ Hydroxy, $R_5$ Isopropyl, n-Octyl, Cyclohexyl oder Phenyl, und $R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkylamino, Niederalkoxycarbonylalkylamino, physiologisch spaltbares verestertes Carboxyalkylamino, Carbamoylniederalkylamino, Hydroxyniederalkylcarbamoylniederalkylamino, α-Carbamoyl-α-(2-hydroxy-1-isobutyl-3-methoxycarbonylpropyl)-carbamoyl-methylamino, Aminoalkylamino, Hydroxyniederalkylaminoniederalkylamino, Diniederalkylaminoniederalkylamino, über ein Stickstoff-Atom gebundenes fünf- oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, Niederalkoxy oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, 4-Morpholinylniederalkylamino, Carboxybenzylamino, Carbamoylbenzylamino, 4- oder 5-Carbamoylmethyl-2-thiazolylamino, Pyridylniederalkylamino, Imidazolylniederalkylamino, Indolylniederalkylamino, Niederalkoxy oder physiologisch spaltbares substituiertes Alkoxy, ferner -Ile-His-, -Ile-Sta- oder -Ala-Sta-, C-terminal substituiert durch Amino, Niederalkylamino, Diniederalkylamino, Hydroxyniederalkylamino, Niederalkoxy oder physiologisch spaltbares substituiertes Alkoxy, darstellt, sowie pharmazeutisch annehmbare Salze von diesen Verbindungen mit salzbildenden Gruppen.

6. Verbindungen gemäss Anspruch 1 der Formel I, worin $R_1$ Wasserstoff, Niederalkanoyl, Aminoalkanoyl, Niederalkoxycarbonylaminoalkanoyl, Benzyloxycarbonylaminoniederalkanoyl, γ-Aminobutyrylaminoacetyl, γ-tert-Butoxycarbonylaminobutyrylaminoacetyl, γ-Benzyloxycarbonylaminobutyrylaminoacetyl, α,ß-Dihydroxy-ß-carboxypro-

pionyl, α,ß-Dihydroxy-ß-methoxycarbonylpropionyl, α,ß-Diacetoxy-ß-methoxycarbonylpropionyl, 3,4,5-Trihydroxycyclohex-1-enylcarbonyl, 2-(o,o-Dichloroanilino)-phenylacetyl, 2-(o,o-Dichloro-N-benzyl-anilino)-phenylacetyl, 3- oder 4-Hydroxypyrrolidinyl-2-carbonyl, 3- oder 4-Hydroxy-1-benzyloxycarbonylpyrrolidinyl-2-carbonyl, 5-Oxo-pyrrolidinyl-2-carbonyl, Niederalkoxycarbonyl, Phenylmethoxycarbonyl mit ein oder zwei Phenylresten, die gegebenenfalls durch Niederalkyl, Niederalkoxy, und/oder Nitro substituiert sind, $X_1$ und $X_2$ zusammen -Phe-His- oder -Phe-Phe-, welche N-terminal mit $R_1$ und C-terminal mit der Gruppe $-NR_2-$ verbunden sind, $R_2$ Wasserstoff, $R_3$ Isobutyl, $R_4$ Hydroxy, $R_5$ Isopropyl, n-Octyl, Cyclohexyl oder Phenyl, und $R_6$ Amino, Niederalkylamino, Diniederalkylamino, Hydroxyniederalkylamino, Carboxyalkylamino, Niederalkoxycarbonylalkylamino, physiologisch spaltbares verestertes Carboxyalkylamino, Aminoalkylamino, Hydroxyniederalkylaminoniederalkylamino, Diniederalkylaminoniederalkylamino, 4-Morpholinylniederalkylamino, 2-Oxo-1-pyrrolidinylniederalkylamino, ferner -Ala- oder -Ile-, C-terminal substituiert durch Amino, Niederalkylamino, Diniederalkylamino, Hydroxyniederalkylamino, Dicarbamoylmethylamino, 2-(2-Hydroxyäthylamino)-äthylamino, 2-(4-Morpholinyl)-äthylamino, 3-Carbamoylbenzylamino, 4-Carbamoylmethyl-2-thiazolylamino, 2-Pyridylmethylamino, 2-(2-Pyridyl)-äthylamino, 3-(2-Pyridyl)-propylamino, 2-(4-Imidazolyl)-äthylamino oder 2-(3-Indolyl)-äthylamino, ferner -Ile-His- oder -Ile-Sta-, C-terminal substituiert durch Amino, oder -Ala-Sta-, C-terminal substituiert durch Niederalkoxy, darstellt, sowie pharmazeutisch annehmbare Salze von diesen Verbindungen mit salzbildenden Gruppen.

7. Verbindungen gemäss Anspruch 1 der Formel I, worin $R_1$ Wasserstoff, Acetyl, Pivaloyl, γ-Aminobutyryl, 8-Aminooctanoyl, γ-tert-Butoxycarbonylaminobutyryl, 8-tert-Butoxycarbonylaminooctanoyl, γ-Benzyloxycarbonylaminobutyryl, 8-Benzyloxycarbonylaminooctanoyl, γ-Aminobutyrylaminoacetyl, γ-tert-Butoxycarbonylaminobutyrylaminoacetyl, γ-Benzyloxycarbonylaminobutyrylaminoacetyl, α,ß-Dihydoxy-ß-carboxypropionyl, α,ß-Dihydroxy-ß-methoxycarbonylpropionyl,

4-Hydroxypyrrolidinyl-2-carbonyl, 1-Benzyloxycarbonyl-4-hydroxypyrrolidinyl-2-carbonyl, 5-Oxopyrrolidinyl-2-carbonyl, tert-Butoxycarbonyl oder Benzyloxycarbonyl, $X_1$ und $X_2$ zusammen -Phe-His-, N-terminal mit $R_1$ und C-terminal mit der Gruppe $-NR_2-$ verbunden, $R_2$ Wasserstoff, $R_3$ Isobutyl, $R_4$ Hydroxy, $R_5$ Isopropyl, und $R_6$ Amino, Niederalkylamino, Dimethylamino, 4-Carboxy-n-butylamino, 7-Carboxy-n-heptylamino, 4-tert-Butoxycarbonyl-n-butylamino, 7-tert-Butoxycarbonyl-n-heptylamino, 7-Pivaloyloxymethoxycarbonyl-n-heptylamino, 3-(2-Oxo-1-pyrrolidinyl)-propylamino, -Ala-, C-terminal substituiert durch 2-(4-Imidazolyl)-äthylamino oder 2-(2-Pyridyl)-äthylamino, -Ile-, C-terminal substituiert durch Dicarbamoylmethylamino, 3-Carbamoylbenzylamino, 4-Carbamoylmethyl-2-thiazolylamino, 2-(2-Pyridyl)-äthylamino, 2-Pyridylmethylamino oder 2-(3-Indolyl)-äthylamino, -Ile-His- oder -Ile-Sta-, C-terminal substituiert durch Amino, oder -Ala-Sta-, C-terminal substituiert durch Methoxy, darstellt, sowie pharmazeutisch annehmbare Salze von diesen Verbindungen mit salzbildenden Gruppen.

8. Benzyloxycarbonyl-Phe-His-[5(S)-amino-4(S)-hydroxy-2(S)-isopropyl-7-methyloctanoyl]-Ile-His-$NH_2$ gemäss Anspruch 1.

9. Benzyloxycarbonyl-Phe-Phe-[5(S)-amino-4(S)-hydroxy-2(S)-isopropyl-7-methyloctanoyl]-Ile-His-$NH_2$ gemäss Anspruch 1.

10. H-Phe-His-[5(S)-amino-4(S)-hydroxy-2(S)-isopropyl-7-methyloctanoyl]-Ile-His-$NH_2$ gemäss Anspruch 1.

11. [(2S,4R)-4-Hydroxypyrrolidinyl-2-carbonyl]-Phe-His-[5(S)-amino-4(S)-hydroxy-2(S)-isopropyl-7-methyloctanoyl]-Ile-His-$NH_2$ gemäss Anspruch 1.

12. [(2S)-5-Oxopyrrolidinyl-2-carbonyl]-Phe-His-[5(S)-amino-4(S)-hydroxy-2(S)-isopropyl-7-methyloctanoyl]-Ile-His-$NH_2$ gemäss Anspruch 1.

13. tert-Butoxycarbonyl-Phe-His-[5(S)-amino-4(S)-hydroxy-2(S)-isopropyl-7-methyloctanoyl]-Ala-Sta-$OCH_3$ gemäss Anspruch 1.

14. Benzyloxycarbonyl-Phe-His-[5(S)-amino-4(S)-hydroxy-2(S)-isopropyl-7-methyloctanoyl]-Ala-Sta-$OCH_3$ gemäss Anspruch 1.

15. γ-Benzyloxycarbonylaminobutyrylaminoacetyl-Phe-His-[5(S)-amino-4(S)-hydroxy-2(S)-isopropyl-7-methyloctanoyl]-Ile-His-$NH_2$ gemäss Anspruch 1.

16. γ-Aminobutyrylaminoacetyl-Phe-His-[5(S)-amino-4(S)-hydroxy-2(S)-isopropyl-7-methyloctanoyl]-Ile-His-$NH_2$ gemäss Anspruch 1.

17. 8-Aminooctanoyl-Phe-His-[5(S)-amino-4(S)-hydroxy-(2S)-isopropyl-7-methyloctanoyl]-Ile-His-$NH_2$ gemäss Anspruch 1.

18. Benzyloxycarbonyl-Phe-His-[5(S)-amino-4(S)-hydroxy-(2S)-isopropyl-7-methyloctanoyl]-Ile-dicarbamoylmethylamid gemäss Anspruch 1.

19. Benzyloxycarbonyl-Phe-His-[5(S)-amino-4(S)-hydroxy-2(S)-isopropyl-7-methyloctanoyl]-2-(3-carbamoyl-4-hydroxy-phenyloxy)-äthylamid gemäss Anspruch 1.

20. α-Benzyloxycarbonylamino-α-methoxycarbonyl-acetyl-Phe-His-[5(S)-amino-4(S)-hydroxy-2(S)-isopropyl-7-methyloctanoyl]-3-(2-pyrrolidinon-1-yl)-propylamid gemäss Anspruch 1.

21. Benzyloxycarbonyl-Phe-His-[5(S)-amino-4(S)-hydroxy-2(S)-isopropyl-7-methyloctanoyl]-methylamid gemäss Anspruch 1.

22. Benzyloxycarbonyl-Phe-His-[5(S)-amino-4(S)-hydroxy-2(S)-isopropyl-7-methylocatnoyl]-4-[tris-(hydroxymethyl)-methylaminocarbonyl]-butylamid gemäss Anspruch 1.

23. Benzyloxycarbonyl-Glu(O-tert-butyl)-His-[5(S)-amino-4(S)-hydroxy-2(S)-isopropyl-7-methyloctanoyl]-Ile-His-$NH_2$ gemäss Anspruch 1.

24. Benzyloxycarbonyl-Phe-His-[5(S)-amino-4(S)-hydroxy-2(S)-isopropyl-7-methyloctanoyl]-carbamoyl-(3-methoxycarbonyl-2-hydroxy-1-isobutyl-propylaminocarbonyl)-methylamid gemäss Anspruch 1.

25. Pharmazeutische Präparate enthaltend Verbindungen der Formel I oder pharmazeutisch verwendbare Salze von Verbindungen der Formel I mit salzbildenden Gruppen zusammen mit einem pharmazeutischen Trägermaterial.

26. Verbindungen der Formel I oder pharmazeutisch verwendbare Salze von Verbindungen der Formel I zur Anwendung in einem Verfahren zur therapeutischen Behandlung des menschlichen oder tierischen Körpers.

27. Verwendung von Verbindungen der Formel I oder pharmazeutisch verwendbare Salze davon zur Hemmung der Wirkung des Enzyms Renin.

28. Verwendung von Verbindungen der Formel I, Hydraten oder pharmazeutisch verwendbaren Salze von Verbindungen der Formel I zur Herstellung von pharmazeutischen Präparaten.

29. Verfahren zur Herstellung von Verbindungen der Formel I, worin die Substituenten die in Anspruch 1 genannten Bedeutungen haben, dadurch gekennzeichnet, dass man
a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, die Ketogruppe in einer Verbindung der Formel

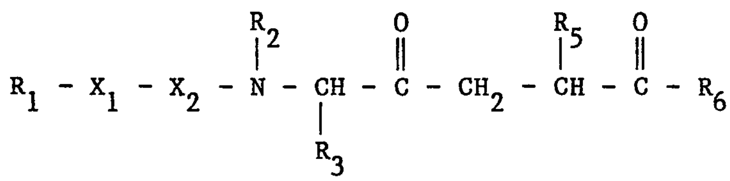

(II),

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) zur Herstellung von Verbindungen der Formel I, worin $R_4$ Hydroxy bedeutet, eine Aldehyd-Verbindung der Formel

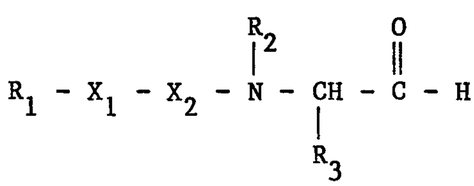

(III),

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

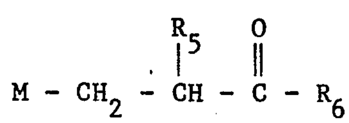

(IV),

worin die Substituenten die genannten Bedeutungen haben und M ein Metallradikal bedeutet, umsetzt und das gebildete Additionsprodukt hydrolysiert, oder

d) in einer Verbindung der Formel

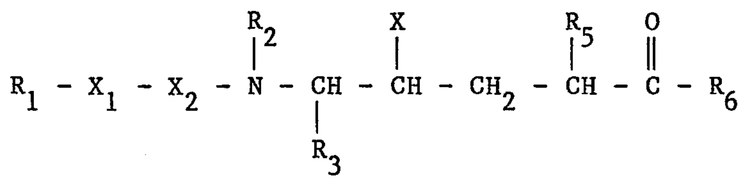
(V),

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder

e) zur Herstellung einer Verbindung der Formel I, worin $R_6$ unsubstituiertes oder substituiertes Amino bedeutet, in einer Verbindung der Formel

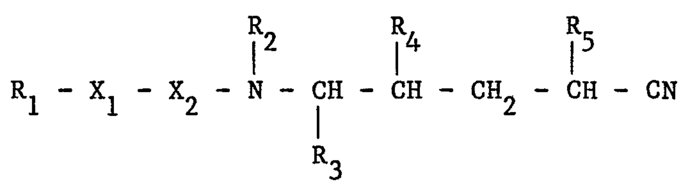
(VI),

worin die Substituenten die genannten Bedeutungen haben, bzw. in einer Verbindung der Formel

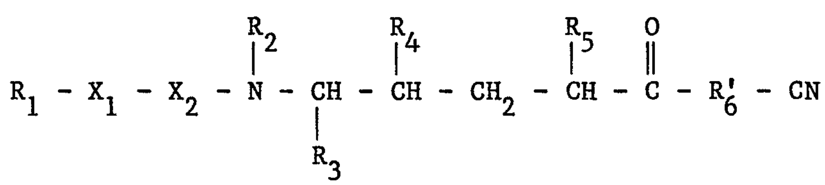
(VIa),

worin die Gruppe $R_6'$-CN den Rest einer in der Natur vorkommenden α-Aminosäure, eines Di- oder eines Tripeptids, worin jeweils die terminale -COOH-Gruppe durch -CN ersetzt ist, bedeutet, die übrigen Substituenten die genannten Bedeutungen haben und vorhandene funktionelle Gruppen gegebenenfalls in geschützter Form vorliegen, die Cyanogruppe in eine unsubstituierte oder N-substituierte Carboxamidogruppe überführt, oder

f) zur Herstellung einer Verbindung der Formel I, worin $R_4$ freies Hydroxy bedeutet, ein Epoxid der Formel

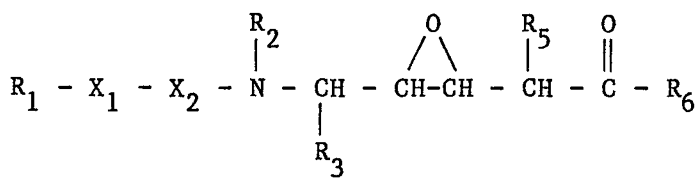

(VII),

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert,
und gewünschtenfalls in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

30. Die nach einem der Verfahren des Anspruchs 29 erhältlichen Verbindungen und ihre Salze.

31. Verbindungen der Formel

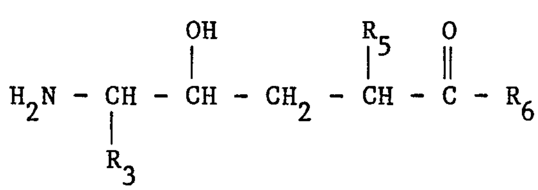

(VIII),

worin die Substituenten die in Anspruch 1 genannten Bedeutungen haben, und Salze davon.

32. Verfahren zur Herstellung von Verbindungen der Formel VIII, worin die Substituenten die in Anspruch 1 genannten Bedeutungen haben, und Salzen davon, dadurch gekennzeichnet, dass man eine Verbindung der Formel

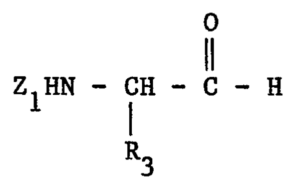 (IX),

worin $R_3$ die genannte Bedeutung hat und $Z_1$ eine Aminoschutzgruppe ist, mit einer Schwefel-Ylid-Verbindung in ein Epoxid der Formel

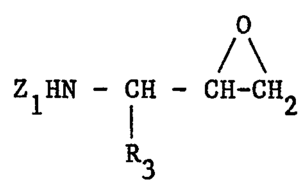 (X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

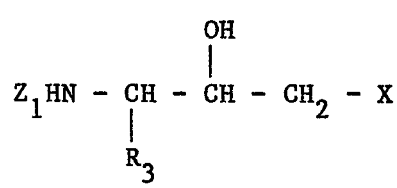 (XI),

worin $R_3$ und $Z_1$ die genannten Bedeutungen haben und X eine nukleofuge Abgangsgruppe ist, mit einer Carbonylverbindung der Formel

$$R_a - (C = O) - R_b \qquad (XII),$$

worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

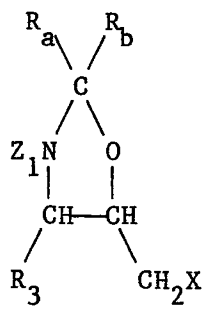 (XIII),

worin die Substituenten die genannten Bedeutungen haben, mit einem Carbonsäureestersalz der Formel

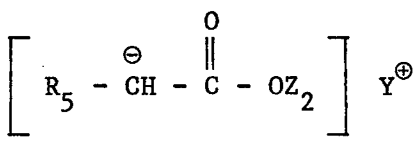 (XIV),

worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium- oder bevorzugt das Lithiumion, ist, umsetzt, und in einer erhältlichen Verbindung der Formel

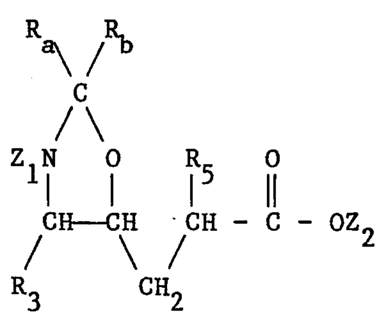 (XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit einer freien Carboxygruppe ($Z_2$=H) je nach Bedeutung des einzuführen-

den Rests $R_6$ amidiert, verestert oder mit einem C-terminal gegebenenfalls amidierten oder veresterten Rest einer Aminosäure, eines Di- oder Tripeptidrest substituiert und in einer erhältlichen Verbindung der Formel

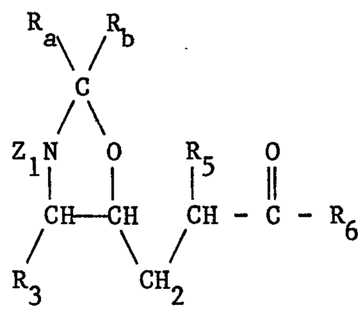

(XVI),

worin die Substituenten die genannten Bedeutungen haben, mit einem geeigneten Solvolysereagens den Ring öffnet und gegebenenfalls die Schutzgruppe $Z_1$ abspaltet.

FO 7.4/KB/gs*

<u>Patentansprüche</u> für den Vertragsstaat AT

1. Verfahren zur Herstellung von substituierten 5-Amino-4-hydroxy-valerylderivaten der Formel

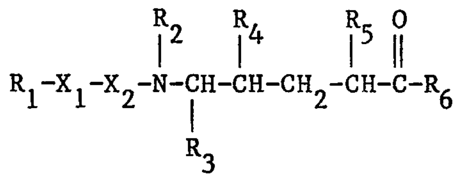

(I),

worin $R_1$ Wasserstoff oder Acyl, $X_1$ einen gegebenenfalls N-alkylierten natürlichen Aminosäurerest, der N-terminal mit $R_1$ und C-terminal mit $X_2$ verbunden ist, $X_2$ einen gegebenenfalls N-alkylierten natürlichen Aminosäurerest, der N-terminal mit $X_1$ und C-terminal mit der Gruppe $-NR_2-$ verbunden ist, $R_2$ Wasserstoff oder Niederalkyl, $R_3$ Wasserstoff, Alkyl, Cycloalkyl, Arylniederalkyl oder Aryl, $R_4$ Hydroxy oder veräthertes oder verestertes Hydroxy, $R_5$ Alkyl, Cycloalkyl, Arylniederalkyl oder Aryl und $R_6$ substituiertes oder freies Amino oder substituiertes Hydroxy darstellen, mit Ausnahme der Verbindungen, worin $R_1$ einen gegebenenfalls N-acylierten Rest der Aminosäure L-Prolin oder worin $X_1$ und $X_2$ zusammen -Val-Val-, -Gly-Gly- oder -Tyr-Gly- bedeuten, ferner von Salzen solcher Verbindungen mit salzbildenden Gruppen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, die Ketogruppe in einer Verbindung der Formel

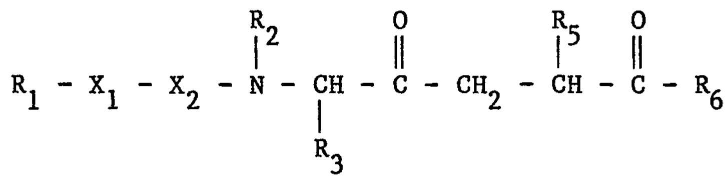

(II),

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) zur Herstellung von Verbindungen der Formel I, worin $R_4$ Hydroxy bedeutet, eine Aldehyd-Verbindung der Formel

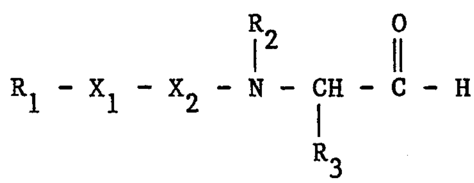

(III),

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

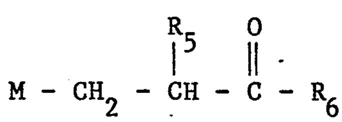

(IV),

worin die Substituenten die genannten Bedeutungen haben und M ein Metallradikal bedeutet, umsetzt und das gebildete Additionsprodukt hydrolysiert, oder

d) in einer Verbindung der Formel

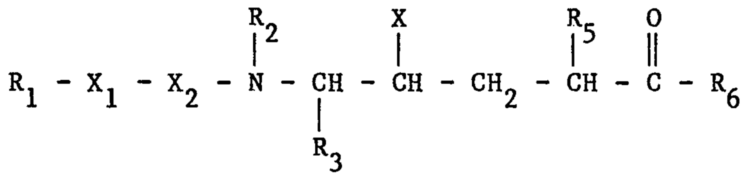 (V),

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X mit einem den Substituenten $R_4$ in nukleophiler Form einführenden Reagens gegen $R_4$ austauscht, oder

e) zur Herstellung einer Verbindung der Formel I, worin $R_6$ unsubstituiertes oder substituiertes Amino bedeutet, in einer Verbindung der Formel

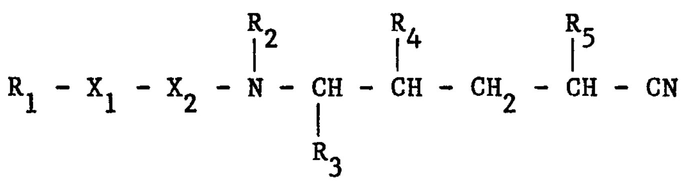 (VI),

worin die Substituenten die genannten Bedeutungen haben, bzw. in einer Verbindung der Formel

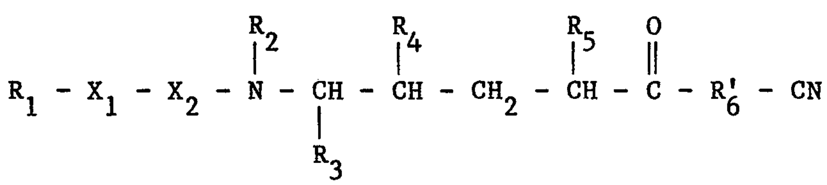 (VIa),

worin die Gruppe $R_6'$-CN den Rest einer in der Natur vorkommenden α-Aminosäure, eines Di- oder eines Tripeptids, worin jeweils die terminale -COOH-Gruppe durch -CN ersetzt ist, bedeutet, die übrigen Substituenten die genannten Bedeutungen haben und vorhandene funktionelle Gruppen gegebenenfalls in geschützter Form vorliegen, die Cyanogruppe in eine unsubstituierte oder N-substituierte Carboxamidogruppe überführt, oder

f) zur Herstellung einer Verbindung der Formel I, worin $R_4$ freies Hydroxy bedeutet, ein Epoxid der Formel

$$R_1 - X_1 - X_2 - \overset{\overset{R_2}{|}}{N} - \underset{\underset{R_3}{|}}{CH} - \overset{O}{\overbrace{CH\text{-}CH}} - \overset{\overset{R_5}{|}}{CH} - \overset{\overset{O}{\|}}{C} - R_6 \qquad (VII),$$

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert,
und gewünschtenfalls in einer erhältlichen Verbindung vorhandene Schutzgruppen abspaltet und/oder gewünschtenfalls nach Ausführung eines der vorstehend genannten Verfahren a) - f) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

2. Verfahren zur Herstellung von substituierten 5-Amino-4-hydroxyvalerylderivaten der Formel I gemäss Anspruch 1, dadurch gekennzeichnet, dass man ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung

kondensiert und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

3. Verfahren zur Herstellung von substituierten 5-Amino-4-hydroxyvalerylderivaten der Formel I gemäss Anspruch 1, dadurch gekennzeichnet, dass man ein Fragment einer Verbindung der Formel I mit einer endständigen Carboxygruppe oder ein davon abgeleiteter aktivierter Ester, reaktionsfähiges Anhydrid oder reaktionsfähiges cyclisches Amid mit einem zur Verbindung der Formel I komplementären Fragment mit einer freien Aminogruppe, wobei in den Reaktionskomponenten vorhandene freie funktionelle Gruppen mit Ausnahme der an der Reaktion teilnehmenden Gruppen gegebenenfalls in geschützter Form vorliegen, in Gegenwart eines Kondensationsmittels ausgewählt aus einem Carbodiimid, Carbonyldiimidazol, 1,2-Oxazoliumverbindungen, einem 1,2-Dihydrochinolin oder einem aktivierten Phosphat, und gewünschtenfalls in Gegenwart einer organischen Base oder von Alkalimetallcarbonaten in einem inerten, polaren, aprotischen Lösungsmittel in einem Temperaturbereich von -40°C bis +100°C kondensiert, wobei aktivierte Ester vom Amino- oder Amidoester-Typ gewünschtenfalls in situ durch Zugabe einer N-Hydroxyamino- oder N-Hydroxyamido-Verbindung gebildet werden, und gewünschtenfalls in einer erhältlichen Verbindung vorhandene Schutzgruppen durch Behandlung mit Säure oder mit Base oder durch Hydrogenolyse abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

4. Verfahren gemäss einem der Ansprüche 1-3, dadurch gekennzeichnet, dass man solche Ausgangsstoffe wählt, dass Verbindungen der Formel I, worin $R_1$ Wasserstoff oder Acyl mit den Teilformeln $R^b$-CO- oder $R^a$-O-CO-, worin $R^a$ einen unsubstituierten oder substituierten, gesättigten oder ungesättigten, aliphatischen, cycloaliphatischen, cycloaliphatisch-aliphatischen Kohlenwasserstoffrest bis einschliesslich 18, bevorzugt bis einschliesslich 10, C-Atomen oder einen unsubstituierten oder substituierten aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen oder einen unsubstituierten oder substituierten, fünf- oder sechsgliedrigen Heterocyclus darstellt und $R^b$ Wasserstoff bedeutet oder die Bedeutungen von $R^a$ hat, $X_1$ einen natürlichen Aminosäurerest, der N-terminal mit $R_1$ und C-terminal mit $X_2$ verbunden ist, $X_2$ einen gegebenenfalls N-alkylierten natürlichen Aminosäurerest, N-terminal mit $X_1$ und C-terminal mit der Gruppe $-NR_2$-verbunden ist, $R_2$ Wasserstoff oder Niederalkyl, $R_3$ Wasserstoff, Niederalkyl, Cycloalkyl, Arylniederalkyl oder Aryl, $R_4$ Hydroxy, $R_5$ Alkyl, Cycloalkyl, Arylniederalkyl oder Aryl, und $R_6$ Amino oder eine Amino-oder Hydroxygruppe, welche durch einen oder gegebenenfalls zwei unsubstituierte oder substituierte, gesättigte oder ungesättigte, aliphatische Kohlenwasserstoffreste bis einschliesslich 18, bevorzugt bis einschliesslich 10, C-Atomen oder einen aromatischen, heteroaromatischen, aromatisch-aliphatischen oder heteroaromatisch-aliphatischen Kohlenwasserstoffrest bis einschliesslich 18, bevorzugt bis einschliesslich 10, C-Atomen substituiert ist, oder einen N-terminal mit der Gruppe -CO- verbundenen und C-terminal gegebenenfalls amidierten oder veresterten Rest einer natürlichen α-Aminosäure oder einen N-terminal mit der Gruppe -CO- verbundenen und C-terminal gegebenenfalls amidierten oder veresterten Di- oder Tripeptidrest, der aus natürlichen α-Aminosäuren und gewünschtenfalls aus Statin besteht, darstellen, mit Ausnahme der Verbindungen, worin $R_1$ einen gegebenen-

falls N-acylierten Rest der Aminosäure L-Prolin oder worin $X_1$ und $X_2$ zusammen -Val-Val-, -Gly-Gly- oder -Tyr-Gly- bedeuten, ferner pharmazeutisch annehmbare Salze von Verbindungen der Formel I mit salzbildenden Gruppen, gebildet werden.

5. Verfahren gemäss einem der Ansprüche 1-3, dadurch gekennzeichnet, dass man solche Ausgangsstoffe wählt, dass Verbindungen der Formel I, worin $R_1$ Wasserstoff, Niederalkanoyl mit 1-7 C-Atomen, Hydroxyniederalkanoyl, Niederalkoxyniederalkanoyl, durch einem gewünschtenfalls acetylierten oder als Acetal geschützten natürlichen Zucker veräthertes Niederalkanoyl, Niederalkanoyloxyniederalkanoyl, Halogenniederalkanoyl, Hydroxysulfonyloxyniederalkanoyl, Carboxyniederalkanoyl, Niederalkoxycarbonylniederalkanoyl, Carbamoylniederalkanoyl, Niederalkylcarbamoylniederalkanoyl, Diniederalkylcarbamoylniederalkanoyl, Aminoalkanoyl mit 1-10 C-Atomen, Niederalkylaminoniederalkanoyl, Diniederalkylaminoniederalkanoyl, Niederalkanoylaminoalkanoyl, Niederalkoxycarbonylaminoalkanoyl, Arylniederalkoxycarbonylaminoalkanoyl, substituiertes Niederalkanoylaminoniederalkanoyl, Mercaptoniederalkanoyl, Niederalkylthioniederalkanoyl, Oxoniederalkanoyl, Hydroxy-carboxy-niederalkanoyl, Hydroxy-niederalkoxycarbonyl-niederalkanoyl, verestertes Hydroxy-niederalkoxycarbonyl-niederalkanoyl, Dihydroxy-carboxyniederalkanoyl, Dihydroxy-niederalkoxycarbonyl-niederalkanoyl, verestertes Dihydroxy-niederalkoxycarbonyl-niederalkanoyl, Hydroxyamino-niederalkanoyl, Hydroxy-acylamino-niederalkanoyl, Carboxyamino-niederalkanoyl, Niederalkoxycarbonyl-amino-niederalkanoyl, Carboxy-acylamino-niederalkanoyl, Niederalkoxycarbonyl-acylaminoniederalkanoyl, Diaminoniederalkanoyl, Diacylaminoniederalkanoyl, Niederalkenoyl mit 3-7 C-Atomen, Niederalkinoyl mit 3-7 C-Atomen, Cycloalkylcarbonyl mit 4-9 C-Atomen, Bicycloalkylcarbonyl mit 6-11 C-Atomen, Tricycloalkylcarbonyl mit 9-11 C-Atomen, Monocycloalkenylcarbonyl mit 4-9 C-Atomen, Bicycloalkenylcarbonyl mit 6-11 C-Atomen, Cycloalkylniederalkanoyl mit 5-11 C-Atomen, Cycloalkylniederalkenoyl mit 6-11 C-Atomen, Cycloalkenylniederalkanoyl mit 5-11 C-Atomen, Hydroxycycloalkylcarbonyl, Carboxycycloalkylcarbonyl, Aminocycloalkylcarbonyl, Acylaminocycloalkylcarbonyl, Hydroxycycloalkenylcarbonyl, Benzoyl, unsubstituiert, mono- oder mehrfachsubstituiert durch Niederalkyl, Hydroxy, Niederalkoxy, Halo und/oder Nitro, Phenylniederalkanoyl, worin Phenyl unsubstituiert, mono- oder mehrfachsubstituiert durch Niederalkyl, Hydroxy, Niederalkoxy, Halo und/oder Nitro sein kann, substituiertes Anilinophenylacetyl, Phenylniederalkenoyl, 2- oder 3-Pyrrolylcarbonyl, 2-, 3- oder 4-Pyridylcarbonyl, 2-, 3- oder 5-Indolylcarbonyl, 2-, 3- oder 4-Chinolinylcarbonyl, 1-, 3- oder 4-Isochinolinylcarbonyl, Phenylhydroxy-niederalkanoyl, unsubstituiertes oder substituiertes Phenyl-amino-niederalkanoyl, unsubstituiertes oder substituiertes Phenyl-acylamino-niederalkanoyl, Pyrrolidinyl-3-carbonyl, Hydroxypyrrolidinylcarbonyl, Hydroxy-1-acylpyrrolidinylcarbonyl, Oxopyrrolidinylcarbonyl, Piperidinyl-2-, -3- oder -4-carbonyl, 1-Niederalkylpiperidinylcarbonyl, 1-Acylpiperidinylcarbonyl, Morpholinyl-2- oder -3-carbonyl, Thiomorpholinyl-2- oder -3-carbonyl, 1-und/oder 4-Niederalkylpiperazinylcarbonyl, ferner Niederalkoxycarbonyl, 2-Halogenniederalkoxycarbonyl, Arylniederalkoxycarbonyl, worin Aryl Phenyl, 1- oder 2-Naphthyl oder durch Niederalkyl, Niederalkoxy, Hydroxy, Halogen und/oder Nitro mono- oder mehrfach substituiertes Phenyl ist,

$X_1$ und $X_2$ unabhängig voneinander Ala, Arg, Asn, Asp, Cys, Gln, Glu, Gly, His, Ile, Leu, Lys, Met, Phe, Pro, Ser, Thr, Trp, Tyr oder Val, $-X_1-X_2-$ aber nicht -Val-Val-, -Gly-Gly- oder -Tyr-Gly-, und worin gegebenenfalls die Carboxylfunktion der Seitenkette in Asp und Glu mit einem Niederalkanol verestert und/oder die Aminofunktion der Seitenkette in Lys durch Niederalkanoyl, durch Niederalkoxycarbonyl oder durch Arylniederalkoxycarbonyl, acyliert ist, $X_1$ N-terminal mit $R_1$ und C-terminal mit $X_2$ und $X_2$ N-terminal mit $X_1$ und C-terminal mit $-NR_2$-verbunden ist und $X_2$ am N-Atom durch Niederalkyl substituiert sein kann,

$R_2$ Wasserstoff oder Niederalkyl,

$R_3$ Wasserstoff, Niederalkyl, Cycloalkyl, Phenylniederalkyl oder Phenyl,

$R_4$ Hydroxy,

$R_5$ Alkyl mit 1-10 C-Atomen, Cycloalkyl, Phenylniederalkyl oder Phenyl,

und $R_6$ Amino, Alkylamino mit 1 bis 10 C-Atomen, Diniederalkylamino, Hydroxyniederalkylamino, Di-(hydroxyniederalkyl)-amino, Niederalkoxyniederalkylamino, Niederalkanoyloxyniederalkylamino, Halogenniederalkylamino, Hydroxysulfonyloxyniederalkylamino, Phenyloxyniederalkylamino oder Phenyloxy-hydroxy-niederalkylamino, worin Phenyl gegebenenfalls durch Niederalkyl, Niederalkoxy, Hydroxy, Carboxy, Niederalkoxycarbonyl oder Carbamoyl substituiert ist, Carboxyalkylamino, Niederalkoxycarbonylalkylamino, physiologisch spaltbares verestertes Carboxyalkylamino, Carbamoylniederalkylamino, Niederalkylcarbamoylniederalkylamino, Hydroxyniederalkylcarbamoylniederalkylamino, Triniederalkylsilyloxyniederalkylcarbamoylniederalkylamino, Niederalkoxycarbonyl-hydroxy-niederalkylcarbamoylniederalkylamino, Diniederalkylcarbamoylniederalkylamino, Sulfoniederalkylamino, Aminoalkylamino, Niederalkylaminoniederalkylamino, Hydroxyniederalkylaminoniederalkylamino, Diniederalkylaminoniederalkylamino, 1-Piperidinylniederalkylamino, 4-Morpholinylniederalkylamino, 1-Pyrrolidinylniederalkylamino, worin Pyrrolidin gegebenenfalls durch Hydroxy und/oder Oxo substituiert ist, Niederalkanoylaminoalkylamino, Niederalkoxycarbonylaminoalkylamino, Niederalkenylamino, Niederalkinylamino, Phenylamino oder Phenylniederalkylamino, worin Phenyl gegebenenfalls durch Niederalkyl, Hydroxy, Niederalkoxy, Niederalkanoyloxy, Halogen, Carboxy, Niederalkoxycarbonyl, Carbamoyl, Amino, Niederalkylamino, Diniederalkylamino, Acylamino, und/oder durch Nitro mono- oder mehrfach substituiert ist, Phenylniederalkylamino, worin Niederalkyl durch

Hydroxy, Cyano, Carboxy, Niederalkoxy und/oder Carbamoyl substituiert ist, Oxazolylamino, Thiazolylamino, das gegebenenfalls durch Carboxyniederalkyl, Niederalkoxycarbonylniederalkyl oder Carbamoylniederalkyl substituiert ist, Pyridylniederalkylamino, Imidazolylniederalkylamino, Indolylniederalkylamino, 1-Benzylpiperidinylamino, 1-Niederalkoxycarbonylpiperidinylamino, ferner Niederalkoxy, Phenyloxy, Phenylniederalkoxy, oder physiologisch spaltbares Alkoxy,

ferner -His-, -Phe-, -Ala-, -Ile-, C-terminal substituiert durch Amino, Alkylamino, Diniederalkylamino, Hydroxyniederalkylamino, Carboxyalkylamino, Niederalkoxycarbonylalkylamino, physiologisch spaltbares, verestertes Carboxyalkylamino, Carbamoylniederalkylamino, Hydroxyniederalkylcarbamoyl-niederalkylamino, Aminoalkylamino, Hydroxyniederalkylamino-niederalkylamino, Diniederalkylaminoniederalkylamino, über ein Stickstoff-Atom gebundenes fünf oder sechsgliedriges Heterocyclylniederalkylamino, Acylaminoniederalkylamino, Phenylniederalkylamino, worin Phenyl durch Carboxy, Niederalkoxycarbonyl oder Carbamoyl und Niederalkyl durch Hydroxy, Cyano oder Carboxy substituiert sein kann, unsubstituiertes oder substituiertes Thiazolylamino, Pyridylniederalkylamino, Imidazolylniederalkylamino, Indolylniederalkylamino, Piperidinylamino, 1-Niederalkoxycarbonylpiperidinylamino, ausserdem durch Niederalkoxy oder physiologisch spaltbares substituiertes Alkoxy,

ferner -Ile-His-, -Ile-Phe-, -Ala-His-, -Gly-Gly-, -Gly-His-, -Gly-Ala-, -Gly-Ser-, -Ala-Sta-, -Ile-Sta-, -Gly-Gly-Gly-, -Gly-His-Lys-, -Gly-Ala-Gly- oder -Ile-His-Lys-, worin die Aminofunktion in der Seitenkette von -Lys- gegebenenfalls durch Niederalkanoyl, Niederalkoxycarbonyl oder Phenylniederalkoxycarbonyl acyliert ist, C-terminal substituiert durch Amino, Niederalkylamino, Diniederalkylamino, Hydroxyniederalkylamino, ferner Niederalkoxy oder physiologisch spaltbares substituiertes Alkoxy darstellt, sowie pharmazeutisch annehmbare Salze von diesen Verbindungen mit salzbildenden Gruppen, gebildet werden.

6. Verfahren gemäss einem der Ansprüche 1-3, dadurch gekennzeichnet, dass man solche Ausgangsstoffe wählt, dass Verbindungen der Formel I, worin $R_1$ Wasserstoff, Niederalkanoyl, Niederalkoxyniederalkanoyl, Mono- oder Diisopropyliden-glucofuranosyl-O-acetyl, Halogenniederalkanoyl, Carboxyniederalkanoyl, Niederalkoxycarbonylniederalkanoyl, Aminoalkanoyl, Niederalkanoylaminoalkanoyl, Niederalkoxycarbonylaminoalkanoyl, Phenylniederalkoxycarbonylaminoalkanoyl, γ-Aminobutyryl-aminoacetyl, γ-Benzyloxycarbonylaminobutyryl-aminoacetyl, (α-Amino-δ-guanidinovaleryl)-aminoacetyl, (α-Benzyloxycarbonylamino-δ-guanidinovaleryl)-aminoacetyl, Oxoniederalkanoyl, Hydroxycarboxy-niederalkanoyl, Hydroxy-niederalkoxycarbonyl-niederalkanoyl, Dihydroxy-carboxy-niederalkanoyl, Dihydroxy-niederalkoxycarbonyl-niederalkanoyl, verestertes Dihydroxyniederalkoxycarbonyl-niederalkanoyl, Hydroxy-amino-niederalkanoyl, Hydroxy-acylamino-niederalkanoyl, Carboxy-amino-niederalkanoyl, Niederalkoxycarbonyl-amino-niederalkanoyl, Niederalkoxycarbonylacylamino-niederalkanoyl, Niederalkenoyl, Niederalkinoyl, Cyclopentyl- oder Cyclohexylcarbonyl, Hydroxycyclohexylcarbonyl, Acylaminocycloalkylcarbonyl, 3,4,5-Trihydroxycyclohex-1-enylcarbonyl, Benzoyl oder durch Halogen, Niederalkoxy und/oder Nitro substituiertes Benzoyl, 2-(o,o-Dichloroanilino)-phenylacetyl, 2-(o,o-Dichloro-N-benzylanilino)-phenylacetyl, Hydroxypyrrolidinylcarbonyl, Hydroxy-1-acylpyrrolidinylcarbonyl, Oxopyrrolidinylcarbonyl, Niederalkoxycarbonyl, 2-Halogenniederalkoxycarbonyl, Arylmethoxycarbonyl mit einem oder zwei Arylresten, worin Aryl gegebenenfalls durch Niederalkyl, Niederalkoxy, Hydroxy, Halogen und/oder Nitro mono-, di- oder trisubstituiertes Phenyl ist,

$X_1$ und $X_2$ zusammen -Phe-His-, -Phe-Phe-, -Phe-(N-methyl-Phe)- -Phe-Leu-, -Phe-Ala-, -Phe-Arg-, -Phe-Gly-, -His-His-, -Arg-Arg-, -Arg-His-, -Arg-Phe-oder -Glu-His-, welche N-terminal mit $R_1$ und C-terminal mit der Gruppe $-NR_2-$ verbunden sind, $R_2$ Wasserstoff, $R_3$ Niederalkyl, $R_4$ Hydroxy, $R_5$ Alkyl, Cyclohexyl oder Phenyl, und

$R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkylamino, Niederalkoxycarbonylalkylamino, physiologisch spaltbares verestertes Carboxyalkylamino, Carbamoylniederalkylamino, Hydroxyniederalkylcarbamoylniederalkylamino, Niederalkoxycarbonyl-hydroxyniederalkoxycarbamoyl-niederalkylamino, Aminoalkylamino, Hydroxyniederalkylaminoniederalkylamino, Diniederalkylaminoniederalkylamino, über ein Stickstoffatom gebundenes fünf- oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, sowie Niederalkoxy, oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, Diniederalkylaminoniederalkylamino, 4-Morpholinylniederalkylamino, Benzylamino, Carboxybenzylamino, Niederalkoxycarbonylbenzylamino, Carbamoylbenzylamino, 2-Phenyläthylamino, 1-Phenylprop-2-ylamino, 3-Phenylpropylamino, 1-Hydroxymethyl-2-phenyläthylamino, 2-Hydroxy-1- oder 2-phenyläthylamino oder 1-(2-Cyano- oder 2-Carboxy-1-hydroxyäthyl)-2-phenyläthylamino, 4- oder 5-Carbamoylmethyl-2-thiazolylamino, Pyridylniederalkylamino, Imidazolylniederalkylamino, Indolylniederalkylamino, 1-Niederalkoxycarbonylpiperidinylamino, Niederalkoxy oder physiologisch spaltbares substituiertes Alkoxy, ferner -Ile-His-, -Ile-Phe, -Ile-Sta-, -Ala-His-, -Ala-Sta- oder -Ile-His-Lys-, worin die Aminofunktion in der Seitenkette von -Lys- gegebenenfalls durch Niederalkanoyl, Niederalkoxcarbonyl oder Phenylniederalkoxycarbonyl acyliert ist, C-terminal substituiert durch Amino, Niederalkylamino, Diniederalkylamino, Hydroxyniederalkylamino, Niederalkoxy oder physiologisch spaltbares substituiertes Alkoxy, darstellt, sowie pharmazeutisch annehmbare Salze von diesen Verbindungen mit salzbildenden Gruppen, gebildet werden.

7. Verfahren gemäss einem der Ansprüche 1-3, dadurch gekennzeichnet, dass man solche Ausgangsstoffe wählt, dass Verbindungen der Formel I, worin $R_1$ Wasserstoff, Niederalkanoyl, Mono- oder Diisopropyliden-glucofuranosyl-O-acetyl, Aminoalkanoyl, Niederalkanoylaminoalkanoyl, Niederalkoxycarbonylaminoalkanoyl, Phenylniederalkoxycarbonylaminoalkanoyl, γ-Aminobutyryl-aminoacetyl, γ-Benzyloxycarbonylaminobutyryl-aminoacetyl, (α-Amino-δ-guanidinovaleryl)-aminoacetyl, (α-Benzyloxycarbonylamino-δ-guanidinovaleryl)-aminoacetyl, Hydroxy-carboxy-niederalkanoyl, Hydroxy-niederalkoxycarbonyl-niederalkanoyl, Dihydroxy-carboxy-niederalkanoyl, Dihydroxy-niederalkoxycarbonyl-niederalkanoyl, verestertes Dihydroxyniederalkoxycarbonyl-niederalkanoyl, Hydroxy-amino-niederalkanoyl, Niederalkoxycarbonyl-acylamino-niederalkanoyl, 3,4,5-Trihydroxycyclohexylcarbonyl, 3,4,5-Trihydroxycyclohex-1-enylcarbonyl, 2-(o,o-Dichloroanilino)-phenylacetyl, 2-(o,o-Dichloro-N-benzylanilino)-phenylacetyl, 3-oder 4-Hydroxypyrrolidinyl-2-carbonyl, 3- oder 4-Hydroxy-1-benzyloxycarbonylpyrrolidinyl-2-carbonyl, 5-Oxopyrroldinyl-2-carbonyl, Niederalkoxycarbonyl, Phenylmethoxycarbonyl mit ein oder zwei Phenylresten, die gegebenenfalls durch Niederalkyl, Niederalkoxy und/oder Nitro substituiert sind, $X_1$ und $X_2$ zusammen -Phe-His-, -Phe-Phe-, -Phe-Leu-, -Arg-His- oder -Arg-Phe-, welche N-terminal mit $R_1$ und C-terminal mit der Gruppe $-NR_2-$ verbunden sind, $R_2$ Wasserstoff, $R_3$ Isopropyl oder Isobutyl, $R_4$ Hydroxy, $R_5$ Isopropyl, n-Octyl, Cyclohexyl oder Phenyl, und $R_6$ Amino, Alkylamino, Diniederalkylamino, Hydroxyniederalkylamino, Niederalkoxyniederalkylamino, 2-(3-Carbamoyl-4-hydroxyphenoxy)-äthylamino, 3-(3-Carbamoylphenoxy)-2-hydroxypropylamino, Carboxyalkylamino, Niederalkoxycarbonylalkylamino, physiologisch spaltbares verestertes Carboxyalkylamino, Carbamoylniederalkylamino, Hydroxyniederalkylcarbamoylniederalkylamino, α-Carbamoyl-α-(2-hydroxy-1-isobutyl-3-methoxycarbonylpropyl)-carbamoyl-methylamino, Aminoalkylamino, Hydroxyniederalkylaminoniederalkylamino, Diniederalkylaminoniederalkylamino, über ein Stickstoff-Atom gebundenes fünf- oder sechsgliedriges Heterocyclylniederalkylamino, Niederalkanoylaminoniederalkylamino, Niederalkoxycarbonylaminoniederalkylamino, Benzylamino, Carboxybenzylamino, Niederalkoxycarbonylbenzylamino, Niederalkoxy oder physiologisch spaltbares Alkoxy, ferner -Ala- oder -Ile-, C-terminal substituiert durch Amino, Alkylamino, Diniederalkylamino, Hydroxyniederalkylamino, Carboxyalkylamino, Carbamoylniederalkylamino, Hydroxyniederalkylcarbamoylniederalkylamino, Aminoalkylamino, Hydroxyniederalkylaminoniederalkylamino, 4-Morpholinylniederalkylamino, Carboxybenzylamino, Carbamoylbenzylamino, 4- oder 5-Carbamoylmethyl-2-thiazolylamino, Pyridylniederalkylamino, Imidazolylniederalkylamino, Indolylniederalkylamino, Niederalkoxy oder physiologisch spaltbares substituiertes Alkoxy, ferner -Ile-His-, -Ile-Sta- oder -Ala-Sta-, C-terminal substituiert durch Amino, Niederalkylamino, Diniederalkylamino, Hydroxyniederalkylamino, Niederalkoxy oder physiologisch spaltbares substituiertes Alkoxy, darstellt, sowie pharmazeutisch annehmbare Salze von diesen Verbindungen mit salzbildenden Gruppen, gebildet werden.

8. Verfahren gemäss einem der Ansprüche 1-3, dadurch gekennzeichnet, dass man solche Ausgangsstoffe wählt, dass Verbindungen der Formel I, worin $R_1$ Wasserstoff, Niederalkanoyl, Aminoalkanoyl, Niederalkoxycarbonylaminoalkanoyl, Benzyloxycarbonylaminoniederalkanoyl, γ-Aminobutyrylaminoacetyl, γ-tert-Butoxycarbonylaminobutyrylaminoacetyl, γ-Benzyloxycarbonylaminobutyrylaminoacetyl, α,ß-Dihydroxy-ß-carboxypropionyl, α,ß-Dihydroxy-ß-methoxycarbonylpropionyl, α,ß-Diacetoxy-ß-methoxycarbonylpropionyl, 3,4,5-Trihydroxycyclohex-1-enylcarbonyl, 2-(o,o-Dichloroanilino)-phenylacetyl, 2-(o,o-Dichloro-N-benzylanilino)-phenylacetyl, 3- oder 4-Hydroxypyrrolidinyl-2-carbonyl, 3- oder 4-Hydroxy-1-benzyloxycarbonylpyrrolidinyl-2-carbonyl, 5-Oxopyrrolidinyl-2-carbonyl, Niederalkoxycarbonyl, Phenylmethoxycarbonyl mit ein oder zwei Phenylresten, die gegebenenfalls durch Niederalkyl, Niederalkoxy, und/oder Nitro substituiert sind, $X_1$ und $X_2$ zusammen -Phe-His- oder -Phe-Phe-, welche N-terminal mit $R_1$ und C-terminal mit der Gruppe $-NR_2$-verbunden sind, $R_2$ Wasserstoff, $R_3$ Isobutyl, $R_4$ Hydroxy, $R_5$ Isopropyl, n-Octyl, Cyclohexyl oder Phenyl, und $R_6$ Amino, Nieder-

alkylamino, Diniederalkylamino, Hydroxyniederalkylamino, Carboxyalkylamino, Niederalkoxycarbonylalkylamino, physiologisch spaltbares verestertes Carboxyalkylamino, Aminoalkylamino, Hydroxyniederalkylaminoniederalkylamino, Diniederalkylaminoniederalkylamino, 4-Morpholinylniederalkylamino, 2-Oxo-1-pyrrolidinylniederalkylamino, ferner -Ala- oder -Ile-, C-terminal substituiert durch Amino, Niederalkylamino, Diniederalkylamino, Hydroxyniederalkylamino, Dicarbamoylmethylamino, 2-(2-Hydroxyäthylamino)-äthylamino, 2-(4-Morpholinyl)-äthylamino, 3-Carbamoylbenzylamino, 4-Carbamoylmethyl-2-thiazolylamino, 2-Pyridylmethylamino, 2-(2-Pyridyl)-äthylamino, 3-(2-Pyridyl)-propylamino, 2-(4-Imidazolyl)-äthylamino oder 2-(3-Indolyl)-äthylamino, ferner -Ile-His- oder -Ile-Sta-, C-terminal substituiert durch Amino, oder -Ala-Sta-, C-terminal substituiert durch Niederalkoxy, darstellt, sowie pharmazeutisch annehmbare Salze von diesen Verbindungen mit salzbildenden Gruppen, gebildet werden.

9. Verfahren gemäss einem der Ansprüche 1-3, dadurch gekennzeichnet, dass man solche Ausgangsstoffe wählt, dass Verbindungen der Formel I, worin $R_1$ Wasserstoff, Acetyl, Pivaloyl, γ-Aminobutyryl, 8-Aminooctanoyl, γ-tert-Butoxycarbonylaminobutyryl, 8-tert-Butoxycarbonylaminooctanoyl, γ-Benzyloxycarbonylaminobutyryl, 8-Benzyloxycarbonylaminooctanoyl, γ-Aminobutyrylaminoacetyl, γ-tert-Butoxycarbonylaminobutyrylaminoacetyl, γ-Benzyloxycarbonylaminobutyrylaminoacetyl, α,ß-Dihydoxy-ß-carboxypropionyl, α,ß-Dihydroxy-ß-methoxycarbonylpropionyl, 4-Hydroxypyrrolidinyl-2-carbonyl, 1-Benzyloxycarbonyl-4-hydroxypyrrolidinyl-2-carbonyl, 5-Oxopyrrolidinyl-2-carbonyl, tert-Butoxycarbonyl oder Benzyloxycarbonyl, $X_1$ und $X_2$ zusammen -Phe-His-, N-terminal mit $R_1$ und C-terminal mit der Gruppe $-NR_2-$ verbunden, $R_2$ Wasserstoff, $R_3$ Isobutyl, $R_4$ Hydroxy, $R_5$ Isopropyl, und $R_6$ Amino, Niederalkylamino, Dimethylamino, 4-Carboxy-n-butylamino, 7-Carboxy-n-heptylamino, 4-tert-Butoxycarbonyl-n-butylamino, 7-tert-Butoxycarbonyl-n-heptylamino, 7-Pivaloyloxymethoxycarbonyl-n-heptylamino, 3-(2-Oxo-1-pyrrolidinyl)-propylamino, -Ala-, C-terminal substituiert durch 2-(4-Imidazolyl)-äthylamino

oder 2-(2-Pyridyl)-äthylamino, -Ile-, C-terminal substituiert durch Dicarbamoylmethylamino, 3-Carbamoylbenzylamino, 4-Carbamoylmethyl-2-thiazolylamino, 2-(2-Pyridyl)-äthylamino, 2-Pyridylmethylamino oder 2-(3-Indolyl)-äthylamino, -Ile-His- oder -Ile-Sta-, C-terminal substituiert durch Amino, oder -Ala-Sta-, C-terminal substituiert durch Methoxy, darstellt, sowie pharmazeutisch annehmbare Salze von diesen Verbindungen mit salzbildenden Gruppen, gebildet werden.

10. Verfahren gemäss einem der Ansprüche 1-3, dadurch gekennzeichnet, dass man solche Ausgangsstoffe wählt, dass Benzyloxycarbonyl-Phe-His-[5(S)-amino-4(S)-hydroxy-2(S)-isopropyl-7-methyloctanoyl]-Ile-His-$NH_2$ gebildet wird.

11. Verfahren gemäss einem der Ansprüche 1-3, dadurch gekennzeichnet, dass man solche Ausgangsstoffe wählt, dass Benzyloxycarbonyl-Phe-Phe-[5(S)-amino-4(S)-hydroxy-2(S)-isopropyl-7-methyloctanoyl]-Ile-His-$NH_2$ gebildet wird.

12. Verfahren gemäss einem der Ansprüche 1-3, dadurch gekennzeichnet, dass man solche Ausgangsstoffe wählt, dass H-Phe-His-[5(S)-amino-4(S)-hydroxy-2(S)-isopropyl-7-methyloctanoyl]-Ile-His-$NH_2$ gebildet wird.

13. Verfahren gemäss einem der Ansprüche 1-3, dadurch gekennzeichnet, dass man solche Ausgangsstoffe wählt, dass [(2S,4R)-4-Hydroxypyrrolidinyl-2-carbonyl]-Phe-His-[5(S)-amino-4(S)-hydroxy-2(S)-isopropyl-7-methyloctanoyl]-Ile-His-$NH_2$ gebildet wird.

14. Verfahren gemäss einem der Ansprüche 1-3, dadurch gekennzeichnet, dass man solche Ausgangsstoffe wählt, dass [(2S)-5-Oxopyrrolidinyl-2-carbonyl]-Phe-His-[5(S)-amino-4(S)-hydroxy-2(S)-isopropyl-7-methyloctanoyl]-Ile-His-$NH_2$ gebildet wird.

15. Verfahren gemäss einem der Ansprüche 1-3, dadurch gekennzeichnet, dass man solche Ausgangsstoffe wählt, dass tert-Butoxycarbonyl-Phe-His-[5(S)-amino-4(S)-hydroxy-2(S)-isopropyl-7-methyloctanoyl]-Ala-Sta-$OCH_3$ gebildet wird.

16. Verfahren gemäss einem der Ansprüche 1-3, dadurch gekennzeichnet, dass man solche Ausgangsstoffe wählt, dass Benzyloxycarbonyl-Phe-His-[5(S)-amino-4(S)-hydroxy-2(S)-isopropyl-7-methyloctanoyl]-Ala-Sta-$OCH_3$ gebildet wird.

17. Verfahren gemäss einem der Ansprüche 1-3, dadurch gekennzeichnet, dass man solche Ausgangsstoffe wählt, dass γ-Benzyloxycarbonylaminobutyrylaminoacetyl-Phe-His-[5(S)-amino-4(S)-hydroxy-2(S)-isopropyl-7-methyloctanoyl]-Ile-His-$NH_2$ gebildet wird.

18. Verfahren gemäss einem der Ansprüche 1-3, dadurch gekennzeichnet, dass man solche Ausgangsstoffe wählt, dass γ-Aminobutyrylaminoacetyl-Phe-His-[5(S)-amino-4(S)-hydroxy-2(S)isopropyl-7-methyloctanoyl]-Ile-His-$NH_2$ gebildet wird.

19. Verfahren gemäss einem der Ansprüche 1-3, dadurch gekennzeichnet, dass man solche Ausgangsstoffe wählt, dass 8-Aminooctanoyl-Phe-His-[5(S)-amino-4(S)-hydroxy-(2S)-isopropyl-7-methyloctanoyl]-Ile-His-$NH_2$ gebildet wird.

20. Verfahren gemäss einem der Ansprüche 1-3, dadurch gekennzeichnet, dass man solche Ausgangsstoffe wählt, dass Benzyloxycarbonyl-Phe-His-[5(S)-amino-4(S)-hydroxy-(2S)-isopropyl-7-methyloctanoyl]-Ile-dicarbamoylmethylamid gebildet wird.

21. Verfahren gemäss einem der Ansprüche 1-3, dadurch gekennzeichnet, dass man solche Ausgangsstoffe wählt, dass Benzyloxycarbonyl-Phe-His-[5(S)-amino-4(S)-hydroxy-2(S)-isopropyl-7-methyloctanoyl]-2-(3-carbamoyl-4-hydroxy-phenyloxy)-äthylamid gebildet wird.

22. Verfahren gemäss einem der Ansprüche 1-3, dadurch gekennzeichnet, dass man solche Ausgangsstoffe wählt, dass α-Benzlyoxycarbonyl-amino-α-methoxycarbonyl-acetyl-Phe-His-[5(S)-amino-4(S)-hydroxy-2(S)-isopropyl-7-methyloctanoyl]-3-(2-pyrrolidinon-1-yl)-propylamid gebildet wird.

23. Verfahren gemäss einem der Ansprüche 1-3, dadurch gekennzeichnet, dass man solche Ausgangsstoffe wählt, dass Benzyloxycarbonyl-Phe-His-[5(S)-amino-4(S)-hydroxy-2(S)-isopropyl-7-methyloctanoyl]-methylamid gebildet wird.

24. Verfahren gemäss einem der Ansprüche 1-3, dadurch gekennzeichnet, dass man solche Ausgangsstoffe wählt, dass Benzyloxycarbonyl-Phe-His-[5(S)-amino-4(S)-hydroxy-2(S)-isopropyl-7-methyloctanoyl]-4-[tris-(hydroxymethyl)-methylaminocarbonyl]-butylamid gebildet wird.

25. Verfahren gemäss einem der Ansprüche 1-3, dadurch gekennzeichnet, dass man solche Ausgangsstoffe wählt, dass Benzyloxycarbonyl-Glu(O-tert-butyl)-His-[5(S)-amino-4(S)-hydroxy-2(S)-isopropyl-7-methyloctanoyl]-Ile-His-$NH_2$ gebildet wird.

26. Verfahren gemäss einem der Ansprüche 1-3, dadurch gekennzeichnet, dass man solche Ausgangsstoffe wählt, dass Benzyloxycarbonyl-Phe-His-[5(S)-amino-4(S)-hydroxy-2(S)-isopropyl-7-methyloctanoyl]-carbamoyl-(3-methoxycarbonyl-2-hydroxy-1-isobuyl-propylaminocarbonyl)-methylamid gebildet wird.

27. Verfahren zur Herstellung von pharmazeutischen Präparaten, dadurch gekennzeichnet, dass man Verbindungen der Formel I oder pharmazeutisch verwendbare Salze von Verbindungen der Formel I mit salzbildenden Gruppen mit einem pharmazeutischen Trägermaterial mischt.

0143746

28. Verfahren zur Herstellung von Verbindungen der Formel

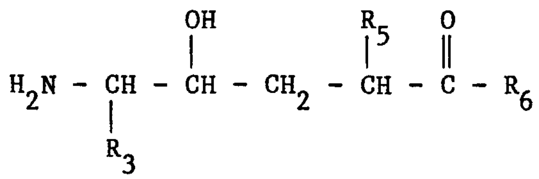

(VIII),

worin die Substituenten die in Anspruch 1 genannten Bedeutungen haben, und Salzen davon, dadurch gekennzeichnet, dass man eine Verbindung der Formel

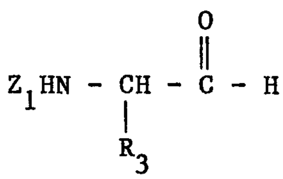

(IX),

worin $R_3$ die genannte Bedeutung hat und $Z_1$ eine Aminoschutzgruppe ist, mit einer Schwefel-Ylid-Verbindung in ein Epoxid der Formel

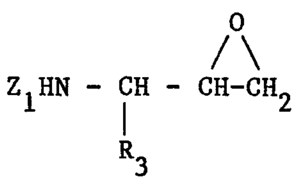

(X),

worin $R_3$ und $Z_1$ die genannten Bedeutungen haben, umwandelt, die erhältliche Verbindung (X) gegebenenfalls nach Trennung der Isomeren mit einem eine nukleofuge Abgangsgruppe X einführenden Reagens umsetzt, die erhältliche Verbindung der Formel

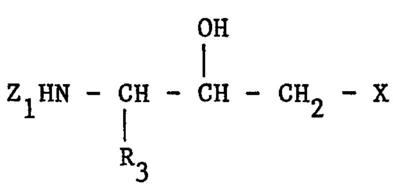

(XI),

worin $R_3$ und $Z_1$ die genannten Bedeutungen haben und X eine nukleofuge Abgangsgruppe ist, mit einer Carbonylverbindung der Formel

$$R_a - (C = O) - R_b \qquad (XII),$$

worin jeder der Reste $R_a$ und $R_b$ unabhängig voneinander Wasserstoff, Niederalkyl, Aryl oder Arylniederalkyl, $R_a$ und $R_b$ zusammen gegebenenfalls überbrücktes Alkyliden mit 4 bis 12 Kohlenstoffatomen darstellen, oder einem reaktionsfähigen Derivat dieser Carbonylverbindung, in Gegenwart eines sauren Reagenses umsetzt, die erhältliche Verbindung der Formel

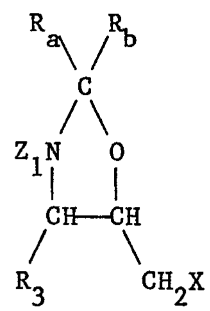

(XIII),

worin die Substituenten die genannten Bedeutungen haben, mit einem Carbonsäureestersalz der Formel

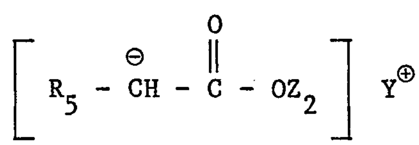

(XIV),

worin $R_5$ die unter Formel I genannte Bedeutung hat, $Z_2$ eine Carboxyschutzgruppe und $Y^{\oplus}$ ein Kation, z.B. ein Alkalimetall-, z.B. das Natrium- oder bevorzugt das Lithiumion, ist, umsetzt, und in einer erhältlichen Verbindung der Formel

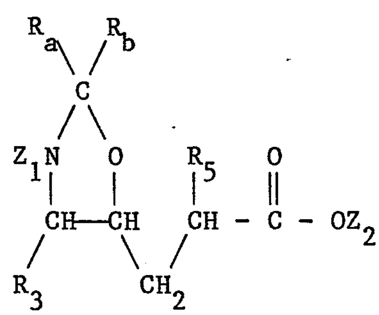

(XV),

worin die Sustituenten die genannten Bedeutungen haben, die Carboxyschutzgruppe $Z_2$ abspaltet und/oder ein erhältliches Isomerengemisch in die einzelnen Isomere auftrennt, die erhältliche Verbindung mit

einer freien Carboxygruppe ($Z_2$=H) je nach Bedeutung des einzuführenden Rests $R_6$ amidiert, verestert oder mit einem C-terminal gegebenenfalls amidierten oder veresterten Rest einer Aminosäure, eines Di- oder Tripeptidrest substituiert und in einer erhältlichen Verbindung der Formel

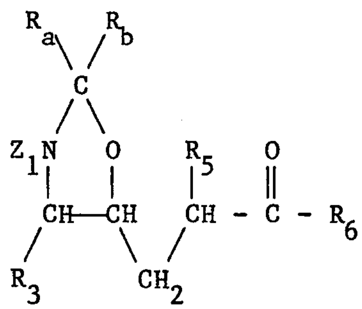

(XVI),

worin die Substituenten die genannten Bedeutungen haben, mit einem geeigneten Solvolysereagens den Ring öffnet und gegebenenfalls die Schutzgruppe $Z_1$ abspaltet.

FO 7.4/KB/gs*